# EUROPEAN PATENT APPLICATION

(11) **EP 1 867 639 A1**
(43) Date of publication of application: **19.12.2007**
(21) Application number: 06745374.6
(22) Date of filing: 23.03.2006
(51) Int. Cl.: C07D 243/14, A61K 31/5513, A61K 45/00, A61P 7/12, A61P 43/00, C07D 401/06, C07D 401/10, C07D 401/12, C07D 401/14, C07D 403/06, C07D 403/10, C07D 403/12, C07D 403/14, C07D 413/10, C07D 413/12

(54) **UREA DERIVATIVE, MEDICINAL COMPOSITION CONTAINING THE SAME, AND MEDICINAL USE OF THESE**

(30) Priority: 25.03.2005 JP 2005090221
(71) Applicant: Kissei Pharmaceutical Co., Ltd., Matsumoto-shi Nagano 399-8710 (JP)
(72) Inventor: SUZUKI, Ritsu, Kissei Pharmaceutical Co. Ltd, Azumino-shi, Nagano 3998304 (JP); YOKOYAMA, Kenji, Kissei Pharmaceutical Co. Ltd, Azumino-shi, Nagano 3998304 (JP); KONDO, Tatsuhiro, Kissei Pharmaceutical Co. Ltd, Azumino-shi, Nagano 3998304 (JP); HIRASAWA, Hideaki, Kissei Pharmaceutical Co. Ltd, Azumino-shi, Nagano 3998304 (JP); KAMBARA, Mikie, Kissei Pharmaceutical Co. Ltd, Azumino-shi, Nagano 3998304 (JP); KOBAYASHI, Hiroaki, Kissei Pharmaceutical Co. Ltd, Azumino-shi, Nagano 3998304 (JP)
(74) Representative: Hayes, Adrian Chetwynd
(86) International application number: PCT/JP2006/305820
(87) International publication number: WO 2006/104008

(57) **Abstract**

Urea derivatives represented by the following general formula (I) : 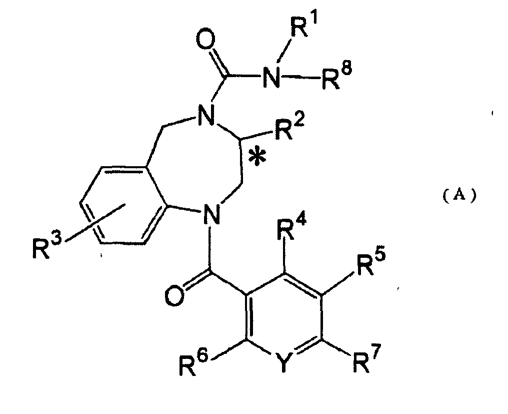 which have an agonism of V2 receptor, are useful as agents for the treatment or prevention of diabetes insipidus, nocturia, nocturnal enuresis, overactive bladder or the like.
In the formula, R¹ represents a hydrogen atom or a C₁₋₆ alkyl group which may have a substituent, R² is a hydrogen atom or a C₁₋₆ alkyl group, R³ is a hydrogen atom, a C₁₋₆ alkyl group or the like, R⁴, R⁵ and R⁶ are independently a hydrogen atom, a halogen atom or the like, R⁷ is a hydrogen atom, a heteroaryl group which may have a substituent, a C₃₋₈ cycloalkyl group, an amino group which may have a substituent or a C₁₋₆ alkoxy group which may have a substituted group, M¹ is a single bond, a C₁₋₄ alkylene group or the like, Y is N or CR^{F} (in the formula, and R^{F} represents a hydrogen atom, a C₁₋₆ alkyl group or the like, or pharmaceutically acceptable salts thereof, or prodrugs thereof, or pharmaceutical compositions comprising the same and pharmaceutical uses thereof.

## Description

### Technical Field

The present invention relates to urea derivatives or pharmaceutically acceptable salts thereof, or prodrugs thereof which are useful as medicaments, or pharmaceutical compositions comprising the same and pharmaceutical uses thereof.

More particularly, the present invention relates to novel urea derivatives having an agonism of a type 2 arginine vasopressin receptor (hereinafter referred to as V2 receptor), or pharmaceutically acceptable salts thereof, or prodrugs thereof, or pharmaceutical compositions comprising the same and pharmaceutical uses thereof.

### Background Art

Arginine vasopressin is one of neurohormones which is biosynthesized in the hypothalamus and is released from the posterior pituitary gland. Arginine vasopressin receptors were classified to V1a, V1b and V2 subtypes. An arginine vasopressin is called an antidiuretic hormone because an arginine vasopressin decreases urine volume due to enhancing water reabsorption at collecting ducts, in which V2 receptor exists and arginine vasopressin shows agonism of V2 receptor via binding this receptor (see Non-patent Reference 1). Therefore, the patients suffer from polyuria because of a deficiency of arginine vasopressin, as a concrete example particularly, central diabetes insipidus, nocturnal enuresis in children, nocturia with aging and the like can be illustrated (see Non-patent References 2 and 3).

Heretofore, a peptide-type compound (see Non-patent Reference 3; desmopressin (1-desamino, D-Arg8) vasopressin, DDAVP) has been used for the treatment of central diabetes insipidus or nocturnal enuresis as a V2 agonist. However, concerning an absorption rate in gastrointestinal tract, it is known that peptide-type compounds have wide individual variability in absorption and the wide variability in plasma concentration of the compounds has been reported (see Non-patent Reference 4). Therefore, it is feared the adverse effects due to these variability will occur and clinical use of the compounds was not necessarily satisfied with safety. It is most preferable to use clinically non-peptide drug, that is, a low molecular V2 agonist for the patients with disorders as mentioned above.

Although a compound represented by the following general formula (D-1) has been reported in Patent Reference 1 so far, the compounds described in the Patent Reference are different from the compounds of the present invention, and do not have a urea structure, and the compounds have not an agonism of vasopressin receptor but antagonism of vasopressin, they have a different action from the compounds of the present invention. 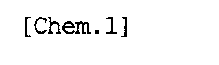 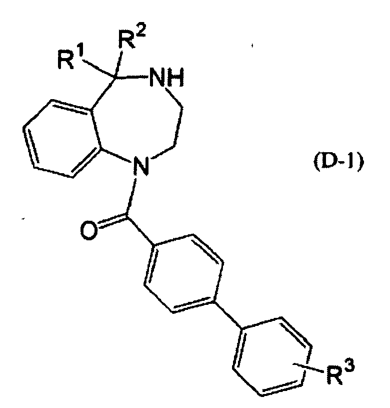 (Regarding the symbols in the formula, see the Patent Reference 1.)

In addition, the following compounds (D-2) and (D-2-1) have been reported in Patent Reference 2 as the compounds which have an agonism of vasopressin receptors. However, the compounds are tricyclic derivatives wherein a benzodiazepine is fused with an aromatic ring, and they have a different chemical structure formula from the compounds of the present invention. 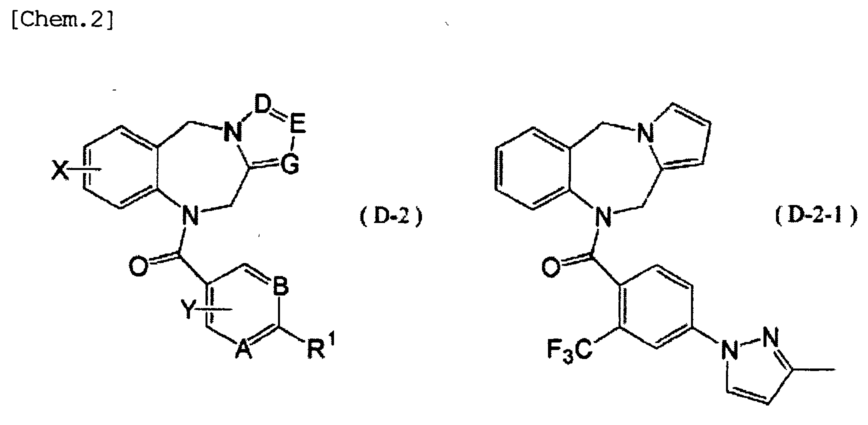 (Regarding the symbols in the formula, see the Patent Reference 2.)

As mentioned above, in any Patent References, there is no disclosure about benzodiazepine derivatives which have a urea structure. The urea derivatives of the present invention are completely new benzodiazepine derivatives which have the urea structure in the structure formula. It has not been reported that the present compounds have an agonism of V2 receptor and are useful as agents for the treatment or prevention for central diabetes insipidus, nocturnal enuresis in children, nocturia with aging and the like.

Patent Reference 1: Japanese Patent Publication H06-016643;
Patent Reference 2: International Publication WO2001/022696 pamphlet;
Non-patent Reference 1: Goodman & Gilman's, The Pharmacological Basis of Therpeutics(Tenth Edition), published by McGraw-Hill Co.,Ltd.;
Non-patent Reference 2: Tsutomu Akikawa and 2 persons, Scand. J. Urol. Nephrol. Suppl, 1999, Vol.202, pp.47-49;
Non-patent Reference 3: Jeffrey P. Weiss and 1 person, J. Urol., Vol. 163, 2000, pp.5-12;
Non-patent Reference 4: Mogens Hammer and 1 person, J. Pharmacol. Exp. Ther., Vol.234, 1985, pp.754-760.

### Disclosure of the Invention

### Problem to be solved by the Invention

The object of the present invention is to provide novel compounds having an agoism of V2 receptor.

### Means of solving the Problems

As a result that the present inventors have studied earnestly to find compounds having an agonism of V2 receptor, they found that a certain urea derivatives represented by the following (A) surprisingly have an agonism of V2 receptor and are excellent medicines having a decreasing activity of urine volume as described below, thereby forming the bases of the present invention.

That is, the present invention relates to:
a urea derivative represented by the general formula (A):

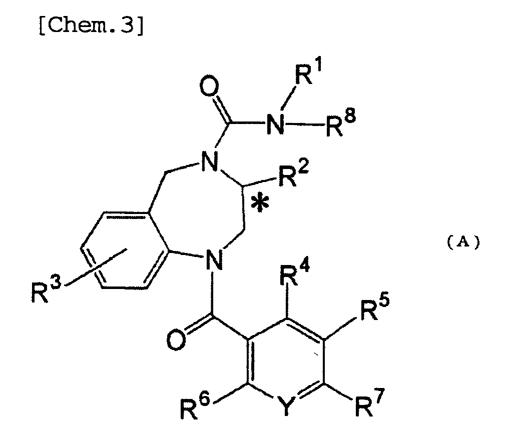

wherein R¹ and R⁸ bind together with the nitrogen atom bound to them to form an alicyclic amine, or are indipendently the following a) to o):
a) a hydrogen atom,
b) a C₃₋₇ cycloalkyl group,
c) a C₁₋₇ alkyl group,
d) a halo(C₁₋₇ alkyl) group,
e) a C₆₋₁₀ aryl group,
f) a heteroaryl group,
g) a hydroxy(C₁₋₇ alkyl) group,
h) a C₃₋₇ cycloalkyl(C₁₋₇ alkyl) group,
i) a C₁₋₆ alkoxy(C₁₋₇ alkyl) group,
j) a C₂₋₇ acyloxy(C₁₋₇ alkyl) group,
k) a C₆₋₁₀ aryl(C₁₋₇ alkyl) group,
l) a heteroaryl(C₁₋₇ alkyl) group,
m) -M¹-COOR¹¹,
n) -M¹-CONR¹²R¹³, or
o) -M¹-NR¹²-SO₂R¹³;
M¹ is a C₁₋₇ alkylene group;
R¹¹ is a hydrogen atom or a C₁₋₇ alkyl group;
R¹² and R¹³ bind together with the nitrogen atom bound to them to form an alicyclic amino group, or are independently the following a) to i):
a) a hydrogen atom,
b) a C₆₋₁₀ aryl group,
c) a C₁₋₇ alkyl group
d) a hydroxy(C₁₋₇ alkyl) group,
e) a C₁₋₆ alkoxy(C₁₋₇ alkyl) group,
f) a heteroaryl(C₁₋₇ alkyl) group,
g) a C₆₋₁₀ aryl-(C₁₋₇ alkyl) group,
h) -M²-CONR¹⁴NR¹⁵, or
i) -M²-NR¹⁶SO₂R¹⁷;
M² is a C₁₋₇ alkylene group;
R¹⁴ and R¹⁵ bind together with the nitrogen atom bound to them to form an alicyclic amino group, or are independently the following a) to f):
a) a hydrogen atom,
b) a C₁₋₇ alkyl group,
c) a hydroxy(C₁₋₇ alkyl) group,
d) a C₁₋₆ alkoxy (C₁₋₇ alkyl) group,
e) a heteroaryl(C₁₋₇ alkyl) group, or
f) a C₆₋₁₀ aryl (C₁₋₇ alkyl) group;
R¹⁶ is a hydrogen atom or a C₁₋₇ alkyl group;
R¹⁷ is a C₁₋₇ alkyl group;
R² is the following a) to g):
a) a hydrogen atom,
b) a C₁₋₇ alkyl group,
c) a hydroxy(C₁₋₇ alkyl) group,
d) a C₁₋₆ alkoxy(C₁₋₇ alkyl) group,
e) a C₆₋₁₀ aryl(C₁₋₇ alkyl) group,
f) -M¹-CONR¹²R¹³ (in the formula, M¹, R¹² and R¹³ have the same meanings as defined above), or
g) -M¹-COOR¹¹ (in the formula, M¹ and R¹¹ have the same meanings as defined above);
R³ is the following a) to d):
a) a hydrogen atom,
b) a halogen atom,
c) a hydroxy group, or
d) a C₁₋₆ alkoxy group;
R⁴, R⁵ and R⁶ are independently the following a) to f):
a) a hydrogen atom,
b) a halogen atom,
c) a C₁₋₇ alkyl group,
e) a C₁₋₆ alkoxy group, or
f) a halo(C₁₋₇ alkyl) group;
R⁷ is the following a) to d):
a) a group represented by the general formula 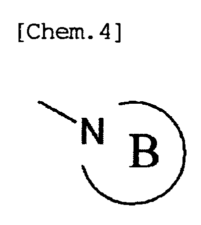 wherein B ring is a heteroaryl group or an alicyclic amino group,
b) a group represented by the general formula 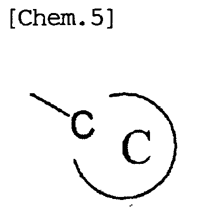 wherein C ring is a C₆₋₁₀ aryl group, a heterocycloalkyl group or a heteroaryl group, or
c) -M³-R⁷¹;
M³ is a single bond, -O-, -C(CH₃)₂- or -CF₂-;
R⁷¹ is the following a) to e):
a) a hydrogen atom,
b) a halogen atom,
c) a C₁₋₇ alkyl group,
d) a halo(C₁₋₇ alkyl) group, or
e) a hydroxy(C₁₋₇ alkyl) group;
Y is N or CH; and
a carbon atom marked with * represents a carbon atom having R-configuration or S-configuration, or a mixture thereof;
or a pharmaceutically acceptable salt thereof, or a prodrug thereof: and the like.

And in another aspect, the present invention relates to a pharmaceutical composition comprising as an active ingredient a urea derivative represented by the above general formula (A) or a pharmaceutically acceptable salt thereof, or a prodrug thereof.

In still another aspect, the present invention relates to a pharmaceutical composition comprising as an active ingredient a urea derivative represented by the above general formula (A) or a pharmaceutically acceptable salt thereof, or a prodrug thereof, which is a V2 receptor receptor agonist.

In still another aspect, the present invention relates to a pharmaceutical composition comprising as an active ingredient a urea derivative represented by the above general formula (A) or a pharmaceutically acceptable salt thereof, or a prodrug thereof, which is an agent for the treatment or prevention of a disease associated with central diabetes insipidus, nocturia or nocturnal enuresis.

In still another aspect, the present invention relates to a pharmaceutical composition comprising as an active ingredient a urea derivative represented by the above general formula (A) or a pharmaceutically acceptable salt thereof, or a prodrug thereof, in combination with at least one agent selected from a group consisting of agents for the treatment of diabetes insipidus, nocturia and nocturnal enuresis other than a V2 agonist.

In still another aspect, the present invention relates to a pharmaceutical composition comprising as an active ingredient a urea derivative represented by the above general formula (A) or a pharmaceutically acceptable salt thereof, or a prodrug thereof, wherein agents selected from a group consisting of agents for the treatment of central diabetes insipidus, nocturia and nocturnal enuresis other than a V2 agonist is an α₁-adrenoceptor blocker, an anticholinergic agent, a cholinergic agent, an antispasmodic agent, an anti-androgen agent, an antidepressant, a calcium antagonist, a potassium-channel opener, a sensory nerve blocking agent, a β-adrenergic agonist, an acetylcholinesterase inhibitor or anti-inflammatory agent. As the agent selected, an α₁-adrenoceptor blocker, a calcium antagonist, a potassium-channel opener, a β-adrenergic agonist and an acetylcholinesterase inhibitor are preferable.

In still another aspect, the present invention relates to a use of a urea derivative represented by the above general formula (A) or a pharmaceutically acceptable salt thereof, or a prodrug thereof, for manufacturing an agent for the treatment or prevention of central diabetes insipidus, nocturia or nocturnal enuresis.

On the compounds represented by the above general formula (A) of the present invention, an agonism of V2 receptor can be confirmed by using cells expressing human V2 receptor, and it was confirmed that the compounds of the present invention has a strong agonism of V2 receptor. In addition, it was confirmed that the compounds represented by the above general formula (A) of the present invention have a strong antidiuretic effect by the confirmation study of antidiuretic effect on the diuretic activity induced by loading hypotonic solution in the anesthetized rats infused with hypotonic solution.

In the present invention, the following terms have the following meanings if not otherwise specified especially.

The term "halogen atom" means a fluorine atom, a chlorine atom, a bromine atom or an iodine atom. A fluorine atom, a chlorine atom or a bromine atom is preferable, and a chlorine atom or a fluorine atom is more preferable.

The term "C₁₋₇ alkyl group" means a straight-chained or branched alkyl group having 1 to 7 carbon atoms such as a methyl group, an ethyl group, a propyl group, an isopropyl group, a n-butyl group, an isobutyl group, a sec-butyl group, a tert-butyl group, a n-pentyl group, a n-hexyl group, a n-heptyl group, an isopentyl group, a neopentyl group, a tert-pentyl group, a 1-methylbutyl group, 2-methylbutyl group, a 1,2-dimethylpropyl group, an isohexyl group or the like. As the C₁₋₇ alkyl group in R¹ and R⁸, an alkyl group represented by a "C₁₋₃ alkyl group" having 1 to 3 carbon atoms is preferable, and a methyl group, an ethyl group, a propyl group or an isopropyl group is more preferable. As the C₁₋₇ alkyl group in R², an alkyl group represented by a "C₁₋₃ alkyl group" having 1 to 3 carbon atoms is preferable, and a methyl group or an ethyl group is more preferable.

The term "halo(C₁₋₇ alkyl) group" means a C₁₋₇ alkyl group substituted by the same or different 1 to 3 halogen atoms as defined above such as a trifluoromethyl group, a 2-chloroethyl group, a 2-fluoroethyl group, a 2,2,2-trifluoroethyl group, a 2,2,2-trichloroethyl group or the like. A C₁₋₇ alkyl group substituted by a fluorine atom is preferable, and a trifluoromethyl group, a 2-fluoroethyl group or a 2, 2, 2-trifluoroethyl group is more preferable.

The term "halo(C₂₋₇ alkyl) group" means a C₂₋₇ alkyl group substituted by the same or different 1 to 3 halogen atoms as defined above such as a 2-chloroethyl group, a 2-fluoroethyl group, a 2,2,2-trifluoroethyl group, a 2,2,2-trichloroethyl group or the like. A C₂₋₇ alkyl group substituted by a fluorine atom is preferable, and a 2-fluoroethyl group or a 2,2,2-trifluoroethyl group is more preferable.

The term "C₃₋₇ cycloalkyl group" means a monocyclic aliphatic alkyl group having 3 to 7 carbon atoms such as a cyclopropyl group, a cyclobutyl group, a cyclopentyl group, a cyclohexyl group, a cycloheptyl group or the like, or a cyclopentyl or cyclohexyl group fused with a benzene ring.

The term "alicyclic amino group" means a 3 to 6-membered cyclic amino group represented by the following group: 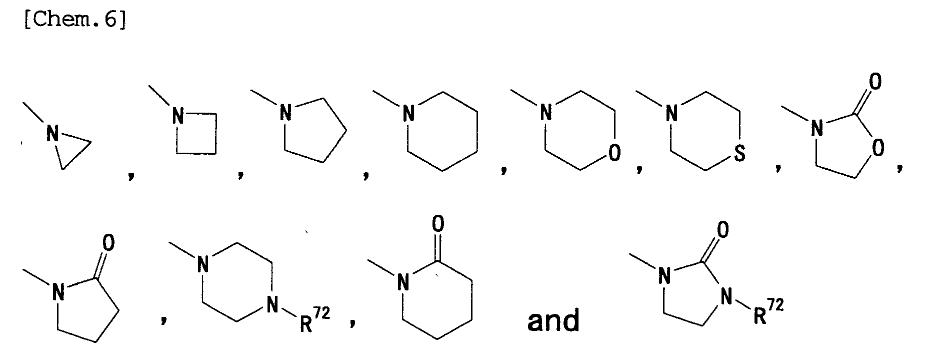 wherein R⁷² is a hydrogen atom, a C₁₋₇ alkyl group, a hydroxy (C₂₋₇ alkyl) group or a C₂₋₇ acyl group, which contains any 1 to 4 hetero atom selected from a group consisting of an oxygen atom, a sulfur atom and a nitrogen atom in the ring, and the ring may have an oxo group or a substituent independently selected from a group consisting of the following Substituent group α.
Substituent group α: a halogen atom, a cyano group, a nitro group, a C₁₋₇ alkyl group, a halo(C₁₋₇ alkyl) group, a C₁₋₆ alkoxy group, -OW¹, -OCOW², -COOW³, -NW⁴W⁵, -NW⁶COW⁷, -CONW⁸W⁹, -SO₂NW¹⁰W¹¹ or -NW¹²-SO₂W¹³; W¹ to W¹³ independently represent a hydrogen atom, a C₁₋₇ alkyl group, a hydroxy(C₁₋₇ alkyl) group or a C₆₋₁₀ aryl(C₁₋₇ alkyl) group, or W⁴ and W⁵, W⁶ and W⁷, W⁸ and W⁹, and W¹⁰ and W¹¹ may bind together with the nitrogen atom bound to them to form an alicyclic amino group.

The term "C₁₋₆ alkoxy group" means a straight-chained or branched alkoxy group having 1 to 6 carbon atoms such as a methoxy group, an ethoxy group, a propoxy group, an isopropoxy group, a butoxy group, an isobutoxy group, a sec-butoxy group, a *tert*-butoxy group, a pentyloxy group, a hexyloxy group or the like. The alkoxy group having 1 to 4 carbon atoms is preferable, and a methoxy group, an ethoxy group or a propoxy group is more preferable.

The term "C₆₋₁₀ aryl group" means an aromatic hydrocarbon group having 6 to 10 carbon atoms such as a phenyl group, a naphthyl group or the like, unsubstituted or substituted by 1 to 5 groups independently selected from the above Substituent group α. A phenyl group unsubstituted or substituted by 1 to 3 groups independently selected from the above Substituent group α is preferable.

The term "heteroaryl group" means a 5 to 10-membered aromatic heterocyclic group represented by the following group: 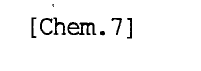 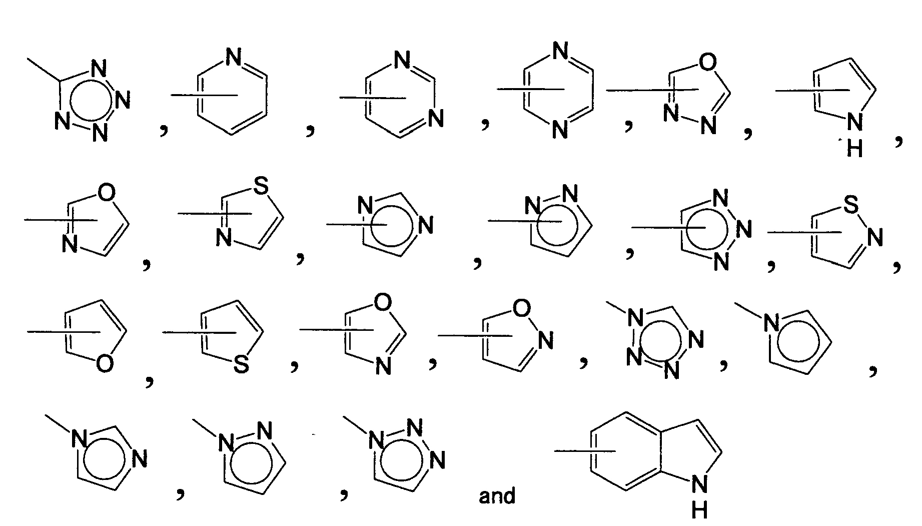 which contains any 1 to 4 hetero atoms selected from a group consisting of an oxygen atom, a sulfur atom and a nitrogen atom in the ring, or an aromatic heterocyclic group consisting of a 6-membered ring fused with a 5 or 6-membered ring containing any 1 to 4 hetero atoms selected from a group consisting of an oxygen atom, a sulfur atom and a nitrogen atom in the ring, which is derived from indole, isoindole, benzofuran, isobenzofuran, benzothiophen, benzoxazole, benzothiazole, indazole, benzimidazole, quinoline, isoquinoline, phthalazine, quinoxaline, quinazoline, cinnoline, indolizine, naphthyridine, pteridine, phthalimide or the like. These aromatic heterocyclic groups are unsubstituted or substituted by 1 to 4 groups selected from the above Substituent group α. In addition, all regioisomers of these aromatic heterocyclic groups can be taken into consideration (for example, a 2-pyridiyl group, a 3-pyridiyl group, a 4-pyridiyl group and the like). The above 5 or 6-membered aromatic heterocyclic group is preferable.

The term "hydroxy (C₁₋₇ alkyl) group" means the above C₁₋₇ alkyl group substituted by a hydroxy group such as a hydroxymethyl group, a 2-hydroxyethyl group, a 1-hydroxyethyl group, a 3-hydroxypropyl group, a 2-hydroxypropyl group, a 1-hydroxypropyl group, 4-hydroxybutyl group or the like. A hydroxymethyl group, a 2-hydroxyethyl group or a 3-hydroxypropyl group is preferable.

The term "hydroxy(C₂₋₇ alkyl) group" means a straight-chained or branched alkyl group having 2 to 7 carbon atoms substituted by a hydroxy group such as a 2-hydroxyethyl group, a 1-hydroxyethyl group, a 3-hydroxypropyl group, a 2-hydroxypropyl group, a 1-hydroxypropyl group, 4-hydroxybutyl group or the like. A hydroxy (C₂₋₇ alkyl) group in R⁷¹ or R⁷² is preferably a 2-hydroxyethyl group or a 3-hydroxypropyl group.

The term "heterocycloalkyl group" means a 5 or 6-membered cyclic alkyl group represented by the following group: 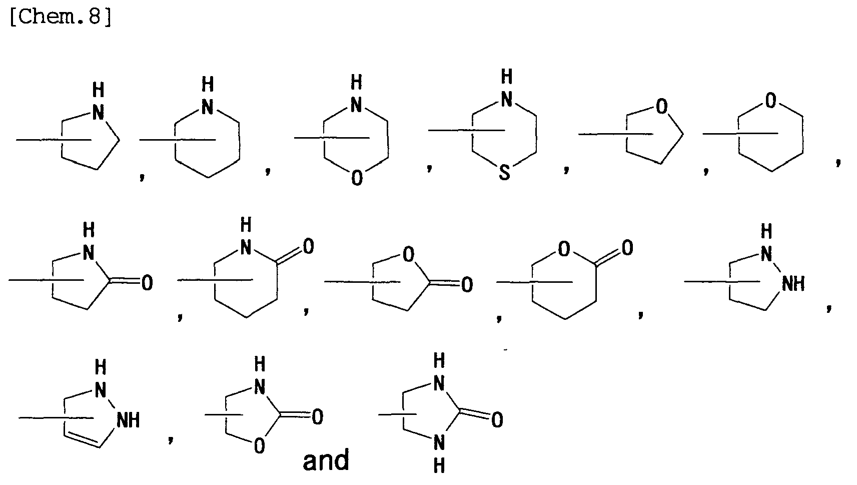 which contains any 1 to 3 hetero atoms selected from a group consisting of a nitrogen atom, an oxygen atom and a sulfur atom in the ring other than at the binding position, which may have an oxo group or a substituent independently selected from the above Substituent group α. In the ring, a single bond and double bond may coexist.

The term "C₁₋₇ alkylene group" means a straight-chained or branched alkylene group having 1 to 7 carbon atoms such as -CH₂-, -CH₂CH₂-, -CH(CH₃)-, -CH₂CH₂CH₂-, -CH(CH₃)CH₂-, -CH₂CH(CH₃)-, -C(CH₃)₂-, -CH(CH₂CH₃)-, -(CH₂)₄, -(CH₂)₅-, -(CH₂)₆-, -(CH₂)₇- or the like. A preferable group is an alkylene group represented by a "C₁₋₄ alkylene group" having 1 to 4 carbon atoms. A more preferable group is -CH₂-, -CH₂CH₂-, -CH₂CH₂CH₂- or -(CH₂)₄-.

The term "C₁₋₆ alkoxy (C₁₋₇ alkyl) group" means a C₁₋₇ alkyl group substituted by the above C₁₋₆ alkoxy group.

The term "C₂₋₇ acyl group" means a straight-chained or branched acyl group having 2 to 7 carbon atoms such as an acetyl group, a propionyl group, a butyryl group, an isobutyryl group, a valeryl group, a pivaloyl group, a hexanoyl group or the like.

The term "C₂₋₇ acyloxy group" means a group represented by C₂₋₇ acyl-O-, which is substituted by the above C₂₋₇ acyl group, for example, a straight-chained or branched acyloxy group having 2 to 7 carbon atoms such as an acetyloxy group, a propionyloxy group, a butyryloxy group, an isobutyryloxy group, a valeryloxy group, a pivaloyloxy group, a hexanoyloxy group or the like.

The term "C₃₋₇ cycloalkyl (C₁₋₇ alkyl) group" means a C₁₋₇ alkyl group substituted by the above C₃₋₇ cycloalkyl group. For example, a cyclopropylmethyl group, a cyclobutylmethyl group, a cyclopentyl group, a cyclohexylmethyl group, a cyclohexylethyl group or the like can be illustrated.

The term "C₂₋₇ acyloxy (C₁₋₇ alkyl) group" means a C₁₋₇ alkyl group substituted by the above C₂₋₇ acyloxy group, for example, a straight-chained or branched acyloxy(C₁₋₇ alkyl) group having 2 to 7 carbon atoms such as an acetyloxymethyl group, an acetyloxyethyl group, a propionyloxymethyl group, a propionyloxyethyl group, a butyryloxymethyl group, a butyryloxyethyl group, an isobutyryloxymethyl group, a valeryloxymethyl group, a pivaloyloxymethyl group, a hexanoyloxymethyl group or the like.

The term "C₆₋₁₀ aryl (C₁₋₇ alkyl) group" means a C₁₋₇ alkyl group substituted by the above C₆₋₁₀ aryl group. For example, a benzyl group, a phenylethyl group can be illustrated.

The term "heteroaryl (C₁₋₇ alkyl) group" means a C₁₋₇ alkyl group substituted by the above heteroaryl group. For example, a 2-pyridylmethyl group, a 3-pyridylmethyl group, a 4-pyridylmethyl group, a 2-pyridylethyl group, a 3-pyridylethyl group, a 4-pyridylethyl group or the like can be illustrated.

As the compounds represented by the above general formula (A) of the present invention have one or more asymmetric carbon atoms, any isomers wherein each asymmetric carbon atom has *R*-configuration or S-configuration in any combination thereof can be also employed in the present invention. In addition, either of a racemic compound, a racemic mixture, a single enantiomer and a diastereomeric compound can be employed in the present invention. As the compounds represented by the above general formula (A) of the present invention have one or more geometrical isomers, either of cis-isomer, trans-isomer and an optional mixture of both isomers can be also employed in the present invention. Moreover, the compounds represented by the above general formula (A) of the present invention include a hydrate and a solvate with a pharmaceutically acceptable solvent such as ethanol or the like.

In the present invention, the term "prodrug" means a compound obtained by modifying a parent compound with a pharmaceutically acceptable group generally used in a prodrug, and such compound can be expected, for example, to have additional characteristics such as improved stability, long action or the like and exert an efficacy after being converted into the parent compound in the body. The prodrugs of the compound represented by the above general formula (A) of the present invention can be prepared by suitably introducing a group forming a prodrug into one or more group optionally selected from a group consisting of a hydroxy group, a carboxy group, an amino group, another group acceptable to form a prodrug of a compound represented by the above general formula (A) using an agent to form a prodrug such as the corresponding halide compound or the like in the usual way and then optionally isolating and purifying in the usual way as an occasion demand (see "Gekkan-yakuji The clinical pharmacokinetics for proper uses of pharmaceutical drugs", Extra edition, March 2000, Vol. 42, No. 4, pp. 669-707 and "New drug delivery system", issued by CMC Co. Ltd., January 31, 2000, pp.67-173).

For example, in case that the compound represented by the above general formula (A) of the present invention have a carboxy group, as the prodrug, an ester which can be formed by replacing a hydrogen atom of the carboxy group by the following group: a C₁₋₇ alkyl group (for example, a methyl group, an ethyl group, a propyl group,an isopropyl group, a butyl group, a tert-butyl group and the like); a C₂₋₇ acyloxymethyl group (for example, a pivaloyloxymethyl group and the like); a 1-(C₂₋₇ acyloxy)ethyl group (for example, a 1-(pivaloyloxy)ethyl group and the like); a C₃₋₇ cycloalkoxycarbonyloxy(C₁₋₇ alkyl) group (for example, a 1-cyclohexyloxycarbonylethyl group and the like); a C₁₋₆ alkoxycarbonyloxymethyl group (for example, a tert-butoxycarbonyloxymethyl group); a 1- (C₁₋₆ alkoxycarbonyloxy)ethyl group (for example, a 1-(tert-butoxycarbonyloxy) ethyl group) ; or a 3-phthalidyl group, can be illustrated.

In addition, in case that the compound represented by the above general formula (A) of the present invention has a hydroxy group, as the prodrug, a compound which can be formed by replacing a hydrogen atom of the hydroxy group by the following group: a C₂₋₇ acyl group (for example, an acetyl group, a propionyl group, a butyryl group, an isobutyryl group, a pivaloyl group and the like); a C₁₋₆ alkoxycarbonyl group (for example, a methoxycarbonyl group, an ethoxycarbonyl group, a propoxycarbonyl group, an isopropoxycarbonyl group, a tert-butoxycarbonyl group and the like) ; a succinoyl group; a C₂₋₇ acyloxymethyl group (for example, a pivaloyloxymethyl group and the like); a 1-(C₂₋₇ acyloxy)ethyl group (for example, a 1-(pivaloyloxy)ethyl group and the like); or a C₁₋₆ alkoxycarbonyloxymethyl group (for example, a tert-butoxycarbonyloxymethyl group); a C₃₋₇ cycloalkoxycarbonyl group (for example, a cyclohexyloxycarbonyl group and the like) can be illustrated.

In addition, in case that the compound represented by the above general formula (A) of the present invention has an amino group, as the prodrug, a compound which can be formed by replacing a hydrogen atom of the amino group by the following group: a C₂₋₇ acyl group (for example, an acetyl group, a propionyl group, a butyryl group, an isobutyryl group, a pivaloyl group and the like); a C₁₋₆ alkoxycarbonyl group (for example, a methoxycarbonyl group, an ethoxycarbonyl group, a propoxycarbonyl group, an isopropoxycarbonyl group, a tert-butoxycarbonyl group and the like); a C₃₋₇ cycloalkoxycarbonyl group (for example, a cyclohexyloxycarbonyl group and the like) can be illustrated.

In the compound represented by the above general formula (A), as for a carbon atom marked with *, the configuration represented by the general formula (A-1): 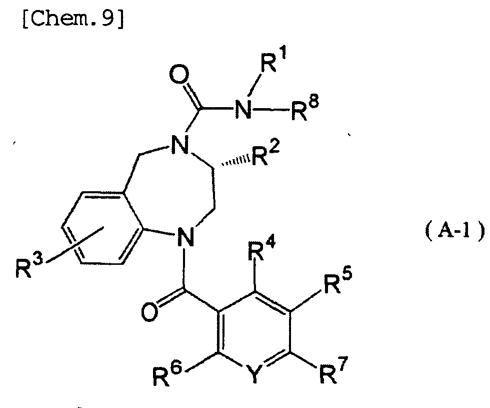 is preferable;
R¹ is preferably the follwing a) to i):
a) a C₁₋₇ alkyl group,
b) a hydroxy(C₁₋₇ alkyl) group,
c) a C₃₋₇ cycloalkyl(C₁₋₇ alkyl) group,
d) a C₁₋₆ alkoxy(C₁₋₇ alkyl) group,
e) a C₂₋₇ acyloxy(C₁₋₇ alkyl) group,
f) a C₆₋₁₀ aryl(C₁₋₇ alkyl) group,
g) a heteroaryl(C₁₋₇ alkyl)group,
h) -M¹-COOR¹¹, or
i) -M¹-CONR¹²R¹³;
M¹ is a C₁₋₇ alkylene group;
R¹¹ is a hydrogen atom or a C₁₋₇ alkyl group;
R¹² and R¹³ are independently the following a) to i):
a) a hydrogen atom,
b) a C₆₋₁₀ aryl group,
c) a C₁₋₇ alkyl group,
d) a hydroxy(C₁₋₇ alkyl) group,
e) a C₁₋₆ alkoxy(C₁₋₇ alkyl) group,
f) a heteroaryl(C₁₋₇ alkyl) group,
g) a C₆₋₁₀ aryl(C₁₋₇ alkyl) group,
h) -M²-CONR¹⁴NR¹⁵, or
i) -M²-NR¹⁶SO₂R¹⁷; or
R¹⁴ and R¹⁵ bind together with the nitrogen atom bound to them to form an alicyclic amino group;
R¹⁶ is preferably a hydrogen atom or a C₁₋₇ alkyl group;
R¹⁷ is preferably a C₁₋₇ alkyl group;
R² is preferably a C₁₋₇ alkyl group;
R³ is preferably a hydrogen atom or a halogen atom;
R⁴ is preferably the following a) to c):
a) a hydrogen atom,
b) a halogen atom, or
c) a halo(C₁₋₇ alkyl) group;
R⁵ and R⁶ are preferably a hydrogen atom;
R⁷ is preferably a group represented by -O-R⁷¹ wherein R⁷¹ is a hydrogen atom, a C₁₋₇ alkyl group, a halo(C₂₋₇ alkyl) group or a hydroxy(C₂₋₇ alkyl) group, or a group selected from a group consisting of the following groups: 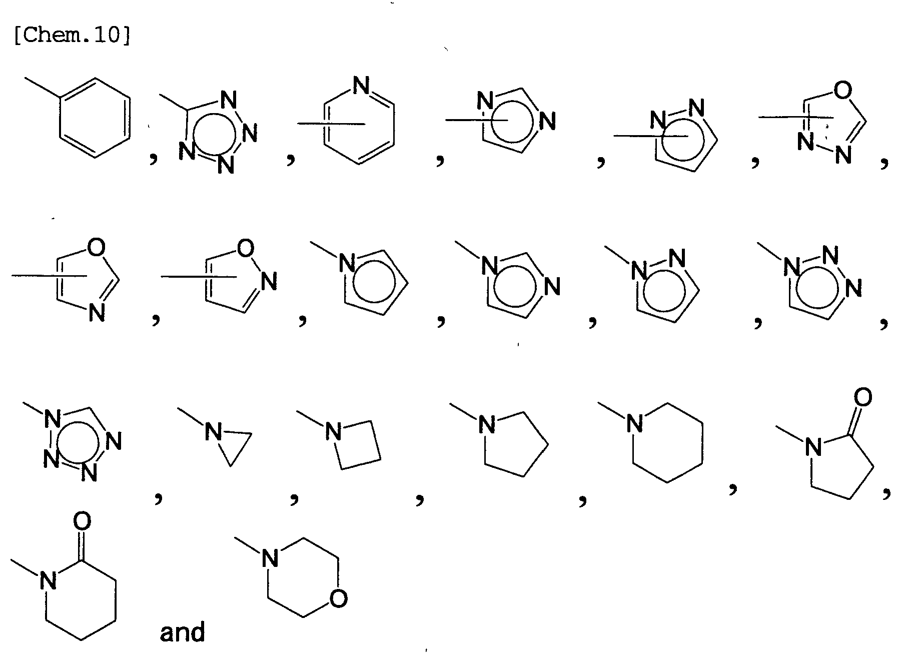 which may be unsubstituted or substituted by a group independently selected from the following groups: a hydroxy group, a halogen atom, a C₁₋₄ alkyl group and a hydroxyl(C₁₋₄ alkyl) group; and
R⁸ is preferably a hydrogen atom.

The compounds represented by the above general formula (A) of the present invention can be prepared, for example, by methods described below in Schemes 1 to 19 or similar methods. In addition, in case that a protective group is necessary depending on a kind of a functional group, introduction and removal procedures can be optionally combined in the usual way. About a kind of a protective group, introduction and removal procedures, for example, the methods described in "Protective Groups in Organic Synthesis (third edition)" written and edited by Green & Wuts can be illustrated.

The typical methods of manufacturing are shown below. There is a case that each process of each scheme described below is executed in combination with multistep reaction, and may be combined with any processes which can be selected by those in the art.

The urea derivatives represented by the above general formula (A) of the present invention can be prepared by using a compound represented by the general formula (VII) as manufacturing intermediates in Scheme 1 described below. The compound (VII) can be prepared by, for example, methods in Scheme 1 described below. In addition, the manufacturing methods described in Scheme 1 can be conducted as the stereoconfiguration is retained, an optically-active compound (VII) wherein the stereoconfiguration is retained can be prepared by using an optically-active starting material or reagent.

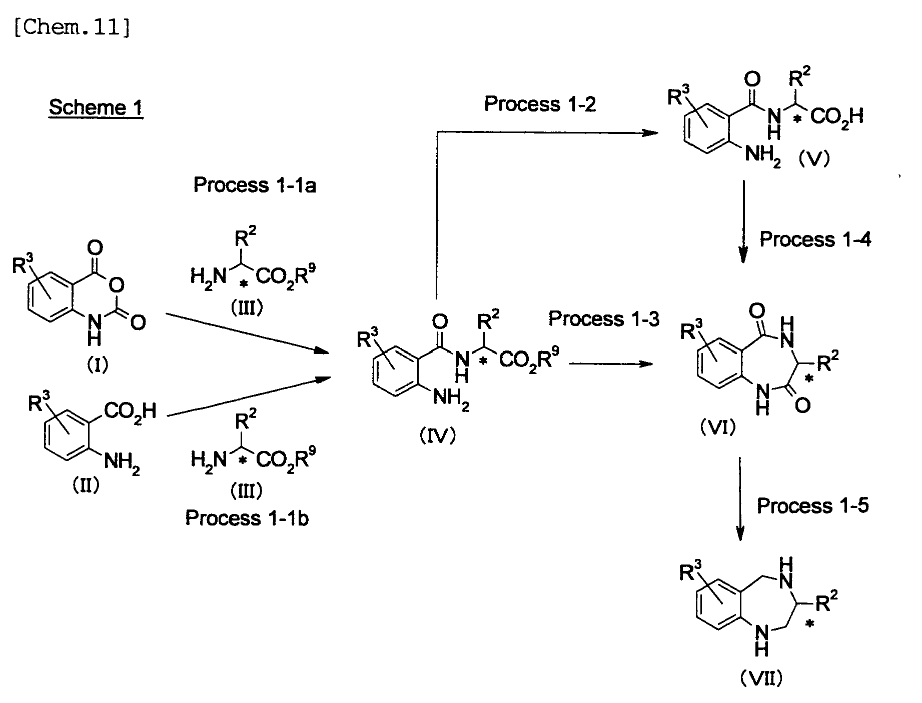

In the formula, R² and R³ have the same meanings as defined above; R⁹ represents a C₁₋₇ alkyl group; and a carbon atom marked with * represents a carbon atom having R-configuration or S-configuration, or a mixture thereof.

### Process 1-1a

A compound represented by the general formula (IV) can be prepared by allowing an isatoic anhydride derivative represented by the general formula (I) to react with an amino acid derivative represented by the general formula (III). The reaction can be conducted in pyridine, triethylamine, dimethylsulfoxide, *N,N*-dimethylacetamide and a mixed solvent thereof. The amino acid derivative represented by the general formula (III) is preferable to use 0.5 to 5 amounts for an isatoic anhydride derivative represented by the general formula (I) . The reaction temperature is usually from 0°C to solvent reflux temperature, and the reaction time is usually from 30 minutes to 3 days, varying based on a used starting material, solvent and reaction temperature.

The compounds represented by the above general formula (IV) can be also prepared in the following methods (Process 1-1b).

### Process 1-1b

A compound represented by the above general formula (IV) can be prepared by subjecting an anthranilic acid derivative represented by the general formula (II) to condensation with the amino acid derivative represented by the above general formula (III) in the presence of a condensation agent such as 1-ethyl-3-(N,N-dimethylaminopropyl)carbodiimide hydrochloride, dicyclohexylcarbodiimide or the like in a solvent, optionally to adding a base such as 4-dimethylaminopyridine, triethylamine or the like. As a solvent used in the reaction, dichloromethane, N,N-dimethylformamide, a mixed solvent thereof or the like can be illustrated. It is preferable to use 0.5 to 5 amounts of the amino acid derivative represented by the above general formula (III), a condensation agent and a base for the anthranilic acid derivative represented by the above general formula (II). The reaction temperature is usually from 0°C to solvent reflux temperature, and the reaction time is usually from 30 minutes to 3 days, varying based on a used starting material, solvent and reaction temperature.

### Process 1-2

A compound represented by the above general formula (V) can be prepared by subjecting the compound represented by the above general formula (IV) to hydrolysis with a base such as lithium hydroxide, sodium hydroxide or the like in a solvent. As a solvent used in the reaction, water, tetrahydrofuran, methanol, ethanol, a mixed solvent thereof or the like can be illustrated. It is preferable to use 1 to 5 amounts of the base for the compound represented by the above general formula (IV). The reaction temperature is usually from 0°C to solvent reflux temperature, and the reaction time is usually from 30 minutes to 3 days, varying based on a used starting material, solvent and reaction temperature.

### Process 1-3

In the present process, a compound represented by the general formula (VI) can be prepared by application of the method (a) or (b) described below.
(a) The compound represented by the above general formula (VI) can be prepared by subjecting the compound represented by the above general formula (IV) to cyclization in the presence or absence of a base such as sodium cyanide, sodium methoxide, n-butyl lithium, sodium hydride or the like in a solvent. As a solvent used in the reaction, tetrahydrofuran, N,N-dimethylacetamide, ethanol, a mixed solvent thereof or the like can be illustrated. It is preferable to use a catalystic amount to 1 amount of a base for the compound represented by the above general formula (IV). The reaction temperature is usually from 0°C to solvent reflux temperature, and the reaction time is usually from 30 minutes to 3 days, varying based on a used starting material, solvent and reaction temperature.
(b) The compound represented by the above general formula (VI) can be prepared by subjecting the compound represented by the above general formula (IV) to cyclization under an acidic condition using acetic acid, hydrogen chloride, sulfonic acid or the like in a solvent or without a solvent. As a solvent used in the reaction, water, toluene, ethanol, a mixed solvent thereof or the like can be illustrated. It is preferable to use a catalystic amount to excessive amounts of an acid for the compound represented by the above general formula (IV). The reaction temperature is usually from 0 to 150°C, and the reaction time is usually from 30 minutes to 3 days, varying based on a used starting material, solvent and reaction temperature.

### Process 1-4

A compound represented by the general formula (VI) can be prepared by applying the method described in the above Process 1-1b.

### Process 1-5

A compound represented by the general formula (VII) can be prepared by subj ecting the compound represented by the above general formula (VI) to reduction using a reducing agent such as lithium aluminium hydride, diboran or the like. As a solvent used in the reaction, tetrahydrofuran, diethylether, 1,2-dimethoxyethene, a mixed solvent thereof or the like can be illustrated. It is preferable to use 1 to 5 amounts of a reducing agent for the compound represented by the above general formula (VI). The reaction temperature is usually from 0°C to solvent reflux temperature, and the reaction time is usually from 30 minutes to 3 days, varying based on a used starting material, solvent and reaction temperature.

The compound represented by the above general formula (VII) can be also prepared in Scheme 2 described below. In addition, the manufacturing methods described in Scheme 2 can be conducted as the stereoconfiguration is retained, an optically-active compound represented by the above general formula (VII) wherein the stereoconfiguration is retained can be prepared by using an optically-active starting material or reagent.

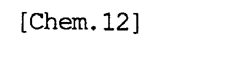

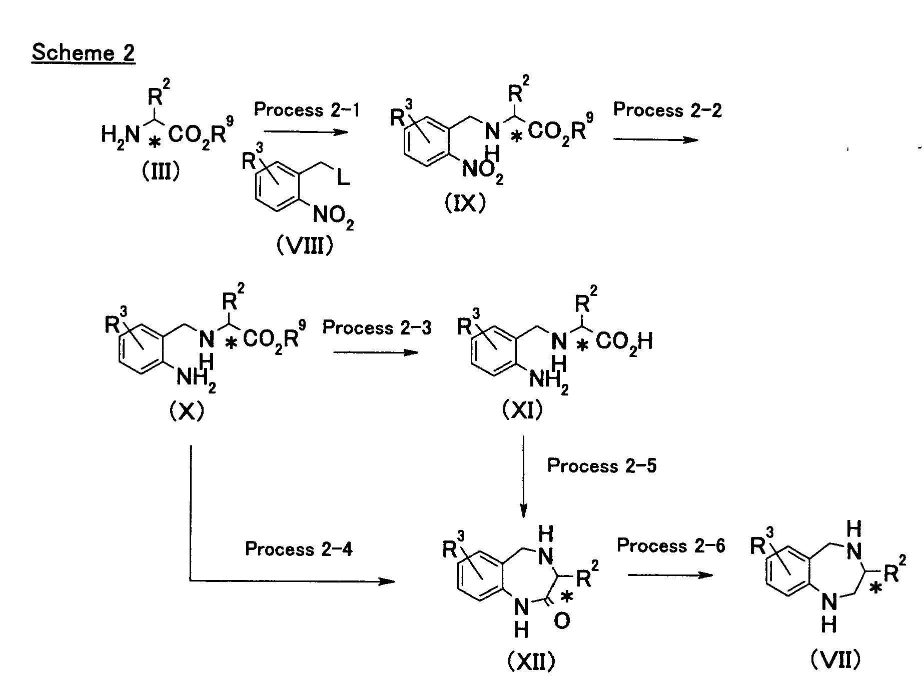

In the formula, R², R³ and R⁹ have the same meanings as defined above; L represents a leaving group such as a chlorine atom, a bromine atom, an iodine atom, a methanesulfonyloxy group, p-toluenesulfonyloxy group or the like; and a carbon atom marked with * represents a carbon atom having R-configuration or S-configuration, or a mixture thereof.

### Process 2-1

A compound represented by the general formula (IX) can be prepared by subjecting an amino acid derivative represented by the above general formula (III) to alkylation with a reactive functional derivative represented by the general formula (VIII) in the presence of a base such as triethylamine, or sodium hydroxide, potassium carbonate or the like in a solvent. As a solvent used in the reaction, N,N-dimethylformamide, acetonitrile, tetrahydrofuran, a mixed solvent thereof or the like can be illustrated. It is preferable to use 0.5 to 5 amounts of a reactive functional derivative represented by the general formula (VIII) and a base for an amino acid derivative represented by the above general formula (III). The reaction temperature is usually from 0°C to solvent reflux temperature, and the reaction time is usually from 30 minutes to 3 days, varying based on a used starting material, solvent and reaction temperature.

### Process 2-2

A compound represented by the general formula (X) can be prepared by application of the present process can be conducted by method (a) or (b) described below.
(a) The compound represented by the above general formula (X) can be prepared by subjecting the compound represented by the above general formula (IX) to reduction using a metal catalyst in a solvent, under a hydrogen gas atmosphere. As a solvent used in the reaction, methanol, ethanol, tetrahydrofuran, a mixed solvent thereof or the like can be illustrated. As a using metal catalyst, palladium on carbon, platinum oxide or the like can be illustrated. It is preferable to use a catalystic amount to 1 amount of a metal catalyst for a compound represented by the above general formula (IX). The reaction temperature is usually from 0°C to solvent reflux temperature, and the reaction time is usually from 30 minutes to 3 days, varying based on a used starting material, solvent and reaction temperature.
(b) The compound represented by the above general formula (X) can be prepared by subj ecting a nitro group of the compound represented by the above general formula (IX) to reduction using a metal such as iron, zinc or the like, or a metal salt, under an acidic condition using acetic acid, hydrochloric acid, sulfonic acid or the like in a solvent. As a solvent used in the reaction, methanol, ethanol, a mixed solvent thereof or the like can be illustrated. It is preferable to use 1 to 5 amounts of a metal or a metal salt for a compound represented by the above general formula (IX). The reaction temperature is usually from 0°C to solvent reflux temperature, and the reaction time is usually from 30 minutes to 3 days, varying based on a used starting material, solvent and reaction temperature.

### Process 2-3

A compound represented by the general formula (XI) can be prepared by applying the method described in Process 1-2.

### Process 2-4

A compound represented by the general formula (XII) can be prepared by applying the method described in Process 1-3.

### Process 2-5

A compound represented by the general formula (XII) can be prepared by applying the method described in Process 1-1b.

### Process 2-6

A compound represented by the general formula (VII) can be prepared by applying the method described in Process 1-5.

The compound represented by the above general formula (VII) can be also prepared in Scheme 3 described below. In addition, the manufacturing methods described in Scheme 3 can be conducted as the stereoconfiguration is retained, an optically-active compound represented by the above general formula (VII) wherein the stereoconfiguration is retained can be prepared by using an optically-active starting material or reagent.

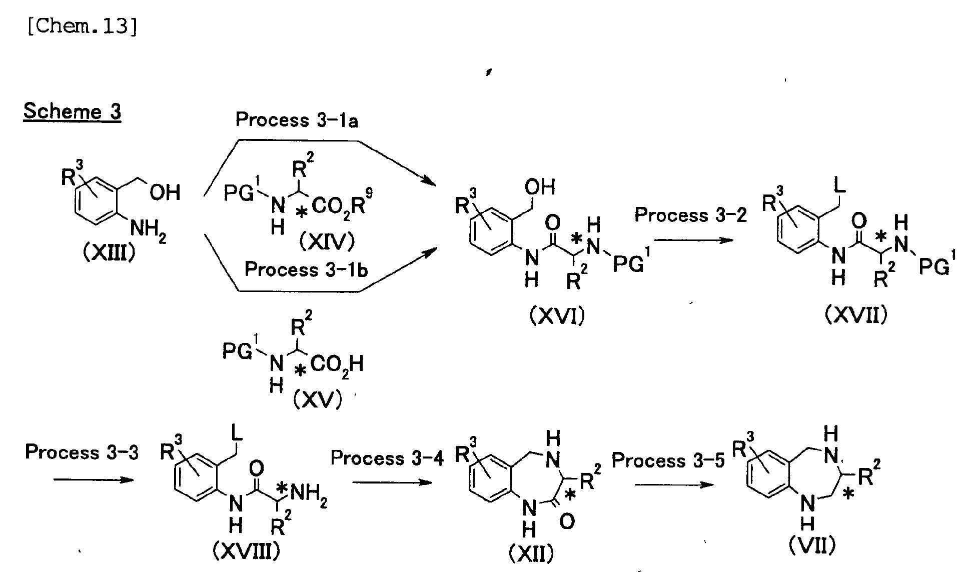

In the formula, R², R³, R⁹ and L have the same meanings as defined above; PG¹ represents a protective group such as a benzyloxycarbonyl group, a tert-butoxycarbonyl group, a triphenylmethyl group or the like; and a carbon atom marked with * represents a carbon atom having R-configuration or S-configuration, or a mixture thereof.

### Process 3-1a

A compound represented by the general formula (XVI) can be prepared by applying the method described in Process 1-3.

### Process 3-1b

A compound represented by the general formula (XVI) can be prepared by applying the method described in Process 1-1b.

### Process 3-2

An alcoholic hydroxy group of the compound represented by the general formula (XVI) can be converted into the leaving group L, for example, by applying the methods described in JIKKEN KAGAKU KOUZA (Fourth Edition of the Experimental Chemistry Course) edited by The Chemical Society of Japan, Vol.19 (Organic Synthesis I), 1992, MARUZEN CO., LTD publication, SHIN JIKKEN KAGAKU KOUZA (New Experimental Chemistry Course) edited by The Chemical Society of Japan, Vol. 14 (Synthesis and Reaction of organic Compounds III), 1978, MARUZEN CO., LTD publication, the corresponding reactive functional derivative (XVII) can be prepared.

### Process 3-3

Removal of a protective group of the reactive functional derivative represented by the above general formula (XVII) can be usually conducted by the methods described in "Protective Groups in Organic Synthesis (third edition)" written and edited by Green & Wuts, WILEY-INTERSCIENCE publication, the corresponding reactive functional derivative represented by the general formula (XVIII) can be prepared.

### Process 3-4

A compound represented by the above general formula (XII) can be prepared by subjecting the reactive functional derivative represented by the above general formula (XVIII) to cyclization in the presence of a base such as potassium carbonate, sodium hydroxide, triethylamine or the like in a solvent. As a solvent used in the reaction, tetrahydrofuran, N,N-dimethylformamide, ethanol, a mixed solvent thereof or the like can be illustrated. It is preferable to use 1 to 5 amounts of a base for the reactive functional derivative represented by the above general formula (XVIII). The reaction temperature is usually from 0°C to solvent reflux temperature, and the reaction time is usually from 30 minutes to 3 days, varying based on a used starting material, solvent and reaction temperature.

### Process 3-5

A compound (VII) can be prepared by applying the method described in Process 1-5.

The compounds represented by the above general formula (VII) can be also prepared in Scheme 4 described below. In addition, the manufacturing methods described in Scheme 4 can be conducted as the stereoconfiguration is retained, an optically-active compound represented by the above general formula (VII) wherein the stereoconfiguration is retained can be prepared by using an optically-active starting material or reagent.

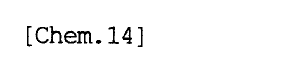

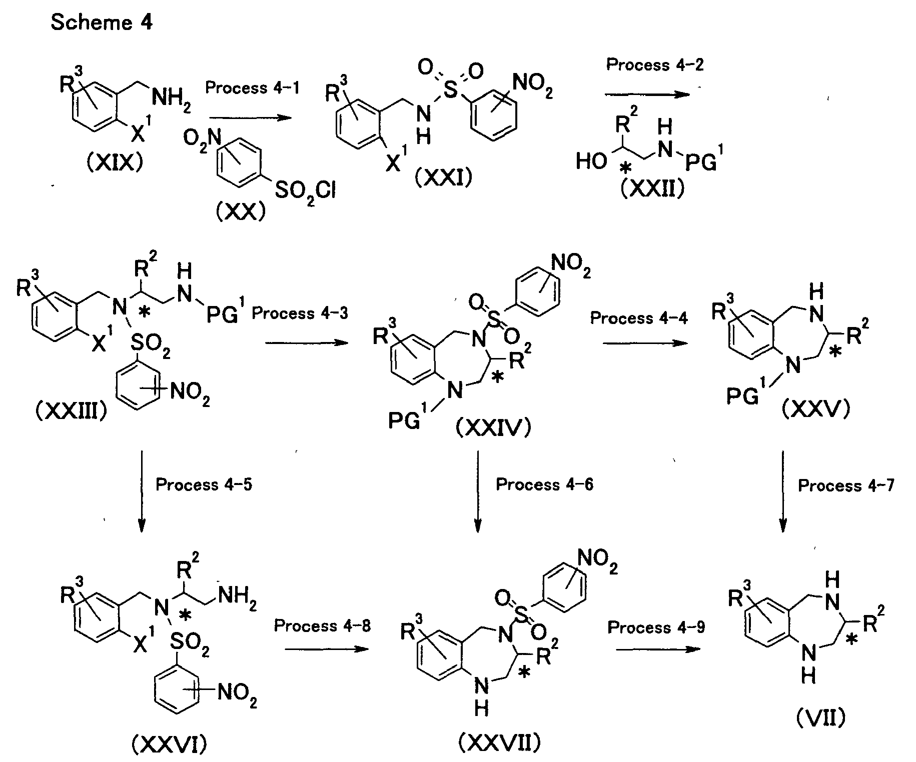

In the formula, R², R³ and PG¹ have the same meanings as defined above; X¹ represents a bromine atom, an iodine atom and a trifluoromethanesulfonyloxy group; and a carbon atom marked with * represents a carbon atom having R-configuration or S-configuration, or a mixture thereof.

### Process 4-1

A compound represented by the general formula (XXI) can be prepared by subjecting a benzylamine derivative represented by the general formula (XIX) to condensation with a benzensulfonylchloride derivative represented by the above general formula (XX) in the presence of a base such as triethylamine, pyridine, potassium carbonate or the like in a solvent. As a solvent used in the reaction, dichloromethane, tetrahydrofuran, a mixed solvent thereof or the like can be illustrated. It is preferable to use 1 to 5 amounts of the benzensulfonylchloride derivative represented by the above general formula (XX) and a base for a compound represented by the above general formula (XIX). The reaction temperature is usually from 0°C to solvent reflux temperature, and the reaction time is usually from 30 minutes to 3 days, varying based on a used starting material, solvent and reaction temperature.

### Process 4-2

A compound represented by the general formula (XXIII) can be prepared by subjecting a compound represented by the above general formula (XXI) to condensation with a compound represented by the general formula (XXII) in the presence of triphenylphosphine and a dehydration-condensation agent such as diethyl azodicarboxylate diisopropyl azodicarboxylate or the like in a solvent. As a solvent used in the reaction, benzene, toluene, tetrahydrofuran, a mixed solvent thereof or the like can be illustrated. It is preferable to use 1 to 5 amounts of the compound represented by the above general formula (XXII), a dehydration-condensation agent and triphenylphosphine for a compound represented by the above general formula (XXI). The reaction temperature is usually from 0°C to solvent reflux temperature, and the reaction time is usually from 30 minutes to 3 days, varying based on a used starting material, solvent and reaction temperature.

### Process 4-3

A compound represented by the above general formula (XXIV) can be prepared by subjecting a compound represented by the above general formula (XXIII) to cyclization in the presence of a base such as cesium carbonate, potassium carbonate, potassium phosphate or the like, a palladium catalyst such as palladium(II) acetate, bis(dibenzylideneacetone) palladium(0) or the like,and 9,9-dimethyl-4,5-bis(diphenylphosphino) xanthene in a solvent. As a solvent used in the reaction, tetrahydrofura 1,4-dioxane, a mixed solvent thereof or the like can be illustrated. It is preferable to use 1 to 5 amounts of a base for the compound represented by the above general formula (XXIII). It is preferable to use a catalystic amount to 1 amount of a palladium catalyst and 9,9-dimethyl-4, 5-bis (diphenylphosphino) xanthene for the compound represented by the above general formula (XXIII). The reaction temperature is usually from room temperature to solvent reflux temperature, and the reaction time is usually from 30 minutes to 3 days, varying based on a used starting material, solvent and reaction temperature.

### Process 4-4

A compound represented by the general formula (XXV) can be prepared by allowing a compound represented by the above general formula (XXIV) to react with nucleophilic reagent such as benzenthiol, methylamine, propylamine or the like in the presence or absence of potassium carbonate or the like in a solvent. As a solvent used in the reaction, N,N-dimethylformamide, dichloromethane, chloroform, a mixed solvent thereof or the like can be illustrated. It is preferable to use 1 to 5 amounts of the using nucleophilic reagent for a compound represented by the above general formula (XXIV). The reaction temperature is usually from 0°C to solvent reflux temperature, and the reaction time is usually from 30 minutes to 3 days, varying based on a used starting material, solvent and reaction temperature.

### Process 4-5

A compound (XXVI) can be prepared by applying the method described in Process 3-3.

### Process 4-6

A compound (XXVII) can be prepared by applying the method described in Process 3-3.

### Process 4-7

A compound (VII) can be prepared by applying the method described in Process 3-3.

### Process 4-8

A compound represented by the above general formula (XXVII) can be prepared by subjecting a compound represented by the above general formula (XXVI) to cyclization in the presence of a base such as cesium carbonate, sodium tert-butoxide or the like, a palladium catalyst such as palladium(II) acetate, and 2,2'-bis(diphenylphosphino) -1,1'-binaphthyl in a solvent. As a solvent used in the reaction, toluene, xylene, a mixed solvent thereof or the like can be illustrated. It is preferable to use 1 to 5 amounts of a base for the compound represented by the above general formula (XXVI). It is preferable to use a catalystic amount to 1 amount of a palladium catalyst or 2,2'-bis(diphenylphosphino)-1,1'-binaphthyl for the compound represented by the above general formula (XXVI). The reaction temperature is usually from room temperature to solvent reflux temperature, and the reaction time is usually from 30 minutes to 3 days, varying based on a used starting material, solvent and reaction temperature.

### Process 4-9

The compounds (VII) can be prepared by applying the method described in Process 4-4.

The compounds represented by the above general formula (VII) can be also prepared in Scheme 5 described below. In addition, because the manufacturing method described in Scheme 5 can be conducted as the stereoconfiguration is retained, an optically-active compound represented by the above general formula (VII) wherein the stereoconfiguration is retained can be prepared by using an optically-active starting material or reagent.

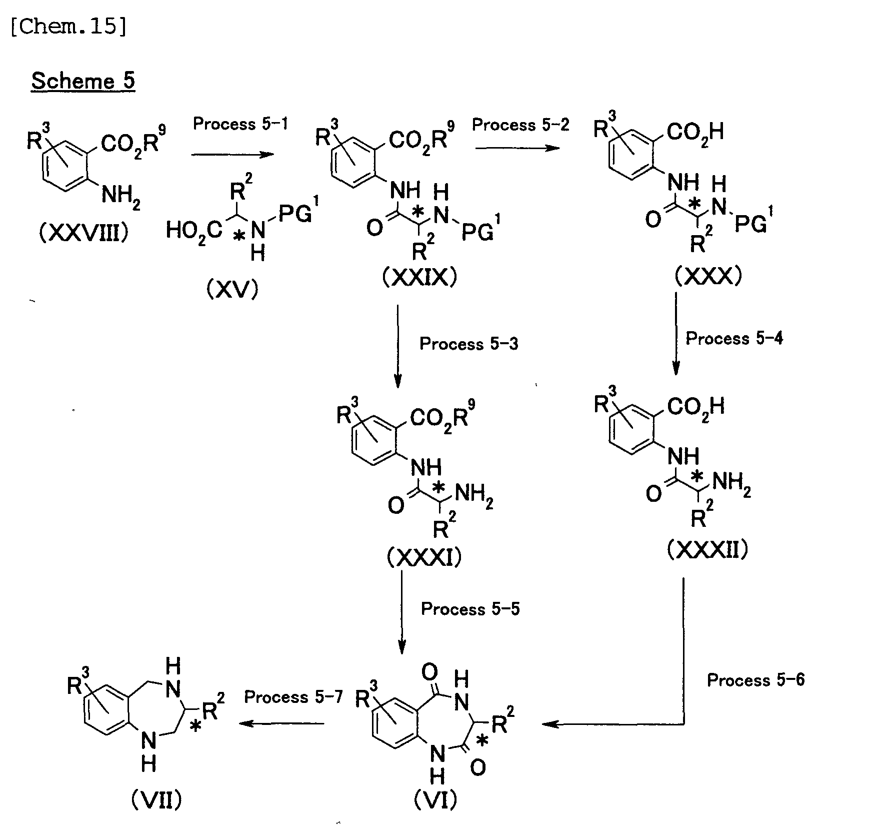

In the formula, R², R³, R⁹ and PG¹ have the same meanings as defined above; and a carbon atom marked with * represents a carbon atom having R-configuration or S-configuration, or a mixture thereof.

### Process 5-1

A compound (XXIX) can be prepared by applying the method described in Process 1-1b.

### Process 5-2

A compound (XXX) can be prepared by applying the method described in Process 1-2.

### Process 5-3

A compound (XXXI) can be prepared by applying the method described in Process 3-3.

### Process 5-4

A compound (XXXII) can be prepared by applying the method described in Process 3-3.

### Process 5-5

A compound (VI) can be prepared by applying the method described in Process 1-3.

### Process 5-6

A compound (VI) can be prepared by applying the method described in Process 1-1b.

### Process 5-7

A compound (VII) can be prepared by applying the method described in Process 1-5.

In addition, among the compounds represented by the above general formula (VII), in case that R² is a hydrogen atom, the compound can be also prepared in Scheme 6 described below.

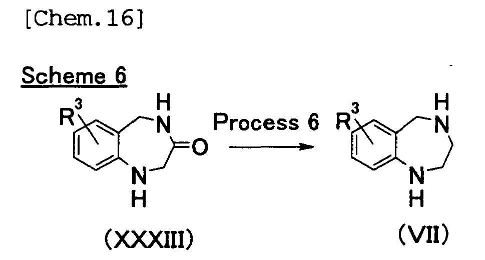

In the formula, R³ has the same meanings as defined above.

### Process 6

A compound (VII) can be prepared by applying the method described in Process 1-5.

The urea derivatives represented by the above general formula (A) of the present invention can be prepared by using a compound represented by the general formula (XLI) as manufacturing intermediates in Scheme 7 described below. A compound represented by the general formula (XLI) can be prepared, for example, in Scheme 7 described below. In addition, the manufacturing method described in Scheme 7 can be conducted as the stereoconfiguration is retained, an optically-active compound wherein the stereoconfiguration is retained can be prepared by using an optically-active starting material or reagent.

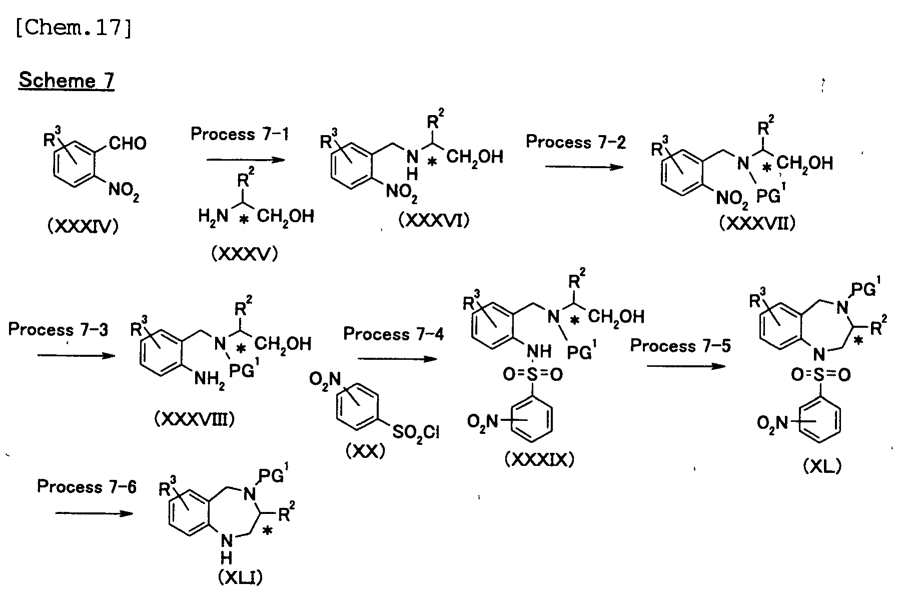

In the formula, R², R³ and PG¹ have the same meanings as defined above; and a carbon atom marked with * represents a carbon atom having R-configuration or S-configuration, or a mixture thereof.

### Process 7-1

A compound represented by the general formula (XXXVI) can be prepared by subjecting an imine which is prepared in a reaction by treating a benzaldehyde derivative represented by the above general formula (XXXIV) with an aminoalchol derivative represented by the above general formula (XXXV) in the presence or absence of a base such as potassium carbonate, potassium hydroxide or the like in a solvent, to reduction using a reducing agent such as lithium aluminium hydride, sodium tetrahydroborate, sodium cyanoborohydride or the like. As a solvent used in the reaction, methanol, ethanol, tetrahydrofuran, a mixed solvent thereof or the like can be illustrated. It is preferable to use 1 to 5 amounts of a aminoalcohol derivative represented by the above general formula (XXXV), a base and a reducing agent for the compoundrepresentedby the above general formula (XXXIV). The reaction temperature is usually from 0°C to solvent reflux temperature, and the reaction time is usually from 30 minutes to 3 days, varying based on a used starting material, solvent and reaction temperature.

### Process 7-2

Introduction of a protective group into a nitrogen atom of a compound represented by the above general formula (XXXVI) can be usually conducted in the methods described in "Protective Groups in Organic Synthesis (third edition)" written and edited by Green & Wuts, WILEY-INTERSCIENCE publication, the corresponding compound represented by the general formula (XXXVII) can be prepared.

### Process 7-3

A compound (XXXVIII) can be prepared by applying the method described in Process 2-2.

### Process 7-4

A compound (XXXIX) can be prepared by applying the method described in Process 4-1.

### Process 7-5

A compound (XL) can be prepared by applying the method described in Process 4-2.

### Process 7-6

A compound (XLI) can be prepared by applying the method described in Process 4-4.

In addition, the compound represented by the above general formula (XLI) can be also prepared in Scheme 8 described below. And the manufacturing method described in Scheme 8 can be conducted as the stereoconfiguration is retained, an optically-active compound represented by the above general formula (XLI) wherein the stereoconfiguration is retained can be prepared by using an optically-active starting material or reagent.

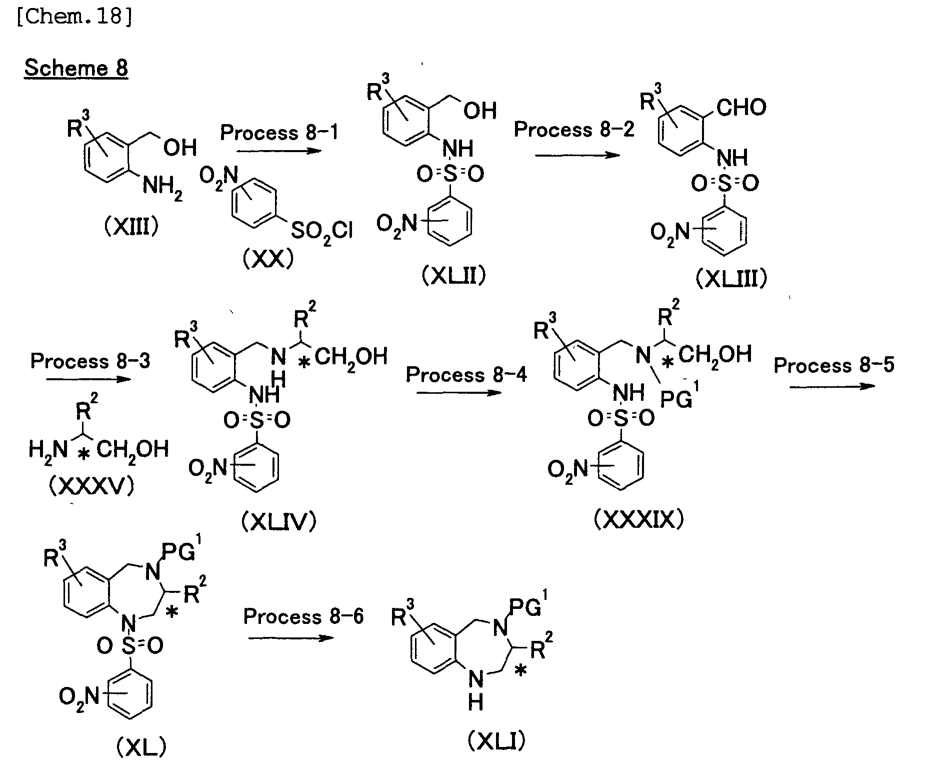

In the formula, R², R³ and PG¹ have the same meanings as defined above; and a carbon atom marked with * represents a carbon atom having R-configuration or S-configuration, or a mixture thereof..

### Process 8-1

A compound (XLII) can be prepared by applying the method described in Process 4-1.

### Process 8-2

A compound represented by the above general formula (XLII) which has an alcoholic hydroxy group can be induced to the corresponding compound represented by the general formula (XLIII) by applying the known oxidative reaction. Specifically, oxidation of an alcoholic hydroxy group using an activated manganese dioxide or the like can be illustrated, these reaction can be conducted by applying the method described in JIKKEN KAGAKU KOUZA (Fourth Edition of the Experimental Chemistry Course) edited by The Chemical Society of Japan, Vol.23 (Organic Synthesis V Oxidation Reaction), 1992, MARUZEN CO., LTD publication.

### Process 8-3

A compound (XLIV) can be prepared by applying the method described in Process 7-1.

### Process 8-4

A compound (XXXIX) can be prepared by applying the method described in Process 7-2.

### Process 8-5

A compound (XL) can be prepared by applying the method described in Process 4-2.

### Process 8-6

A compound (XLI) can be prepared by applying the method described in Process 4-4.

The urea derivatives represented by the above general formula (A) of the present invention can be prepared by using a compound represented by the general formula (LIV) as manufacturing intermediates in Scheme 9 described below. A compound represented by the general formula (LIV) can be prepared by methods in Scheme 9 described below. In addition, the manufacturing methods described in Scheme 9 can be conducted as the stereoconfiguration is retained, an optically-active compound represented by the general formula (LIV) wherein the stereoconfiguration is retained can be prepared by using an optically-active starting material or reagent.

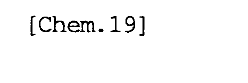

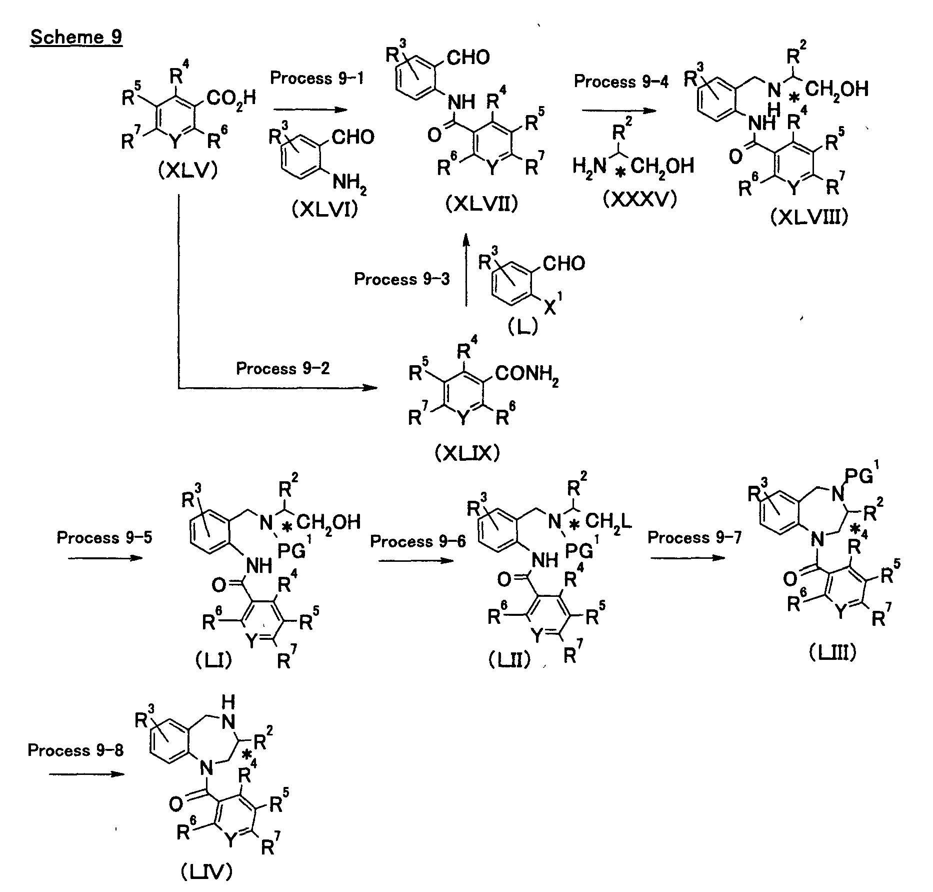

In the formula, R², R³, R⁴, R⁵, R⁶, R⁷, X¹, Y, L and PG¹ have the same meanings as defined above; and a carbon atom marked with * represents a carbon atom having R-configuration or S-configuration, or a mixture thereof.

### Process 9-1

A compound (XLVII) can be prepared by applying the method described in Process 1-1b.

### Process 9-2

A compound (XLIX) can be prepared by applying the method described in Process 1-1b.

### Process 9-3

A compound (XLVII) can be prepared by applying the method described in Process 4-3.

### Process 9-4

A compound (XLVIII) can be prepared by applying the method described in Process 7-1.

### Process 9-5

A compound (LI) can be prepared by applying the method described in Process 7-2.

### Process 9-6

A compound (LII) can be prepared by applying the method described in Process 3-2.

### Process 9-7

A compound represented by the general formula (LIII) can be prepared by subjecting the compound represented by the above general formula (LII) to cyclization in the presence of a base such as sodium hydride, lithium diisopropylamide, lithium bis(trimethylsilyl)-amide or the like in a solvent. As a solvent used in the reaction, tetrahydrofuran, N,N-dimethylformamide, a mixed solvent thereof or the like can be illustrated. It is preferable to use a catalytic amount to 1 amount of a base for the compound represented by the above general formula (LII). The reaction temperature is usually from -78 °C to solvent reflux temperature, and the reaction time is usually from 30 minutes to 3 days, varying based on a used starting material, solvent and reaction temperature.

### Process 9-8

A compound (LIV) can be prepared by applying the method described in Process 3-3.

In addition, the compounds represented by the above general formula (LIV) can be also prepared in Scheme 10 described below. And the manufacturing methods described in Scheme 10 can be conducted as the stereoconfiguration is retained, an optically-active compound represented by the above general formula (LIV) wherein the stereoconfiguration is retained can be prepared by using an optically-active starting material or reagent.

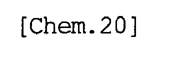

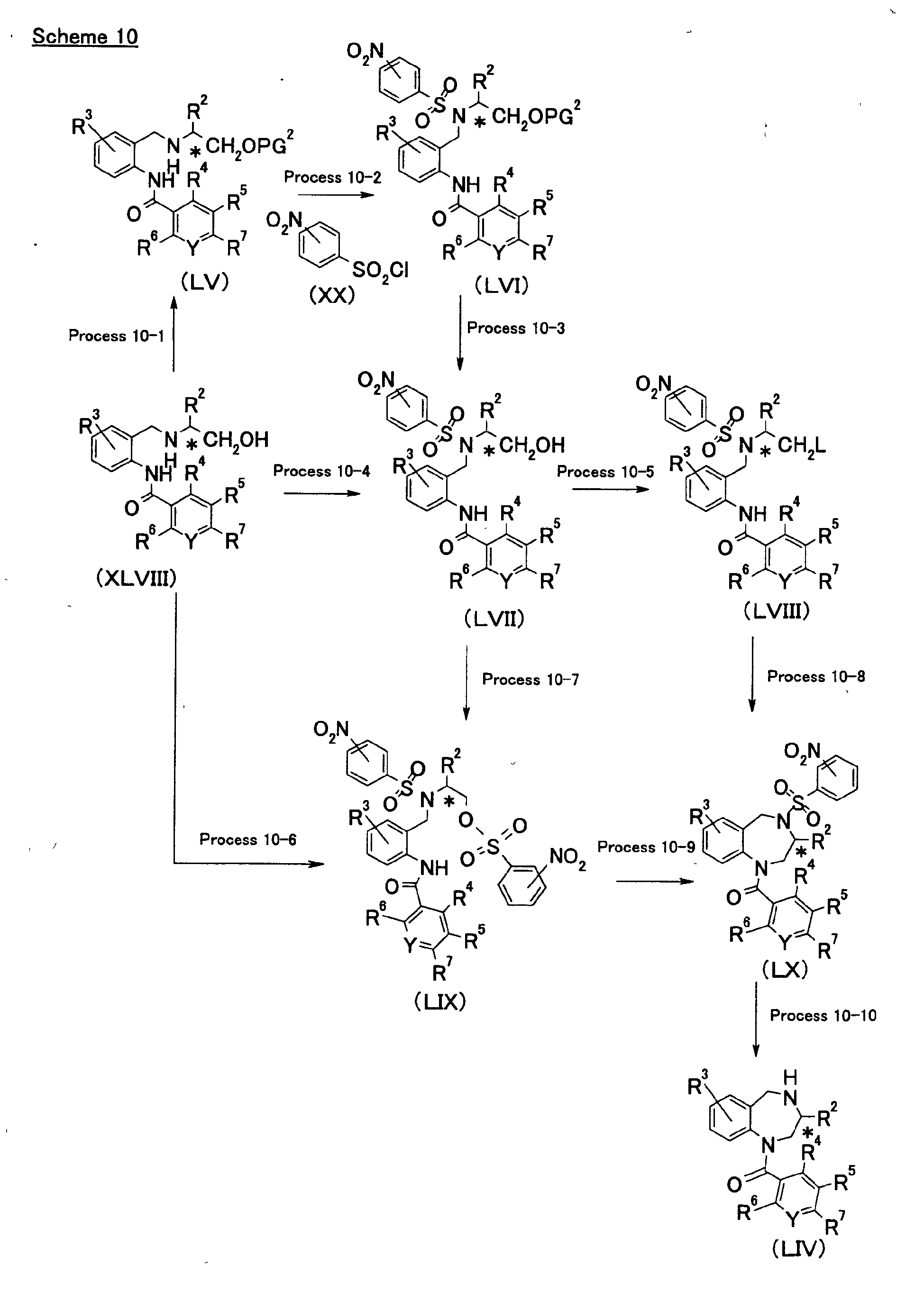

In the formula, R², R³, R⁴, R⁵, R⁶, R⁷, X¹, L and Y have the same meanings as defined above; PG² represents a protective group such as a tert-buthyldimethylsilyl group or the like; and a carbon atom marked with * represents a carbon atom having R-configuration or S-configuration, or a mixture thereof.

### Process 10-1

A protection of an alcoholic hydroxy group of the compound represented by the above general formula (XLVIII) can be usually conducted in the methods described in "Protective Groups in Organic Synthesis (third edition)" written and edited by Green & Wuts, WILEY-INTERSCIENCE publication, the corresponding compound represented by the general formula (LV) can be prepared.

### Process 10-2

A compound (LVI) can be prepared by applying the method described in Process 4-1.

### Process 10-3

A removal of a protective group of the compound represented by the above general formula (LVI) can be usually conducted in the methods described in "Protective Groups in Organic Synthesis (third edition)" written and edited by Green & Wuts, WILEY-INTERSCIENCE publication, the corresponding alcohol compound represented by the general formula (LVII) can be prepared.

### Process 10-4

A compound (LVII) can be prepared by applying the method described in Process 4-1.

### Process 10-5

A compound (LVIII) can be prepared by applying the method described in Process 3-2.

### Process 10-6

A compound (LIX) can be prepared by applying the method described in Process 4-1.

### Process 10-7

A compound (LIX) can be prepared by applying the method described in Process 4-1.

### Process 10-8

A compound (LX) can be prepared by applying the method described in Process 9-7.

### Process 10-9

A compound (LX) can be prepared by applying the method described in Process 9-7.

### Process 10-10

A compound (LIV) can be prepared by applying the method described in Process 4-4.

Among the compounds represented by the above general formula (LIV) which are useful as intermediates manufacturing the urea derivatives represented by the above general formula (A) of the present invention, in case that R⁷ is a heteroaryl group or a alicyclic amino group, the compound can be also prepared in Scheme 11 described below. In addition, the manufacturing methods described in Scheme 11 can be conducted as the stereoconfiguration is retained, and therefore, an optically-active compound (LXX) wherein the stereoconfiguration is retained can be prepared by using an optically-active starting material or reagent.

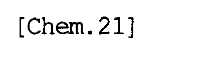

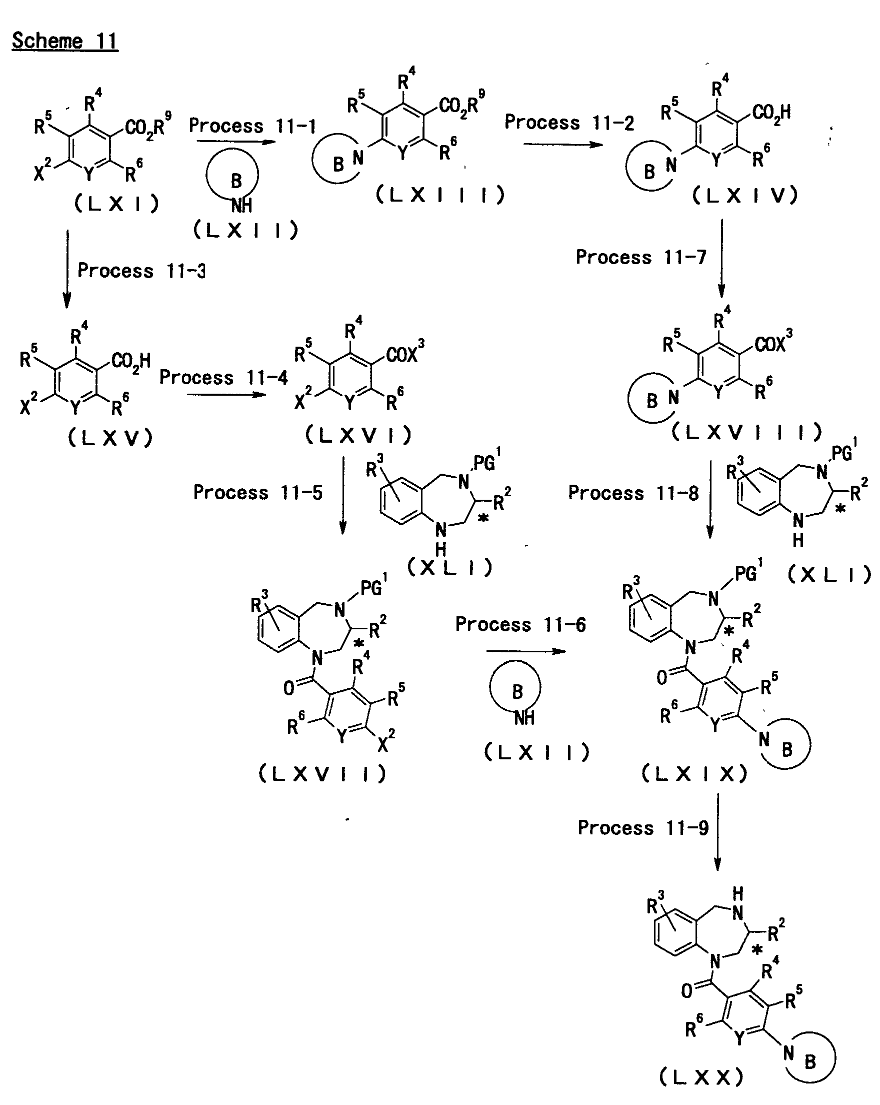

In the formula, R², R³, R⁴, R⁵, R⁶, R⁹, Y, B ring and PG¹ have the same meanings as defined above; X² represents a halogen atom or a trifluoromethanesulfonyloxy group; X³ represents a halogen atom; and a carbon atom marked with * represents a carbon atom having R-configuration or S-configuration, or a mixture thereof.

### Process 11-1

A compound (LXIII) can be prepared by application of a method (a) or (b) described below.
(a) A compound represented by the general formula (LXIII) can be prepared by allowing a compound represented by the general formula (LXI) to react with a compound represented by the general formula (LXII) in the presence or absence of a base such as potassium carbonate, cesium carbonate or the like in a solvent. As a solvent used in the reaction, dimethylsulfoxide, N,N-dimethylacetamide, N,N-dimethylformamide, a mixed solvent thereof or the like can be illustrated. It is preferable to use 1 to 5 amounts of the compound represented by the general formula (LXII) and the base for the compound represented by the general formula (LXI). The reaction temperature is usually from 0°C to solvent reflux temperature, and the reaction time is usually from 30 minutes to 3 days, varying based on a used starting material, solvent and reaction temperature.
(b) A compound (LXIII) can be prepared by applying the method described in Process 4-8.

### Process 11-2

A compound (LXIV) can be prepared by applying the method described in Process 1-2.

### Process 11-3

A compound (LXV) can be prepared by applying the method described in Process 1-2.

### Process 11-4

An acid halide represented by the general formula (LXVI) can be prepared by allowing a benzoic acid derivative represented by the above general formula (LXV) to react with a halogenating agent such as thionyl chloride, oxalyl chloride or the like in a solvent or without. As a solvent used in the reaction, dichloromethane, benzene, toluene, a mixed solvent thereof or the like can be illustrated. It is preferable to use 1 amount to excessive amounts of the the halogenating agent for the benzoic acid derivative represented by the general formula (LXV). The reaction temperature is usually from 0 to 200°C, and the reaction time is usually from 30 minutes to 3 days, varying based on a used starting material, solvent and reaction temperature. In addition, the present reaction may be optionally conducted by adding a catalyst amount of N,N-dimethylformamide, N-methylpyrrolidone or the like.

### Process 11-5

A compound represented by the general formula (LXVII) can be prepared by subjecting a compound represented by the above general formula (XLI) which can be manufactured by methods described in Scheme 7 or 8, or a compound represented by the above general formula (XLI) which can be prepared by introduction of a protective group into a nitrogen atom at 4-position of a benzodiazepine derivative represented by the above general formula (VII) which can be manufactured by methods described in Schemes 1 to 6 in the usual way to condensation with an acid halide represented by the above general formula (LXVI) in the presence of abase such as triethylamine, pyridine, potassium carbonate or the like in a solvent. As a solvent used in the reaction, dichloromethane, tetrahydrofuran, a mixed solvent thereof or the like can be illustrated. It is preferable to use 1 to 5 amounts of the acid halide represented by the above general formula (LXVI) and the base for the compound represented by the above general formula (XLI). The reaction temperature is usually from 0°C to solvent reflux temperature, and the reaction time is usually from 30 minutes to 3 days, varying based on a used starting material, solvent and reaction temperature.

### Process 11-6

A compound (LXIX) can be prepared by applying the method described in Process 11-1.

### Process 11-7

A compound (LXVIII) can be prepared by applying the method described in Process 11-4.

### Process 11-8

A compound (LXIX) can be prepared by applying the method described in Process 11-5.

### Process 11-9

A compound (LXX) can be prepared by applying the method described in Process 3-3.

The urea derivatives represented by the above general formula (A) of the present invention can be prepared by using a compound represented by the general formula (LXXVIII) as manufacturing intermediates in Scheme 12 described below. The compounds (LXXVIII) can be prepared, for example, in Scheme 12 described below. In addition, the manufacturing methods described in Scheme 12 can be conducted as the stereoconfiguration is retained,an optically-active compound wherein the stereoconfiguration is retained can be prepared by using an optically-active starting material or reagent.

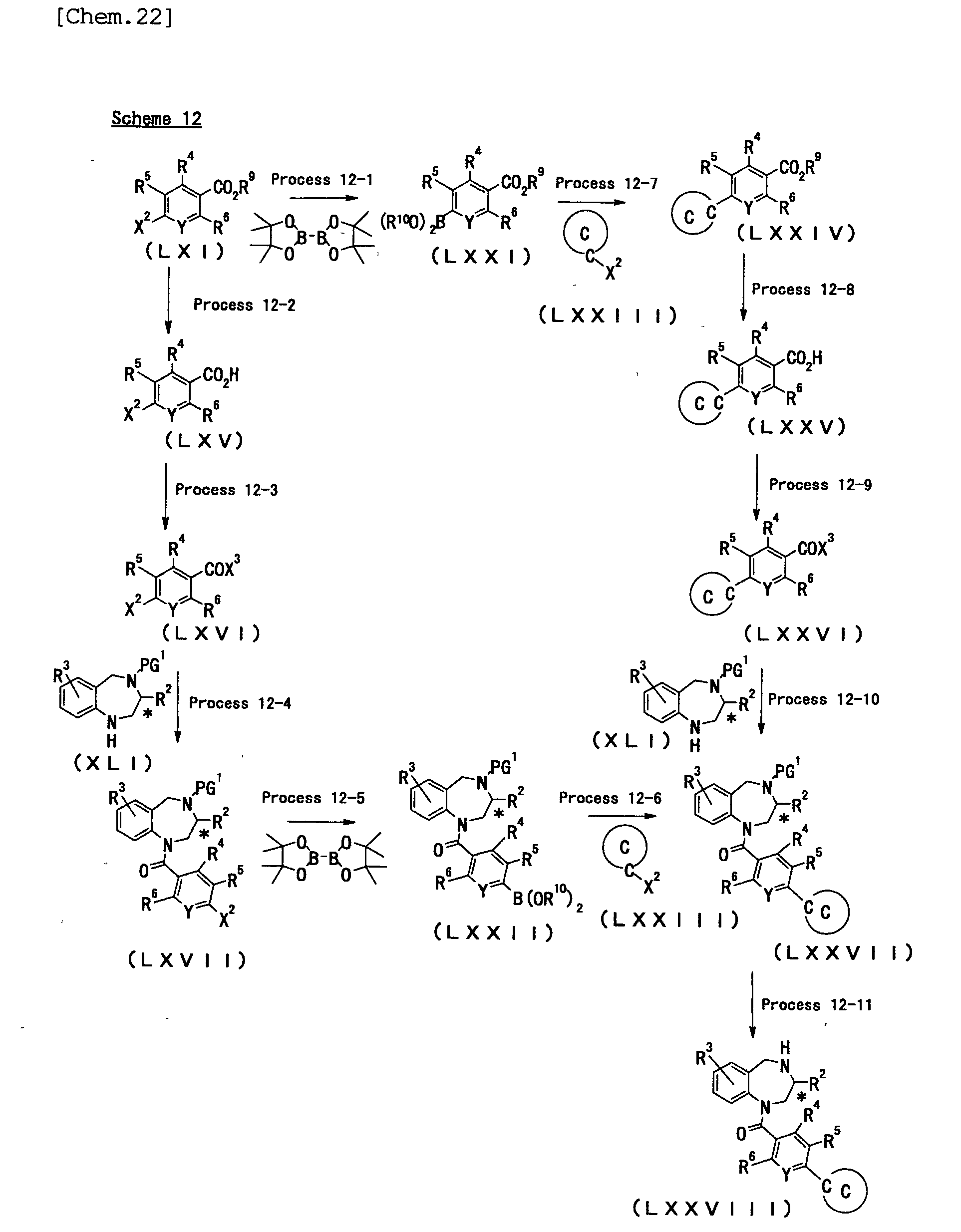

In the formula, R², R³, R⁴, R⁵, R⁶, R⁹, Y, X², X³, C ring and PG¹ have the same meanings as defined above; R¹⁰ represents a hydrogen atom or 2 groups of R¹⁰ form a group represented by -C(CH₃)₂-C(CH₃)₂-; and a carbon atom marked with * represents a carbon atom having R-configuration or S-configuration, or a mixture thereof.

### Process 12-1

A compound represented by the general formula (LXXI) can be prepared by allowing a compound represented by the above general formula (LXI) to react with a bis(pinacolato)diboron in a solvent, in the presence of a base such as potassium acetate or the like, and a palladium catalyst such as dichlorobis(triphenylphosphine) palladium (II) or the like. As a solvent used in the reaction, dimethylsulfoxide, N,N-dimethylformamide, 1,4-dioxane, a mixed solvent thereof or the like can be illustrated. It is preferable to use 1 to 5 amounts of the bis(pinacolato)diboron and the base for the compound represented by the above general formula (LXI). The reaction temperature is usually from room temperature to solvent reflux temperature, and the reaction time is usually from 30 minutes to 3 days, varying based on a used starting material, solvent and reaction temperature. In addition, the present reaction may be optionally conducted by adding a ligand such as bis(diphenylphosphino)ferrocene or the like.

### Process 12-2

A compound (LXV) can be prepared by applying the method described in Process 1-2.

### Process 12-3

A compound (LXVI) can be prepared by applying the method described in Process 11-4.

### Process 12-4

A compound (LXVII) can be prepared by applying the method described in Process 11-5.

### Process 12-5

A compound (LXXII) can be prepared by applying the method described in Process 12-1.

### Process 12-6

A compound represented by the general formula (LXXVII) can be prepared by allowing a boronic acid derivative represented by the above general formula (LXXII) to react with a compound represented by the above general formula (LXXIII) in a solvent, in the presence of a base such as sodium carbonate, cesium fluoride or the like, and a palladium catalyst such as dichlorobis(triphenylphosphine) palladium(II), tetrakis(triphenylphosphine)palladium(0)or the like. As a solvent used in the reaction, toluene, N,N-dimethylformamide, 1,4-dioxane, a mixed solvent thereof or the like can be illustrated. It is preferable to use 1 to 5 amounts of the compound represented by the above general formula (LXXIII) and the base for the boronic acid derivative represented by the above general formula (LXII). The reaction temperature is usually from room temperature to solvent reflux temperature, and the reaction time is usually from 30 minutes to 3 days, varying based on a used starting material, solvent and reaction temperature. In addition, the present reaction may be optionally conducted by adding a ligand such as bis(diphenylphosphino)ferrocene or the like.

### Process 12-7

A compound (LXXIV) can be prepared by applying the method described in Process 12-6.

### Process 12-8

A compound (LXXV) can be prepared by applying the method described in Process 1-2.

### Process 12-9

A compound (LXXVI) can be prepared by applying the method described in Process 11-4.

### Process 12-10

A compound (LXXVII) can be prepared by applying the method described in Process 11-5.

### Process 12-11

A compound (LXXVIII) can be prepared by applying the method described in Process 3-3.

In addition, the compounds represented by the above general formula (LXXVIII) can be also prepared in Scheme 13 described below. And the manufacturing methods described in Scheme 13 can be conducted as the stereoconfiguration is retained, an optically-active compound represented by the above general formula (LXXVIII) wherein the stereoconfiguration is retained can be prepared by using an optically-active starting material.

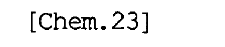

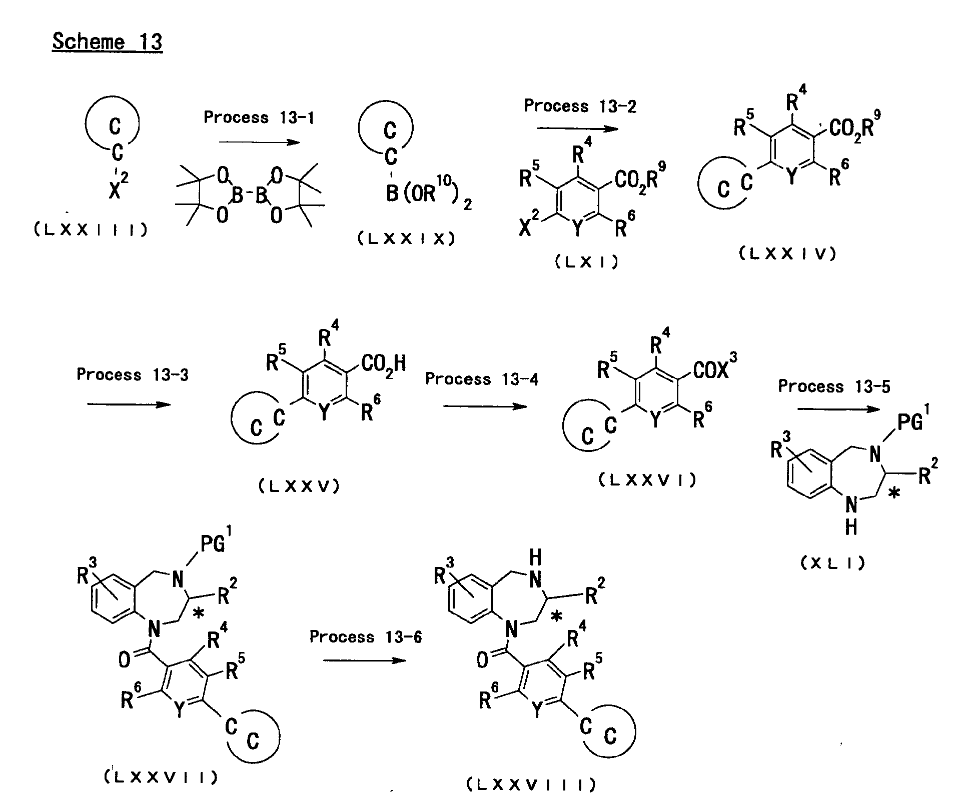

In the formula, R² R³, R⁴, R⁵, R⁶, R⁹, R¹⁰, Y, X², X³, PG¹, and C ring have the same meanings as defined above; and a carbon atom marked with * represents a carbon atom having R-configuration or S-configuration, or a mixture thereof.

### Process 13-1

A compound (LXXIX) can be prepared by applying the method described in Process 12-1.

### Process 13-2

A compound (LXXIV) can be prepared by applying the method described in Process 12-6.

### Process 13-3

A compound (LXXV) can be prepared by applying the method described in Process 1-2.

### Process 13-4

A compound (LXXVI) can be prepared by applying the method described in Process 11-4.

### Process 13-5

A compound (LXXVII) can be prepared by applying the method described in Process 11-5.

### Process 13-6

A compound (LXXVIII) can be prepared by applying the method described in Process 3-3.

In addition, the compound represented by the above general formula (LXXVIII) can be also prepared in Scheme 14 described below. And the manufacturing methods described in Scheme 14 can be conducted as the stereoconfiguration is retained, an optically-active compound represented by the above general formula (LXXVIII) wherein the stereoconfiguration is retained can be prepared by using an optically-active starting material.

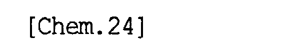

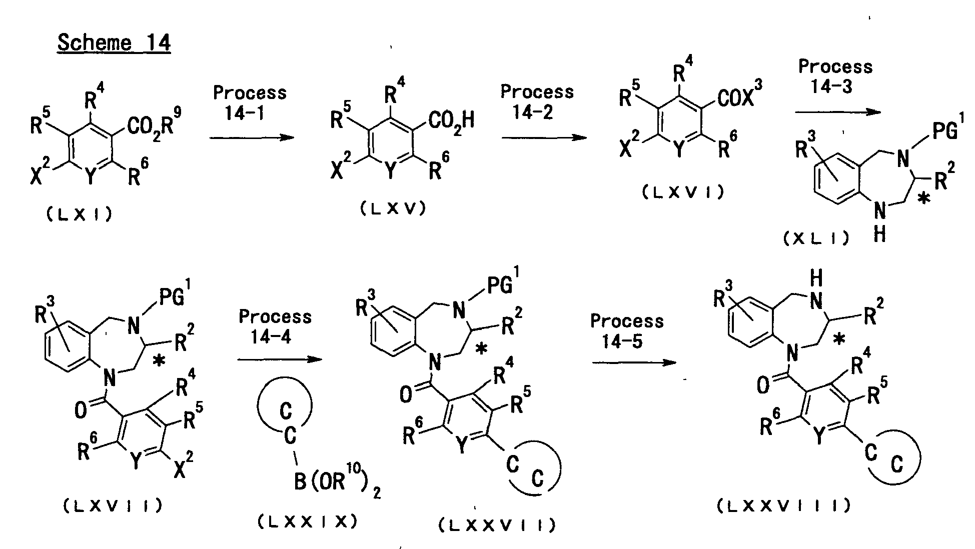

In the formula, R², R³, R⁴, R⁵, R⁶, R⁹, R¹⁰, Y, X², X³, PG¹ and C ring have the same meanings as defined above; and a carbon atom marked with * represents a carbon atom having R-configuration or S-configuration, or a mixture thereof.

### Process 14-1

A compound (LXV) can be prepared by applying the method described in Process 1-2.

### Process 14-2

A compound (LXVI) can be prepared by applying the method described in Process 11-4.

### Process 14-3

A compound (LXVII) can be prepared by applying the method described in Process 11-5.

### Process 14-4

A compound (LXXVII) can be prepared by applying the method described in Process 12-6.

### Process 14-5

A compound (LXXVIII) can be prepared by applying the method described in Process 3-3.

In addition, the compounds represented by the above general formula (LXXVIII) can be also prepared in Scheme 15 described below. And the manufacturing methods described in Scheme 15 can be conducted as the stereoconfiguration is retained, an optically-active compound represented by the above general formula (LXXVIII) wherein the stereoconfiguration is retained can be prepared by using an optically-active starting material.

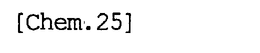

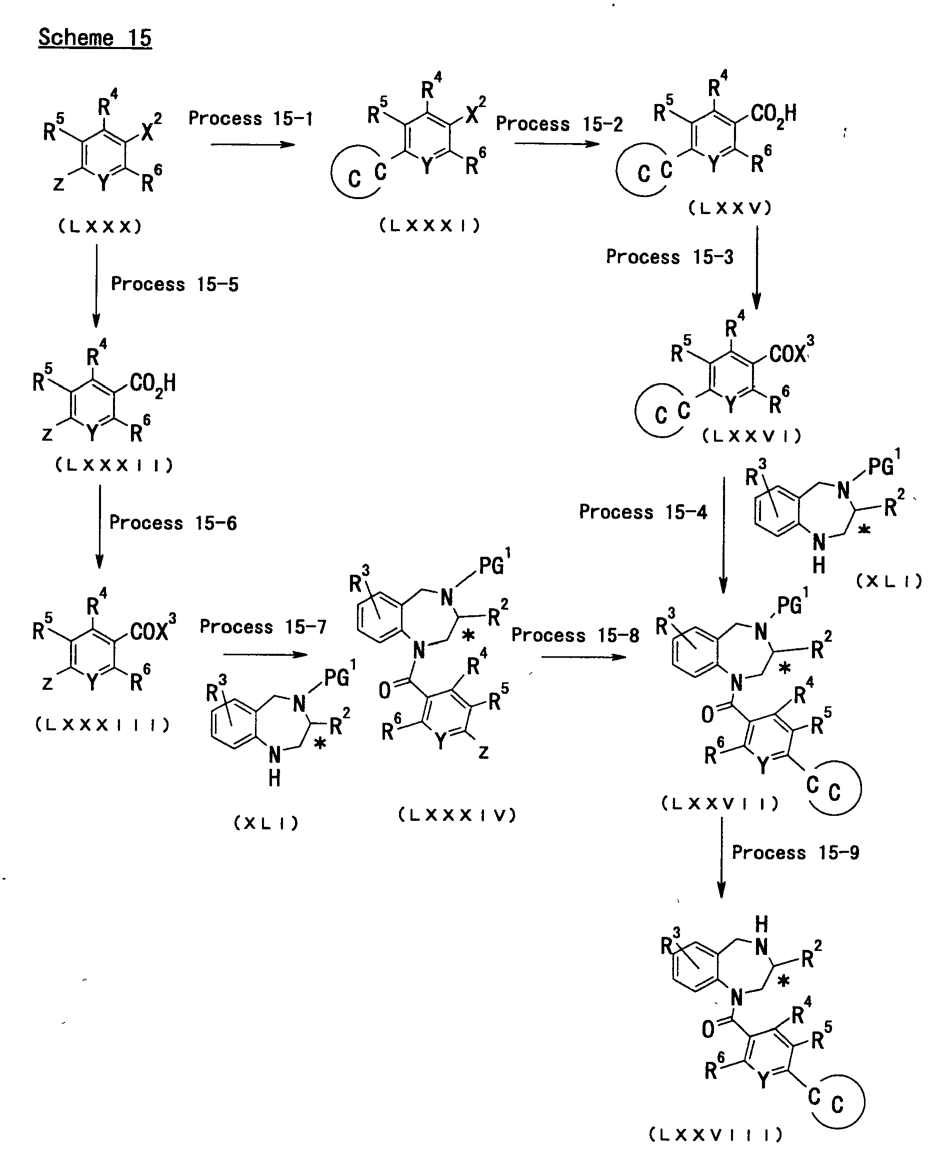

In the formula, R², R³, R⁴, R⁵, R⁶, R⁹, R¹⁰, Y, X², X³, PG¹ and C ring have the same meanings as defined above; Z represents a functional group which can be induced into C ring; and a carbon atom marked with * represents a carbon atom having R-configuration or S-configuration, or a mixture thereof.

### Process 15-1

A reaction by which a functional group Z of a compound represented by the general formula (LXXX) is induced into a C₆₋₁₀ aryl group or a heteroaryl group can be usually conducted in the methods described in "SHINHEN HETEROKANKAGOUBUTSU OUYOUHEN (New edition of heterocyclic compound applied chapter), first edition" and "SHINHEN HETEROKANKAGOUBUTSUKISOHEN (New edition of heterocyclic compound basic chapter), first edition", Koudansha Scientific CO., LTD publication, or the like. A functional group Z such as a cyano group, a carboxy group, an acyl group or the like can be converted into a C₆₋₁₀ aryl group or a heteroaryl group by the manufacturing method, a compound represented by the general formula (LXXXI) can be prepared.

### Process 15-2

A compound represented by the above general formula (LXXV) can be prepared by subjecting a compound represented by the above general formula (LXXXI) to lithiation in a solvent, in the presence of a base such as n-buthyllithium, to reaction with carbon dioxide. As a solvent used in the reaction, tetrahydrofuran, diethylether a mixed solvent thereof or the like can be illustrated. It is preferable to use 1 to 5 amounts of the base for the compound represented by the above general formula (LXXXI) . It is preferable to use large excessive amounts of carbon dioxide for the compound represented by the above general formula (LXXXI) . The reaction temperature is usually from -78°C to room temperature, and the reaction time is usually from 30 minutes to 3 days, varying based on a used starting material, solvent and reaction temperature.

### Process 15-3

A compound (LXXVI) can be prepared by applying the method described in Process 11-4.

### Process 15-4

A compound (LXXVII) can be prepared by applying the method described in Process 11-5.

### Process 15-5

A compound (LXXXII) can be prepared by applying the method described in Process 15-2.

### Process 15-6

On the compound (LXXXII), in case that Z is a functional group other than a carboxy group, the acid halide represented by the above general formula (LXXXII) can be prepared by applying the method described in Process 11-4.

### Process 15-7

A compound (LXXXIV) can be prepared by applying the method described in Process 11-5.

### Process 15-8

A compound (LXXVII) can be prepared by applying the method described in Process 15-1.

### Process 15-9

A compound (LXXVIII) can be prepared by applying the method described in Process 3-3.

The urea derivatives represented by the above general formula (A) of the present invention can be prepared by using a compound represented by the general formula (LIV) as manufacturing intermediates in Scheme 9 or 10. A compound represented by the general formula (A) can be prepared by methods in Scheme 16 described below. In addition, the manufacturing methods described in Scheme 16 can be conducted as the stereoconfiguration is retained, an optically-active compound represented by the general formula (A) wherein the stereoconfiguration is retained can be prepared by using an optically-active starting material or reagent.

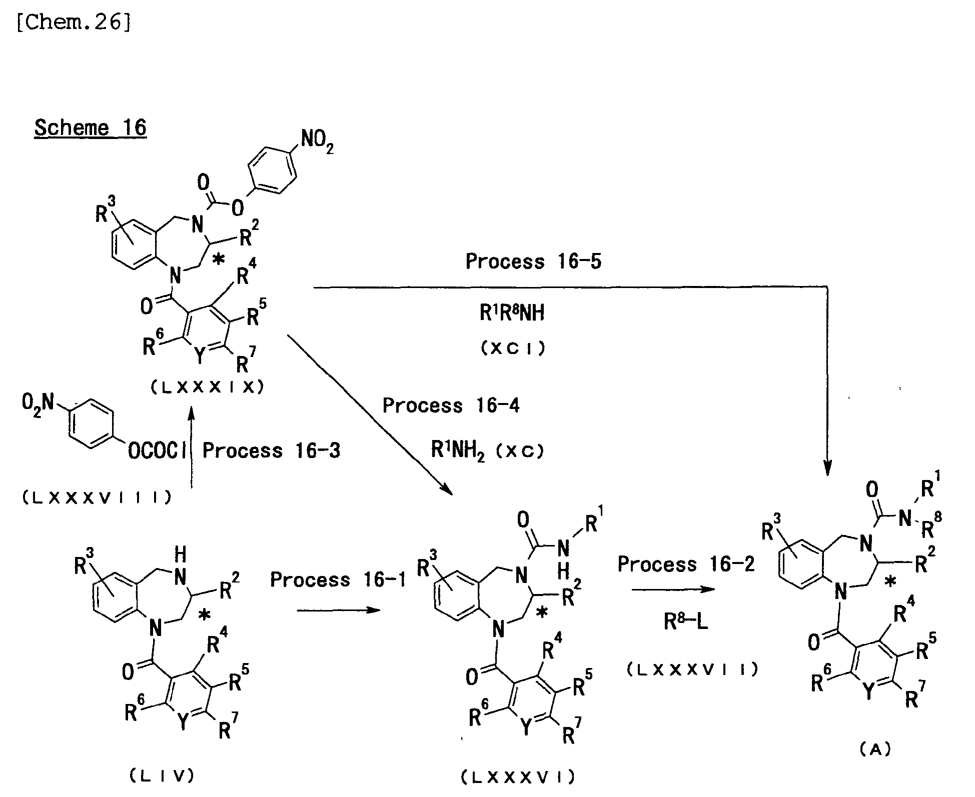

In the formula, R¹, R², R³, R⁴, R⁵, R⁶, R⁷, R⁸, L and Y have the same meanings as defined above; and a carbon atom marked with * represents a carbon atom having R-configuration or S-configuration, or a mixture thereof.

### Process 16-1

A compound (LXXXVI) can be prepared by applying a method (a) or (b) described below.
(a) A compound represented by the above general formula (LXXXVI) can be prepared by allowing a compound represented by the above general formula (LIV) to react with an isocyanate compound represented by the general formula (LXXXV) in the presence or absence of a base such as triethylamine, pyridine or the like in a solvent. As a solvent used in the reaction, dichloromethane, tetrahydrofuran, ethyl acetate, a mixed solvent thereof or the like can be illustrated. It is preferable to use 1 to 5 amounts of the isocyanate compound represented by the above general formula (LXXXV) and the base for the compound represented by the general formula (LIV). The reaction temperature is usually from 0°C to solvent reflux temperature, and the reaction time is usually from 30 minutes to 3 days, varying based on a used starting material, solvent and reaction temperature. In addition, a compound represented by the above general formula (LXXXVI) in which R¹ is a hydrogen atom can be prepared by allowing a compound represented by the above general formula (LIV) to react with a trimethylsilyl isocyanate.
(b) A compound represented by the above general formula (LXXXVI) can be prepared by allowing a compound represented by the general formula (XC) or salt thereof to react with triphosgene in the presence or absence of a base such as triethylamine, N,N-diisopropylethylamine or the like in a solvent. As a solvent used in the reaction, dichloromethane, ethyl acetate, tetrahydrofuran, a mixed solvent thereof or the like can be illustrated. It is preferable to use 0.3 to 1 amounts of triphosgene for the compound represented by the above general formula (XC). It is preferable to use 1 to 5 amounts of the base for the compound represented by the above general formula (XC). And it is preferable to use 1 to 5 amounts of the compound represented by the above general formula (LIV) for the compound represented by the above general formula (XC) . The reaction temperature is usually from -20°C to solvent reflux temperature, and the reaction time is usually from 30 minutes to 3 days, varying based on a used starting material, solvent and reaction temperature. In addition, the present process can be conducted, for example, using an activating reagent such as 1,1*'*-carbonylbis-1H-imidazole or the like instead of triphosgene.

### Processes 16-1 and 16-2

The urea derivatives represented by the above general formula (A) of the present invention can be prepared by subj ecting the compound represented by the above general formula (LIV) to condensation with an amine derivative represented by the general formula (XCI) in a solvent, in one step, by the manufacturing method described in Process 16-1b.

### Process 16-2

A urea derivative represented by the above general formula (A) of the present invention can be prepared by subjecting a compound represented by the above general formula (LXXXVI) by treating a base such as sodium hydride, n-butyllithium or the like in a solvent to alkylation with an alkylating agent represented by the general formula (LXXXVII). As a solvent used in the reaction, tetrahydrofuran, diethylether a mixed solvent thereof or the like can be illustrated. It is preferable to use 1 to 5 amounts of the base and the alkylating agent for the compound represented by the above general formula (LXXXVI). The reaction temperature is usually from -78°C to solvent reflux temperature, and the reaction time is usually from 30 minutes to 3 days, varying based on a used starting material, solvent and reaction temperature.

### Process 16-3

An activated ester compound represented by the general formula (LXXXIX) can be prepared by allowing a compound represented by the above general formula (LIV) to react with an activated ester reagent represented by the general formula (LXXXVIII) in the presence of a base such as triethylamine, pyridine or the like in a solvent. As a solvent used in the reaction, dichloromethane, tetrahydrofuran, ethyl acetate, a mixed solvent thereof or the like can be illustrated. It is preferable to use 1 to 5 amounts of the activated ester reagent represented by the general formula (LXXXVIII) and the base for the compound represented by the above general formula (LIV). Thereaction temperature is usually from 0°C to solvent reflux temperature, and the reaction time is usually from 30 minutes to 3 days, varying based on a used starting material, solvent and reaction temperature.

### Process 16-4

A compound represented by the above general formula (LXXXVI) can be prepared by subjecting an activated ester compound represented by the above general formula (LXXXIX) to condensation with an amine derivative represented by the general formula (XC) or the salt thereof in the presence or absence of a base such as triethylamine, pyridine or the like in a solvent. As a solvent used in the reaction, dichloromethane, tetrahydrofuran, ethyl acetate, a mixed solvent thereof or the like can be illustrated. It is preferable to use 1 to 5 amounts of the the amine derivative represented by the general formula (XC) or the salt thereof and the base for a compound represented by the above general formula (LXXXIX). The reaction temperature is usually from 0°C to solvent reflux temperature, and the reaction time is usually from 30 minutes to 3 days, varying based on a used starting material, solvent and reaction temperature.

### Process 16-5

A urea derivative represented by the above general formula (A) of the present invention can be prepared by applying the method described in Process 16-4.

Among the urea derivatives represented by the above general formula (A) of the present invention, a urea derivative represented by the general formula (XCV), a urea derivative represented by the general formula (XCVI), a urea derivative represented by the general formula (XCVIII) or a urea derivative represented by the general formula (XCVII) can be prepared by using the compound represented, by the general formula (LXVII) as manufacturing intermediates in Scheme 11, for example, can be prepared by methods in Scheme 17 described below. In addition, the manufacturing methods described in Scheme 17 can be conducted as the stereoconfiguration is retained, an optically-active compound represented by the general formula (XCVI) and compound represented by the general formula (XCVIII) wherein the stereoconfiguration is retained can be prepared by using an optically-active starting material or reagent.

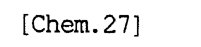

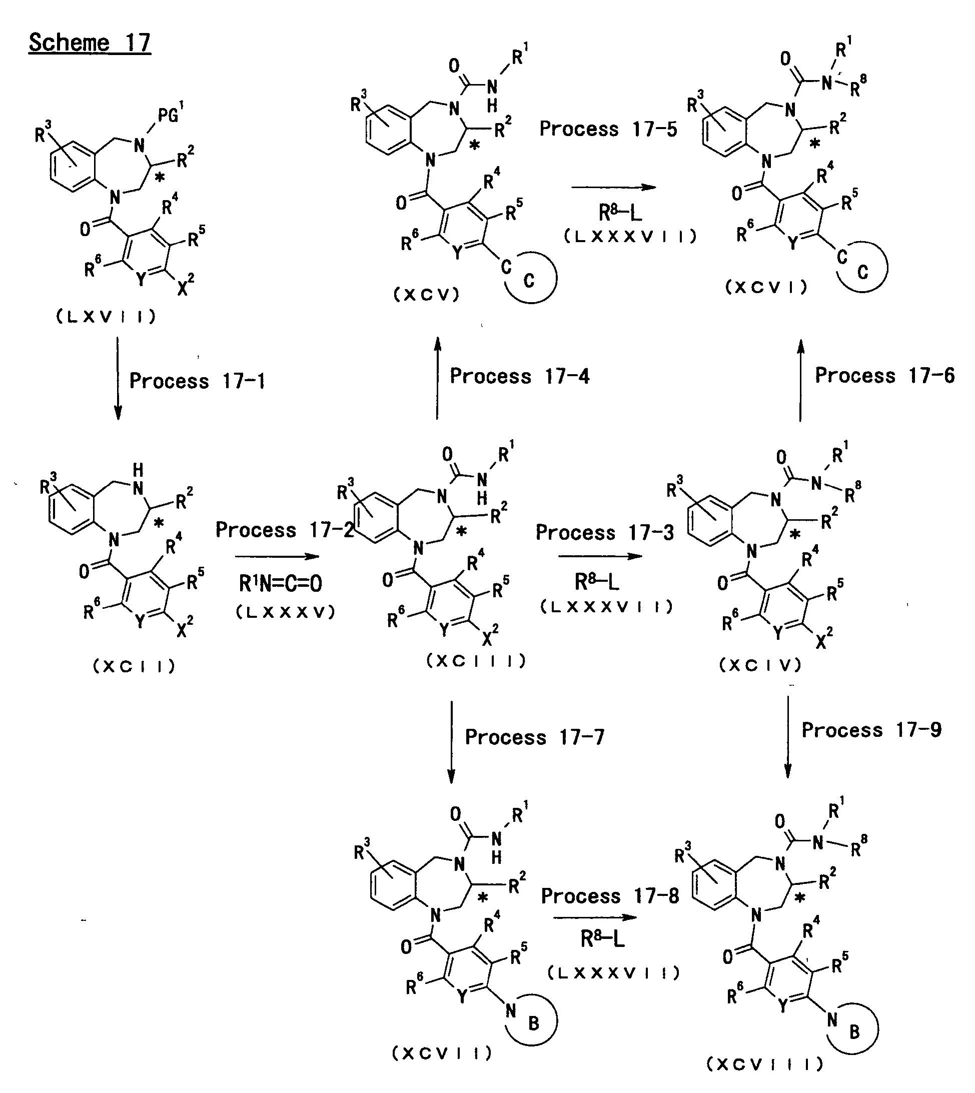

In the formula, R¹, R², R³, R⁴, R⁵, R⁶, R⁸, L, Y, X², PG¹, B ring and C ring have the same meanings as defined above; and a carbon atom marked with * represents a carbon atom having R-configuration or S-configuration, or a mixture thereof.

### Process 17-1

A compound (XCII) can be prepared by applying the method described in Process 3-3.

### Process 17-2

(a) A compound (XCIII) can be prepared by applying the method described in Process 16-1a.
(b) A compound (XCIII) can be prepared by applying the method described in Process 16-1b.

### Processes 17-2 and 17-3

A compound represented by the above general formula (XCIV) can be prepared in one step by subjecting the compound represented by the general formula (XCI) and the compound represented by the above general formula (XCII) to condensation with an amine derivative represented by'the general formula (XCI), with the manufacturing method described in Process 16-1b.

### Process 17-3

A compound (XCIV) can be prepared by applying the method described in Process 16-2.

### Process 17-4

A compound (XCV) can be prepared by applying the method described in Process 12-1 and 12-6.

### Process 17-5

A compound (XCVI) can be prepared by applying the method described in Process 16-2.

### Process 17-6

A compound (XCVI) can be prepared by applying the method described in Process 12-1 and 12-6.

### Process 17-7

A compound (XCVII) can be prepared by applying the method described in Process 11-1.

### Process 17-8

A compound (XCVIII) can be prepared by applying the method described in Process 16-2.

### Process 17-9

A compound (XCVIII) can be prepared by applying the method described in Process 11-1.

A urea derivative represented by the above general formula (XCV), the above general formula (XCVI), the above general formula (XCVII) and the above general formula (XCVIII) of the present invention can be prepared by methods in Scheme 18 described below by using the compound represented by the general formula (LXVII) as manufacturing intermediates in Scheme 11. In addition, the manufacturing methods described in Scheme 18 can be conducted as the stereoconfiguration is retained, an optically-active compound represented by the general formula (XCVI) and compound represented by the general formula (XCVIII) wherein the stereoconfiguration is retained can be prepared by using an optically-active starting material or reagent.

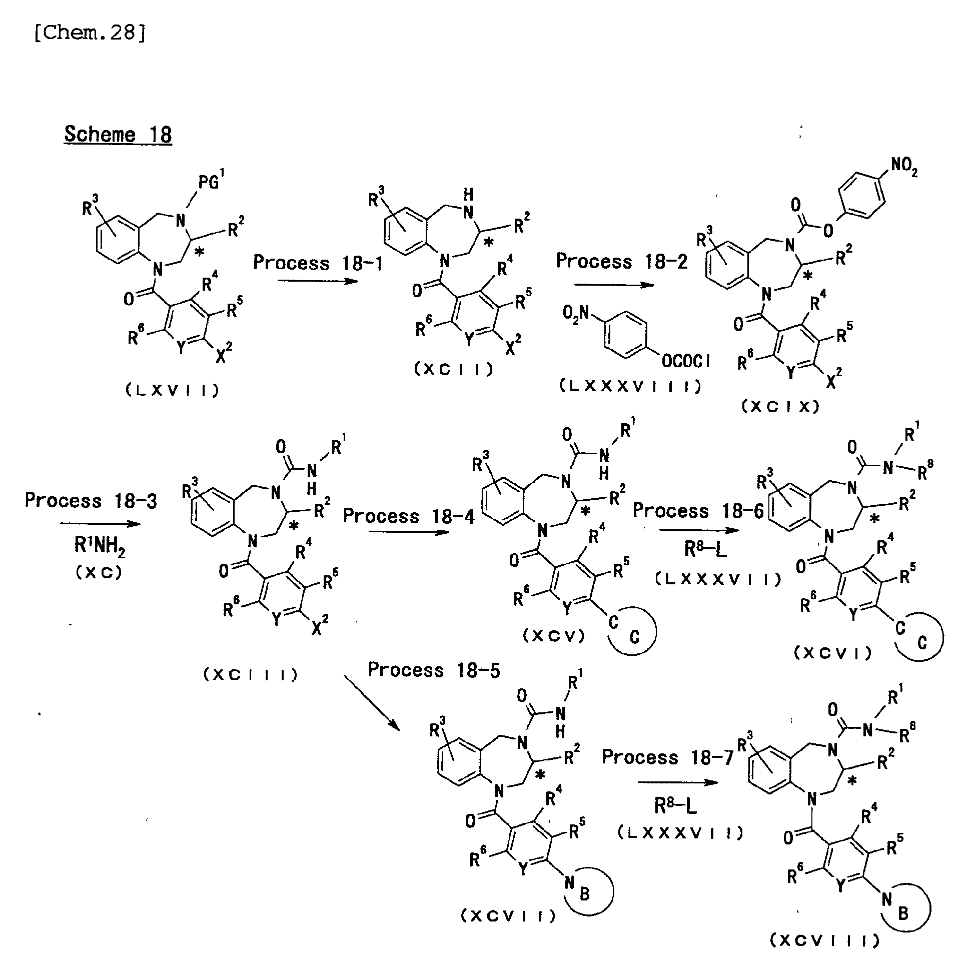

In the formula, R¹, R², R³, R⁴, R⁵, R⁶, Y, X², PG¹, B ring and C ring have the same meanings as defined above; and a carbon atom marked with * represents a carbon atom having R-configuration or S-configuration, or a mixture thereof.

### Process 18-1

A compound (XCII) can be prepared by applying the method described in Process 3-3.

### Process 18-2

A compound (XCIX) can be prepared by applying the method described in Process 16-3.

### Process 18-3

A compound (XCIII) can be prepared by applying the method described in Process 16-4.

### Process 18-4

A compound (XCV) can be prepared by applying the method described in Process 12-1 and 12-6.

### Process 18-5

compound (XCVII) can be prepared by applying the method described in Process 11-1.

### Process 18-6

A compound (XCVI) can be prepared by applying the method described in Process 16-2.

### Process 18-7

A compound (XCVIII) can be prepared by applying the method described in Process 16-2.

A urea derivative represented by the above general formula (XCVI) and the above general formula (XCVIII) of the present invention can be also prepared by using the compound represented by the general formula (LXVII) as manufacturing intermediates in Scheme 11, by methods in Scheme 19 described below. In addition, the manufacturing methods described in Scheme 19 can be conducted as the stereoconfiguration is retained, an optically-active compound represented by the general formula (XCVI) and compound represented by the general formula (XCVIII) wherein the stereoconfiguration is retained can be prepared by using an optically-active starting material or reagent.

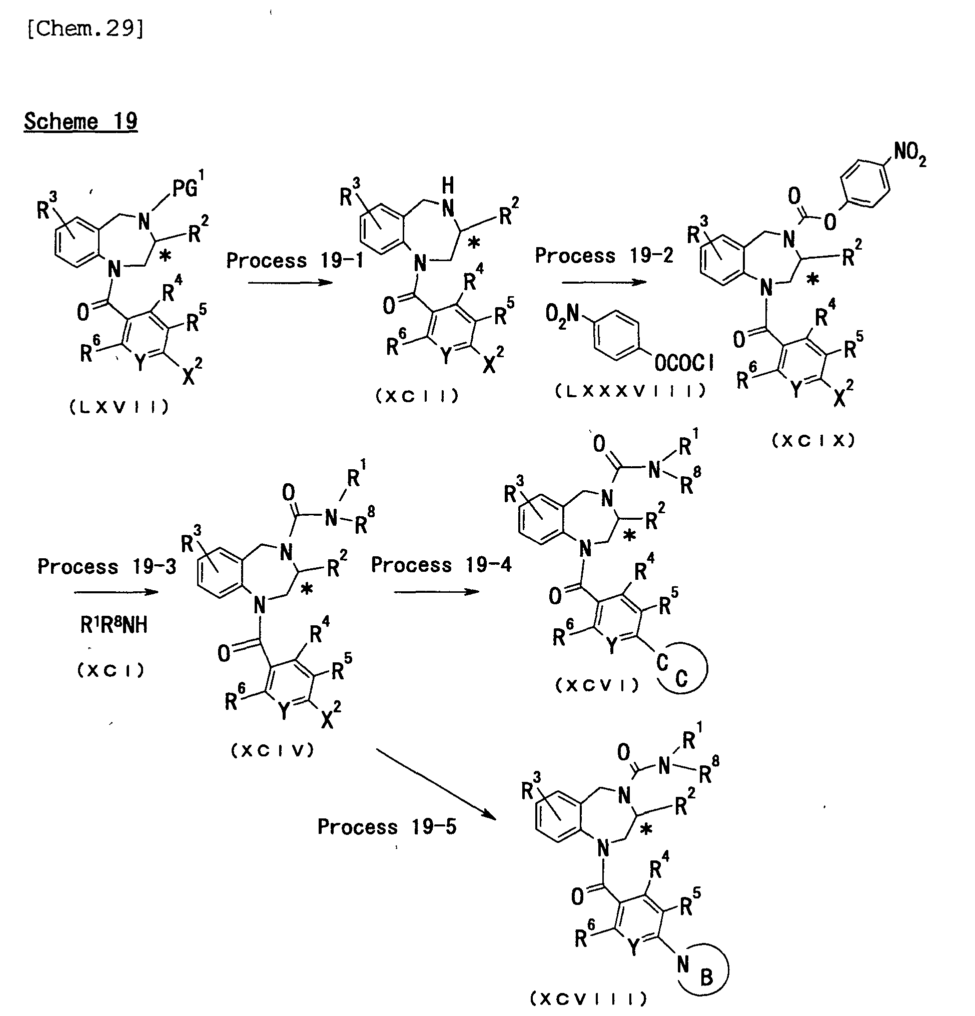

In the formula, R¹, R², R³, R⁴, R⁵, R⁶, R⁸, Y, X², PG¹, B ring and C ring have the same meanings as defined above; and a carbon atom marked with * represents a carbon atom having R-configuration or S-configuration, or a mixture thereof.

### Process 19-1

A compound (XCII) can be prepared by applying the method described in Process 3-3.

### Process 19-2

A compound (XCIX) can be prepared by applying the method described in Process 16-3.

### Process 19-3

A compound (XCIV) can be prepared by applying the method described in Process 16-5.

### Process 19-4

A compound (XCVI) can be prepared by applying the method described in Process 12-1 and 12-6.

### Process 19-5

A compound (XCVIII) can be prepared by applying the method described in Process 11-1.

The schemes shown above are some illustrations of the methods for manufacturing the compounds of the present invention or the manufacturing intermediates thereof. They can be variously modified to the schemes easily understood by those in the art.

The urea derivatives represented by the above general formula (A) of the present invention and intermediates for the use in manufacturing the derivatives can be isolated and purified, optionally by using a operation of a solvent extraction, a recrystallization, chromatography, a preparative high performance liquid chromatography or the like well-known in those in the art in the field as the methods of isolation and purification.

The pharmaceutical compositions comprising as an active ingredient a compound represented by the above general formula (A) of the present invention, or pharmaceutically acceptable salts thereof are used in various dosage forms according to the usage. As the dosage forms, for example, powders, fine granules, granules, dry syrups, tablets, capsules, injections, solutions, ointments, suppositories, poultices, sublingual formulation or the like can be illustrated, and these are administered orally or parenterally.

These pharmaceutical compositions can be prepared by suitably' admixing or by diluting and dissolving with appropriate pharmaceutical additives such as excipients, disintegrators, binders, lubricants, diluents, buffers, isotonicities, antiseptics, moistening agents, emulsifiers, dispersing agents, stabilizing agents, dissolving aids and the like by method well-known in the galenical pharmacy depending on the formulation.

The term "V2 receptor agonist" of the present invention means an agent having an agonism of V2 receptor and acting as an agonist or a partial agonist of V2 receptor. The compounds represented by the above general formula (A) of the present invention can act as an agonist or a partial agonist of V2 receptor.

The compounds represented by the above general formula (A) of the present invention, for example, in a binding experiment for human V2 receptor and a study to confirm the agonism of human V2 receptor, exerted a strong agonism of human V2 receptor. Thence the compounds represented by the above general formula (A) of the present invention can decrease urine volume significantly. Therefore the compounds represented by the above general formula (A) of the present invention can be applied to all symptoms caused by an increseing of urine volume. In addition, the compounds represented by the above general formula (A) have a releasing activity of coagulation factor VIII and von-Wiliebrand factor, and can be used for the treatment of bleeding diseases. Thence, the pharmaceutical compositions comprising as an active ingredient a compound represented by the above general formula (A) of the present invention, or a pharmaceutically acceptable salt thereof, or a prodrug thereof, for example, are useful for various disorder with urination, a large volume of urine and a bleeding tendency, and are preferable as agents for the treatment or prevention of micturition, urinary incontinence, enuresis, central diabetes insipidus, nocturia, spontaneous bleeding, hemophilia, von-Wiliebrand disease, congenital/acquired dysfunction of blood platelets or the like.

The present invention can provide methods for the treatment, prevention or alleviation of a disease improved by stimulating V2 receptor. As these methods for stimulating V2 receptor, for example, methods for the treatment, prevention or alleviation of a disease such as micturition, urinary incontinence, enuresis, central diabetes insipidus, nocturia, spontaneous bleeding, hemophilia, von-Wiliebrand disease, congenital/acquired dysfunction of blood platelets or the like can be illustrated.

The compounds represented by the above general formula (A) of the present invention or the pharmaceutically acceptable salts thereof can be used in combination with at least one agent described below. As agents which can be used in combination with the compounds represented by the above general formula (A) of the present, an α₁-adrenoceptor blocker, a cholinergic blocking agent, a cholinergic agent, an antispasmodic agent, an anti-androgen agent, an antidepressant, a calcium antagonist, a potassium-channel opener, a sensory nerve blocking agent, a β-adrenergic agonist, an acetylcholinesterase inhibitor, anti-inflammatory agent and the like can be illustrated.

In case of uses of the compound represented by the above general formula (A) of the present invention in combination with the above one or more other drugs, either dosage form of simultaneous administration as a single preparation or separated preparations in way of the same or different administration route, and administration at different dosage intervals as separated preparations in way of the same or different administration route can be adopted, a pharmaceutical composition comprising in combination with the compound of the present invention and the above agent can adopt dosage form of a single preparation or combination with separated preparations as follows.

The compounds represented by the above general formula (A) of the present invention can obtain more advantageous effects than additive effects in the prevention or treatment of the above diseases When using suitably in combination with the above one or more drugs. Also, the administration dose can be decreased in comparison with administration of either drug alone, or adverse effects of coadministrated drugs can be avoided or declined.

The concrete compounds as the drugs used for combination and preferable diseases to be treated are exemplified as follows. However, the present invention is not limited thereto, and the concrete compounds include their free compounds, and their pharmaceutically acceptable salts.

As α₁-adrenoceptor blockers, for example, terazosin, bunazosin, urapidil, tamsulosin, bunitrolol, doxazosin, prazosin, carvedilol, bevantolol, WY-21901, naftopidil, alfuzosin, levobunolol, silodosin, IDR-16804, fiduxosin, SPM-969, (S)-doxazosin, KRG-3332 or the like can be illustrated.

As anticholinergic agents, for example, propiverine, oxybutynin, tolterodine, solifenacin or the like can be illustrated.

As cholinergic drugs, for example, besacolin or the like can be illustrated.

As antispasmodic agents, for example, flavoxate or the like can be illustrated.

As anti-androgen drugs, for example, chlormadinone acetate, allylestrenol or the like can be illustrated.

As antidepressants, for example, imipramine or the like can be illustrated.

As calcium antagonists, for example, fasudil, nifedipine, nimodipine, nilvadipine, bepridil, manidipine, barnidipine, nitrendipine, benidipine, isradipine, nicardipine, lercanidipine, amlodipine, nisoldipine, efonidipine, gallopamil, diltiazem, cilnidipine, azelnidipine, felodipine, lacidipine, aranidipine, pranidipine, ranolazine, IQB-875D, iganidipine or the can be illustrated.

As potassium-channel opners, for example, NS-8, nicorandil, tilisolol, pinacidil, levcromakalim, GKE-841, PNU-83757, NN-414, KCO-912, AZD-0947 ABT-598 or the like can be illustrated.

As sensory nerve blocking agents, for example, KW-7158, capsaicin, resiniferatoxin or the like can be illustrated.

As β-adrenergic agonists, for example, mabuterol, ritodrine, fenoterol, denopamine, docarpamine, clenbuterol, formoterol, procaterol, pirbuterol, KWD-2183, xamoterol, terbutaline, tulobuterol, salmeterol, dopexamine, levalbuterol, ephedrine, meluadrine, SR-58611, arformoterol, CHF-4226, KUR-1246, KUC-7483, KTO-7924, YM-178, QAB-149, TD-3327, LY-362884, GW-427353, N-5984, KUL-7211 or the like can be illustrated.

As acetylcholinesterase inhibitors, for example, donepezil, itopride, rivastigmine, metrifonate, galantamine, phenoserine, KA-672, CHF-2819, T-82, EN-101, ZT-1, TAK-802, ladostigil or the like can be illustrated.

As anti-inflammatory agents, suplatast tosilate or the like can be illustrated.

The dosage of a compound represented by the above general formula (A) or a pharmaceutically acceptable salt thereof is appropriately decided depending on the age, sex, body weight and degree of symptoms and treatment of each patient, which is approximately within the range of from 0.01 to 1,000 mg per day per adult human in the case of oral administration and approximately within the range of from 0.01 to 1,000 mg per day per adult human in the case of parenteral administration, and the daily dose can be divided into one to several doses per day and administered suitably.

As pharmaceutical compositions comprising the compound represented by the above general formula (A) or a pharmaceutically acceptable salt thereof in combination with at least one agent selected from a group consisting of a therapeutic agent for diabetes insipidus, nocturia and nocturnal enuresis other than a V2 agonist, the dosage of an agent can be appropriately selected depending on the age, sex, body weight of each patient, the symptom, a dosing period, a dosage form, an administration method, a combination of agents or the like.

### Effect of the Invention

The compounds represented by the above general formula (A) of the present invention, for example, in a binding experiment for human V2 receptor and a study to confirm the agonism of human V2 receptor, exerted a strong agonism of human V2 receptor. Thence the compounds represented by the above general formula (A) of the present invention can decrease urine volume significantly. Therefore the compounds represented by the above general formula (A) of the present invention have a profile based on the effect such as antidiuretic activity and a releasing activity of coagulation factor VIII and von-Wiliebrand factor, and are useful for a various problems of urination, and a large volume of urine and a bleeding tendency, and are preferable as agents for the treatment or prevention of micturition, urinary incontinence, enuresis, central diabetes insipidus, nocturia, spontaneous bleeding, hemophilia, von-Wiliebrand disease, congenital/acquired dysfunction of blood platelets or the like.

### Best Mode to practice the Invention

The present invention'is further illustrated in more detail by way of the following Test Examples. However, the present invention is not limited thereto. In addition, among symbols used in each of Reference exapmles, each of Examples and each of Tables, "Ref.No." means'the number of Reference example, "Ex.No." means the number of Example, "Strc" means a chemical structure formula, and "1H-NMR" means proton nuclear magnetic resonance spectrum. In addition, "CDCl3" means chloroform-d, "DMSO-d6" means dimethylsulfoxide-d₆, and "CD3OD" means methanol-d₄. In addition, "MS" means the mass spectrometry.

### Examples

### Reference example 1

### tert-Butyl 4-fluoro-2-trifluoromethylbenzoate

To a solution of 4-fluoro-2-trifluoromethylbenzoic acid (5.00 g) in tetrahydrofuran (72.0 mL) were successively added tert-butyl 2,2,2-trichloroacetoimidate (8.18mL) and boron trifluoride diethyl ether complex (0.304 mL) under ice-cooling, and the reaction mixture was stirred at room temperature for 18 hours. To the reaction mixture was added 1 mol/L aqueous solution of sodium hydroxide and the mixture was extracted with ethyl acetate. The organic layer was washed with 1 mol/L aqueous solution of sodium hydroxide, water, brine, and dried over anhydrous magnesium sulfate. After filtration, the filtrate was concentrated under reduced pressure. To this residue were added diisopropyl ether-hexane and the insoluble was removed by filtration. This filtrate was concentrated under reduced pressure. The obtained crude product was purified by column chromatography on silica gel (eluent:ethyl acetate-hexane) to give tert-butyl 4-fluoro-2-trifluoromethylbenzoate (3.13 g).
1H-NMR(CDCl3) δ ppm:
1.58 (9H, s), 7.20-7.30 (1H, m), 7.35-7.45 (1H, m), 7.75-7.85 (1H, m).

### Reference example 2-1

### Ethyl 3-chloro-4-pyrazol-1-ylbenzoate

To a suspension of ethyl 3-chloro-4-fluorobenzoate (0.500 g) and potassium carbonate (0.682 g) in N,N-dimethylformamide (5.0 mL) was added 1H-pyrazole (0.185 g) at room temperature and this mixture was stirred at 120 °C for an hour. To the reaction mixture were added water and ethyl acetate. The organic layer was separated and the aqueous layer was extracted with ethyl acetate. The combined organic layer was washed with water and brine, dried over anhydrous magnesium sulfate. After filtration, the filtrate was concentrated under reduced pressure. The obtained crude product was purified by column chromatography on silica gel (eluent:ethyl acetate-hexane) to give ethyl 3-chloro-4-pyrazol-1-ylbenzoate (0.474 g).
1H-NMR(CDCl3) δ ppm:
1.43 (3H, t, J=7.1 Hz), 4.42 (2H, q, J=7.1Hz), 6.50-6.60 (1H, m), 7.74 (1H, d, J=8.3Hz), 7.78 (1H, d, J=1.7Hz), 8.00-8.10 (2H, m), 8.21 (1H, d, J=1.7Hz).

### Reference examples 2-2 to 2-6

The following compounds of Reference examples 2-2 to 2-6 were obtained with the use of the corresponding ester derivatives and amine derivatives in a similar manner to that described in Reference example 2-1. The structure formula and physical data of these compounds were shown in Table 1.

**[Table 1]**

| Ref No. | Strc | Physical data |
|---|---|---|
| 2-2 | 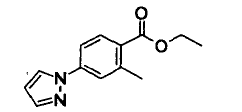 | 1H-NMR (CDCl3) δ ppm 1.41 (3H, t, J=7.2Hz), 2 67 (3H, s), 4.38, (2H, q, J=7.2Hz), 6 45-6 55 (1H, m), 7.50-7.85 (3H, m), 7.95-8 10 (2H, m). |
| 2-3 | 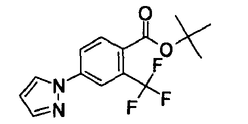 | 1H-NMR (CDCl3) δ ppm. 1 60 (9H,s), 6 54 (1H, d, J=1.6Hz), 7.78 (1H, d, J=1 6Hz), 785-7 95 (2H, m), 8 00 (1H, d, J=2.5Hz), 8.10 (1H, d, J=2 2Hz) |
| 2-4 | 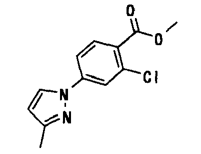 | 1H-NMR (CDCl3) δ ppm 2.38 (3H, s), 3 94 (3H, s), 6 30 (1H, d, J=2.5Hz), 7 60 (1H, dd, J=8 6, 2 2Hz), 7.82 (1H, d, J=2 2Hz), 7.86 (1H, d, J=2.5Hz), 7 97 (1H, d, J=8.6Hz) |
| 2-5 | 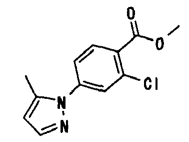 | 1H-NMR (CDCl3) δ ppm. 2 44 (3H, s), 3.96 (3H, s), 6 20-6.30 (1H, m), 7.47 (1H, dd, J=8.5, 2.1Hz), 7.61 (1H, d, J=1 4Hz), 7 66 (1H, d, J=2 1Hz), 7.98 (1H, d, J=8 5Hz) |
| 2-6 | 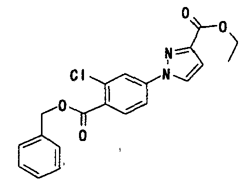 | 1H-NMR (CDCl3) δ ppm. 1 43 (3H, t, J=6 9Hz), 4 46 (2H, q, J=6.9Hz), 5.40 (2H, s), 7.03 (1H, d, J=2.6Hz), 7 30-7.50 (5H, m), 7.71 (1H, dd, J=2.2Hz), 7 90-8.05 (3H, m) |

### Reference example 3

### Ethyl 2-methoxy-4-(4,4,5,5-tetramethyl-1,3,2-dioxaborolan-2-yl)benzoate

A suspension of ethyl 4-chloro-2-methoxybenzoate (1.68 g), bis(pinacolato)diboron(2.98 g), [1,1'-bis(diphenylphosphino)ferrocene]dichloropalladium(II)-dichloromethane adducts (0.192 g), 1,1'-bis(diphenylphosphino)ferrocene (0.130 g), and potassium acetate (3.84 g) in 1,4-dioxane (30 mL) was stirred at 115°C for 66 hours under an argon atmosphere. The reaction mixture was diluted with ethyl acetate. The insoluble was removed by filtration, and the filtrate was concentrated under reduced pressure. To the residue were added water and ethyl acetate, and the insoluble was removed by filtration again. The organic layer of the filtrate was separated and the aqueous layer was extracted with ethyl acetate. The organic layer was collected and the layer was washed with water and brine, dried over anhydrous magnesium sulfate. After filtration, the filtrate was concentrated under reduced pressure. The obtained crude product was purified by column chromatography on silica gel (eluent:ethyl acetate-hexane) to give ethyl 2-methoxy-4-(4,4,5,5-tetramethyl-1,3,2-dioxaborolan-2-yl)benzoate (2.37 g).
1H-NMR(CDCl3) δ ppm:
1.30-1.45 (15H, m), 3.95 (3H, s), 4.36 (2H, q, J=7.1Hz), 7.35-7.45 (2H, m), 7.75 (1H, d, J=7.6Hz).

### Reference example 4

### 4-Ethoxycarbonyl-3-methoxyphenylboronic acid

To a suspension of ethyl 2-methoxy-4-(4,4,5,5-tetramethyl-1,3,2-dioxaborolan-2-yl)benzoate (1.00 g) in tetrahydrofuran (12 mL) -methanol (3.0 mL) was added sodium metaperiodate (2.10 g) at room temperature and this mixture was stirred at room temperature for 4 hours. To the reaction mixture was added sodiummetaperiodate (2.10 g) at room temperature. This mixture was stirred at room temperature for 2 hours, and at 50 °C for 2.5 hours. To the reaction mixture was added water and the mixture was stirred at room temperature overnight. To this mixture was added 2 mol/L hydrochloric acid (1.96 mL) and this suspension was stirred at room temperature for an hour. To this mixture were added water and ethyl acetate and the insoluble was removed by filtration. The organic layer of the filtrate was separated, and the aqueous layer was extracted with ethyl acetate. The organic layer was collected and the layer was washed with water and brine,dried over anhydrous magnesium sulfate. After filtration, the filtrate was concentrated under reduced pressure. The obtained residue was passed through column chromatography on silica gel (eluent:ethyl acetate-hexane) to give 4-ethoxycarbonyl-3-methoxyphenylboronic acid (0.644 g).

### Reference example 5

### Ethyl 2-methoxy-4-pyrazol-1-ylbenzoate

A mixture of 4-ethoxycarbonyl-3-methoxyphenylboronic acid (0.644 g), 1H-pyrazole (0.0978 g), copper (II) acetate (0.391 g), pyridine (0.232 mL) and molecular sieves 4A (0.200 g) was stirred in dichloromethane (6.0 mL) at room temperature for 2 days. The reaction mixture was passed through a Celite pad and the filtrate was concentrated under reduced pressure. The obtained crude product was purified by column chromatography on silica gel (eluent:ethyl acetate-hexane) to give ethyl 2-methoxy-4-pyrazol-1-ylbenzoate (0.381 g).
1H-NMR(CDCl3) δ ppm:
1.40 (3H, t, J=7.1Hz), 4.00 (3H, s), 4.37 (2H, q, J=7.1Hz), 6.50-6.55 (1H, m), 7.19 (1H, dd, J=8.5, 2.0Hz), 7.51 (1H, d, J=2.0Hz), 7.76 (1H, d, J=1.6Hz), 7.93 (1H, d, J=8.5Hz), 7.95-8.05 (1H, m).

### Reference example 6

### Benzyl 4-(2-acetoxyethoxy)-2-chlorobenzoate

2-Bromoethyl acetate (56.9 g) was added dropwise to a stirred suspension of benzyl 2-chloro-4-hydroxybenzoate (81.4 g), cesium carbonate (111g) and sodium iodide (9.29g) in N,N-dimethylformamide (814 mL) under water-cooling, and the reaction mixture was stirred at an internal temperature of 83 ° C for 2 hours 40 minutes. To the reaction mixture were successively added cesium carbonate (10.1 g), sodium iodide (4.65g) and 2-bromoethyl acetate (5.18g) at an internal temperature of 68 ° C and the mixture was stirred at an internal temperature of 83 °C for 45 minutes again. After the reaction mixture was allowed to cool to room temperature, the reaction mixture was poured into water, and the mixture was extracted with ethyl acetate. The aqueous layer was extracted with ethyl acetate again. The organic layer was collected and the layer was washed with water and brine. The organic layer was additionally washed with 1 mol/L hydrochloric acid, water, brine and dried over anhydrous magnesium sulfate. After filtration, the filtrate was concentrated under reduced pressure to give benzyl 4-(2-acetoxyethoxy)-2-chlorobenzoate (115 g).
1H-NMR(CDCl3) δ ppm:
2.10 (3H, s), 4.15-4.25 (2H, m), 4.40-4.45 (2H, m), 5.35 (2H, s), 6.82 (1H, dd, J=8.8, 2.5Hz), 6.99 (1H, d, J=2.5Hz), 7.30-7.50 (5H, m), 7.92 (1H, d, J=8.8Hz).

### Reference example 7-1

### 3-Chloro-4-pyrazol-1-ylbenzoic acid

A solution of ethyl 3-chloro-4-pyrazol-1-yl-benzoate (0.474 g) and 5 mol/L aqueous solution of sodium hydroxide (1.13 mL) in ethanol (5.0 mL) was refluxed for 30 minutes. To the reaction mixture was added 2 mol/L hydrochloric acid (2.84 mL) at room temperature. Ethanol was removed under reduced pressure. The precipitate was collected by filtration and washed with water to give 3-chloro-4-pyrazol-1-ylbenzoic acid (0.420 g).
1H-NMR(DMSO-d6)δ ppm:
6.50-6.60 (1H, m), 7.75 (1H, d, J=8.3Hz), 7.83 (1H, d, J=1.8Hz), 8.02 (1H, dd, J=8.3, 1.8Hz), 8.12 (1H, d, J=1.8Hz), 8.28 (1H, d; J=2.5Hz), 13.45-13.65 (1H, brs).

### Reference examples 7-2 to 7-14

The following compounds of Reference examples 7-2 to 7-14 were obtained with the use of the corresponding ester derivatives in a similar manner to that described in Reference example 7-1. The structure formula and physical data of these compounds were shown in Tables 2 and 3.

**[Table 2]**

| Ref No | Strc | Physical data |
|---|---|---|
| 7-2 | 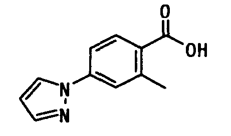 | 1H-NMR (CDCl3) δ ppm : 2.61 (3H, s), 6 55-6 65 (1H, m), 7.70-7:90 (3H, m) 7.97 (1H, d, J=8 5Hz), 8 61 (1H, d, J=2.5Hz), 12 80-13.00 (1H, brs). |
| 7-3 | 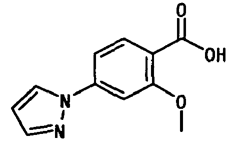 | 1H-NMR(DMSO-d6)δ ppm 3.92 (3H, s), 6.55-6 65 (1H, m), 7 49 (1H, dd, J=2 0, 8.5Hz), 7.57 (1H, d, J=1 9Hz), 7 75-7.85 (2H, m), 8 67 (1H, d, J=2.5Hz), 12 55-12.75 (1H, br s) |
| 7-4 | 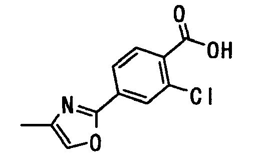 | 1H-NMR (DMSO-d6) δ ppm. 2.19 (3H, s), 7.80-8.10 (4H, m), 12 00-14 50 (1H, br) MS(ESI, m/z): 238(M+H)+ |
| 7-5 | 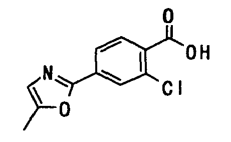 | 1H-NMR (CDCl3) δ ppm: 2.35-2 45 (3H, m), 7 05-7 15 (1H, m), 7 90-8 00 (3H, m), 13 55-13 65 (1H, br) |
| 7-6 | 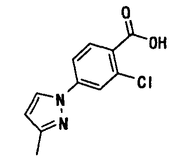 | 1H-NMR (DMSO-d6) δ ppm: 2.28 (3H, s), 6 41 (1H, d, J=2.4Hz), 7 80-7 95 (2H, m), 7.98 (1H, d, J=2 1Hz), 8.55 (1H, d, J=2 4Hz), 13.20-13.60 (1H,br) MS(ESI, m/z) 237(M+H)+ |
| 7-7 | 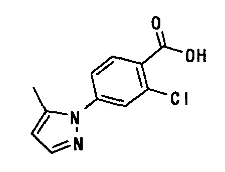 | 1H-NMR (CDCl3) δ ppm 2.46 (3H, s), 6.25-6 30 (1H, m), 7 52 (1H, dd, J=8.5, 2.1Hz), 7.65 (1H, d, J=1.5Hz), 7 71 (1H, d, J=2 1Hz), 8.14 (1H, d, J=8 5Hz) MS(ESI, m/z) :237(M+H)+ |

**[Table 3]**

| Ref No | Strc | Physical data |
|---|---|---|
| 7-8 | 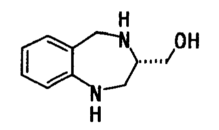 | 1H-NMR (CDCl3) δ ppm 2 70-4.20 (7H, m), 6 60-6.90 (2H, m), 7 00-7 30 (2H, m) |
| 7-9 | 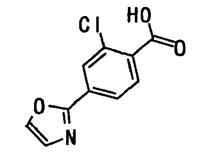 | 1H-NMR (CD3OD) δ ppm: 7.30-7 40 (1H, m), 7.85 (4H, m) |
| 7-10 | 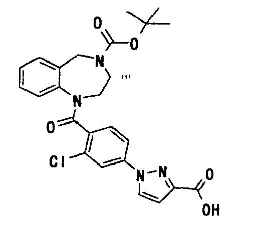 | 1H-NMR (CDCl3) δ ppm 0.75-1 65 (12H, m), 2.85-5.25 (5H, m), 6 45-8 05 (9H, m) |
| 7-11 | 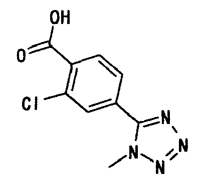 | 1H-NMR (DMSO-d6) δ ppm 4.20 (3H, s), 7.91 (1H, dd, J=8 2, 16Hz), 7 99 (1H, d, J=8.2Hz), 8.30 (1H. d, J=1 6Hz), 13 76 (1H, brs) |
| 7-12 | 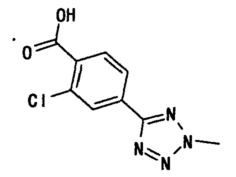 | 1H-NMR (DMSO-d6) δ ppm: 4 46 (3H, s), 7 98 (1H, d, J=8.2Hz), 8.08 (1H, dd, J=8.2, 1 6Hz), 8.12 (1H, d, J=1 6Hz), 13 63 (1H, brs) |
| 7-13 | 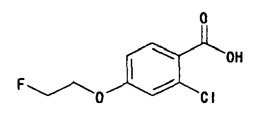 | 1H-NMR (DMSO-d6) δ ppm: 4.20-4.30 (2H, m), 4.65-4 85 (2H, m), 6 95-7.05 (1H, m), 7 10-7 20 (1H, m), 7 80-7 90 (1H, m), 13 04 (1H, brs) |
| 7-14 | 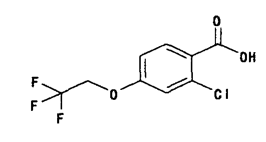 | 1H-NMR (DMSO-d6) δ ppm 4 90 (2H, q, J=8.8Hz), 7.12 (1H, dd, J=8 7, 2.7Hz), 7.28 (1H, d, J=2 8Hz), 7 85 (1H, d, J=8.5Hz), 13 14 (1H, s) |

### Reference example 8

### 4-Pyrazol-1-yl-2-trifluoromethylbenzoic aid

A solution of tert-butyl 4-pyrazol-1-yl-2-trifluoromethylbenzoate (0.651g) indichloromethane (4.0mL)-trifluoroacetic acid (4.0mL) was stirred at room temperature for 6 hours. Thereaction solution was concentrated under reduced pressure to give 4-pyrazol-1-yl-2-trifluoromethylbenzoic acid (0.525 g).
1H-NMR(DMSO-d6) δ ppm:
6.60-6.70 (1H, m), 7.85-7.90 (1H, m), 8.01 (1H, d, J=8.5Hz), 8.20-8.35 (2H, m), 8.78 (1H, d, J=2.4Hz), 13.50-13.85 (1H, brs).

### Reference example 9

### 4-(2-Acetoxyethoxy)-2-chlorobenzoic acid

To a solution of benzyl 4-(2-acetoxyethoxy)-2-chlorobenzoate (80 g) in tetrahydrofuran (960 mL) was added 10% palladium-carbon (7.32 g) under an argon gas atmosphere, while ice-cooling, and the suspension was stirred at room temperature for 8.5 hours under a hydrogen gas atmosphere. To the reaction mixture was added 10% palladium-carbon (4.88 g) under an argon atmosphere, and the suspension was stirred at room temperature for 1.5 hours under a hydrogen gas atmosphere. The catalyst was removed by passsing through a Celite pad, and the solvent was removed under reduced pressure to give 4-(2-acetoxyethoxy)-2-chlorobenzoic acid (47.6 g).
1H-NMR(DMSO-d6) δ ppm:
2.04 (3H, s), 4.20-4.40 (4H, m), 7.01 (1H, dd, J=8.8, 2.5Hz), 7.13 (1H, d, J=2.5Hz), 7.83 (1H, d, J=8.8Hz), 13.04 (1H, brs).

### Reference example 10-1

### 2-Chloro-4-pyrazol-1-ylbenzoyl chloride

To a stirred solution of thionyl chloride (0.832 mL) and catalytic amount of N,N-dimethylformamide in dichloromethane (6.8 mL) was added 2-chloro-4-pyrazol-1-ylbenzoic acid (0.508 g) under ice-cooling, and this mixture was stirred at 40 °C for 3 hours. The reaction solution was concentrated under reduced pressure to give 2-chloro-4-pyrazol-1-ylbenzoyl chloride (0.550 g).
1H-NMR (CDCl3) δ ppm:
6.55-6.60 (1H, m), 7.70-7.75 (1H, m), 7.75-7.85 (1H, m), 7.90-7.95 (1H, m), 8.00-8.05 (1H, m), 8.20-8.30 (1H, m).

### Reference example 10-2

### 3-Chloro-4-pyrazol-1-ylbenzoyl chloride

To a suspension of 3-chloro-4-pyrazol-1-ylbenzoic acid (0.0552 g) in dichloromethane (0.70 mL) were added thionyl chloride (0.0900 mL) and a catalytic amount of N-methylpyrrolidone at room temperature, and this mixture was refluxed at 40 °C for 15 hours.
The reaction solution was concentrated under reduced pressure to give 3-chloro-4-pyrazol-1-ylbenzoyl chloride (0.0597 g).

### Reference example 10-3

### 2-Methyl-4-pyrazol-1-ylbenzoyl chloride

To a suspension of 2-methyl-4-pyrazol-1-ylbenzoic acid (0.111 g) in dichloromethane (1.0 mL) were added thionyl chloride (0.200 mL) and a catalytic amount of N-methylpyrrolidone at room temperature, and this suspension was refluxed at 40 °C for an hour. The reaction solution was concentrated under reduced pressure to give 2-methyl-4-pyrazol-1-ylbenzoyl chloride (0.121 g).

### Reference example 10-4

### 2-Methoxy-4-pyrazol-1-ylbenzoyl chloride

To a suspension of 2-methoxy-4-pyrazol-1-ylbenzoic acid (0.0600 g) in dichloromethane (1.0 mL) were added thionyl chloride (0.100 mL) and a catalytic amount of N-methylpyrrolidone at room temperature, and this suspension was refluxed at 40 °C for 1.5 hours.
The reaction solution was concentrated under reduced pressure to give 2-methoxy-4-pyrazol-1-ylbenzoyl chloride (0.0649 g).

### Reference example 10-5

### 3-Chlorobiphenyl-4-carbonyl chloride

To a suspension of 3-chlorobiphenyl-4-carboxylic acid (0.0787 g) in dichloromethane (1.0 mL) were added thionyl chloride (0.120 mL) and a catalytic amount of N-methylpyrrolidone at room temperature, and this suspension was stirred at room temperature for 1.5 hours. The reaction solution was concentrated under reduced pressure to give 3-chlorobiphenyl-4-carbonyl chloride (0.0849 g).

### Reference example 10-6

### 2-Chloro-4-pyrrolidin-1-ylbenzoyl chloride

To a suspension of 2-chloro-4-pyrolidin-1-ylbenzoic acid (0.0733 g) in dichloromethane (1.0 mL) were added thionyl chloride (0.236 mL) and a catalytic amount of N-methylpyrrolidone at room temperature, and this suspension was stirred at room temperature for 4.5 hours. The reaction solution was concentrated under reduced pressure to give 2-chloro-4-pyrrolidin-1-ylbenzoyl chloride (0.0793 g).

### Reference example 10-7

### Ethyl 2-(3-chloro-4-chlorocarbonylphenoxy)acetate

Ethyl 2-(3-chloro-4-chlorocarbonylphenoxy)acetate was obtained with the use of the corresponding 4-substituted benzoic acid derivative in a similar manner to that described in reference example 10-1.
1H-NMR(CDC13) δ ppm:
2.11 (3H, s), 4.20-4.30 (2H, m), 4.40-4.50 (2H, m), 6.91 (1H, dd, J=9.0, 2.5Hz), 7.03 (1H, d, J=2.5Hz), 8.20 (1H, d, J=9.0Hz).

### Reference example 11

### 2-Chloro-N-(2-formylphenyl)-4-pyrrolidin-1-ylbenzamide

To a solution of 2-chloro-4-pyrrolidin-1-ylbenzoic acid (0.473 g) in thionyl chloride (6.0 mL) was added a catalytic amount of N-methylpyrrolidone, and this solution was stirred at room temperature for 1.5 hours. The reaction solution was concentrated under reduced pressure. This residue was dissolved in dichloromethane (15 mL) (solution A). To a solution of 2-aminobenzaldehyde (0.266 g) and triethylamine (1.17 mL) in dichloromethane (15 mL) was added solution A under ice-cooling and this solution was stirred at room temperature for 66 hours. To the reaction solution was added water and the organic layer was separated. The extracted solution was concentrated under reduced pressure. The obtained crude product was purifiedby column chromatography on silica gel (eluent:ethyl acetate-hexane) to give 2-chloro-N-(2-formylphenyl)-4-pyrrolidin-1-ylbenzamide (0.276 g).
1H-NMR(CDCl3)δ ppm:
2.00-2.10 (4H, m), 3.25-3.40 (4H, m), 6.48 (1H, dd, J=8.8H, 2.4Hz), 6.57 (1H, d, J=8.4Hz), 7.20-7.30 (1H, m), 7.60-7.75 (3H, m), 8.92 (1H, d, J=8.5Hz), 9.94 (1H, s), 11.59 (1H, s).

### Reference example 12

### N-(2-Hydroxymethylphenyl)-2-nitrobenzenesulfonamide

To a solution of 2-aminophenylmethanol (3.00 g) and pyridine (3.94mL) indichloromethane (20mL) was added2-nitrobenzenesulfonyl chloride (5.67 g) under ice-cooling and this solution was stirred at room temperature overnight. To the reaction mixture were added 2 mol/L hydrochloric acid and water, and the mixture was stirred for a few minutes. The insoluble was removed by filtration and to the filtrate was added ethyl acetate. The organic layer was separated and the layer was washed with brine, dried over anhydrous magnesium sulfate. After filtration, the filtrate was removed under reduced pressure. The obtained crude product was purified by column chromatography on silica gel (eluent:ethyl acetate-hexane) to give N-(2-hydroxymethylphenyl)-2-nitrobenzenesulfonamide (6.78 g).
1H-NMR(CDCl3)δ ppm:
2.20-2.30 (1H, br), 4.60-4.65 (2H, m), 7.15-7.30 (3H, m), 7.40-7.45 (1H, m), 7.55-7.65 (1H, m), 7.65-7.75 (1H, m), 7.85-7.90 (2H, m), 8.30-8.45 (1H, brs).

### Reference example 13

### N-(2-Formylphenyl)-2-nitrobenzenesulfonamide

To a solution of N-(2-hydroxymethylphenyl)-2-nitrobenzenesulfonamide (6.78 g) in dichloromethane (40 mL) was added activatedmanganese oxide (IV) (13.4 g) and the suspension was stirred at room temperature for 13 hours. To the reaction mixture was added activatedmanganeseoxide (IV) (5.74 g) and this suspension was stirred at room temperature for 4.5 hours. To the reaction mixture was added dichloromethane and the precipitate was removed by filtration. The filtrate was concentrated under reduced pressure. To the residue was added diethyl ether, and this suspension was stirred at room temperature for a few minutes. The precipitate was collected by filtration to give N-(2-formylphenyl)-2-nitrobenzenesulfonamide (3.42 g).
1H-NMR(CDCl3)δ ppm:
7.20-7.30 (1H, m), 7.50-7.60 (1H, m), 7.65-7.80 (3H, m), 7.80-7.90 (2H, m), 8.15-8.25 (1H, m), 9.91 (1H, s), 11.30-11.45 (1H, brs).

### Reference example 14-1

### 2-Chloro-N-{2-[((R)-2-hydroxy-1-methylethylimino)methyl]-phenyl}-4-pyrrolidin-1-ylbenzamide

To a suspension of 2-chloro-N-(2-formylphenyl)-4-pyrrolidin-1-ylbenzamide (0.450 g) and potassium carbonate (0.195 g) in ethanol (10 mL) was added D-alaninol (0.109 mL) at room temperature and the mixture was refluxed for an hour. After the reaction mixture was allowed to cool to room temperature, the raction mixture was filtered. To the filtrate was added water, and the mixture was extracted with ethyl acetate. The organic layer was washed with water and brine, dried over anhydrous magnesium sulfate. After filtration, the filtrate was concentrated under reduced pressure. The obtained crude product was purified by column chromatography on silica gel (eluent:ethyl acetate-methanol) to give 2-chloro-N-{2-[((R)-2-hydroxy-1-methylethylimino)methyl]phenyl}-4-pyrrolidin-1-ylbenzamide (0.508 g).
1H-NMR(CDCl3) δ ppm:
1.19 (3H, d, J=6.4Hz), 1.45-1.50 (1H, m), 2.00-2.10 (4H, m), 3.30-3.40 (4H, m), 3.40-3.50 (1H, m), 3.50-3.70 (2H, m), 6.44 (1H, dd, J=8.7, 2.4Hz), 6.57 (1H, d, J=2.4Hz), 7.10-7.15 (1H, m), 7.36 (1H, dd, J=7.7, 1.5Hz), 7.40-7.50 (1H, m), 7.57 (1H, d, J=8.7Hz), 8.42 (1H, s), 8.89 (1H, d, J=8.4Hz), 12.9-13.0 (1H, brs).
MS(ESI, m/z) : 386(M+H)+

### Reference example 14-2

### N-{2-[((R)-2-Hydroxy-1-methylethylimino)methyl]phenyl}-2-nitrobenzenesulfonamide

To a suspension of N-(2-formylphenyl)-2-nitrobenzenesulfonamide (2.43 g) and potassium carbonate (1.13 g) in ethanol (50 mL) was added D-alaninol (0.631 mL) at an external temperature of 90 °C and this solution was refluxed for 16 hours. After the reaction mixture was allowed to cool to room temperature, the reaction mixture was filtered. The filtrate was used for the next reaction as a solution of N-{2-[((R)-2-hydroxy-1-methylethylimino)methyl]phenyl}-2-nitrobenzenesulfonamide in ethanol.

### Reference example 14-3

### 2-Chloro-N-{2-[(2-hydroxyethylimino)methyl]phenyl}-4-pyrrolidin-1-ylbenzamide

A mixture of 2-chloro-N-(2-formylphenyl)-4-pyrrolidin-1-yl-benzamide (169 mg), 2-aminoethanol(31.0 uL), potassium carbonate (73.3 mg) in ethanol (3.0mL)was refluxed at an external temperature of 95 ° C for 30 minutes. After the reaction mixture was cooled to room temperature, the reaction mixture was filtered. The filtrate was used for the next reaction as a solution of 2-chloro-N-{2-[(2-hydroxyethylimino)methyl]phenyl}-4-pyrrolidin-1-ylbenzamide in ethanol.

### Reference example 14-4

### N-{2-[((R)-2-Hydroxy-1-methylethylimino)methyl]phenyl}-4-nitrobenzenesulfonamide

To a suspension of N-(2-formylphenyl)-4-nitorbenzenesulfonamide (700 mg) and potassium carbonate (325 mg) in ethanol (5.0 mL) was added D-alaninol (0.182 mL) at room temperature, and this mixture was refluxed at an external temperature of 90 °C for 5 hours. After the reaction mixture was allowed to cool to room temperature, the reaction mixture was filtered. The filtrate was used for the next reaction as a solution of N-{2-[((R)-2-hydroxy-1-methylethylimino)methyl]phenyl}-4-nitrobenzenesulfonamide in ethanol.

### Reference example 15-1

### 2-Chloro-N-{2-[((R)-2-hydroxy-1-methylethylamino)methyl]-phenyl}-4-pyrrolidin-1-ylbenzamide

To a solution of 2-chloro-N-{2-[((R)-2-hydroxy-1-methylethylimino)methyl]phenyl}-4-pyrrolidin-1-ylbenzamide(0.50 8 g) in ethanol (10 mL) was added sodium tetrahydroborate (0.0598 g), and this mixture was refluxed for an hour. To the reaction mixture was added ethanol (5.0 mL) and the mixture was refluxed for an hour. After the reaction mixture was cooled to room temperature, sodium tetrahydroborate (0.0598 g) was added to the mixture, and this mixture was stirred at room temperature overnight. The reaction mixture was concentrated under reduced pressure. To this residue was added water, and the mixture was extracted with ethyl acetate. The organic layer was washed with water and brine, dried over anhydrous magnesium sulfate. After filtration, the filtrate was concentrated under reduced pressure. The obtained crude product was purified by column chromatography on silica gel (eluent: ethyl acetate-methanol) to give 2-chloro-N-{2-[((R)-2-hydroxy-1-methylethylamino)methyl]-phenyl}-4-pyrrolidin-1-ylbenzamide (0.333 g).
1H-NMR(CDCl₃) δ ppm:
1.01 (3H, d, J=6.4Hz), 2.00-2.05 (4H, m), 2.75-2.85 (1H, m), 3.25-3.35 (4H, m), 3.35-3.60 (2H, m), 4.00 (2H, m), 6.44 (1H, dd, J=8.7, 2.4Hz), 6.52 (1H, d, J=2.4Hz), 6.95-7.05 (1H, m), 7.10-7.20 (1H, m), 7.25-7.35 (1H, m), 7.61 (1H, d, J=8.7Hz), 8.25-8.35 (1H, m), 10.60-10.70 (1H, brs).
MS(ESI, m/z) : 388(M+H)+

### Reference examples 15-2 to 15-4

The following compounds of Reference examples 15-2 to 15-4 were obtained with the use of the corresponding 2-benzylideneaminoethanol derivatives in a similar manner to that described in Reference example 15-1. The structure formula and physical data of these compounds were shown in Table 4.

**[Table 4]**

| Ref No | Strc | Physical data |
|---|---|---|
| 15-2 | 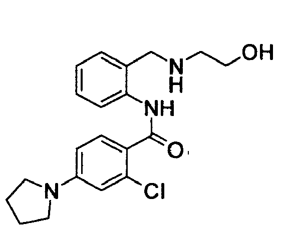 | 1H-NMR (CDCl3) δ ppm 2 00-2.10 (4H, m), 2 75-2 80 (2H, m), 3.25-3 35 (4H, m), 3 65-3 75 (2H, m), 3 90 (2H, s), 6 46 (1H, dd, J=8.7, 2 4Hz), 6.52 (1H, d, J=2.4Hz), 7.00-7 05 (1H, m), 7 10-7 20 (1H, m), 7.25-7 40 (1H, m), 7.64 (1H, d, J=8 7Hz), 8 25-8 40 (1H, m), 10 65-10 75 (1H, brs). MS(ESI, m/z): 374(M+H)+ |
| 15-3 | 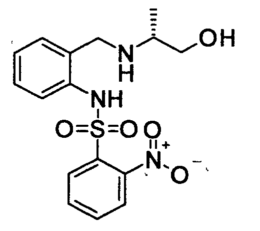 | 1H-NMR (CDCl3) δ ppm : 1.16(3H, d, J=6 5Hz), 2 90-3 05 (1H, m), 3 55-3.80 (2H, m), 3 80-4.10 (2H, m), 6 85-6 95 (1H, m); 7.00-7.10 (1H, m), 7 10-7 20 (1H, m), 7 25-7.30 (1H, m), 7.55-7.70 (3H, m), 8.05-8 15 (1H, m). MS(ESI, m/z): 366(M+H)+ |
| 15-4 | 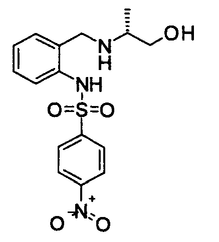 | 1H-NMR (DMSO-d6) δ ppm 1 21(3H, d, J=6 6Hz), 3.05-3.15 (1H, m), 3 40-3.70 (2H, m), 3 95-4 15 (2H, m), 5.20-550 (1H. br), 6 55-6.65 (1H. m), 6 95-7 15 (3H, m), 7.95-8 05 (2H, m), 820-830 (2H, m), 8 80-9 50 (2H, br). MS(ESI, m/z): 366(M+H)+ |

### Reference example 16

### N-(2-{[(R)-2-(tert-Butyldimethylsilanyloxy)-1-methylethylamino]methyl}phenyl)-2-chloro-4-pyrrolidin-1-ylbenzamide

To a solution of 2-chloro-N-(2-{[(R)-2-hydroxy-1-methylethylamino]methyl}phenyl)-4-pyrrolidin-1-ylbenzamide (10.6 g) and 1H-imidazole (2.80 g) in N,N-dimethylformamide (150 mL) was added tert-butyldimethylchlorosilane (4.96 g) at room temperature, and this mixture was stirred at room temperature overnight. To the reaction solution was added water and the mixture was extracted with ethyl acetate. The organic layer was washed with brine, dried over anhydrous magnesium sulfate. After filtration, the filtrate was concentrated under reduced pressure. The obtained crude product was purified by column chromatography on silica gel (eluent:ethyl acetate-hexane) to give N-(2-{[(R)-2-(tert-butyldimethylsilanyloxy)-1-methylethylamino]methyl}phenyl)-2-chloro-4-pyrrolidin-1-ylbenzamide (13.0 g).
1H-NMR(CDCl3) δ ppm:
-0.05-0.05 (6H, m), 0.85 (9H, s), 0.94 (3H, d, J=6.3Hz), 2.00-2.10 (4H, m), 2.65-2.90 (1H, m), 3.25-3.60 (6H, m), 3.75-3.95 (2H, m), 6.43 (1H, dd, J=8.7, 2.3Hz), 6.52 (1H, d, J=2.3Hz), 6.95-7.05 (1H, m), 7.10-7.20 (1H, m), 7.25-7.35 (1H, m), 7.58 (1H, d, J=8.7Hz), 8.30-8.45 (1H, m), 11.00-11.20 (1H, brs).

### Reference example 17-1

### tert-Butyl 2-(2-chloro-4-pyrrolidin-1-ylbenzoylamino)benzyl-((R)-2-hydroxy-1-methylethyl)carbamate

To a solution of 2-chloro-N-{2-[((R)-2-hydroxy-1-methylethylamino)methyl]phenyl}-4-pyrrolidin-1-ylbenzamide (0.333g) in dichloromethane (10 mL) was added di-tert-butyl dicarbonate (0.197 g) at room temperature, and this solution was stirred at room temperature for 9 hours. To the reaction solution was added di-tert-butyl dicarbonate (0.0562 g), and this solution was stirred at room temperature for 12 hours. The reaction solution was concentrated under reduced pressure. The obtained residue was purified by column chromatography on silica gel (eluent:ethyl acetate-hexane) to give tert-butyl 2-(2-chloro-4-pyrrolidin-1-yl-benzoylamino)benzyl((R)-2-hydroxy-1-methylethyl)carbamate (0.298 g).
1H-NMR(CDC13) δ ppm:
1.12 (3H, d, J=7.0Hz), 1.32 (9H, s), 2.00-2.10 (4H, m), 3.25-3.35 (4H, m), 3.50-3.70 (2H, m), 3.75-3.90 (1H, m), 4.40-4.55 (2H, m), 6.47 (1H, dd, J=8.7, 2.3Hz), 6.52 (1H, d, J=2.3Hz), 7.10-7,20 (1H, m), 7.25-7.35 (2H, m), 7.72 (1H, d, J=8.7Hz), 7.80-8.10 (1H, m), 8.40-9.50 (1H, br).
MS(ESI, m/z) : 488 (M+H) +

### Reference examples 17-2 to 17-7

The following compounds of Reference examples 17-2 to 17-7 were obtained with the use of the corresponding amine derivatives in a similar manner to that described in Reference example 17-1. The structure formula and physical data of these compounds were shown in Table 5.

**[Table 5]**

| Ref No. | Strc | Physical data |
|---|---|---|
| 17-2 | 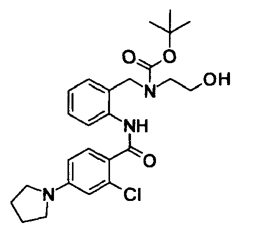 | 1H-NMR (CDCl3) δ ppm. 1.34 (9H, s), 2 00-2 10 (4H, m), 3 25-3.40 (6H, m), 3.60-3.70 (2H, m), 4 55 (2H, s), 6.40-6 50 (1H, m), 6 53 (1H, d, J=2 3Hz), 7.10-7.20 (1H, m), 7 20-7.30 (1H, m), 7.30-7 40 (1H, m), 7.60-7 75 (1H, m), 7 80-8.40 (1H, m), 9 00-9 60 (1H, br) |
| 17-3 | 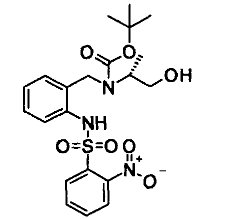 | 1H-NMR (CDCl3) δ ppm : 1.10 (3H, d, J=7.0Hz), 1.44 (9H, s), 3.50-3.70 (2H, m), 3 80-4 00 (1H, m), 4.48 (2H, s), 6 90-7.25 (3H, m), 7.30-7 40 (1H, m), 7.55-7 95 (4H, m). MS(ESI, m/z) 466(M+H)+ |
| 17-4 | 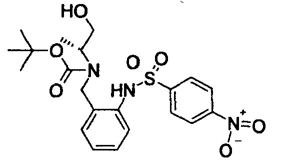 | 1H-NMR (CDCl3) δ ppm : 1.05 (3H, d, J=6.7Hz), 1.52 (9H, s), 3.45-3 75 (3H, m), 4 00-4.25 (2H, m), 7 05-7.20 (2H, m), 7.25-7 30 (1H, m), 7 55-7 60 (1H, m), 7 95-8.05 (2H, m), 8 20-8 30 (2H, m), 940-1040(1H, br) MS(ESI, m/z) 466(M+H)+ |
| 17-5 | 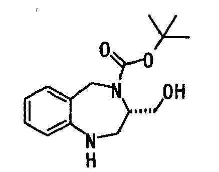 | 1H-NMR (CDCl3) δ ppm: 1.30-1 50 (9H, m), 2 30-4 80 (7H, m), 6.55-6 80 (2H, m), 6 95-715 (2H, m) |
| 17-6 | 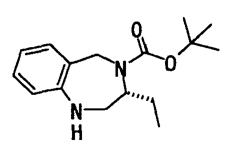 | 1H-NMR (CDCl3) δ ppm: 0 96 (3H, t, J=7 6Hz), 1 50-1 55 (11H, m), 3.15-3 40 (2H, m), 3 75-3.85 (1H, m), 4.25-4.45 (2H, m), 6 56 (1H, d, J=7 9Hz), 6 65-6 80 (1H, m), 6 95-7.15 (2H, m) |
| 17-7 | 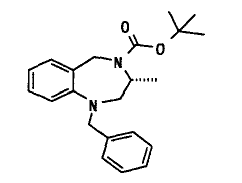 | 1H-NMR (CDCl3) δ ppm: 0 95-1 70 (12H, m), 2.95-3 10 (1H, m), 3 35-3 55 (1H. m), 4 25-4 85 (5H, m), 6 65-6.80 (2H, m), 6 95-7 40 (7H, m) |

### Reference example 18-1

### 2-Chloro-N-(2-{[(R)-2-hydroxy-1-methylethyl(4-nitrobenzenesulfonyl)amino]methyl}phenyl)-4-pyrrolidin-1-ylbenzamide

To a solution of 2-chloro-N-(2-{[(R)-2-hydroxy-1-methylethylamino]methyl}phenyl)-4-pyrrolidin-1-ylbenzamide (0.177 g) and pyridine (0.0340 mL) in dichloromethane (3.0 mL) was added 4-nitrobenzenesulfonyl chloride (0.845 g) under ice-cooling, and the mixture was stirred at room temperature for 13 hours. The reaction mixture was concentrated under reduced pressure. The obtained residue was purified by column chromatography on silica gel (eluent:ethyl acetate-hexane) to give 2-chloro-N-(2-{[(R)-2-hydroxy-1-methylethyl(4-nitrobenzenesulfonyl)amino]methyl}pheny 1)-4-pyrrolidin-1-ylbenzamide (0.0645 g).
1H-NMR (CDCl3) δ ppm:
1.04 (3H, d, J=6.9Hz), 1.70-2.10 (4H, m), 3.25-3.55 (6H, m), 4.10-4.20 (1H, m), 4.30-4.60 (2H, m), 6.45-6.55 (2H, m), 7.10-7.20 (1H, m), 7.25-7.75 (4H, m), 7.86 (2H, d, J=8.4Hz), 8.22 (2H, d, J=8.4Hz), 8.45-8.55 (1H, brs).
MS(ESI, m/z) : 573(M+H)+

### Reference example 18-2

### N-[2-({[(R)-2-(tert-Butyldimethylsilanyloxy)-1-methylethyl]-(4-nitrobenzenesulfonyl)amino}methyl)phenyl]-2-chloro-4-pyrrolidin-1-ylbenzamide

N-[2-({[(R)-2-(tert-Butyldimethylsilanyloxy)-1-methylethyl]-(4-nitrobenzenesulfonyl)amino}methyl)phenyl]-2-chloro-4-pyrrolidin-1-ylbenzamide was obtained with the use of the corresponding N-benzylaminoethanol derivative and nosyl chloride in a similar manner to that described in Reference example 18-1.
1H-NMR(CDCl3)δ ppm: -0.10-0.00 (6H, m), 0.78 (9H, s), 1.09 (3H, d, J=7.0Hz), 2.00-2.10 (4H, m), 3.25-3.65 (7H, m), 4.45-4.60 (2H, m), 6.49 (1H, dd, J=8.8, 2.2Hz), 6.54 (1H, d, J=2.4Hz), 7.00-7.10 (1H, m), 7.25-7.35_{:} (2H, m), 7.70-7.85 (4H, m), 8.15-8.25 (2H, m), 8.60-8.65 (1H, brs).
MS(ESI, m/z) : 687(M+H)+

### Reference example 18-3

### (R)-2-{[2-(2-Chloro-4-pyrrolidin-1-ylbenzoylamino)benzyl]-(4-nitrobenzenesulfonyl)amino}propyl 4-nitrobenzenesulfonate

(R)-2-{[2-(2-Chloro-4-pyrrolidin-1-ylbenzoylamino)-benzyl]-(4-nitrobenzenesulfonyl)amino}propyl 4-nitrobenzenesulfonate was obtained with the use of the corresponding N-benzylaminoethanol derivative and nosyl chloride in a similar manner to that described in Reference example 18-1.
1H-NMR(CDCl3) δ ppm:
1.00-1.35 (3H, m), 2.00-2.10 (4H, m), 2.95-4.60 (9H, m), 6.40-8.50 (15H, m).

### Reference example 19

### 2-Chloro-N-(2-{[(R)-2-hydroxy-1-methylethyl(4-nitrobenzenesulfonyl)amino]methyl}phenyl)-4-pyrrolidin-1-ylbenzamide

To a solution of N-(2-{[(R)-2-(tert-butyldimethylsilanyloxy)-1-methylethyl(4-nitrobenzenesulfonyl)amino]methyl}-phenyl)-2-chloro-4-pyrrolidin-1-ylbenzamide (0.270 mg) in tetrahydrofuran (5.0 mL) was added tetra-n-butylammonium fluoride (1mol/L solution in tetrahydrofuran) (0.410 mL) at room temperature, and this solution was stirred at room temperature for 30 minutes. To the reaction solution was added water, and the mixture was extracted with ethyl acetate. The organic layer was washed with brine, dried over anhydrous magnesium sulfate. After filtration, the filtrate was concentrated under reduced pressure. The obtained residue was purified by column chromatography on aminopropylsilylated silica gel (eluent:ethyl acetate-hexane) to give 2-chloro-N-(2-{[(R)-2-hydroxy-1-methylethyl(4-nitrobenzenesulfonyl)amino]methyl}-phenyl)-4-pyrrolidin-1-ylbenzamide (0.132 g).
1H-NMR(CDCl3)δ ppm:
1.04 (3H, d, J=6.9Hz), 1.70-2.10 (5H, m), 3.25-3.55 (6H, m), 4.10-4.20 (1H, m), 4.30-4.60 (2H, m), 6.45-6.55 (2H, m), 7.10-7.20 (1H, m), 7.25-7.75 (4H, m), 7.86 (2H, d, J=8.4Hz), 8.22 (2H, d, J=8.4Hz), 8.45-8.55 (1H, brs).
MS (ESI, m/z) : 573(M+H)+

### Reference example 20-1

### (R)-2-{tert-Butoxycarbonyl[2-(2-chloro-4-pyrrolidin-1-yl-benzoylamino)benzyl]amino}propyl methanesulfonate

To a solution of tert-butyl 2-(2-chloro-4-pyrrolidin-1-ylbenzoylamino)benzyl[(R)-2-hydroxy-1-methylethyl]carbamate (0.280 g) and triethylamine (0.120 mL) in tetrahydrofuran (6.0 mL) was added methanesulfonyl chloride (0.0530 mL) under ice-cooling, and this reaction mixture was stirred at 0 °C for 25 minutes, at room temperature for 20 minutes, and at 45 °C for 30 minutes. To the reaction mixture was added water, and the mixture was extracted with ethyl acetate. The organic layer was washed with water and brine, dried over anhydrous magnesium sulfate. After filtration, the filtrate was concentrated under reduced pressure. The obtained residue was purified by column chromatography on silica gel (eluent:ethyl acetate-hexane) to give (R)-2-{tert-butoxycarbonyl[2-(2-chloro-4-pyrrolidin-1-ylbenzoylamino)benzyl]-amino}propyl methanesulfonate (0.160 g).
1H-NMR(CDCl3)δ ppm:
1.19 (3H, d, J=7.0Hz), 1.32 (9H, s), 2.00-2.10 (4H, m), 2.82 (3H, s), 3.30-3.40 (4H, m), 3.70-4.80 (5H, m), 6.50-6.65 (2H, m), 7.10-7.40 (3H, m), 7.50-9.50 (3H, m).
MS(ESI, m/z) : 566(M+H)+

### Reference example 20-2

### 2-{tert-Butoxycarbonyl-[2-(2-chloro-4-pyrrolidin-1-ylbenzoylamino)benzyl]amino}ethyl methanesulfonate

2-{tert-Butoxycarbonyl-[2-(2-chloro-4-pyrrolidin-1-ylbenzoylamino)benzyl]amino}ethyl methanesulfonate was obtained with the use of the corresponding 2-benzylaminoethanol derivative and methanesulfonyl chloride in a similar manner to that described in Reference example 20-1.
1H-NMR(CDCl3) δ ppm:
1.33 (9H, s), 2.00-2.10 (4H, m), 2.91 (3H, s), 3.25-3.35 (4H, m), 3.50 (2H, t, J=5.6Hz), 4.10-4.25 (2H, m), 4.54 (2H, s), 6.40-6.50 (1H, m), 6.53 (1H, d, J=2.3Hz), 7.05-7.20 (1H, m), 7.20-7.25 (1H, m), 7.30-7.40 (1H, m), 7.55-7.75 (1H, m), 8.10-8.45 (1H, m 9.10-9.50 (1H, br).
MS(ESI, m/z) : 552 (M+H)+

### Reference example 20-3

### (R)-2-{[2-(2-Chloro-4-pyrrolidin-1-ylbenzoylamino)benzyl]-(4-nitrobenzenesulfonyl)amino}propyl methanesulfonate

(R)-2-{[2-(2-Chloro-4-pyrrolidin-1-ylbenzoylamino)-benzyl]-(4-nitrobenzenesulfonyl)amino}propyl methanesulfonate was obtained with the use of the corresponding 2-benzylaminoethanol derivative and methanesulfonyl chloride in a similar manner to that described in Reference example 20-1.
1H-NMR(CDCl3) δ ppm:
1.10 (3H, d, J=7.0Hz), 2.00-2.10 (4H, m), 2.79 (3H, s), 3.25-3.40 (4H, m), 3.90-4.00 (1H, m), 4.05-4.15 (1H, m), 4.25-4.40 (1H, m), 4.40-4.60 (2H, m), 6.49 (1H, dd, J=8.8, 2. 4Hz), 6.53 (1H, d, J=2.4Hz), 7.15-7.25 (1H, m), 7.30-7.45 (2H, m), 7.60-7.70 (1H, m), 7.76 (1H, d, J=8.8Hz), 7.90-8.00 (2H, m), 8.25-8.35 (2H, m), 8.40-8.50 (1H, brs).
MS(ESI, m/z) : 651(M+H)+

### Reference example 21-1

### tert-Butyl (R)-1-(2-chloro-4-pyrrolidin-1-ylbenzoyl)-3-methyl-1,2,3,5-tetrahydrobenzo[e]-1,4-diazepin-4-carboxylate

To a solution of (R)-{tert-butoxycarbonyl[2-(2-chloro-4-pyrrolidin-1-ylbenzoylamino)benzyl]amino}propyl methanesulfonate (0.160 g) in tetrahydrofuran (5.0 mL) was added sodium hydride (dispersion in oil ca 60% : 0.0203 g) under ice-cooling, and this mixture was stirred at room temperature for 60 hours. The reaction mixture was poured into water, and the mixture was extracted with ethyl acetate. The organic layer was washed with brine, dried over anhydrous magnesium sulfate. After filtration, the filtrate was concentrated under reduced pressure. The obtained crude product was purified by column chromatography on silica gel (eluent:ethyl acetate-hexane) to give tert-butyl (R)-1-(2-chloro-4-pyrrolidin-1-ylbenzoyl)-3-methyl-1,2,3,5-tetrahydrobenzo[e]-1,4-diazepin-4-carboxylate (0.0922 g).
1H-NMR(CDCl3) δ ppm:
1.00-1.60 (12H, m), 1.90-2.00 (4H, m), 2.70-3.50 (5H, m), 4.10-5.40 (4H, m), 6.00-8.00 (7H, m).
MS(ESI, m/z) : 470 (M+H) +

### Reference example 21-2

### tert-Butyl 1-(2-chloro-4-pyrrolidin-1-ylbenzoyl)-1,2,3,5-tetrahydrobenzo[e]-1,4-diazepin-4-carboxylate

tert-Butyl 1-(2-chloro-4-pyrrolidin-1-ylbenzoyl)-1,2,3,5-tetrahydrobenzo[e]-1,4-diazepin-4-carboxylate was obtained with the use of the corresponding 2-(2-benzoylamino-benzylamino) ethyl methanesulfonate derivative in a similar manner to that described in Reference example 21-1.
1H-NMR(CDCl3) δ ppm:
1.42 (9H, s), 1.90-2.00 (4H, m), 2.70-5.20 (10H, m), 6.05-7.65 (7H, m).
MS(ESI, m/z) : 456(M+H)+

### Reference example 21-3

### (2-Chloro-4-pyrrolidin-1-ylphenyl)-[(R)-3-methyl-4-(4-nitrobenzenesulfonyl)-2,3,4,5-tetrahydrobenzo[e]-1,4-diazepin-1-yl]-methanone

(2-Chloro-4-pyrrolidin-1-ylphenyl)-[(R)-3-methyl-4-(4-nitrobenzenesulfonyl)-2,3,4,5-tetrahydrobenzo[e]-1,4-diazepin-1-yl]methanone was obtained with the use of the corresponding 2-(2-benzoylaminobenzylamino)ethyl methanesulfonate derivative in a similar manner to that described in Reference example 21-1.
1H-NMR(CDC13) δ ppm:
1.30-1.50 (3H, m), 1.90-2.10 (4H, m), 2.40-3.35 (5H, m), 4.30-4.45 (1H, m), 4.60-5.15 (3H, m), 6.10-6.40 (2H, m), 6.60-7.20 (4H, m), 7.30-7.50 (1H, m), 7.80-7.90 (2H, m), 8.15-8.25 (2H, m).

### Reference example 22-1

### tert-Butyl (R)-3-methyl-1-(2-nitrobenzenesulfonyl)-1,2,3,5-tetrahydrobenzo[e]-1,4-diazepin-4-carboxylate

To a solution of tert-butyl (R)-2-hydroxy-l-methylethyl[2-(2-nitrobenzenesulfonylamino)benzyl]carbamate (2.14 g) and triphenylphosphine (1.33g) in benzene (50mL) was added diisopropyl azodicarboxylate (40% toluene solution: 2.30mL) atroomtemperature, and this solution was stirred at room temperature for 10 minutes. The reaction solution was concentrated under reduced pressure. The obtained residue was purified by column chromatography on silica gel (eluent: ethyl acetate-hexane) to give tert-butyl (R)-3-methyl-1-(2-nitrobenzenesulfonyl) -1,2,3,5-tetrahydrobenzo[e]-1,4-diazepin-4-carboxylate (1.53 g).
1H-NMR(CDCl3) δ ppm:
1.35-1.75 (12H, m), 3.40-3.70 (1H, m), 3.95-4.90 (4H, m), 6.60-6.80 (1H, m), 6.95-8.10 (7H, m).

### Reference example 22-2

### tert-Butyl (R)-3-methyl-1-(4-nitrobenzensulfonyl)-1,2,3,5-tetrahydrobenzo[e]-1,4-diazepin-4-carboxylate

tert-Butyl (R)-3-methyl-1-(4-nitrobenzensulfonyl)-1,2,3,5-tetrahydrobenzo[e]-1,4-diazepin-4-carboxylate was obtained with the use of the corresponding nitrobenzenesulfonamide derivative in a similar manner to that described in Reference example 22-1.
1H-NMR (CDCl3) δ ppm:
1.25-1.40 (12H, m), 3.50-4.70 (5H, m), 7.15-7.30 (4H, m), 7.85-7.95 (2H, m), 8.25-8.35 (2H, m).

### Reference example 23

### tert-Butyl (R)-3-methyl-1,2,3,5-tetrahydrobenzo[e]-1,4-diazepin-4-carboxylate

To a suspension of tert-butyl (R)-3-methyl-1-(2-nitrobenzenesulfonyl)-1,2,3,5-tetrahydrobenzo[e]-1,4-diazepine-4-carboxylate (1.53 g) and potassium carbonate (2.36 g) in N,N-dimethylformamide (20 mL) was added benzenethiol (0.700 mL) at room temperature, and this mixture was stirred at room temperature for 30 minutes. To the reaction mixture was added water, and the mixture was extracted with ethyl acetate. The organic layer was washed with water and brine, dried over anhydrous magnesium sulfate. After filtration, the filtrate was concentrated under reduced pressure. The obtained residue was purifiedby column chromatography on silica gel (eluent:ethyl acetate-hexane) to give tert-butyl (R)-3-methyl-1,2,3,5-tetrahydrobenzo[e]-1,4-diazepin-4-carboxylate (0.803 g).
1H-NMR(CDCl3) δ ppm: 1.10-1.50 (12H, m), 3.05-3.50 (2H, m), 3.75-3.95 (1H, m), 4.25-4.75 (3H, m), 6.56 (1H, d, J=7.7Hz), 6.65-6.75 (1H, m), 6.95-7.15 (2H, m).
MS(ESI, m/z) : 263(M+H)+

### Reference example 24

### Ethyl (R)-2-(2-amino-5-fluorobenzoylamino)propionate

To a mixture of 2-amino-5-fluorobenzoic acid (0.500 g), hydroxybenzotriazole monohydrate (0.740 g) and N,N-dimethylformamide (7.0 mL) were added 1-ethyl-3-(3-dimethylaminopropyl)carbodiimide hydrochloride(0.927g)and triethylamine(0.540 mL) at room temperature, and this mixture was stirred at room temperature for 3 hours. To the reaction mixture was added water, and the mixture was extracted with ethyl acetate. The organic layer was washed with water and brine, dried over anhydrous magnesium sulfate. After filtration, the filtrate was concentrated under reduced pressure. The obtained residue was purified by column chromatography on silica gel (eluent: ethyl acetate-hexane). The obtained solid was recrystallized from diethyl ether - hexane to give ethyl (R)-2-(2-amino-5-fluorobenzoylamino)propionate (0.676 g).
1H-NMR (CDCl3) δ ppm:
1.32 (3H, t, J=7.1Hz), 1.51 (3H, d, J=7.2Hz), 4.25 (2H, q, J=7.1Hz), 4.71 (1H, quint, J=7.3Hz), 5.28 (1H, brs), 6.55-6.70 (2H, m), 6. 90-7.05 (1H, m), 7.12 (1H, dd, J=9.3, 2.9Hz).

### Reference example 25

### (R)-7-Fluoro-3-methyl-3,4-dihydro-1H-benzo[e]-1,4-diazepin-2,5-dione

A solution of ethyl (R)-2-(2-amino-5-fluorobenzoylamino)-propionate (0.533 g) in acetic acid (7.0 mL) was refluxed at 120 °C for 19 hours. The reaction mixture was concentrated under reduced pressure. The obtained residue was purified by column chromatography on aminopropylsilylated silica gel (eluent:ethyl acetate) to give (R)-7-fluoro-3-methyl-3,4-dihydro-1H-benzo[e]-1,4-diazepin-2,5-dione (0.340 g).
1H-NMR(DMSO-d6)δ ppm:
1.22 (3H, d, J=6.8Hz), 3.75-3.90 (1H, m), 7.13 (1H, dd, J=8.9, 4.9Hz), 7.35-7.50 (2H, m), 8.54 (1H, d, J=5.2Hz), 10.37 (1H, s).
MS(ESI, m/z) : 209(M+H)+

### Reference example 26

### (R)-2-(2-Amino-5-fluorobenzoylamino)propionic acid

To a solution of ethyl (R)-2-(2-amino-5-fluorobenzoylamino)-propionate (0.300 g) in ethanol (4.00 mL) was added 2 mol/L aqueous solution of sodium hydroxide (1.15 mL) at room temperature, and the reaction mixture was stirred at room temperature for an hour. To the reaction mixture were added 2 mol/L hydrochloric acid (1.15 mL) and toluene at room temperature. This mixture was concentrated under reduced pressure to give (R)-2-(2-amino-5-fluorobenzoylamino)-propionic acid (0.267 g).
1H-NMR (DMSO-d6) δ ppm:
1.36 (3H, d, J=7.3Hz), 4.33 (1H, quint, J=7.3Hz), 6.20-6.50 (2H, brs), 6.65-6.75 (1H, m), 7.00-7.10 (1H, m), 7.40-7.50 (1H, m), 8.40-8.50 (1H, m), 12.30-13.00 (1H, brs).

### Reference example 27

### (R)-7-Fluoro-3-methyl-3,4-dihyrdo-1H-benzo[e]-1,4-diazepin-2,5-dione

To a suspension of (R)-2-(2-amino-5-fluorobenzoylamino)-propionic acid (0.267 g) in N,N-dimethylformamide (10 mL) were added hydroxybenzotriazole monohydrate (0.271 g) and 1-ethyl-3-(3-dimethylaminopropyl)carbodiimide hydrochloride (0.339 g) at room temperature, and this mixture was stirred at 30 °C for 3 hours. To the reaction mixture was added water at room temperature and the mixture was extracted with ethyl acetate. The organic layer was washed with water and brine, dried over anhydrous magnesium sulfate and filtered. The filtrate was concentrated under reduced pressure, and the obtained residue was purified by column chromatography on aminopropylsilylated silica gel (eluent:ethyl acetate-ethanol) to give (R)-7-fluoro-3-methyl-3,4-dihyrdo-1H-benzo[e]-1,4-diazepin-2,5-dione (0.0750 g).
1H-NMR(DMSO-d6)δ ppm:
1.22 (3H, d, J=6.8Hz), 3.75-3.90 (1H, m), 7.13 (1H, dd, J=8.9, 4.9Hz), 7.35-7.50 (2H, m), 8.54 (1H, d, J=4.9Hz), 10.37 (1H, s).
MS(ESI, m/z) : 209(M+H)+

### Reference example 28

### (R)-7-Fluoro-3-methyl-2,3,4,5-tetrahydro-1H-benzo[e]-1,4-diazepine

To a suspension of (R)-7-fluoro-3-methyl-3,4-dihyrdo-1H-benzo[e]-1,4-diazepin-2,5-dione (0.0750 g) in dimethoxyethane (3.0 mL) was added lithiumaluminiumhydride (0.0547g) atroomtemperature, and the reaction mixture was refluxed for 16 hours. To the reaction mixture was added lithium aluminium hydride (0.0270 g) at room temperature and the reaction mixture was refluxed for 2 hours. To the reaction mixture was added lithium aluminium hydride (0.0270 g) at room temperature and the reaction mixture was refluxed for 5 hours. This reaction mixture was stirred at room temperature for 3 days. To the reaction mixture were added water (0.110 mL), 15wt% aqueous solution of sodium hydroxide (0.110 mL) and water (0.220 mL). This mixture was passed through a Celite pad, and the filtrate was concentrated under reduced pressure. The obtained residue was purified by column chromatography on aminopropylsilylated silica gel (eluent:ethyl acetate) to give (R)-7-fluoro-3-methyl-2,3,4,5-tetrahydro-1H-benzo[e]-1,4-diazepine (0.0075 g).
1H-NMR(CDC13) δ ppm:
1.09 (3H, d, J=6.6Hz), 2.52 (1H, dd, J=13.0, 9.0Hz), 2.95-3.10 (1H, m), 3.28 (1H, dd, J=9.0, 2.6Hz), 3.88 (2H, s), 6.69 (1H, dd, J=8.5, 4.9Hz), 6.77 (1H, td, J=8.5, 2.9Hz), 6.82 (1H, dd, J=8.5, 2.9Hz).
MS(ESI, m/z) : 181(M+H)+

### Reference example 29-1

### tert-Butyl (R)-3-methyl-1,2,3,5-tetrahydrobenzo[e]-1,4-diazepin-4-carboxylate

To a solution of (R)-3-methyl-2,3,4,5-tetrahydro-1H-benzo [e] -1, 4-diazepine (1. 81 g) in tetrahydrofuran (11 mL) was added di-tert-butyl dicarbonate (2.56 g) at room temperature, and this solution was stirred at room temperature for an hour. The reaction solution was concentrated under reduced pressure. To the residue was added diethyl ether and the precipitate was collected by filtration to give tert-butyl (R)-3-methyl-1,2,3,5-tetrahydrobenzo[e]-1,4-diazepin-4-carboxylate (2.17 g).
1H-NMR(CDCl3)δ ppm:
1.10-1.50 (12H, m), 3.05-3.50 (2H, m), 3.75-3.95 (1H, m), 4.25-4.75 (3H, m), 6.56 (1H, d, J=7.6Hz), 6.65-6.75 (1H, m), 6.95-7.15 (2H, m).
MS(ESI, m/z) : 263(M+H)+

### Reference example 29-2

### tert-Butyl (R)-7-fluoro-3-methyl-1,2,3,5-tetrahydrobenzo[e]-1,4-diazepin-4-carboxylate

tert-Butyl (R)-7-fluoro-3-methyl-1,2,3,5-tetrahydro-benzo[e]-1,4-diazepin-4-carboxylate was obtained with the use of the corresponding 2,3,4,5-tetrahydro-1H-benzo[e]-1,4- diazepine derivative in a similar manner to that described in Reference example 29-1.
1H-NMR(CDCl3)δ ppm:
1.10-1.55 (12H, m), 3.05-3.90 (3H, m), 4.25-4.70 (3H, m), 6.45-6.95 (3H, m).

### Reference example 30-1

### tert-Butyl (R)-3-methyl-1,2,3,5-tetrahydrobenzo[e]-1,4-diazepin-4-carboxylate

To a solution of tert-butyl (R)-3-methyl-1,2,3,5-tetrahydro-benzo[e]-1,4-diazepin-4-carboxylate (0.0657 g) and N,N-diisopropylethylamine (0.174 mL) in dichloromethane (1.0 mL) was added a suspension of 2-chloro-4-pyrrolidin-1-ylbenzoyl chloride (0.079 g) in dichloromethane (1.0 mL) and this solution was stirred at 30 ° C overnight. The reaction mixture was concentrated under reduced pressure. The obtained residue was purified by column chromatography on silica gel (eluent:ethyl acetate-hexane) to give tert-butyl (R)-3-methyl-1,2,3,5-tetrahydrobenzo[e]-1,4-diazepin-4-carboxylate (0.0314 g).
1H-NMR(CDCl3) δ ppm:
1.00-1.60 (12H, m), 1.90-2.00 (4H, m), 2.70-3.50 (5H, m), 4.10-5.40 (4H, m), 6.00-8.00 (7H, m).
MS(ESI, m/z) : 470(M+H)+

### Reference examples 30-2 to 30-32

The following compounds of Reference examples 30-2 to 30-32 were obtained with the use of the corresponding tert-butyl 1, 2, 3, 5-tetrahydrobenzo[e]-1,4-diazepine-4-carbonate derivatives in a similar manner to that described in Reference example 30-1. The structure formula and physical data of these compounds were shown in Tables 6 to 10.

**[Table 6]**

| Ref No | Strc | Physical data |
|---|---|---|
| 30-2 | 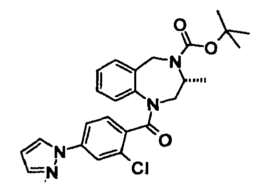 | 1H-NMR (CDCl3) δ ppm: 120-1.70 (12H, m), 2 80-5.35 (5H, m), 6.40-8 10 (10H, m). |
| 30-3 | 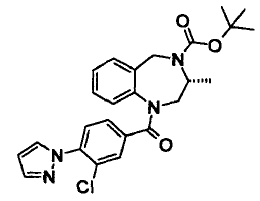 | 1H-NMR (CDCl3) δ ppm : 1.10-1.50 (12H, m), 2.90-3.25 (1H, m), 4.25-5.20 (4H, m), 6.45-7.60 (8H, m), 7.65-7.70 (1H, m), 7.85-7.80 (1H, m). |
| 30-4 | 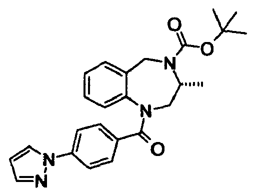 | 1H-NMR (CDCl3) δ ppm: 1.00-1.50 (12H, m), 2.85-3.30 (1H, m), 4 00-5.80 (4H, m), 6.40-7.60 (9H, m), 7 65-8 00 (2H, m) |
| 30-5 | 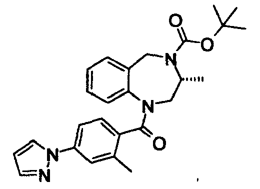 | 1H-NMR (CDCl3) δ ppm 1.10-1 50 (12H, m), 2.25-2.65 (3H, m), 2.80-3.70 (1H, m), 4.00-5 50 (4H, m), 6 30-7.60 (8H, m), 7 65-7.80 (1H, m), 7.80-7.90 (1H, m). |
| 30-6 | 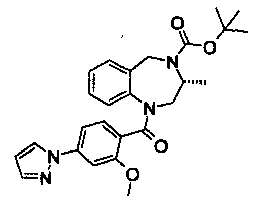 | 1H-NMR (CDCl3) δ ppm: 1.35-1 55 (12H, m), 2.80-4 10 (4H, m), 4.20-5.40 (4H, m), 6 40-8.00 (10H, m). MS(ESI, m/z) : 463(M+H)+ |
| 30-7 | 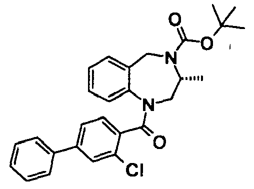 | 1H-NMR (CDCl3) δ ppm: 1.20-1.60 (12H, m), 2.90-3.80 (1H, m), 4.10-5 40 (4H, m), 6.50-8 00 (12H, m) MS(ESI, m/z) : 477(M+H)+ |

**[Table 7]**

| Ref No | Strc | Physical data |
|---|---|---|
| 30-8 | 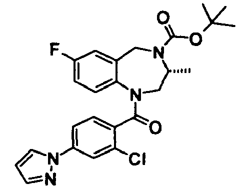 | MS(ESI, m/z) 485(M+H)+ |
| 30-9 | 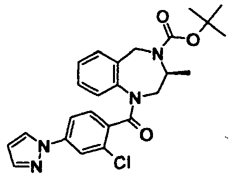 | 1H-NMR (CDCl3) δ ppm 1.10-1 65 (12H, m), 2 90-5 15 (5H, m), 6 40-8 00 (10H, m) |
| 30-10 | 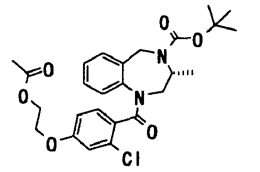 | 1H-NMR (CDCl3) δ ppm 1 00-1 55 (12H, m),2.05-2 15 (3H, m), 2 90-5 60 (9H, m), 6 20-7.60 (7H, m) |
| 30-11 | 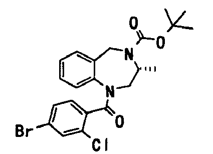 | 1H-NMR (CDCl3) δ ppm 1 00-1 50 (12H, m), 2 80-5 30 (5H, m), 6 30-7.80 (7H, m) |
| 30-12 | 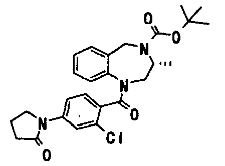 | 1H-NMR (CDCl3) δ ppm 1 00-1 60 (12H, m), 1 95-2.20 (2H, m), 2 30-2 65 (2H, m), 2 80-5 30 (7H, m), 6 30-8 20 (7H, m) MS(ESI, m/z). 484(M+H)+ |
| 30-13 | 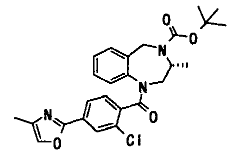 | 1H-NMR (CDCl3) δ ppm 0 80-1 60 (12H, m), 2 15-2 30 (3H, m), 2.80-5 50 (5H, m), 6 30-8 20 (8H, m) MS(ESI, m/z): 482(M+H)+ |
| 30-14 | 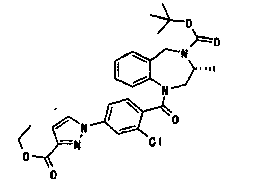 | 1H-NMR (CDCl3) δ ppm 1 00-1 60 (15H, m), 2.80-5 30 (7H, m), 6 40-8 20 (9H, m) MS(ESI, m/z) 539(M+H)+ |

**[Table 8]**

| Ref. No | Strc | Physical data |
|---|---|---|
| 30-15 | 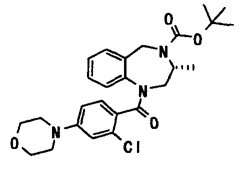 | 1H-NMR (CDCl3) δ ppm: 0 90-1 60 (12H, m), 2 80-3 30 (5H, m), 3.75-3 95 (4H, m), 4.10-5 30 (4H, m), 6.30-7.60 (7H, m) MS(ESI, m/z): 486(M+H)+ |
| 30-16 | 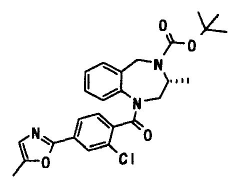 | 1H-NMR (CDCl3) δ ppm: 1.10-160 (12H, m), 2.30-2.45 (3H, m), 2.90-3 45 (1H, m), 4 15-5.30 (4H, m), 6.45-8.20 (8H, m) MS(ESI, m/z): 482(M+H)+ |
| 30-17 | 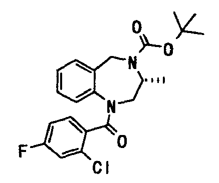 | 1H-NMR (CDCl3) δ ppm. 1.00-1 55 (12H, m), 2 80-5.30 (5H, m), 6 30-7.90 (7H, m) MS(ESI, m/z) 419(M+H)+ |
| 30-18 | 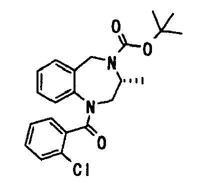 | 1H-NMR (CDCl3) δ ppm. 0.80-1.60 (12H, m), 2 80-5 40 (5H, m), 6 40-7.90 (8H, m) MS(ESI, m/z) 401 (M+H)+ |
| 30-19 | 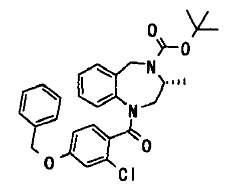 | 1H-NMR (CDCl3) δ ppm. 0 90-1.60 (12H, m), 2.70-5.30 (7H, m), 6.20-7 80 (12H, m) MS(ESI, m/z) 507(M+H)+ |
| 30-20 | 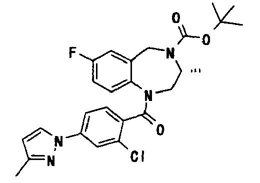 | 1H-NMR (CDCl3) δ ppm: 1 00-1 60 (12H, m), 2 30-2 40 (3H, m), 2 70-5.30 (5H, m), 6 20-8 00 (8H, m) MS(ESI, m/z) 499(M+H)+ |

**[Table 9]**

| Ref No | Strc | Physical data |
|---|---|---|
| 30-21 | 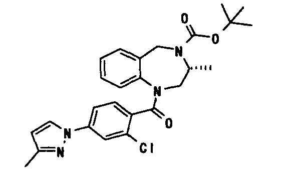 | 1H-NMR (CDCl3) δ ppm: 1.00-1.60 (12H, m), 2 30-2 40 (3H, m), 2 90-5.30 (5H, m), 6.20-8.00 (9H, m) |
| 30-22 | 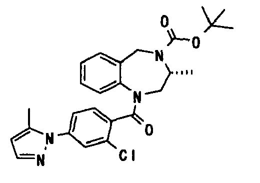 | 1H-NMR (CDCl3) δ ppm: 1.00-1 65 (12H, m), 2 30 (3H, s), 2.95-5 40 (5H, m), 6.15-8 00 (9H, m) MS(ESI, m/z) 481 (M+H)+ |
| 30-23 | 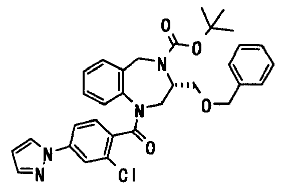 | 1H-NMR (CDCl3) δ ppm: 1.35-1 50 (9H, m), 3.00-5.35 (9H, m), 6.40-8.20 (15H, m) |
| 30-24 | 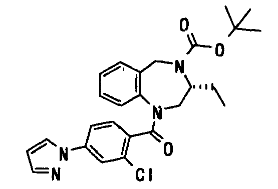 | 1H-NMR (CDCl3) δ ppm: 1.00-1.10 (3H, m), 1 43 (9H, s), 1.80-2.10 (3H, m), 3 00-5 40 (7H, m), 6 40-6.50 (1H, m), 6.55-8.10 (9H, m) |
| 30-25 | 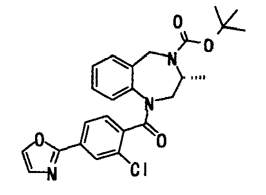 | 1H-NMR (CDCl3) δ ppm: 0.95-1 70 (12H, m), 2.80-5 40 (5H, m), 6 35-8 30 (9H, m) |
| 30-26 | 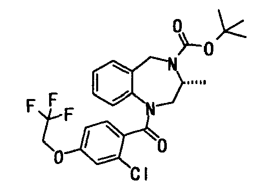 | 1H-NMR (CDCl3) δ ppm: 1 00-1 60 (12H, m), 2.70-5.35 (7H, m), 6.20-7 70 (7H, m) |

**[Table 10]**

| Ref. No | Strc | Physical data |
|---|---|---|
| 30-27 | 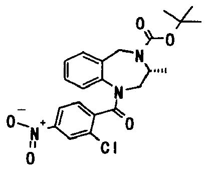 | 1H-NMR (CDCl3) δ ppm 0 85-1 60 (12H, m), 2 85-5.30 (5H, m), 6 45-8 40 (7H, m) |
| 30-28 | 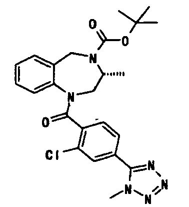 | 1H-NMR (CDCl3) δ ppm: 1.00-1 70 (12H, m), 2 80-5 30 (8H, m), 6.45-7 95 (7H, m) |
| 30-29 | 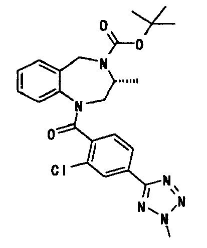 | 1H-NMR (CDCl3) δ ppm 0 95-1 65 (12H, m), 2 80-5.25 (8H, m), 6 50-8 30 (7H, m) |
| 30-30 | 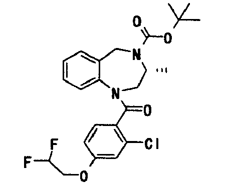 | 1H-NMR (CDCl3) δ ppm 0.95-1.60 (12H, m), 2.85-5.30 (7H, m), 5 85-7.70 (7H, m) MS(ESI, m/z) 481(M+H)+ |
| 30-31 | 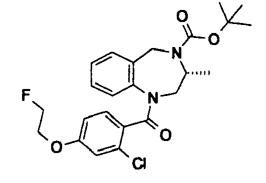 | 1H-NMR (CDCl₃) δ ppm. 0 80-1.60 (12H, m), 2.70-5.20 (9H, m), 6 25-7.70 (7H, m) MS(ESI, m/z) 463(M+H)+ |
| 30-32 | 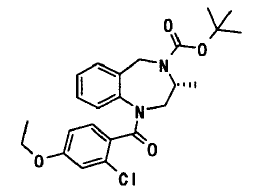 | 1H-NMR (CDCl₃) δ ppm 0 60-1 80 (15H, m), 2 60-5 20 (7H, m), 6 40-7 40 (7H, m) |

### Reference example 31-1

### 2-Chloro-4-pyrrolidin-1-ylphenyl((R)-3-methyl-2,3,4,5-tetrahydrobenzo[e]-1,4-diazepin-1-yl)methanone

A solution of tert-butyl (R)-1-(2-chloro-4-pyrrolidin-1-ylbenzoyl)-3-methyl-1,2,3,5-tetrahydrobenzo[e]-1,4-diazepin-4-carboxylate (0.0314 g) in trifluoroacetic acid (1.0 mL)-dichloromethane (1.0 mL) was stirred at room temperature for an hour. The reaction solution was concentrated under reduced pressure. The obtained residue was purified by column chromatography on aminopropylsilylated silica gel (eluent:ethyl acetate-hexane) to give 2-chloro-4-pyrrolidin-1-ylphenyl((R)-3-methyl-2,3,4,5-tetrahydrobenzo[e]-1,4-diazepin-1-yl)methanone (0.0225 g).
1H-NMR(CDCl3)δ ppm:
0.85-1.25 (3H, m), 1.90-2.00 (4H, m), 2.40-2.55 (1H, m), 3.05-3.45 (6H, m), 3.85-4.30 (2H, m), 5.02 (1H, d, J=13.2Hz), 6.05-6.60 (2H, m), 6.65-7.40 (5H, m).
MS(ESI, m/z) : 370(M+H)+

### Reference examples 31-2 to 31-38

The following compounds of Reference examples 31-2 to 31-38 were obtained with the use of the corresponding amine derivatives in a similar manner to that described in Reference example 31-1. The structure formula and physical data of these compounds were shown in Tables 11 to 16.

**[Table 11]**

| Ref No | Strc | Physical data |
|---|---|---|
| 31-2 | 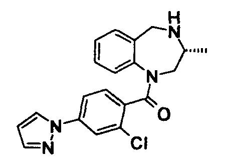 | MS(ESI, m/z) : 367(M+H)+ |
| 31-3 | 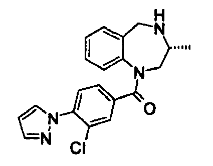 | 1H-NMR (CDCl3) δ ppm 1 05-1 25 (3H, m), 2 45-2 65 (1H, m), 3 00-3 45 (1H, m), 3 85-4 25 (2H, m), 4 95-5 10 (1H, m), 6 40-7 95 (10H, m) MS(ESI, m/z) 367(M+H)+ |
| 31-4 | 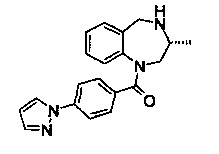 | 1H-NMR (CDCl3) δ ppm 1 05-1.25 (3H, m), 2.40-2.65 (1H, m), 3 00-3 45 (1H, m), 3.95-4 30 (2H, m), 5 00-5 15 (1H, m), 6 40-6.55 (1H, m), 660-8.00 (10H, m) MS(ESI, m/z) 333(M+H)+ |
| 31-5 | 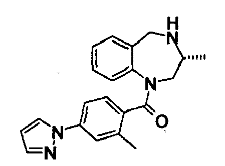 | 1H-NMR(CDCl3) δ ppm: 0 80-1.30 (3H, m), 2 45-2 60 (4H; m), 3 25-3 45 (1H, m), 3 90-4 20 (2H, m), 4 95-5 10 (1H, m), 6 35-6 55 (1H, m), 6 60-8 00 (9H, m) MS(ESI, m/z) 347(M+H)+ |
| 31-6 | 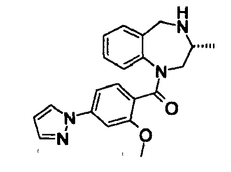 | 1H-NMR (CDCl3) δ ppm 0 85-1.25 (3H, m), 2.40-2 55 (1H, m), 3 20-3 80 (4H, m), 3 90-4 30 (2H, m), 4 98 (1H, dd, J=13.3, 2 1Hz), 6.40-8 00 (10H, m). MS(ESI, m/z) 363(M+H)+ |
| 31-7 | 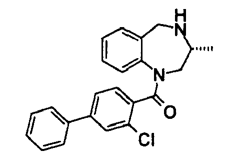 | 1H-NMR (CDCl3) δ ppm 0 85-1 25 (3H, m), 2 50-2 65 (1H, m), 3 30-3 45 (1H, m), 3 90-4 30 (2H, m), 4 95-5 05 (1H, m), 6 90-7.75 (12H, m) MS(ESI, m/z) 377(M+H)+ |
| 31-8 | 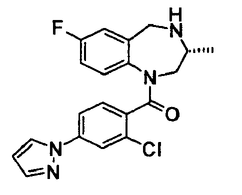 | 1H-NMR (CDCl3) δ ppm 1 20 (3H, d, J=6 5Hz), 2 54 (1H, dd, J=13 4, 10 5Hz), 3 30-3.45 (1H, m), 3 91 (1H, d, J=14 5Hz), 4 10-4 25 (1H, m), 4 94 (1H, dd, J=13 4,1 9Hz), 6 40-8 05 (9H,m) MS(ESI, m/z) 385(M+H)+ |

**[Table 12]**

| Ref No | Strc | Physical data |
|---|---|---|
| 31-9 | 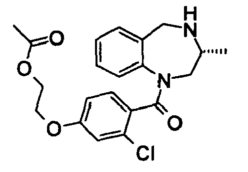 | 1H-NMR (CDCl3) δ ppm 0 85-1 25 (3H, m), 2.05-2 15 (3H, m), 2 45-5 00 (10H, m), 6 50-7 40 (7H, m) |
| 31-10 | 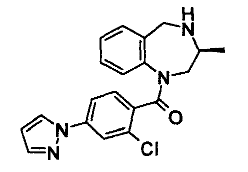 | 1H-NMR (CDCl3) δ ppm 0 85-1 25 (3H, m), 2 50-5 00 (6H, m), 6 40-8 00 (10H, m). |
| 31-11 | 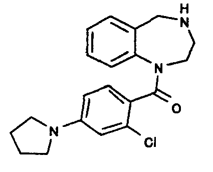 | 1H-NMR (CDCl3) δ ppm 1 85-2 05 (4H, m), 2 50-3 70 (8H, m), 4 10-4 40 (2H, m), 5.00-5 25 (1H, m), 6 10-6 65 (2H, m), 6 80-7 60 (5H, m) MS(ESI, m/z) 356(M+H)+ |
| 31-12 | 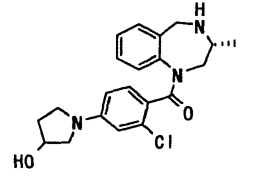 | 1H-NMR (CDCl3) δ ppm 0 80-1 30 (3H, m), 1 95-2 20 (2H, m), 2 40-2.55 (1H. m), 3 05-3 60 (5H, m), 380-425 (2H, m), 4 45-5 10 (2H, m), 605-760 (7H, m) MS(ESI, m/z) : 386(M+H)+ |
| 31-13 | 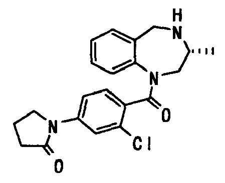 | 1H-NMR (CDCl3) δ ppm 0 80-1 25 (3H, m), 2 05-2 30 (2H, m), 2 45-3 00 (3H, m), 3 10-3 45 (1H, m), 3 55-4 30 (4H, m), 4 35-5 05 (1H, m), 6 70-8 00 (7H, m) MS(ESI, m/z) : 384(M+H)+ |
| 31-14 | 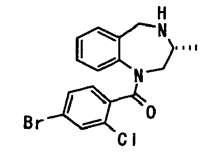 | 1H-NMR (CDCl3) δ ppm. 0 80-1 25 (3H, m), 2 45-5.05 (5H, m), 6 60-7 80 (7H, m) |

**[Table 13]**

| Ref No | Strc | Physical data |
|---|---|---|
| 31-15 | 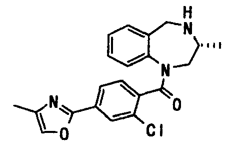 | 1H-NMR (CDCl3) δ ppm: 0 75-1.30 (3H, m), 2 15-2 30 (3H, m), 2 50-2 65 (1H, m), 3 30-3 45 (1H, m), 3.80-3 90 (1H, m), 4 15-4.30 (1H, m), 4 90-5 05 (1H, m), 6 70-8 20 (8H, m) MS(ESI, m/z) 382(M+H)+ |
| 31-16 | 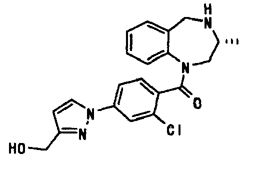 | 1H-NMR (CDCl3) δ ppm: 0 80-1 30 (3H, m), 2 50-2 60 (1H. m), 3 30-3 75 (1H, m), 390-4 30 (2H, m), 4 70-4 85 (2H, m), 4 90-5 00 (1H, m), 6 40-8.00 (9H, m) MS(ESI, m/z) 397(M+H)+ |
| 31-17 | 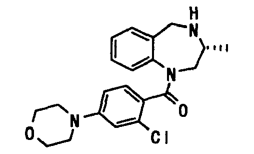 | 1H-NMR (CDCl3) δ ppm 0 85-1 25 (3H, m), 2 40-5 10 (13H, m), 6 45-7 60 (7H, m) MS(ESI, m/z) : 386(M+H)+ |
| 31-18 | 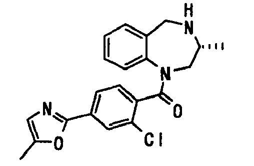 | MS(ESI, m/z) 382(M+H)+ |
| 31-19 | 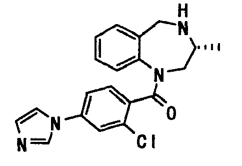 | 1H-NMR (CDCl3) δ ppm 0 80-1 40 (3H, m), 2 40-5 20 (5H, m), 6 30-8 00 (10H, m) MS(ESI, m/z) 367(M+H)+ |
| 31-20 | 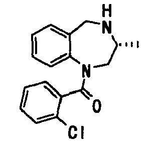 | 1H-NMR (CDCl3) δ ppm 0.80-1 40 (3H, m), 2 40-5.20 (5H, m), 6 70-8 00 (8H, m) MS(ESI, m/z) 301(M+H)+ |
| 31-21 | 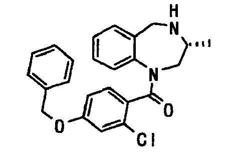 | 1H-NMR (CDCl3) δ ppm 0 80-1 25 (3H, m), 2 40-3.00 (1H, m), 3 25-3 75 (1H. m), 3 85-4 25 (2H, m), 4 85-5 15 (3H, m), 6 55-7 60 (12H, m) MS(ESI, m/z) 407(M+H)+ |

**[Table 14]**

| Ref No | Strc | Physical data |
|---|---|---|
| 31-22 | 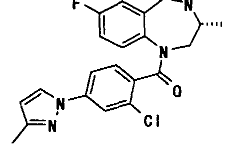 | 1H-NMR (CDCl3) δ ppm 1 19 (3H, dd, J=6 5Hz), 2 30-2 40 (3H, m), 2 45-2 60 (1H, m), 330-340 (1H, m), 3 80-3.95 (1H, m), 3.90-4 25 (1H, m), 4 94 (1H. dd, J=13 4, 1 9Hz), 6 20-6 35 (1H, m), 6 60-6 70 (1H. m), 6 85-7 15 3H, m), 7.30-7 40 (1H, m), 7 62 (1H, s), 7.70-7.90 (1H. m) |
| 31-23 | 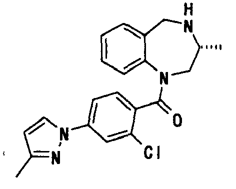 | 1H-NMR (CDCl3) δ ppm 1 20-1 35 (3H, m), 2.30-2 40 (3H, m), 2 65-5 05 (5H, m), 6.20-7 90 (9H, m) MS(ESI, m/z) .381(M+H)+ |
| 31-24 | 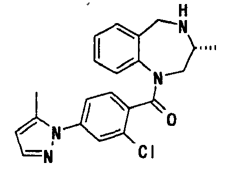 | MS(ESI, m/z) 381(M+H)+ |
| 31-25 | 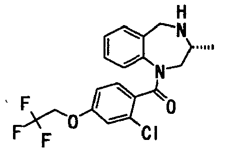 | 1H-NMR (CDCl3) δ ppm 0 85-1 25 (3H, m), 2 40-3 00 (1H, m), 3 10-3 40 (1H, m), 3 85-4 50 (4H, m), 4 85-5 15 (1H, m), 6 50-7 25 (7H, m) |
| 31-26 | 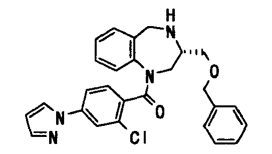 | 1H-NMR (CDCl3) δ ppm 2 75-5 50 (9H, m), 6 40-7 95 (15H, m) |
| 31-27 | 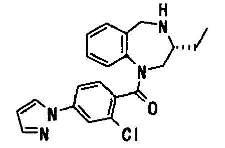 | 1H-NMR (CDCl3) δ ppm 1 08 (3H, t, J=7 6Hz), 1 20-1 55 (2H, m), 2 40-3 65 (2H, m), 3 95-4 25 (2H, m), 4 90-5 20 (1H, m), 6 40-6 60 (1H, m), 6 90-8 00 (9H, m) |

**[Table 15]**

| Ref No. | Strc | Physical data |
|---|---|---|
| 31-28 | 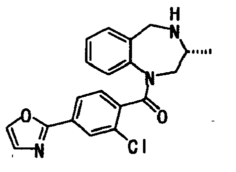 | 1H-NMR (CDCl3) δ ppm 0 80-1 30 (3H, m), 2.45-5 10 (5H, m), 660-825 (9H, m) |
| 31-29 | 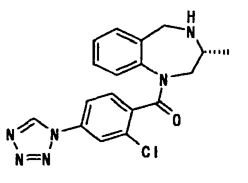 | 1H-NMR (CDCl3) δ ppm 1.21 (3H, d, J=6 6Hz), 2 50-2 65 (1H, m), 2 85-5 10 (4H, m), 690-800 (7H, m), 8 90-9.15 (1H, m) MS(ESI, m/z) 369(M+H)+ |
| 31-30 | 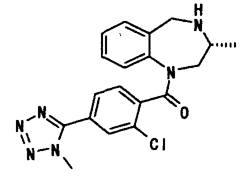 | 1H-NMR (CDCl3) δ ppm 1 21 (3H, d, J=6 6Hz), 2 45-2.70 (1H, m), 3 20-4 40 (6H, m), 485-505 (1H, m), 6 70-8 05 (7H, m) MS(ESI, m/z) 383(M+H)+ |
| 31-31 | 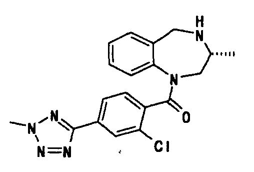 | 1H-NMR (CDCl3) δ ppm 0 80-1 25 (3H, m), 2 50-2 65 (1H, m), 3 25-4.55 (6H, m), 4 90-5 05 (1H, m), 6 85-8 30 (7H, m) MS(ESI, m/z) 383(M+H)+ |
| 31-32 | 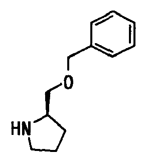 | 1H-NMR (CDCl3) δ ppm 1 35-1.45 (1H, m), 1 65-1 85 (3H, m), 2 80-3 05 (2H, m), 3.25-3.50 (3H, m), 4 45-4 60 (2H, m), 7 00-7 60 (5H, m) MS(ESI, m/z) 192(M+H)+ |

**[Table 16]**

| Ref No | Strc | Physical data |
|---|---|---|
| 31-33 | 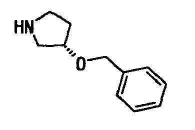 | 1H-NMR (CDCl3) δ ppm 1.80-1 95 (1H, m), 2 00-2 15 (1H, m), 2 45-3.20 (5H, m), 4 05-4.20 (1H, m), 4 40-4 55 (2H, m), 7.20-7 40 (5H, m) MS(ESI, m/z) 178(M+H)+ |
| 31-34 | 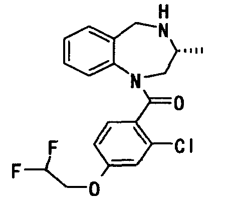 | 1H-NMR (CDCl3) δ ppm 0 80-1.25 (3H, m), 2 53 (1H, dd, J=12 9,10.7 Hz), 3 25-3 40 (1H, m), 3 90-4 30 (4H, m), 4.90-5.05 (1H, m), 6 02 (1H, tt, J=54 9, 4.1Hz), 6 50-7 05 (5H, m), 7 08 (1H, dt, J=7.3, 1 3Hz), 7 18 (1H, dd, J=7 6, 1 3Hz) |
| 31-35 | 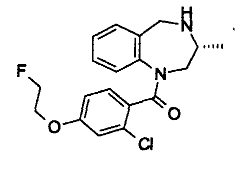 | 1H-NMR(CDCl3) δ ppm 0 80-1 25 (3H, m), 2 40-3 40 (2H, m), 3 85-5 05 (7H, m), 6 50-7.60 (7H, m) MS(ESI, m/z) 363(M+H)+ |
| 31-36 | 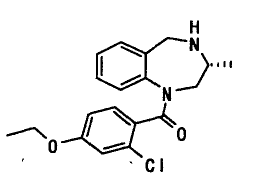 | 1H-NMR (CDCl3) δ ppm 0 70-1.50 (6H, m), 2 40-2 60 (1H, m), 3 25-3 40 (1H, m), 3 80-4 25 (4H, m), 4 90-5 05 (1H, m), 6 45-6 60 (1H, m), 6 70-7 40 (7H, m) MS(ESI, m/z) : 345(M+H)+ |
| 31-37 | 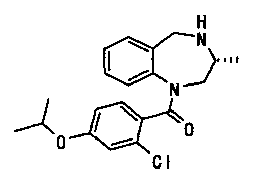 | 1H-NMR (CDCl3) δ ppm 0 75-1 40 (9H, m), 2 20-3 00 (1H, m), 3 25-3 45 (1H, m), 3 60-4 25 (2H, m), 4 35-4 65 (1H m), 4 90-5 05 (1H; m), 6 40-6 60 (1H, m), 6 70-7 55 (7H, m) MS(ESI, m/z) 359(M+H)+ |
| 31-38 | 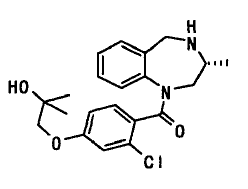 | 1H-NMR (CDCl3) δ ppm 1 18 (3H, d, J=6 4Hz), 1 29 (6H, s), 1 95-2 15 (1H, br), 2.45-3 00 (1H, m), 3 15-3 40 (1H, m), 3.60-3 75 (2H, m), 3 85-4 25 (2H, m), 4 90-5 05 (1H, m), 6 50-7 60 (7H, m) MS(ESI, m/z) 389(M+H)+ |

### Reference example 32-1

### 2-Chloro-4-pyrrolidin-1-ylphenyl((R)-3-methyl-2,3,4,5-tetrahydrobenzo[e]-1,4-diazepin-1-yl)methanone

To a suspension of 2-chloro-4-pyrrolidin-1-ylphenyl[(R)-3-methyl-4-(4-nitrobenzenesulfonyl)-2,3,4,5-tetrahydrobenzo[e]-1,4-diazepin-1-yl]methanone (0.908 g) and potassium carbonate (1.13 g) in acetonitrile (20 mL) was added 1-dodecanethiol (1.17 mL) at room temperature, and this suspension was refluxed for 5 hours. To the reaction mixture was added water and the mixture was extracted with ethyl acetate. The organic layer was washed with brine, dried over anhydrous magnesium sulfate. After filtration, the filtrate was concentrated under reduced pressure. The obtained residue was purified by column chromatography on silica gel (eluent:ethyl acetate-methanol) to give 2-chloro-4-pyrrolidin-1-ylphenyT((R)-3-methyl-2,3,4,5-tetrahydrobenzo[e]-1,4-diazepin-1-yl)methanone (0.439 g).
1H-NMR (CDCl3) δ ppm:
0.85-1.25 (3H, m), 1.90-2.00 (4H, m), 2.40-2.85 (1H, m), 3.05-3.45 (6H, m), 3.85-4.30 (2H, m), 5.02 (1H, d, J=13.2Hz), 6.05-7.55 (8H, m).
MS(ESI, m/z) : 370(M+H)+

### Reference example 32-2

### tert-Butyl (R)-3-methyl-1,2,3,5-tetrahydrobenzo[e]-1,4-diazepin-4-carboxylate

tert-Butyl (R)-3-methyl-1,2,3,5-tetrahydrobenzo[e]-1,4-diazepin-4-carboxylate was obtained with the use of the corresponding nitrosulfonamide derivative in a similar manner to that described in Reference example 32-1.
1H-NMR(CDCl3)δ ppm:
1.10-1.50 (12H, m), 3.05-3.50 (2H, m), 3.75-3.95 (1H,m), 4.25-4.75 (3H, m), 6.56 (1H, d, J=7.6Hz), 6.65-6.75 (1H, m), 6.95-7.15 (2H, m) .
MS (ESI, m/z) : 263 (M+H) +

### Reference example 33

### Ethyl 3-chlorobiphenyl-4-carboxylate

A solution of ethyl 4-bromo-3-chlorobenzoate (400 mg), phenylboronic acid (241 mg), tetrakis(triphenylphosphine)-palladium(0) (87.7 mg), cesium fluoride (1.38 g) in 1,4-dioxane-ethanol-water(6.0-1.5-1.5 mL)was stirred at an external temperature of 100 °C under an argon gas atmosphere for 13 hours. After the reaction mixture was allowed to cool, the reaction mixture was concentrated under reduced pressure. To the residue were added water and ethyl acetate, and the organic layer was separated. After the organic layer was washed with brine, the solvent was removed under reduced pressure . The obtained residue was purified by column chromatography on aminopropylsilylated silica gel (eluent:hexane) to give ethyl 3-chlorobiphenyl-4-carboxylate (383 mg).
1H-NMR (CDCl₃) δ ppm:
1.43 (3H, t, J=7.2Hz), 4.42 (2H, q, J=7.2Hz), 7.40-7.50 (3H, m), 7.53 (1H, dd, J=1.7, 8.1Hz), 7.55-7.65 (2H, m), 7.68 (1H, d, J=1.7Hz), 7.92 (1H, d, J=8.1Hz).

### Reference example 34

### tert-Butyl (R)-1-[2-chloro-4-(3-hydroxypyrrolidin-1-yl)-benzoyl]-3-methyl-1,2,3,5-tetrahydrobenzo[e]-1,4-diazepin-4-carboxylate

To a suspension of tert-butyl (R)-1-(4-bromo-2-chlorobenzoyl)-3-methyl-1,2,3,5-tetrahydrobenzo[e]-1,4-diazepin-4-carboxylate (0.070 g), palladium acetate (II) (1.6 mg), 2,2'-bis(diphenylphosphino)-1,1'-binaphthyl (6.8 mg), cesium carbonate (0.0951 g) in toluene (2 mL) was added DL-3-pyrrolidinol (15.0 uL) at room temperature, and the suspension was stirred at an external temperature of 100 °C for 12 hours under an argon gas atmosphere. After the suspension was allowed to cool, the suspension was passed through a Celite pad, and the filtrate was concentrated under reduced pressure. The obtained crude product was purified by column chromatography on silica gel (eluent:ethyl acetate-hexane) to give tert-butyl (R)-1-[chloro-4-(3-hydroxypyrrolidin-1-yl)-benzoyl]-3-methyl-1,2,3,5-tetrahydrobenzo[e]-1,4-diazepin-4-carboxylate (51.1 mg).
1H-NMR(CDCl3) δ ppm:
0.90-1.60 (12H, m), 1.80-2.20 (2H, m), 2.70-5.30 (10H, m), 5.90-7.70 (7H, m).
MS(ESI, m/z) : 486(M+H)+

### Reference example 35

### Methyl 2-chloro-4-(3-ethoxycarbonylpropylamino)benzoate

To a mixture of methyl 4-amino-2-chlorobenzoate (0.500 g) and sodium iodide (0.404 g) in N,N-dimethylformamide (8.0 mL) were successively added 2, 6-lutidine (0.433 g) and ethyl 4-bromobutylate (0.578 g) at room temperature, and the solution was stirred at an external temperature of 80 °C for 14 hours. To the solution were added water and ethyl acetate at room temperature. The organic layer was separated. The organic layer was successively washed with water and brine, dried over anhydrous magnesium sulfate. The solvent was removed under reduced pressure. The obtained crude product was purified by column chromatography on aminopropylsilylated silica gel (eluent:ethyl acetate-hexane) to give methyl 2-chloro-4-(3-ethoxycarbonylpropylamino) benzoate (0.178 g). ethoxycarbonylpropylamino)benzoate (0.178 g).
1H-NMR(CDCl3) δ ppm:
1.26 (3H, t, J=7.2Hz), 1.90-2.00 (2H, m), 2.43 (2H, t, J=6.9Hz), 3.15-3.25 (2H, m), 3.85 (3H, s), 4.15 (2H, q, J=7.2Hz), 4.35-4.45(1H, br), 6.44 (1H, dd, J=8.7, 2.4Hz), 6.59 (1H, d, J=2.4Hz), 7.79 (1H, d, J=8.7Hz).
MS(ESI, m/z) : 300 (M+H)+

### Reference example 36

### 2-Chloro-4-(2-oxopyrrolidin-1-yl)benzoic acid

To a suspension of methyl 2-chloro-4-(3-ethoxycarbonylpropylamino) benzoate (0.178 g) in ethanol (2. 5 mL) was added 5 mol/L,aqueous solution of sodium hydroxide (0.124 mL), and the solution was refluxed at an external temperature of 85 °C for 14 hours. To the stirred solution was added 2 mol/L hydrochloric acid (0.310 mL) at room temperature. The mixture was concentrated under reduced pressure. The residue was collected by filtration. The residue was washed with water, and dried under reduced pressure to give 2-chloro-4-(2-oxopyrrolidin-1-yl)benzoic acid (0.0850 g).
1H-NMR(DMSO-d6) δ ppm:
2.00-2.15 (2H, m), 2.54 (2H, t, J=8.1Hz), 3.86 (2H, t, J=7.1Hz), 7.65 (1H, dd, J=8.7, 2.2Hz), 7.87 (1H, d, J=8.7Hz), 7.96 (1H, d, J=2.2Hz), 12.00-14.00 (1H, br).

### Reference example 37

### 4-Benzyl 1-ethyl 2-chloroterephthalate

A mixture of ethyl 4-bromo-2-chlorobenzoate (1.67 g), palladium acetate (II) (0.142 g), 1, 3-bis (diphenylphosphino) propane (0.261g), N,N-diisopropylethylamine (2.42 mL), benzylalcohol (1.97 mL) indimethylsulfoxide (15mL) was stirred at an external temperature of 100 °C for 9 hours under a carbon monoxide atmosphere. After the mixture was allowed to cool, to the mixture were added water and ethyl acetate. The mixture was passed through a Celite pad. The organic layer of the filtrate was separated, and the layer was washed with water, brine, and dried over anhydrous magnesium sulfate. The solvent was removed under reduced pressure. The obtained crude product was purified by column chromatography on silica gel (eluent:ethyl acetate-hexane) to give 4-benzyl 1-ethyl 2-chloroterephthalate (1.17 g).
1H-NMR (CDCl3) δ ppm:
1.41 (3H, t, J=7.2Hz), 4.42 (2H, q, J=7.2Hz), 5.38 (2H, s), 7.30-7.50 (5H, m), 7.80-7.90 (1H, m), 7.98 (1H, dd, J=8.1, 1.6Hz), 8.10-8.15 (1H, m).
MS(ESI, m/z) : 319 (M+H) +

### Reference example 38-1

### Ethyl 2-chloro-4-(2-hydroxy-1-methylethyl)aminocarbonylbenzoate

To a solution of 1-ethyl 2-chloroterephthalate (0.177 g), DL-2-aminopropanol (67.0 uL), hydroxybenzotriazole monohydrate (0.154 g) in N,N-dimethylformamide (3.0 mL) was added 1-ethyl-3-(3-dimethylaminopropyl) carbodiimide hydrochloride (0.193 g) at room temperature, and the solution was stirred at room temperature for 4 days. The solution was diluted with water, and the mixture was extracted with ethyl acetate. The organic layer was separated. The aqueous layer was extracted with ethyl acetate, and the combined organic layer was washed with brine. The layer was dried over anhydrous magnesium sulfate, concentrated under reduced pressure. The obtained crude product was purified by column chromatography on aminopropylsilylated silica gel (eluent:ethyl acetate-hexane-methanol) to give Ethyl 2-chloro-4-(2-hydroxy-1-methylethyl)-aminocarbonylbenzoate (0.194 g).
1H-NMR(CDCl3) δ ppm:
1.32 (3H, d, J=6.8Hz), 1.42 (3H, t, J=7.2Hz), 2.30-2.45 (1H, br), 3.60-3.90 (2H, m), 4.20-4.35 (1H, m), 4.42 (2H, q, J=7.2Hz), 6.30-6.45 (1H, br), 7.69 (1H, dd, J=8.1, 1.7Hz), 7.80-7.90 (2H, m).
MS(ESI, m/z) : 286(M+H)+

### Reference examples 38-2 to 38-7

The following compounds of Reference examples 38-2 to 38-7 were obtained with the use of the corresponding carboxylic acid derivatives and amine derivatives in a similarmanner to that described in Reference example 38-1. The structure formula and physical data of these compounds were shown in Table 17.

**[Table 17]**

| Ref No | Strc | Physical data |
|---|---|---|
| 38-2 | 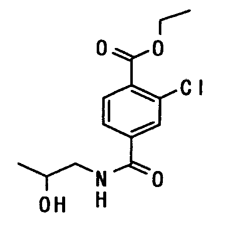 | 1H-NMR (CDCl3) δ ppm: 1.27 (3H, d, J=6 3Hz), 1.42 (3H, t, J=7.1Hz), 2 35-2.50 (1H, br), 3.20-3.35 (1H, m), 3 60-3.75 (1H, m), 3.95-4.10 (1H, m), 4.42 (2H, q, J=7.1Hz), 6 65-6.85 (1H, br), 7.69 (1H, dd, J=81, 1 6Hz), 7 80-7.90 (2H, m) MS(ESI, m/z) : 286(M+H)+ |
| 38-3 | 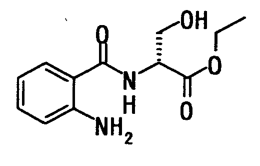 | 1H-NMR (CDCl3) δ ppm 1.33 (3H, t, J=6.9Hz), 2 61 (1H, t, J=6 0Hz), 4.06 (2H, dd, J=6 0, 3.5Hz), 4.29 (2H, q, J=6.9Hz), 4.75-4.85 (1H, m), 5 52 (2H, brs), 6 65-6 75 (2H, m), 7 02 (1H, d, J=6.3Hz), 720-7.30 (1H, m), 7.40-7 50 (1H, m) |
| 38-4 | 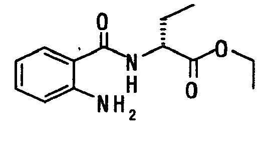 | 1H-NMR (CDCl₃) δ ppm. 0.98 (3H, t, J=7.3Hz), 1.31 (3H, t, J=6.9Hz), 1.75-1.90 (1H, m), 1 95-2.05 (1H, m), 4.20-4.30 (2H, m), 4 70-4.75 (1H, m), 5 49 (2H, brs), 6 55-6 70 (3H, m), 7.20-7.25 (1H, m), 7.40-7 45 (1H, m) |
| 38-5 | 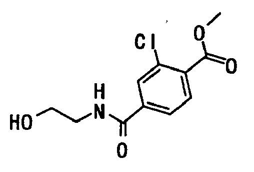 | 1H-NMR (CDCl₃) δ ppm: 2.00-2.25 (1H, br), 3 60-3.70 (2H, m), 3.80-3 90 (2H, m), 3.96 (3H, s), 6 55-6 65 (1H, br), 7.70 (1H, dd, J=8 1, 17Hz), 7.85-7 95 (2H, m) |
| 38-6 | 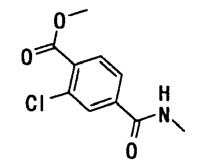 | 1H-NMR (CDCl3) δ ppm 3.03 (3H, d, J=5.0Hz), 3.95 (3H, s), 6 05-6 35 (1H, m), 7 67 (1H, dd, J=8 2, 1 6Hz), 7 84 (1H, d, J=1 6Hz), 7.87 (1H, d, J=8.2Hz) |
| 38-7 | 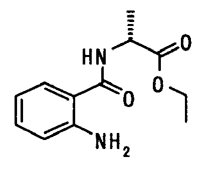 | 1H-NMR (DMSO-d6) δ ppm: 1.19 (3H, t, J=7.3Hz), 1 37 (3H, d, J=7 3Hz), 4.00-4.20 (2H, m), 4 30-4.45 (1H, m), 6 37 (2H, brs), 6.45-6 55 (1H, m), 6 65-6 75 (1H, m), 7 10-7 20 (1H, m), 7.50-7.60 (1H, m), 8.40-8 55 (1H, m) |

### Reference example 39

### Ethyl 2-chloro-4-(1-methyl-2-oxoethyl)aminocarbonylbenzoate

To a solution of ethyl 2-chloro-4-(2-hydroxy-1-methylethyl)-aminocarbonylbenzoate (0.194 g) in dichloromethane (2 mL) was added Dess-Martin periodinane (0.433 g) at room temperature, and the suspension was stirred for 30 minutes under the same condition. The mixture was diluted with dichloromethane. The resulting white precipitate was removed by Celite filtration, and the filtrate was concentrated under reduced pressure. The obtained crude product was purified by column chromatography on silica gel (eluent:ethyl acetate-hexane) to give ethyl Ethyl 2-chloro-4-(1-methyl-2-oxoethyl)aminocarbonylbenzoate (0.154 g).
1H-NMR(CDCl3) δ ppm:
1.42 (3H, t, J=7.1Hz), 1.53 (3H, d, J=7.5Hz), 4.43 (2H, q, J=7.1Hz), 4.70-4.80 (1H, m), 6.80-6.95 (1H, br), 7.73 (1H, dd, J=8.1, 1.7Hz), 7.85-7.95 (2H, m), 9.66 (1H, s).

### Reference example 40

### Ethyl 2-chloro-4-(4-methyloxazol-2-yl)benzoate

A solution of triphenylphosphine (0.430 g) and hexachloroethane (0.386 g) in acetonitrile (3.0 mL) was stirred at room temperature for 5 minutes. To the solution was added a solution of ethyl 2-chloro-4-(1-methyl-2-oxoethyl)aminocarbonylbenzoate (0.154g) inacetonitrile (1.0mL) atroomtemperature, and the solution was stirred for 10 minutes. To the solution was added pyridine (0.264 mL) at room temperature, and the solution was stirred for 30 minutes. The solution was concentrated under reduced pressure. The obtained crude product was purified by column chromatography on silica gel (eluent:ethyl acetate-hexane) to give ethyl 2-chloro-4-(4-methyloxazol-2-yl)benzoate (90.9 mg).
MS(ESI, m/z) : 266(M+H)+

### Reference example 41

### Benzyl 4-amino-2-chlorobenzoate

A mixture of benzyl 2-chloro-4-nitrobenzoate (4.34 g) and tin chloride (II) dihydrate (10.1 g) in ethanol (100 mL) was heated to reflux for an hour. After the mixture was allowed to cool, the mixture was concentrated under reduced pressure. To the residue was added ethanol (50 mL) and the mixture was heated to reflux for another an hour. After the mixture was allowed to cool, the mixture was concentrated under reduced pressure. The residue was diluted with dichloromethane. Then water was added. The bulk of dichloromethane was removed under reduced pressure. The precipitate was collected by filtration, washed with hexane, dried under reduced pressure to give benzyl 4-amino-2-chlorobenzoate (3.81 g).
1H-NMR (CDCl3) δ ppm:
5.24 (2H, s), 5.80-6.60 (3H, m), 6.64 (1H, d, J=2.2Hz), 7.30-7.50 (5H, m), 7.69 (1H, d, J=8.7Hz).

### Reference example 42

### Benzyl 2-chloro-4-hydrazinobenzoate hydrochloride

To a suspension of benzyl 4-amino-2-chlorobenzoate (2.00 g) in concentrated hydrochloric acid (10 mL) were added water (1.0 mL) and concentrated hydrochloric acid (1.0 mL) under ice- cooling. The mixture was stirred at room temperature for 5 minutes, at an external temperature of -5 °C for 20 minutes. A mixture of sodium sulfate (0.554 g) in water was added dropwise to the mixture, while an internal temperature was kept below 0 °C. After dropwise addition, the mixture was stirred at room temperature for 2.5 hours. After the mixture was ice-cooled, a mixture of tin chloride (II) dihydrate (6.90 g) and concentrated sulfuric acid (20mL) was added dropwise to the mixture as an internal temperature was kept below 4 °C. After dropwise addition, the mixture was stirred at room temperature for 2 hours. The resulting precipitate was collected by filtration and successively washed with concentrated hydrochloric acid, hexane and diethylether, dried under reduced pressure to give benzyl 2-chloro-4-hydrazinobenzoate hydrochloride (2.35 g).
1H-NMR(DMSO-d6) δ ppm:
5.30 (2H, s), 6.96 (1H, dd, J=8.6, 1.8Hz), 7.12 (1H, d, J=1.8Hz), 7.30-7.55 (5H, m), 7.85 (1H, d, J=8.6Hz), 9.00-9.30 (1H, br), 10.00-11.50 (3H, br).
MS(ESI, m/z) : 277(M+H-Cl)+

### Reference example 43

### Ethyl 1-(4-benzyloxycarbonyl-3-chlorophenyl)-1H-pyrazol-3-carboxylate

To a mixture of benzyl 2-chloro-4-hydrazinobenzoate hydrochloride (0.752 g) in ethanol (3.0 mL) was added a mixture of (E)-1,1,1-trichloro-4-ethoxybuta-3-en-2-on (0.435 g) in ethanol (2.0 mL) at room temperature and the mixture was heated to reflux overnight. After the mixture was allowed to cool, the mixture was passed through a celite pad, and the filtrate was concentrated under reduced pressure. The obtained crude product was purified by column chromatography on silica gel (eluent:ethyl acetate-hexane) to give ethyl 1-(4-benzyloxycarbonyl-3-chlorophenyl)-1H-pyrazol-3-carboxylate (57.5 mg).
1H-NMR(CDCl3) δ ppm:
1.43 (3H, t, J=7.2Hz), 4.46 (2H, q, J=7.2Hz), 5.39 (2H, s), 7.03 (1H, d, J=2.6Hz), 7.30-7.50 (5H, m), 7.71 (1H, dd, J=8.6, 2.2Hz), 7.96 (1H, d, J=2.2H), 7.99 (1H, d, J=2.6Hz), 8.02 (1H, d, J=8.6Hz).
MS(ESI, m/z) : 385(M+H)+

### Reference example 44

### Ethyl 2-chloro-4-(2-oxopropyl)aminocarbonylbenzoate

To a solution of oxalyl chloride (0.347 g) in dichloromethane (15 mL) was added dimethylsulfide (0.320g) at an external temperature of -78 °C, and the solution was stirred at the same temperature for 20 minutes. A solution of ethyl 2-chldro-4-(2-hydroxypropyl)-aminocarbonylbenzoate (0.390 g) in dichloromethane (6 mL) was added at the same temperature, and the solution was stirred at the same temperature for 30 minutes. Triethylamine (1.52 mL) was added at the same temperature and the mixture was stirred at room temperature for 10 minutes. To the solution was added water. The organic layer was separated. The aqueous layer was extracted with dichloromethane and the combined organic layer was washed with brine, dried over anhydrous magnesium sulfate. The solvent was removed under reduced pressure. The obtained crude product was purified by column chromatography on silica gel (eluent:ethyl acetate) to give Ethyl 2-chloro-4-(2-oxopropyl)aminocarbonylbenzoate (0.366 g).
1H-NMR(CDCl3)δ ppm:
1.42 (3H, t, J=7.1Hz), 2.30 (3H, s), 4.37 (1H, d, J=4.3Hz), 4.43 (2H, q, J=7.1Hz), 6.85-7.00 (1H, br), 7.72 (1H ,dd, J=8.1, 1.7Hz), 7.88 (1H, d, J=8.1Hz), 7.90 (1H, d, J=1.7Hz).
MS (ESI, m/z) : 284 (M+H) + ,

### Reference example 45

### Ethyl 2-chloro-4-(5-methyloxazol-2-yl)benzoate

A solution of ethyl 2-chloro-4-(2-oxopropyl)-aminocarbonylbenzoate (0.366 g) and phosphorus oxychloride (0.593 g) in toluene (5.0 mL) was stirred at an external temperature of 100 °C overnight . After the mixture was allowed to cool, to the mixture were successively added ice and a saturated aqueous solution of sodium hydrogen carbonate. The organic layer was separated and washed with brine, then dried over magnesium sulfate. The solvent was removed under reduced pressure. The obtained crude product was purified by column chromatography on silica gel (eluent:ethyl acetate-hexane) to give ethyl 2-chloro-4-(5-methyloxazol-2-yl)benzoate (0.296 g).
1H-NMR(CDCl3) δ ppm:
1.42 (3H, t, J=7.1Hz), 2.35-2.50 (3H, m), 4.42 (2H, q, J=7.1Hz), 6.85-6.95 (1H, m), 7.85-8.00 (3H, m), 8.05-8.15 (1H, m).
MS(ESI, m/z) : 266(M+H)+

### Reference example 46

### tert-Butyl (R)-1-(2-chloro-4-imidazol-1-ylbenzoyl)-3-methyl-1,2,3,5-tetrahydrobenzo[e]-1,4-diazepin-4-carboxylate

A suspension of tert-butyl (R)-1-(2-chloro-4-fluoro-1-yl-benzoyl)-3-methyl-1,2,3,5-tetrahydrobenzo[e]-1,4-diazepin-4-carboxylate (80.0 mg), imidazole (15.6 mg),potassium carbonate (39.6 mg) in N,N-dimethylformamide (1.0 mL) was stirred at an external temperature of 120 °C for 42 hours. To the mixture was added water at room temperature. The resulting precipitate was collected by filtration, dried under reduced pressure to give tert-butyl (R)-1-(2-chloro-4-imidazol-1-ylbenzoyl)-3-methyl-1,2,3,5-tetrahydrobenzo[e]-1,4-diazepin-4-carboxylate (66.2 mg).
1H-NMR(CDCl3) δ ppm:
1.00-1.60 (12H, m), 2.90-3.90 (1H, m), 4.20-5.30 (4H, m), 6.30-7.90 (10H, m).
MS(ESI, m/z) : 467 (M+H) +

### Reference example 47

### tert-Butyl (R)-1-[2-chloro-4-(3-hydroxymethylpyrazol-1-yl)-benzoyl-3-methyl-1,2,3,5-tetrahydrobenzo[e]-1,4-diazepin-4-carboxylate

To a solution of tert-butyl (R)-1-[4-(3-carboxypyrazol-1-yl)-2-chlorobenzoyl]-3-methyl-1,2,3,5-tetrahydrobenzo[e]-1,4-diazepin-4-carboxylate (45.0 mg) and diisopropylethylamine (9.4 uL) in tetrahydrofuran (0.80 mL) was added isobutyl chloroformate (6.80 mg) under ice-cooling, and the mixture was stirred at room temperature for 1.5 hours. To a stirred mixture was added lithium tetrahydroborate (3.90 mg) under ice-cooling and the mixture was stirred under the same condition for an hour. To a stirred mixture was added lithium tetrahydroborate (3.90 mg) under ice-cooling and the mixture was stirred under the same condition for 30 minutes. To the mixture was added an aqueous solution of ammonium chloride under ice-cooling. Ethyl acetate was additionally added to the mixture, and the organic layer was separated. After the layer was washed with brine, the layer was dried over anhydrous magnesium sulfate. The solvent was removed under reduced pressure. The obtained crude product was purified by column chromatography on silica gel (eluent:ethyl acetate-hexane) to give tert-butyl (R)-1-[2-chloro-4-(3-hydroxymethylpyrazol-1-yl)benzoyl-3-methyl-1,2,3,5-tetrahydrobenzo[e]-1,4-diazepin-4-carboxylate (12.3 mg).
1H-NMR(CDCl3) δ ppm:
0.80-1.60 (12H, m), 2.01 (1H, t, J=5.8Hz), 2.90-5.30 (7H, m), 6.40-8.00 (9H, m).
MS(ESI, m/z) : 497 (M+H)+

### Reference example 48

### Benzyl N-2-fluoroethyl-N-2-methoxyethylcarbamate

To a solution of benzyl 2-methoxyethylcarbamate (0.200 g) in N, N-dimethylformamide (2.6 mL) was added sodium hydride (purity 60%, 27 . 5 mg) at room temperature under an argon atmosphere, and the mixture was stirred under the same condition for 10 minutes. 1-Bromo-2-fluoroethane (160 uL) was added to the mixture at room temperature and the mixture was stirred at an external temperature of 90 °C overnight. After the reaction mixture was allowed to cool, the reaction was quenched by water addition to the reaction mixture. Ethyl acetate was added to the mixture, and the organic layer was separated. The organic layer was concentrated under reduced pressure, and the residue was purified by column chromatography on silica gel (eluent:ethyl acetate-hexane) to give benzyl N-2-fluoroethyl-N-2-methoxyethylcarbamate (74.4 mg).
1H-NMR(CDCl3) δ ppm:
3.25-3.40 (3H, m), 3.45-3.75 (6H, m), 4.40-4.65 (2H, m), 5.15 (2H, s), 7.30-7.40 (5H, m).
MS(ESI, m/z) : 256(M+H)+

### Reference example 49

### tert-Butyl (R)-1-(4-amino-2-chlorobenzoyl)-3-methyl-1,2,3,5-tetrahydrobenzo[e]-1,4-diazepin-4-carboxylate

To a solution of tert-butyl (R)-1-(2-chloro-4-nitrobenzoyl)-3-methyl-1,2,3,5-tetrahydrobenzo[e]-1,4-diazepin-4-carboxylate (100 mg) in acetic acid (1.14 mL) was added zinc (147 mg) under water-cooling. Two mol/L hydrochloric acid (0.113 mL) was added dropwise to the mixture. The reaction mixture was stirred at room temperature for an hour, followed by stirring at an external temperature of 50 °C for an hour. To the stirred reaction mixture were added ethyl acetate and 28% aqueous solution of ammonia (1.35 mL) at room temperature. The separated organic layer was washed with a saturated aqueous solution of sodium hydrogen carbonate, and brine. The organic layer was dried over anhydrous magnesium sulfate and after filtration, the filtrate was concentrated under reduced pressure. The obtained crude product was purified by column chromatography on aminopropylsilylated silica gel (eluent:ethyl acetate-hexane) to give tert-butyl (R)-1-(4-amino-2-chlorobenzoyl)-3-methyl-1,2,3,5-tetrahydrobenzo[e]-1,4-diazepin-4-carboxylate (55.8 mg).
1H-NMR(CDCl3)δ ppm:
1.05-1.60 (12H, m), 2.75-5.20 (7H, m), 6.15-7.50 (7H, m).

### Reference example 50

### tert-Butyl (R)-1-(2-chloro-4-tetrazol-1-ylbenzoyl)-3-methyl-1,2,3,5-tetrabenzo[e]-diazepin-4-carboxylate

To a mixture of tert-butyl (R) -1- (4-amino-2-chlorobenzoyl)-3-methyl-1,2,3,5-tetrabenzo[e]-1,4-diazepin-4-carboxylate (50.0 mg), diethoxymethoxyethane (53.4 mg) and sodium azide (9.40 mg) was added acetic acid (0.15 mL) at room temperature and the reaction mixture was stirred at an external temperature of 80 °C for 5 hours. To the stirred reaction mixture were successively added water (0.124 mL), concentrated hydochloric acid (13 uL) and 25% aqueous solution of sodium sulfite (9 uL) at room temperature, and the mixture was stirred for an hour under ice-cooling. To the stirred mixture were added water and ethyl acetate at room temperature. The separated organic layer was dried over anhydrous magnesium sulfate, filtrated, and the filtrate was concentrated under reduced pressure. The obtained crude product was purified by column chromatography on aminopropylsilylated silica gel (eluent:ethyl acetate-hexane) to give tert-butyl(R)-1-(2-chloro-4-tetrazol-1-ylbenzoyl)-3-methyl-1,2,3,5-tetrabenzo[e]-diazepin-4-carboxylate (43.7 mg).
1H-NMR(CDCl3)δ ppm:
0.85-1.70 (12H, m), 2.90-5.25 (5H, m), 6.40-8.00 (7H, m), 8.90-9.10 (1H, m).

### Reference example 51

### Methyl 2-chloro-4-(1-methyl-1H-tetrazol-5-yl)benzoate

To a suspension of methyl 2-chloro-4-methylaminocarbonylbenzoate (290 mg) in toluene (10 mL) was added phosphorus pentachloride (292 mg) under ice-cooling and the mixture was stirred at an external temperature of 40 °C for 2 hours. To the mixture was added trimethylsilyldiazomethane (0.264 mg) at room temperature, and the reactionmixture was stirred at the same temperature overnight. Then the reaction mixture was stirred at an external temperature of 80 °C for 4 hours and additionally at room temperature for 4 days. To the reaction mixture was added a saturated solution of sodium hydrogen carbonate and the organic layer was separated. The aqueous layer was extracted with ethyl acetate. The organic layer was combined and washed with brine. The organic layer was dried over anhydrous magnesium sulfate, filtered, and the filtrate was concentrated under reduced pressure. The obtained crude product was purified by column chromatography on aminopropylsilylated silica gel (eluent:ethyl acetate-hexane) to give methyl 2-chloro-4-(1-methyl-1H-tetrazol-5-yl)benzoate (94.0 mg).
1H-NMR(CDCl3) δ ppm:
3.99 (3H, s), 4.22 (3H, s), 7.73 (1H, dd, J=8.2, 1.6Hz), 7.89 (1H, d, J=1.6Hz), 8.02 (1H, d, J=8.2Hz).

### Reference example 52

### Methyl 2-chloro-4-cyanobenzoate

To a suspension of methyl 4-aminocarbonyl-2-chlorobenzoate (123 mg) and triethylamine (235 mg) in dichloromethane (2.0 mL) was added trifluoroacetic acid anhydride (254 mg) under ice-cooling, and the mixture was stirred at the same temperature for 2 hours. To the reaction mixture was added water and the mixture was extracted with dichloromethane. The organic layer was concentrated under reduced pressure. The obtained crude product was purified by column chromatography on silica gel (eluent:ethyl acetate-hexane) to give methyl 2-chloro-4-cyanobenzoate (109 mg).
1H-NMR(CDCl3) δ ppm:
3.97 (3H, s), 7.61 (1H, dd, J=8.2, 1.6Hz), 7.76 (1H, d, J=1.6Hz), 7.90 (1H, d, J=8.2Hz).

### Reference example 53

### Methyl 2-chloro-4-(1H-tetrazol-5-yl)benzoate

To a solution of methyl 2-chloro-4-cyanobenzoate (104 mg) and trimethylamine hydrochloride (55. 9 mg) in N-methylpyrrolidone (2.0 mL) was added sodium azide (38.0 mg) at room temperature, and the reaction mixture was stirred at 130 °C for 7 hours. After the reaction mixture was allowed to cool to around room temperature, the mixture was poured into 1 mol /L hydrochloric acid and the mixture was extracted with ethyl acetate. The organic layer was washed with water and brine. The organic layer was dried over anhydrous magnesium sulfate, filtered,and the filtrate was concentrated under reduced pressure to give methyl 2-chloro-4-(1H-tetrazol-5-yl)benzoate (107 mg).
1H-NMR(DMSO-d6) δ ppm:
3.91 (3H, s), 7.95-8.25 (3H, m).

### Reference example 54

### Methyl 2-chloro-4-(2-methyl-2H-tetrazol-5-yl)benzoate

To a suspension of methyl 2-chloro-4-(1H-tetrazol-5-yl)-benzoate (99.0 mg) and potassium carbonate (143 mg) in N,N-dimethylformamide (2.0 mL) was added methyl iodide (118 mg) at room temperature, and the reaction mixture was stirred at 70 °C for 4 hours. After the reaction mixture was allowed to cool to around room temperature, the mixture was poured into water and extracted with ethyl acetate. The organic layer was washed with water and brine. The organic layer was dried over anhydrous magnesium sulfate filtered, and the filtrate was concentrated under reduced pressure. The obtained crude product was purified by column chromatography on silica gel (eluent:ethyl acetate-hexane) to 2-chloro-4-(2-methyl-2H-tetrazol-5-yl)benzoate (85.6 mg)
1H-NMR(CDCl3) δ ppm:
3.97 (3H, s), 4.43 (3H, s), 7.95 (1H, d, J=8.2Hz), 8.08 (1H, dd, J=8.2, 1.6Hz), 8.26 (1H, d, J=1.6Hz).

### Reference example 55

### (R)-1-Benzyl-3-methyl-3,4-dihydro-1H-benzo[e]-1,4-diazepin-2,5-dione

To a suspension of (R)-methyl-3,4-dihydro-1H-benzo[e]-1,4-diazepin-2,5-dione (10.0 g) and potassium carbonate (7.99 g) in N,N-dimethylformamide (150 mL) was added benzyl bromide (9.89 g) under ice-cooling, and the reaction mixture was stirred at an internal temperature of around 80 °C overnight. After the reaction mixture was allowed to cool to about room temperature, to the stirred reaction mixture was added 2-(2-hydroxyethylamino)ethanol (2.76 g) under water-cooling, and the mixture was stirred at room temperature for an hour. To the mixture were added ethyl acetate (200 mL), water (75 mL) and tetrahydrofuran (50 mL). The mixture was stirred at room temperature for an hour. The resulting insoluble was removed by filtration and the organic layer of the filtrate was separated. The aqueous layer was extracted with ethyl acetate again. The organic layer was combined and successively washed with 1 mol/L aqueous solution of sodium hydroxide, water and brine. The organic layer was dried over anhydrous magnesium sulfate, filtered, and the filtrate was concentrated under reduced pressure to give (R)-1-benzyl-3-methyl-3,4-dihydro-1H-benzo[e]-1,4-diazepin-2,5-dione (10.4g).
1H-NMR(DMSO-d6) δ ppm:
1.28 (3H, d, J=6.6Hz), 3.90-4.00 (1H, m), 4.97 (1H, d, J=16.1Hz), 5.33 (1H, d, J=16.1Hz), 7.05-7.35 (6H, m), 7.40-7.55 (2H, m), 7.60-7.70 (1H, m), 8.60 (1H, d, J=5. 7Hz) .

### Reference example 56

### tert-Butyl (S)-3-benzyloxymethyl-1,2,3,5-tetrahydrobenzo[e]-1,4-diazepin-4-carboxylate

To a mixture of tert-butyl (S)-3-hydroxymethyl-1,2,3,5-tetrahydrobenzo[e]-1,4-diazepin-4-carboxylate (79.1 mg) in N,N-dimethylformamide (1.0mL) was added sodium hydride (purity 60%, 14.8 mg) at room temperature. The suspension was stirred under the same condition for 10 minutes, and benzyl bromide (58.3 mg) was added to the mixture. The mixture was stirred under the same condition for 5 hours, then the reaction was quenched by water addition. Ethyl acetate was added to the mixture and the organic layer was separated. The organic layer was successively washed with water, brine, dried over anhydrous magnesium sulfate, and the solvent was removed under reduced pressure. The residue was purified by column chromatography on silica gel (eluent:ethyl acetate-hexane) to give tert-butyl (S)-3-benzyloxymethyl-1,2,3,5-tetrahydrobenzo[e]-1,4-diazepin-4-carboxylate (48.3 mg).
1H-NMR(CDCl3) δ ppm:
1.30-1.45 (9H, m), 3.20-3.35 (1H, m), 3.50-3.95 (4H, m), 4.35-4.80 (4H, m), 6.50-6.60 (1H, m), 6.65-6.75 (1H, m), 6.90-7.10 (2H, m), 7.20-7.40 (5H, m).

### Reference example 57

### Methyl 2-chloro-4-(4,5-dihydroxazol-2-yl)benzoate

To a stirred solution of methyl 2-chloro-4-(2-hydroxyethyl)aminocarbonylbenzoate (120 mg) in dichloromethane (2.0 mL) was added thionyl chloride (66.5 mg) at room temperature. The mixture was stirred under the same condition for 30 minutes, and the solvent was removed under reduced pressure. The residue was purified by column chromatography on silica gel (eluent:ethyl acetate-hexane) to give methyl 2-chloro-4-(4,5-dihydroxazol-2-yl)benzoate (70.0 mg).
1H-NMR(CDCl3) δ ppm:
3.95 (3H, s), 4.10 (2H, t, J=9.6Hz), 4.47 (2H, t, J=9.6Hz), 7.85-7.90 (2H, m), 8.00-8.10 (1H, m).

### Reference example 58

### Methyl 2-chloro-4-oxazol-2-ylbenzoate

A mixture of methyl 2-chloro-4-(4,5-dihydroxazol-2-yl)-benzoate (181 mg), 4,5-dichloro-3,6-dioxocyclohexa-1,4-dien-1,2-dicarbonitrile (189 mg) and toluene (35 mL) was heated to reflux for 16 hours. After the mixture was allowed to cool, the solvent was removed under reduced pressure. The residue was purified by column chromatography on silica gel (eluent:ethyl acetate-hexane) to give methyl 2-chloro-4-oxazol-2-ylbenzoate (18.3 mg).
1H-NMR(CDCl3) δ ppm:
3.96 (3H, s), 7.25-7.35 (1H, m), 7.75-7.80 (1H, m), 7.93 (1H, d, J=8.2Hz), 7.98 (1H, dd, J=8.2, 1.6Hz), 8.15 (1H, d, J=1.6Hz).

### Reference example 59-1

### 4-Bromo-2-chlorobenzoyl chloride

To a solution of 4-bromo-2-chlorobenzoic acid (0.331 g) in dichloromethane (3.0 mL) were added thionyl chloride (510 uL) and a catalytic amount of N-methylpyrrolidone at room temperature, and the mixture was heated to reflux at 40 °C for 30 minutes, additionally stirred at 35 °C for 12 hours. The mixture was concentrated under reduced pressure to give 4-bromo-2-chlorobenzoyl chloride (355 mg) .

### Reference example 59-2

### 2-Chloro-4-(2-oxopyrrolidin-1-yl)benzoyl chloride

To a solution of 2-chloro-4-(2-oxopyrrolidin-1-yl)benzoic acid (83.7mg) in dichloromethane (1.0mL) were added thionyl chloride (127 uL) and a catalytic amount of N-methylpyrrolidone at room temperature, and the mixture was stirred under the same condition for 4 hours. The mixture was concentrated under reduced pressure to give 2-chloro-4-(2-oxopyrrolidin-1-yl)benzoyl chloride (90.0 mg).

### Reference example 59-3

### 2-Chloro-4-(4-methyloxazol-2-yl)benzoyl chloride

To a suspension of 2-chloro-4-(4-methyloxazol-2-yl)benzoic acid (81.3mg) in dichloromethane (1.0mL) were added thionyl chloride (250 uL) and a catalytic amount of N-methylpyrrolidone at room temperature, and the suspension was stirred at 32 °C for an hour. Thionyl chloride (250 uL) was additionally added at room temperature and the suspension was stirred at 32 °C overnight. The solvent was removed under reduced pressure to give 2-chloro-4- (4-methyloxazol-2-yl)benzoyl chloride (87.0 mg).

### Reference example 59-4

### 2-Chloro-4-fluorobenzoyl chloride

To a mixture of 2-chloro-4-fluorobenzoic acid (159 mg) in dichloromethane (2.0 mL) were added thionyl chloride (332 uL) and a catalytic amount of N-methylpyrrolidone at room temperature, and the mixture was stirred at room temperature for an hour, followed by stirring at 38 °C for 1.5 hours. The solvent was removed under reduced pressure to give 2-chloro-4-fluorobenzoyl chloride (175 mg).

### Reference example 59-5

### 2-Chlorobenzoyl chloride

To a mixture of 2-chlorobenzoic acid (50.0 mg) in dichloromethane (1.0 mL) were added thionyl chloride (116 uL) and a catalytic amount of N-methylpyrrolidone at room temperature, and the mixture were stirred at room temperature for an hour. The solvent was removed under reduced pressure to give 2-chlorobenzoyl chloride (55.0 mg).

### Reference example 59-6

### 4-Benzyloxy-2-chlorobenzoyl chloride

To a solution of 4-benzyloxy-2-chlorobenzoic acid (180.0 mg) in thionyl chloride (1.48 mL) was added a catalytic amount of N-methylpyrrolidone at room temperature, and the mixture was stirred at room temperature for an hour. The solvent was removed under reduced pressure to give 4-benzyloxy-2-chlorobenzoyl chloride (191 mg).

### Reference example 59-7

### 2-Chloro-4-oxazol-2-ylbenzoyl chloride

To a mixture of 2-chloro-4-oxazol-2-ylbenzoic acid (31.4 mg) in thionyl chloride (1.0 mL) was added a catalytic amount of N-methylpyrrolidone at room temperature, and the reaction mixture was stirred at 40 °C for 3 hours. The solvent was removed under reduced pressure to give 2-chloro-4-oxazol-2-ylbenzoyl chloride (34.0 mg).

### Reference example 59-8

### 2-Chloro-4-(2,2,2-trifluoroethoxy)benzoyl chloride

To a mixture of 2-chloro-4-(2,2,2-trifluoroethoxy)benzoic acid (757 mg) in thionyl chloride (2.0 mL) was added a catalytic amount of N-methylpyrrolidone at room temperature, and the reaction mixture was stirred at 40 °C for 3 hours. The solvent was removed under reduced pressure to give 2-chloro-4-(2,2,2-trifluoroethoxy)-benzoyl chloride (810 mg).

### Reference example 59-9

### 2-Chloro-4-(1-methyl-1H-tetrazol-5-yl)benzoyl chloride

To a suspension of 2-chloro-4-(1-methyl-1H-tetrazol-5-yl)benzoic acid (55.0 mg) in dichloromethane (1.0 mL) were added thionyl chloride (131 mg) and N-methylpyrrolidone (5.00 uL) at room temperature, and the mixture was stirred at room temperature for 20 minutes. Thionyl chloride (131 mg) was added at room temperature and the mixture was stirred at an external temperature of 40 °C for 3 hours. The mixture was concentrated under reduced pressure to give 2-chloro-4- (1-methyl-1H-tetrazol-5-yl) benzoyl chloride (58.0 mg).

### Reference example 59-10

### 2-Chloro-4-(2-methyl-2H-tetrazol-5-yl)benzoyl chloride

To a suspension of 2-chloro-4-(2-methyl-2H-tetrazol-5-yl)benzoic acid (61.0 mg) in dichloromethane (1.0 mL) were added thionyl chloride (290 mg) and N-methylpyrrolidone (15.0 uL) under ice-cooling, and the mixture was stirred at an external temperature of 40 °C for 3 hours. The mixture was concentrated under reduced pressure to give 2-chloro-4-(2-methyl-2H-tetrazol-5-yl)benzoyl chloride (64.0 mg).

### Reference example 59-11

### 2-Chloro-4-(2-fluoroethoxy)benzoyl chloride

A mixture of 2-chloro-4- (2-fluoroethoxy) benzoic acid (500 mg) and a catalytic amount of N-methylpyrrolidone and thionyl chloride (2.0 mL) was stirred at an external temperature of 40 °C for an hour. The mixture was concentrated under reduced pressure to give 2-chloro-4-(2-fluoroethoxy)benzoyl chloride (0.54 g).

### Reference examples 59-12 to 59-16

The following compounds of Reference examples 59-12 to 59-16 were obtained with the use of the corresponding benzoic acid derivatives in a similar manner to that described in Reference example 59-1. The structure formula and physical data of these compounds were shown in Table 18.

**[Table 18]**

| Ref. No | Strc | Physical data |
|---|---|---|
| 59-12 | 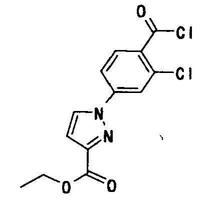 | 1H-NMR (DMSO-d6) δ ppm: 1 33 (3H, t, J=7 1Hz), 4 34 (2H, q, J=7 1Hz), 7 07 (1H, d, J=2 6Hz), 795-8 05 (2H, m), 8.10-8.15 (1H, m), 8 82 (1H, d, J=2 6Hz) |
| 59-13 | 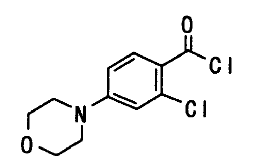 | 1H-NMR (DMSO-d6) δ ppm 3 20-3.30 (4H, m), 3 65-3.75 (4H, m), 6.93 (1H, dd, J=8 9, 2 4Hz), 6 98 (1H, d, J=2.4Hz), 7 77 (1H, d, J=8 9Hz) |
| 59-14 | 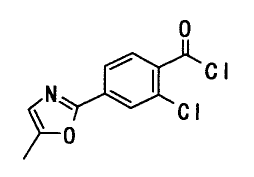 | 1H-NMR (DMSO-d6) δ ppm. 2.35-2 45 (1H, m), 705-7.15 (1H, m), 7.90-8 00 (3H, m) |
| 59-15 | 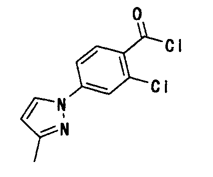 | 1H-NMR (CDCl3) δ ppm 2 39 (3H, s), 6 30-6 40 (1H, m), 7 65-7.75 (1H, m), 7.85-7 95 (2H, m), 8 20-830 (1H, m) |
| 59-16 | 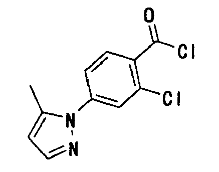 | 1H-NMR (CDCl3) δ ppm 2 49 (3H, s), 6.25-6.30 (1H, m), 7 60 (1H, dd, J=8 6, 2 1Hz), 7.60-7 70 (1H, m), 776 (1H, d, J=2 1Hz), 8 26 (1H, d, J=8 6Hz) |

### Reference example 60-1

### 1-Ethyl 2-chloroterephthalate

To a solution of 4-benzyl 1-ethyl 2-chloroterephthalate (251 mg) in tetrahydorfuran (5.0 mL) was added palladium-carbon (10%, 56% water content, 50.3 mg) at room temperature under an argon gas atmosphere, and the mixture was stirred at room temperature for 2 hours under a hydrogen gas atmosphere. Palladium-carbon (10%, 56% water content, 50.3 mg) was added to the mixture at room temperature, and the mixture was stirred at room temperature for an hour under a hydrogen gas atmosphere. The mixture was passed through a Celite pad. The filtrate was concentrated under reduced pressure to give 1-ethyl 2-chloroterephthalate (177 mg).
1H-NMR(CDCl3) δ ppm:
1.42 (3H, t, J=7.2Hz), 4.42 (2H, q, J=7.2Hz), 5.38 (2H, s), 7.87 (1H, d, J=8.1Hz), 8.02 (1H, d, J=8.1Hz), 8.17 (1H, s).

### Reference examples 60-2 to 60-8

The following compounds of Reference examples 60-2 to 60-8 were obtained with the use of the corresponding N-benzylamine derivatives, benzyl ester derivatives and benzyl ether derivatives in a similar manner to that described in reference example 60-1. Meanwhile, in the cases of Reference examples 60-7 and 60-8, the corresponding de-benzyl forms were obtained as tosic acid salts by addition of tosic acid. The structure formula and physical data of these compounds were shown in Table 19.

**[Table 19]**

| Ref No. | Strc | Physical data |
|---|---|---|
| 60-2 | 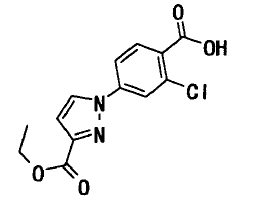 | 1H-NMR (DMSO-d6) δ ppm. 1 33 (3H, t, J=7 1Hz), 4 34 (2H, q, J=7 1Hz), 7 07 (1H, d, J=2.6Hz), 7 95-8 05 (2H, m), 8.10-8 15 (1H, m), 8.81 (1H, d, J=2.6Hz), 13 40-13 75 (1H, br) MS(ESI, m/z) : 295(M+H)+ |
| 60-3 | 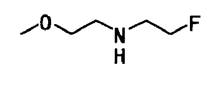 | 1H-NMR (CD3OD) δ ppm. 2 75-2 95 (4H, m), 3 35 (3H, s), 3 45-3.55 (2H, m), 4 40-4 50 (2H, m), |
| 60-4 | 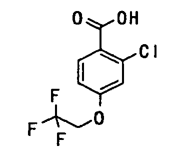 | 1H-NMR (DMSO-d6) δ ppm. 4 91 (2H, q, J=8 8Hz), 7 12 (1H, dd, J=8 8, 2,6Hz), 7.29 (1H, d, J=2 6Hz), 7 85 (1H, d, J=8 8Hz), 13 17 (1H, brs) MS(ESI, m/z) :255(M+H)+ |
| 60-5 | 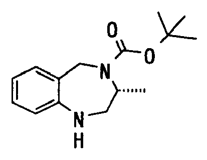 | 1H-NMR (CDCl3) δ ppm 1.10-1 50 (12H, m), 3 05-3 45 (2H, m), 3 75-3 90 (1H, m), 4.25-4 75 (3H, m), 6 50-6 60 (1H, m), 6 65-6 75 (1H, m), 6 90-7 15 (2H, m) |
| 60-6 | 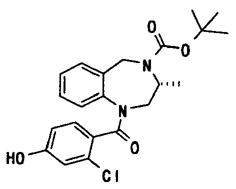 | 1H-NMR (CDCl3) δ ppm 0 90-1.60 (12H, m), 2 80-3 70 (1H, m), 4 10-8 50 (11H, m) MS(ESI, m/z) :415(M-H)- |
| 60-7 | 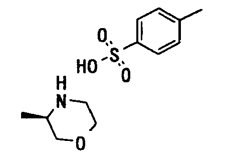 | 1H-NMR (DMSO-d6) δ ppm 1 12 (3H, t, J=6 3Hz), 2.29 (3H, s), 3 00-3 45 (4H, m), 3 50-3 65 (1H, m), 3 80-3 95 (2H, m), 7 05-7 15 (2H, m), 7 40-7 55 (2H, m), 8 59 (1H, brs), 8 85 (1H, brs) |
| 60-8 | 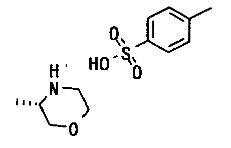 | 1H-NMR (DMSO-d6) δ ppm 1 12 (3H, t, J=6 3Hz), 2 29 (3H, s), 3 00-3 45 (4H, m), 3 50-3 65 (1H, m), 3 80-3 95 (2H, m), 7 05-7 15 (2H, m), 7.40-7 55 (2H, m), 8 59 (1H, brs), 8 85 (1H, brs) |

### Reference example 61-1

### (R)-2,5-dioxo-2,3,4,5-tetrahydro-1H-benzo[e]-1,4-diazepin-3-ylmethyl acetate

A solution of ethyl (R)-2-(2-aminobenzoylamino)-3-hydroxypropionate (1.01g) in acetic acid (20 mL) was heated to reflux for 13 hours. After the mixture was allowed to cool, the mixture was concentrated under reduced pressure. The residue was suspended in ethyl acetate. The precipitate was removed by filtration. The filtrate was concentrated under reduced pressure. The residue was purified by column chromatography on silica gel (eluent:ethyl acetate-ethanol) to give (R)-2,5-dioxo-2,3,4,5-tetrahydro-1H-benzo[e]-1,4-diazepin-3-ylmethyl acetate (303 mg).
1H-NMR(DMSO-d6) δ ppm:
2.00 (3H, s), 3.95-4.05 (1H, m), 4.15-4.25 (1H, m), 4.30-4.40 (1H, m), 7.11 (1H, d, J=7.9Hz), 7.20-7.30 (1H, m), 7.50-7.60 (1H, m), 7.70-7.80 (1H, m), 8.60-8.75 (1H, m), 10.51 (1H, brs).

### Reference example 61-2

### (R)-3-Ethyl-3,4-dihydro-1H-benzo[e]-1,4-diazepin-2,5-dione

(R)-3-Ethyl-3,4-dihydro-1H-benzo[e]-1,4-diazepin-2,5-dione was obtained with the use of the corresponding compound in a similar manner to that described in Reference example 61-1.
1H-NMR(CDCl3) δ ppm:
0.90 (3H, t, J=7.6Hz), 1.50-1.65 (1H, m), 1.70-1.85 (1H, m), 3.45-3.55 (1H, m), 7.05-7.15 (1H, m), 7.15-7.25 (1H, m), 7.45-7.55 (1H, m), 7.74 (1H, dd, J=7.9, 1.6Hz), 8.44 (1H, d, J=6.0Hz), 10.36 (1H, brs).
MS (ESI, m/z): 205(M+H)+

### Reference example 61-3

### (R)-3-Methyl-3,4-dihydro-1H-benzo[e]-1,4-diazepin-2,5-dione

(R)-3-methyl-3,4-dihydro-1H-benzo[e]-1,4-diazepin-2,5-dione was obtained with the use of the corresponding compound in a similar manner to that described in Reference example 61-1.
1H-NMR(DMSO-d6)δ ppm:
1.23 (3H, d, J=6.9Hz), 3.75-3.85 (1H, m), 7.09 (1H, d, J=8.1Hz), 7.74 (1H, d, J=7.8Hz), 8.38 (1H, d, J=4.9Hz), 10.33 (1H, brs).

### Reference example 62-1

### (R)-3-Ethyl-2,3,4,5-tetrahydro-1H-benzo[e]-1,4-diazepine

To a mixture of (R)-3-ethyl-3,4-dihydro-1H-benzo[e]-1,4-diazepin-2,5-dione (300 mg) and lithium aluminium hydride (149 mg) was added 1,2-dimethoxyethane (8.0 mL) under ice-cooling, and the mixture was heated to reflux for 7 hours. After the reaction mixture was allowed to cool, to the reaction mixture were successively added water (0.149 mL), 15% aqueous solution of sodium hydroxide (0.149 mL) and water (0. 447 mL). The mixture was filtered and the filtrate was concentrated under reduced pressure. The residue was purified by column chromatography on aminopropylsilylated silica gel (eluent:ethyl acetate-hexane) to give (R)-3-ethyl-2,3,4,5-tetrahydro-1H-benzo[e]-1,4-diazepine (110 mg).
1H-NMR(CDCl3) δ ppm:
0.99 (3H, t, J=7.6Hz), 1.35-1.50 (2H, m), 2.60-2.70 (1H, m), 2.75-2.85 (1H, m), 3.30-3.45 (1H, m), 3.80-4.05 (2H, m), 6.70-6.85 (2H, m), 7.00-7.15 (2H, m).

### Reference example 62-2

### (R)-1-Benzyl-3-methyl-2,3,4,5-tetrahydro-1H-benzo[e]-1,4-diazepine

(R)-1-Benzyl-3-methyl-2,3,4,5-tetrahydro-1H-benzo[e]-1,4-diazepine was obtained with the use of the corresponding amide derivative in a similar manner to that described in Reference example 62-1.
1H-NMR(CDC13) δ ppm:
0.90 (3H, d, J=6.6Hz), 2.46 (1H, dd, J=13.9, 9.1Hz), 2.90-3.00 (1H, m), 3.11 (1H, dd, J=13.9, 2.8Hz), 3.98 (1H, d, J=14.5Hz), 4.04 (1H, d, J=14.5Hz), 4.22 (1H, d, J=14.2Hz), 4.54 (1H, d, J=14.2Hz), 6.85-7.00 (2H, m), 7.10-7.45 (7H, m).

### Reference example 63

### tert-Butyl 2-chloro-4-fluorobenzoate

To a suspension of magnesium sulfate (4.81g) in dichloromethane (40 mL) was added concentrated sulfuric acid (0.981 g) at room temperature. The mixture was stirred at room temperature for 15 minutes. To the mixture were added 2-chloro-4-fluorobenzoic acid (1.75 g) and 2-methylpropan-2-ol (3.71 g). The mixture was stirred at room temperature for 15 hours. The reaction was quenched by addition of an aqueous solution of sodium hydrogen carbonate to the mixture, and ethyl acetate was added to the mixture, the organic layer was separated. The organic layer was washed with brine, dried over anhydrous sodium sulfate. The residue was purified by column chromatography on silica gel (eluent: ethyl acetate-hexane) to give tert-butyl 2-chloro-4-fluorobenzoate (1.98 g).
1H-NMR (CDCl3) δ ppm:
1.60 (9H, s), 6.95-7.05 (1H, m), 7.16 (1H, dd, J=8.5, 2.5Hz), 7.80 (1H, dd, J=8.7, 6.1Hz).

### Reference example 64-1

### tert-Butyl 2-chloro-4-(2,2-difluoroethoxy)benzoate

To a suspension of sodium hydride (purity 60%, 251 mg) in N,N-dimethylformamide (9.0 mL) was added 2,2-difluoroethanol (833 mg) under ice-cooling and the mixture was stirred for 10 minutes under ice-cooling. To the mixture was added tert-butyl 2-chloro-4-fluorobenzoate (1.12g) underice-cooling, and the mixture was stirred at room temperature for 3 hours. The reaction was quenched by addition of 2 mol/L hydrochloric acid to the mixture. To the reaction mixture was added ethyl acetate and the organic layer was separated. The organic layer was successively washed with an aqueous solution of sodium hydrogen carbonate and brine, and dried over anhydrous sodium sulfate. The mixture was concentrated under reduced pressure. The residue was purifiedby column chromatography on silica gel (eluent:ethyl acetate-hexane) to give tert-butyl 2-chloro-4-(2, 2-difluoroethoxy) benzoate (1.03 g).
1H-NMR(CDCl3) δ ppm:
1.50-1.65 (9H, m), 4.05-4.35 (2H, m), 5.90-6.20 (1H, m), 6.75-6.85 (1H, m), 6.90-7.00 (1H, m), 7.20-7.30 (1H, m)

### Reference example 64-2

### 2-Fluoroethyl 2-chloro-4-(2-fluoroethoxy)benzoate

2-Fluoroethyl 2-chloro-4-(2-fluoroethoxy)benzoate was obtained with the use of the corresponding 4-fluorobenzoic acid ester derivative and alcohol derivative in a similarmanner to that described in Reference example 64-1.
1H-NMR(CDCl3) δ ppm:
4.20-4.35 (2H, m), 4.50-4.60 (2H, m), 4.65-4.85 (4H, m), 6.87 (1H, dd, J=2.5, 8.8Hz), 7.02 (2H, d, J=8.8Hz), 7.95 (1H, d, J=8.8Hz).
MS(ESI, m/z) : 265(M+H)+

### Reference example 64-3

### Ethyl 2-chloro-4-(2,2,2-trifluoroethoxy)benzoate

Ethyl 2-chloro-4-(2,2,2-trifluoroethoxy)benzoate was obtained with the use of the corresponding 4-fluorobenzoic acid ester derivative and alcohol derivative in a similarmanner to that described in Reference example 64-1.
1H-NMR(CDCl3) δ ppm:
1.40 (3H, t, J=7.1Hz), 4.39 (2H, q, J=7.9Hz), 4.38 (2H, q, J=7.1Hz), 6.88 (1H, dd, J=8.7, 2.6Hz), 7.03 (1H, d, J=2.6Hz), 7.90 (1H, d, J=8.7Hz).
MS(ESI, m/z) :283(M+H)+

### Reference example 65

### 2-Chloro-4-(2,2-difluoroethoxy)benzoyl chloride

To a solution of tert-butyl 2-chloro-4-(2,2-difluoroethoxy)-benzoate (985 mg) in thionyl chloride (1.0 mL) was added N-methylpyrrolidone (5.00 uL) at room temperature. The mixture was stirred at an external temperature of 40 °C for 2 hours. To the mixture was added water (10.0 uL) at room temperature. The mixture was stirred at an external temperature of 50 °C for 4 hours. To the mixture was added toluene, and the solvent was removed with toluene as an azeotropy to give 2-chloro-4- (2,2-difluoroethoxy)benzoyl chloride (85.0 mg). 1H-NMR(CDCl3) δ ppm:
4.27 (2H, dt, J=12.6, 4.1Hz), 6.11 (1H, tt, J=4.1, 54.9Hz), 6.94 (1H, dd, J=8.8, 2.5Hz), 7.05 (1H, d, J=2.5Hz), 8.22 (1H, d, J=8.8Hz).

### Reference example 66-1

### tert-Butyl (R)-1-(2-chloro-4-ethoxybenzoyl)-3-methyl-1,2,3,5-tetrahydrobenzo[e]-1,4-diazepin-4-carboxylate

A mixture of tert-butyl (R)-1-(2-chloro-4-hydroxybenzoyl)-3-methyl-1,2,3,5-tetrahydrobenzo[e]-1,4-diazepin-4-carboxylate (70.0 mg), ethyliodide (39.3 mg), potassium carbonate (46.4 mg), N,N-dimethylformamide (0.50 mL) was stirred at an external temperature of 60 °C overnight. After the reaction mixture was allowed to cool, to the reaction mixture was added water and the mixture was extracted with ethyl acetate. The organic layer was washed with 10% aqueous solution of sodium carbonate and brine. The extract was dried over anhydrous magnesium sulfate and filtered. The filtrate was concentrated under reduced pressure. The residue was purified by column chromatography on silica gel (eluent:ethyl acetate-hexane) to give tert-butyl (R)-1-(2-chloro-4-ethoxybenzoyl)-3-methyl-1,2,3,5-tetrahydrobenzo[e]-1,4-diazepin-4-carboxylate (54.6 mg).
1H-NMR(CDC13) δ ppm:
0.70-1.70 (15H, m), 2.80-3.80 (1H, m), 3.85-4.10 (2H, m), 4.15-5.25 (4H, m), 6.30-7.70 (7H, m).
MS(ESI, m/z) : 487 (M+Na)+

### Reference examples 66-2 to 66-4

The following compounds of Reference examples 66-2 to 66-4 were obtained with the use of the corresponding phenol derivatives and alkyl halide derivatives in a similar manner to that described in Reference example 66-1. Meanwhile, in Reference example 66-3, 2,2,2-trifluoroethyl toluene-4-sulfonate was used instead of alkyl halide, and in Reference example 66-4, 2,2-dimethyloxirane was used instead of alkyl halide. The structure formula and physical data of these compounds were shown in Table 20.

**[Table 20]**

| Ref No. | Strc | Physical data |
|---|---|---|
| 66-2 | 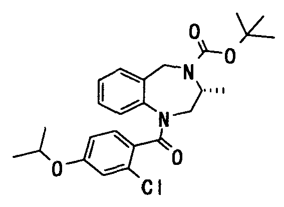 | 1H-NMR (CDCl3) δ ppm: 0.95-1 60 (18H, m), 2 70-3 80 (1H, m), 4 15-5 20 (5H, m), 6 20-7.80 (7H, m) MS(ESI, m/z). 481(M+Na)+ |
| 66-3 | 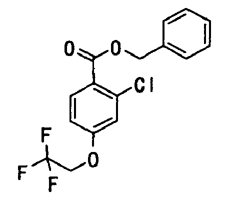 | 1H-NMR (CDCl3) δ ppm: 4 39 (2H, q, J=8.2Hz), 5 36 (2H, s), 6 87 (1H, dd, J=8 9, 2 6Hz), 7 03 (1H, d, J=2.6Hz), 7 30-7 50 (5H, m), 7 94 (1H, d, J=8 9Hz) MS(ESI, m/z) 345(M+H)+ |
| 66-4 | 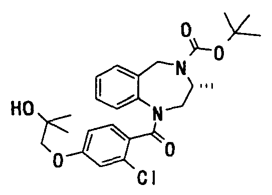 | 1H-NMR (DMSO-d6) δ ppm 1.19 (6H, s), 1.28 (3H, d, J=6.8Hz), 1.40 (9H, s), 3.50-4 90 (8H, m), 6 70-7.35 (7H, m) MS(ESI, m/z) 489(M+H)+ |

### Reference example 67

To a solution of DL-ornithine hydrochloride (100 mg) in acetonitrile (2.5 mL) was added hexamethyldisilazane (1.25 mL) at room temperature. The mixture was heated to reflux for 47 hours under an argon gas atmosphere. The mixture was stirred at room temperature overnight under an argon gas atmosphere. The mixture was poured into coldmethanol and the mixture was concentrated under reduced pressure. To the residue was added chloroform, and the mixture was passed through a Celite pad. The filtrate was concentrated under reduced pressure to give 3-aminopiperidin-2-one (42.0 mg).
MS(ESI, m/z) : 115 (M+H) +

### Reference example 68

### tert-Butyl (2-ethoxyethyl)ethylcarbamate

A solution of tert-butyl (2-ethoxyethyl)carbamate (300 mg) in N,N-dimethylformamide (3.0 mL) was added dropwise to a suspension of sodium hydride (95.2 mg, purity 60%) in N,N-dimethylformamide (2.0 mL) under ice-cooling and the mixture was stirred at room temperature for an hour. To the mixture was added ethyl iodide (371 mg) under ice-cooling and the mixture was stirred at room temperature for an hour. To the mixture was added water and the miuxture was extracted with ethyl acetate. The organic layer was washed with water and brine. The organic layer was dried over anhydrous magnesium sulfate, filtered and the filtrate was concentrated under reduced pressure to give tert-butyl (2-ethoxyethyl) ethylcarbamate (328 mg). 1H-NMR(CDC13)5 ppm:
0.95-1.30 (6H, m), 1.35-1.45 (9H, m), 3.10-3.50 (8H; m).

### Reference example 69

### (2-Ethoxyethyl)ethylamine hydrochloride

To a solution of tert-butyl (2-ethoxyethyl)ethylcarbamate (120 mg) in ethanol (1.2 mL) was added 38wt% ethanolic solution of hydrogen chloride (1.20mL) underice-coolingandthereactionmixture was stirred at room temperature overnight. The reaction mixture was concentrated under reduced pressure and the addition of ethyl acetate gaveaprecipitate. The solid was collected by filtration, and washed with a mixed solvent of ethyl acetate/hexane=1/1 to give (2-ethoxyethyl)ethylamine hydrochloride (41.0 mg).
1H-NMR(DMSO-d6)δ ppm:
1.15 (3H, t, J=7.3Hz), 1.19 (3H, t, J=7.3Hz), 2.85-3.15 (4H, m), 3.49 (2H, q, J=7.3Hz), 3.55-3.65 (2H, m), 8.76 (2H, brs).

### Example 1-1

### Ethyl {[(R)-1-(2-chloro-4-pyrazol-1-ylbenzoyl)-3-methyl-1,2,3,5-tetrahydrobenzo[e]-1,4-diazepin-4-carbonyl]amino}acetate

To a solution of 2-chloro-4-pyrazol-1-ylphenyl((R)-3-methyl-2,3,4,5-tetrahydrobenzo[e]-1,4-diazepin-1-yl)methanone (0.0600 g) in tetrahydrofuran (0.80 mL) was added ethyl isocyanatoacetate (0.0280 mL) under ice-cooling, and the mixture was stirred at room temperature for an hour. The reaction solution was purified by column chromatography on silica gel (eluent:ethyl acetate-hexane) to give ethyl {[(R)-1-(2-chloro-4-pyrazol-1-ylbenzoyl)-3-methyl-1,2,3,5-tetrahydrobenzo[e]-1,4-diazepin-4-carbonyl]amino}acetate (0.0855 g).
1H-NMR(CDCl3) δ ppm: 1.20-1.65 (6H, m), 2.90-5.40 (9H, m), 6.40-8.05 (10H, m).
MS(ESI, m/z) : 496(M+H)+

### Example 1-2

### 2-{3-Chloro-4-((R)-3-methyl-4-propylcarbamoyl-2,3,4,5-tetrahydrobenzo[e]-1,4-diazepin-1-carbonyl)phenoxy}ethyl acetate

To the mixture of 2-[3-chloro-4-((R)-3-methyl-2,3,4,5-tetrahydrobenzo[e]-1,4-diazepin-1-carbonyl)phenoxy]ethyl acetate (0.0300 g) and dichloromethane (0.80 mL) was added 1-isocyanatopropane (0.0115 g) at room temperature and this mixture was stirred at room temperature for 15 hours. The reaction mixture was concentrated under reduced pressure. The obtained residue was purified by column chromatography on aminopropylsilylated silica gel (eluent:ethyl acetate) to give 2-[3-chloro-4-((R)-3-methyl-4-propylcarbamoyl-2,3,4,5-tetrahydrobenzo[e]-1,4-diazepin-1-carbonyl)phenoxy]ethyl acetate (0.0395 g).

### Examples 1-3 to 1-53

The following compounds of Examples 1-3 to 1-53 were obtained with the use of the corresponding 2,3,4,5-tetrahydro-1H-benzo[e]-1,4-diazepine derivatives and isocyanate derivatives in a similar manner to that described in Example 1-1. The structure formula and physical data of these compounds were shown in Tables 21 to 28.

| No | Strc | Physical data |
|---|---|---|
| Ex 1-3 | 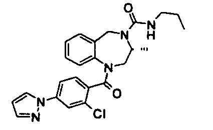 | MS(ESI, m/z) 452(M+H)+ |
| Ex 1-4 | 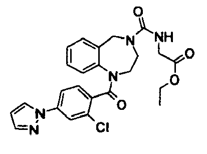 | MS(ESI, m/z) 482(M+H)+ |
| Ex 1-5 | 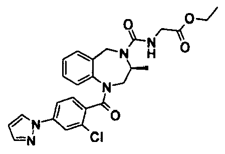 | MS(ESI, m/z) 496(M+H)+ |
| Ex 1-6 | 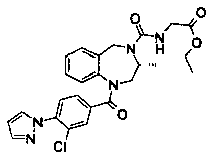 | 1H-NMR (CDCl3) δ ppm 1 15-160 (6H, m), 295-530 (9H, m), 640-800 (10H, m) MS(ESI, m/z) 496(M+H)+ |
| Ex 1-7 | 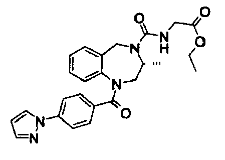 | 1H-NMR(CDCl3) δ ppm 1 10-160 (6H, m), 290-570 (9H, m), 6 40-7 90 (11H, m) MS(ESI, m/z) 461(M+)+ |
| Ex 1-8 | 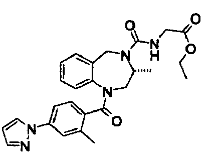 | MS(ESI, m/z) 476(M+)+ |
| Ex 1-9 | 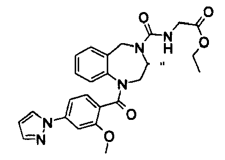 | MS(ESI, m/z) 492(M+H)+ |

**[Table 22]**

| No | Strc | Physical data |
|---|---|---|
| Ex 1-10 | 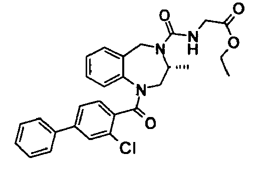 | MS(ESI, m/z) 506(M+H)+ |
| Ex 1-11 | 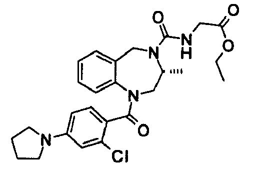 | MS(ESI, m/z). 499(M+H)+ |
| Ex. 1-12 | 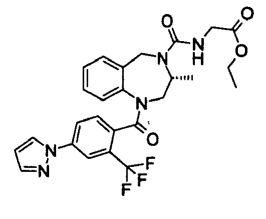 | 1H-NMR (CDCl3) δ ppm 115-1 65 (6H, m), 2.95-3 35 (1H, m), 3.60-5 35 (8H, m), 6 40-8.30 (10H, m) |
| Ex. 1-13 | 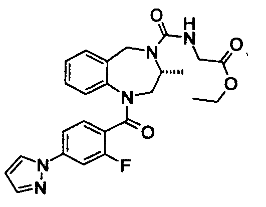 | 1H-NMR (CDCl3) δ ppm. 1.20-1 60 (6H, m), 2 90-3 30 (1H, m), 3 65-5.25 (8H, m), 6.40-7 95 (10H, m). MS(ESI, m/z) 480(M+H)+ |
| Ex. 1-14 | 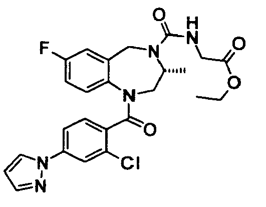 | 1H-NMR (CDCl3) δ ppm. 1 15-1 65 (6H, m), 2.90-5.35 (9H, m), 6 40-8 10 (9H, m) MS(ESI, m/z) 514(M+H)+ |
| Ex. 1-15 | 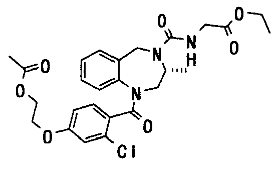 | 1H-NMR (CCl3) δ ppm 0 85-1 65 (6H, m), 1 80-2.20 (3H, m), 2 85-5 40 (14H, m), 6 20 -7 80 (7H, m) |

**[Table 23]**

| No | Strc | Physical data |
|---|---|---|
| Ex 1-16 | 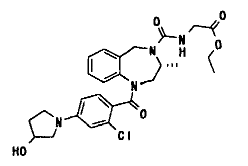 | MS(ESI, m/z) 515(M+H)⁺ |
| Ex 1-17 | 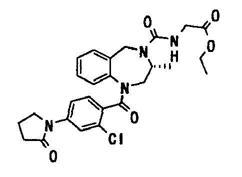 | MS(ESI, m/z) 513(M+H)⁺ |
| Ex 1-18 | 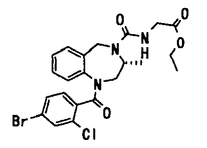 | 1H-NMR (CDCl₃) δ ppm 1 00-1 60 (6H, m), 2 80-5 40 (9H, m), 6.30-7 80 (7H, m) MS(ESI, m/z). 508(M+H)+ |
| Ex 1-19 | 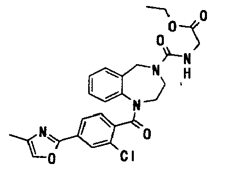 | MS(ESI, m/z) 511(M+H)+ |
| Ex 1-20 | 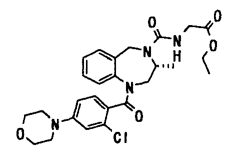 | MS(ESI, m/z) 515(M+H)+ |
| Ex 1-21 | 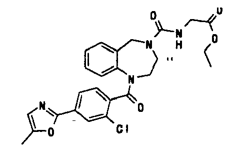 | MS(ESI, m/z) 511M+H)+ |
| Ex 1-22 | 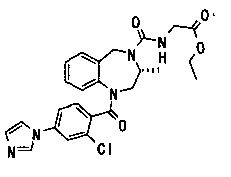 | MS(ESI, m/z) 496(M+H)+ |

**[Table 24]**

| No | Strc | Physical data |
|---|---|---|
| Ex. 1-23 | 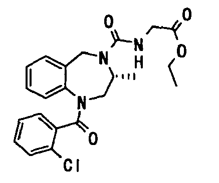 | 1H-NMR (CDCl3) δ ppm 0 80-1 80 (6H, m), 2 80-5 50 (9H, m), 6.40-8 00 (8H, m) MS(ESI, m/z) 430(M+H)+ |
| Ex 1-24 | 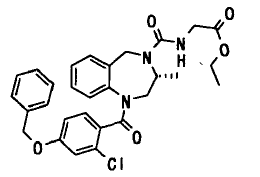 | MS(ESI, m/z): 508(M+H)+ |
| Ex 1-25 | 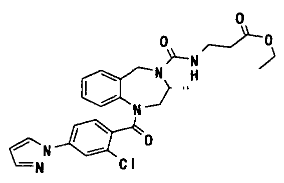 | MS(ESI, m/z) :510(M+H)+ |
| Ex 1-26 | 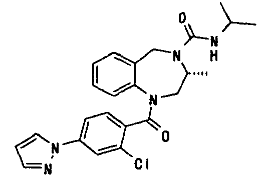 | 1H-NMR (CDCl3) δ ppm 0 90-1 60 (9H, m), 2 70-5 50 (6H, m), 6 40-8.00 (10H, m) MS(ESI, m/z) :452(M+H)+ |
| Ex 1 -27 | 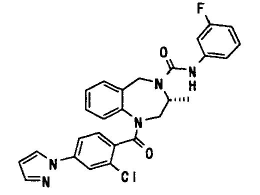 | 1H-NMR (CDCl3) δ ppm 1.05-1 80 (3H, m), 2 80-5 60 (5H, m), 6.40-8 00 (14H, m) MS(ESI, m/z) -504(M+H)+ |
| Ex 1-28 | 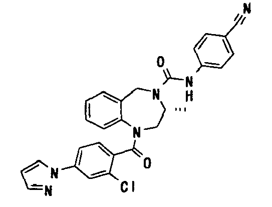 | 1H-NMR (CDCl3) δ ppm 1.05-1 80 (3H, m), 2.80-5 60 (5H, m), 6 20-8 20 (14H, m) |

**[Table 25]**

| No | Strc | Physical data |
|---|---|---|
| Ex 1-29 | 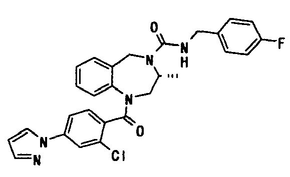 | 1H-NMR (CDCl3) δ ppm 1 00-1.70 (3H, m), 2 80-5 50 (7H, m), 6.40-8.20 (14H, m) MS(ESI, m/z) 518(M+H)+ |
| Ex 1-30 | 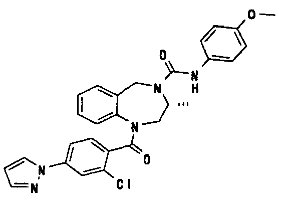 | 1H-NMR (CDCl3) δ ppm. 1 00-1 70 (3H, m), 2.80-5 50 (8H, m), 6 20-8.20 (14H, m) MS(ESI, m/z) : 516(M+H)+ |
| Ex 1-31 | 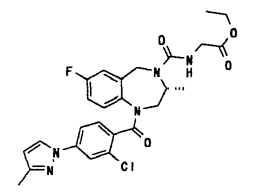 | 1H-NMR (CDCl3) δ ppm 1 00-1.65 (6H, m), 2 30-2 40 (3H, m), 2.95-5 40 (9H, m), 6 20-8 00 (8H, m) |
| Ex 1-32 | 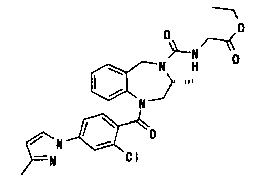 | MS(ESI, m/z) 510(M+H)+ |
| Ex 1-33 | 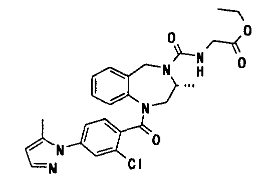 | 1H-NMR (CDCl3) δ ppm: 1 00-1 60 (6H, m), 2.30 (3H, s), 2 90-5.30 (9H, m), 6.10-8 00 (9H, m) MS(ESI, m/z) 510(M+H)+ |
| Ex 1-34 | 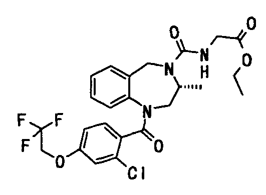 | 1H-NMR (CDCl3) δ ppm: 1 00-1 65 (6H, m), 2 80-5 50 (11H, m), 640-780 (7H, m) MS(ESI, m/z) 528(M+H)+ |

**[Table 26]**

| No | Strc | Physical data |
|---|---|---|
| Ex 1-35 | 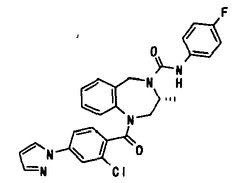 | 1H-NMR (CDCl3) δ ppm 1 05-1 75 (3H, m), 2 75-5 65 (5H, m), 6 15-7 50 (7H, m) MS(ESI, m/z) 504(M+H)+ |
| Ex 1-36 | 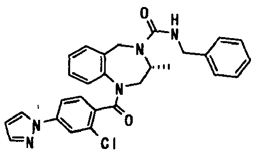 | 1H-NMR (CDCl3) δ ppm 1 00-1 65 (3H, m), 2 95-5 35 (7H, m), 6 40-8 15 (15H, m) MS(ESI, m/z) 500(M+H)+ |
| Ex. 1-37 | 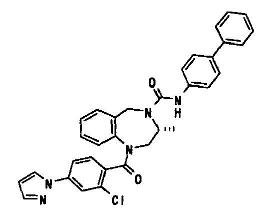 | 1H-NMR (DMSO-d6) δ ppm 1.20-1 55 (3H, m), 2 95-3 60 (1H, m), 4 45-5 35 (4H, m), 6 45-8 90 (19H, m) MS(ESI, m/z) 562(M+H)+ |
| Ex 1-38 | 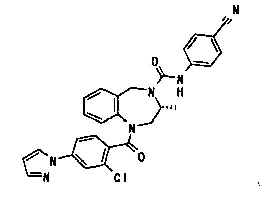 | 1H-NMR (DMSO-d6) δ ppm 1.20-1 55 (3H, m), 2 90-3 60 (1H, m), 4.10-5 35 (4H, m), 6 50-9 50 (14H, m) MS(ESI, m/z) 511(M+H)+ |
| Ex 1-39 | 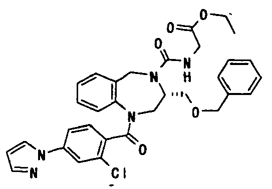 | 1H-NMR (CDCl3) δ ppm 1 20-1 30 (3H, m), 3 00-5 20 (13H, m), 5 35-6 15 (1H, m), 6 40-8 00 (15H, m) |
| Ex 1-40 | 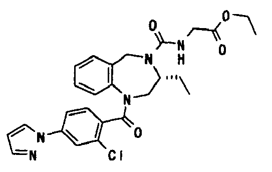 | 1H-NMR (CDCl3) δ ppm 1 05-1 20 (3H, m), 1 20-1 30 (3H, m), 1 60-1 80 (1H, m), 1 90-2 10 (1H, m), 300-5 50 (9H, m), 645-8 00 (10H, m) MS(ESI, m/z) 510(M+H)+ |

**[Table 27]**

| No | Strc | Physical data |
|---|---|---|
| Ex 1-41 | 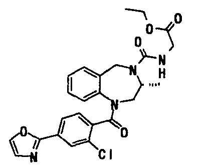 | 1H-NMR (CDCl3) δ ppm 1 00-1.70 (6H, m), 270-540 (9H, m), 620-820 (9H, m) |
| Ex 1-42 | 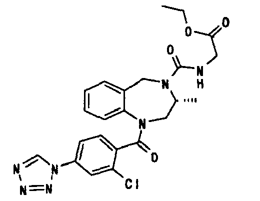 | 1H-NMR (CDCl3) δ ppm 1 05-1 70 (6H, m), 295-525 (9H, m), 620-830 (7H, m), 890-915 (1H, m) MS(ESI, m/z) 498(M+H)+ |
| Ex 1-43 | 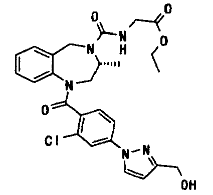 | 1H-NMR (CDCl3) δ ppm 0 75-1 70 (6H, m), 1 90-2 10 (1H, m), 2 95-5 45 (11H, m), 6 40-8 10 (9H, m) MS(ESI, m/z) 526(M+H)+ |
| Ex 1-44 | 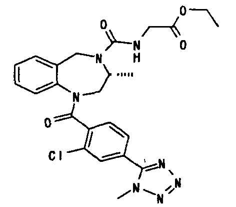 | 1H-NMR (CDCl3) δ ppm 1 00-1 85 (6H, m), 2 85-5 45 (12H, m), 6 30-815 (7H, m) MS(ESI, m/z) 512(M+H)+ |
| Ex 1-45 | 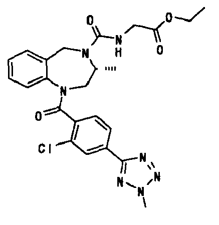 | 1H-NMR (CDCl3) δ ppm 100-170 (6H, m), 2 80-5 25 (12H, m), 650-830 (7H, m) MS(ESI, m/z) 512(M+H)+ |
| Ex 1-46 | 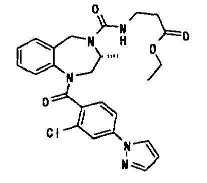 | 1H-NMR (CDCl3) δ ppm 1 00-1 60 (6H, m), 2 40-2 60 (2H, m), 280-570 (10H, m), 6 40-8 00 (10H, m) MS(ESI, m/z) 510(M+H)+ |

**[Table 28]**

| No | Strc | Physical data |
|---|---|---|
| Ex 1-47 | 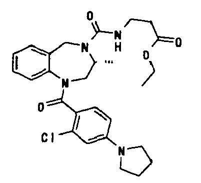 | 1H-NMR (CDCl3) δ ppm 1 00-1 60 (6H, m), 1 90-2 05 (4H, m), 2 40-2 55 (2H, m), 2 70-5 50 (13H, m), 6 00-7 50 (7H, m) |
| Ex 1-48 | 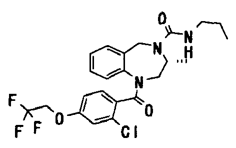 | 1H-NMR (CDCl3) δ ppm D 70-1 60 (8H, m), 2 80-5 40 (9H, m), 6 30-7 50 (7H, m) MS(ESI, m/z) 484(M+H)+ |
| Ex 1-49 | 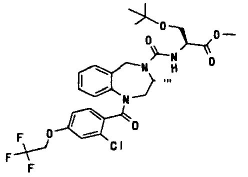 | 1H-NMR (DMSO-d6) δ ppm D 90-1 45 (12H, m), 2 70-5 05 (13H, m), 6 20-7 60 (7H, m) MS(ESI, m/z) 600(M+H)+ |
| Ex 1-50 | 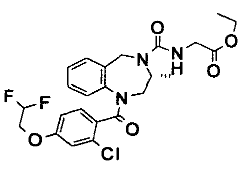 | 1H-NMR (CDCl3) δ ppm 1 00-160 (6H, m), 2 90-5 40 (11H, m), 6 03 (1H, tt, J=54 9,4 41Hz), 620-770 (7H, m) |
| Ex 1-51 | 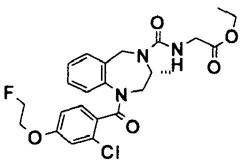 | 1H-NMR (CDCl3) δ ppm 1 00-160 (6H, m), 2 80-5 60 (13H, m), 6 35-7.75 (7H, m) MS(ESI, m/z) 492(M+H)+ |
| Ex 1-52 | 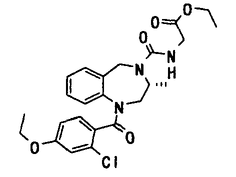 | 1H-NMR (CDCl3) δ ppm 080-170 (9H, m), 260-530 (11H, m), 6 40-7 20 (7H, m) MS(ESI, m/z) 474(M+H)+ |
| Ex 1 -53 | 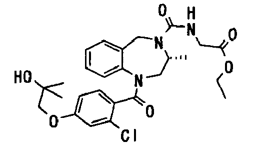 | 1H-NMR (CDCl3) δ ppm 1 00-1 75 (12H, m), 2 00-2 20 (1H, m), 2 70-5 30 (11H, m), 6 30-7 80 (7H, m) MS(ESI, m/z) 518(M+H)+ |

### Example 2

### N-Methyl-N-propyl-(R)-1-(2-chloro-4-pyrazol-1-ylbenzoyl)-3-methyl-1,2,3,5-tetrahydrobenzo[e]-1,4-diazepin-4-carboxamide

To a solution of N-propyl-(R)-1-(2-chloro-4-pyrazol-1-ylbenzoyl)-3-methyl-1,2,3,5-tetrahydrobenzo[e]-1,4-diazepine (0.0200 g) in tetrahydrofuran (0.20 mL) was added sodium hydride (dispersion in oil ca 60% : 0.0035 g) under ice-cooling and the mixture was stirred at room temperature for an hour. To the reaction mixture was added iodomethane (0.0055 mL) under ice-cooling and the mixture was stirred at room temperature for 18 hours. To the reaction mixture was added water, and the mixture was extracted with ethyl acetate. The organic layer was washed with brine, dried over anhydrous magnesium sulfate. After filtration, the filtrate was concentrated under reduced pressure. The obtained crude product was purified by column chromatography on silica gel (eluent: ethyl acetate-hexane) to give N-methyl-N-propyl-(R)-1-(2-chloro-4-pyrazol-1-ylbenzoyl)-3-methyl-1,2,3,5-tetrahydrobenzo[e]-1,4-diazepin-4-carboxamide (0.0151 g).
1H-NMR(CDCl3) δ ppm:
0.70-0.95 (3H, m), 1.20-1.70 (5H, m), 2.70-2.90 (3H, m), 2.95-3.30 (3H, m), 4.05-5.10 (4H, m), 6.40-6.55 (1H, m), 6.55-8.00 (9H, m).
MS(ESI, m/z) : 466(M+H)+

### Example 3-1

### {[(R)-1-(2-Chloro-4-pyrazol-1-ylbenzoyl)-3-methyl-1,2,3,5-tetrahydrobenzo[e]-1,4-diazepin-4-carbonyl]amino}acetic acid

To a solution of ethyl {[(R)-1-(2-chloro-4-pyrazol-1-ylbenzoyl)-3-methyl-1,2,3,5-tetrahydrobenzo[e]-1,4-diazepin-4-carbonyl]-amino}acetate (0.0200g) inethanol (0.20mL) was added 5 mol/L aqueous solution of sodium hydroxide (0.0177 mL) at room temperature, and the mixture was stirred at room temperature for 24 hours. To the reaction mixture was added 1 mol/L hydrochloric acid (0.100 mL) and the mixture was extracted with ethyl acetate. The organic layer was washed with water and brine, dried over anhydrous magnesium sulfate. After filtration, the filtrate was concentrated under reduced pressure to give {[(R)-1-(2-chloro-4-pyrazol-1-ylbenzoyl)-3-methyl-1,2,3,5-tetrahydrobenzo[e]-1,4-diazepin-4-carbonyl]-amino}acetic acid (0.0156 g).
1H-NMR(CDCl3) δ ppm:
1.10-1.85 (3H, m), 2.85-3.40 (1H, m), 3.50-4.00 (2H, m), 4.20-6.10 (6H, m), 6.35-8.05 (10H, m).
MS (ESI, m/z) : 468 (M+H) +

### Examples 3-2 to 3-53

The following compounds of Examples 3-2 to 3-53 were obtained with the use of the corresponding ester derivatives in a similar manner to that described in Example 3-1. The structure formula and physical data of these compounds were shown in Tables 29 to 36.

**[Table 29]**

| No | Strc | Physical data |
|---|---|---|
| Ex 3-2 | 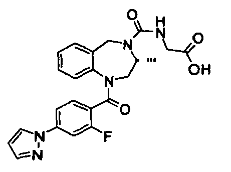 | MS(ESI, m/z) 452(M+H)+ |
| Ex 3-3 | 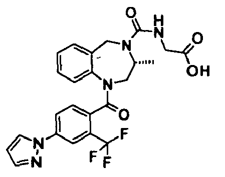 | MS(ESI, m/z) 502(M+H)+ |
| Ex 3-4 | 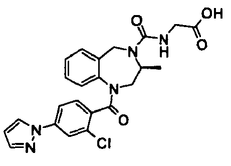 | MS(ESI, m/z) 468(M+H)+ |
| Ex 3-5 | 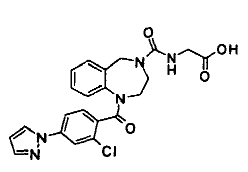 | MS(ESI, m/z) 454(M+H)+ |
| Ex 3-6 | 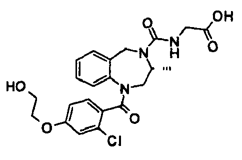 | MS(ESI, m/z) 462(M+H)+ |
| Ex 3-7 | 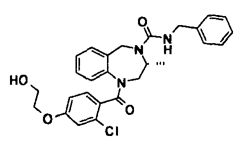 | MS(ESI, m/z) 494(M+H)+ |
| Ex 3-8 | 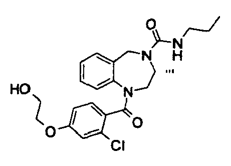 | MS(ESI, m/z) 446(M+H)+ |

**[Table 30]**

| No | Strc | Physical data |
|---|---|---|
| Ex 3-9 | 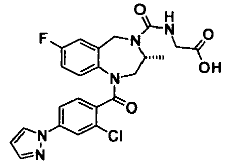 | MS(ESI, m/z) 486(M+H)+ |
| Ex 3-10 | 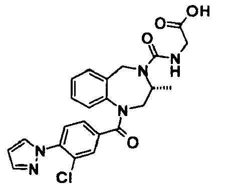 | MS(ESI, m/z) 468(M+H)+ |
| Ex 3-11 | 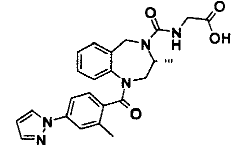 | MS(ESI, m/z) 448(M+H)+ |
| Ex 3-12 | 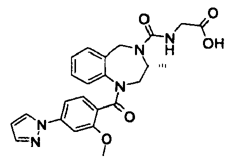 | MS(ESI, mlz) 464(M+H)+ |
| Ex 3-13 | 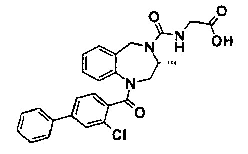 | MS(ESI, m/z) 478(M+H)+ |
| Ex 3-14 | 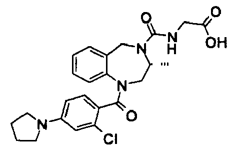 | MS(ESI, m/z) 471 (M+H)+ |
| Ex 3-15 | 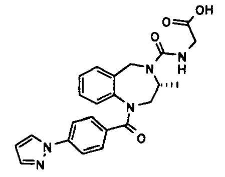 | MS(ESI, m/z) 434(M+H)+ |

**[Table 31]**

| No | Strc | Physical data |
|---|---|---|
| Ex 3-16 | 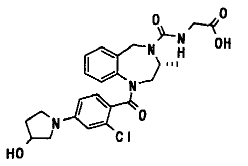 | MS(ESI, m/z) 487(M+H)⁺ |
| Ex 3-17 | 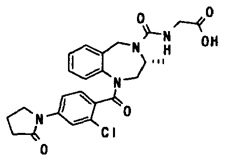 | 1H-NMR (CDCl3) 6 ppm 0 80-1.40 (5H, m), 2 00-2 20 (2H, m), 2 40-2 70 (2H, m), 2 80-5 70 (7H, m), 6 30-8 00 (7H, m) MS(ESI, m/z) 485(M+H)+ |
| Ex 3-18 | 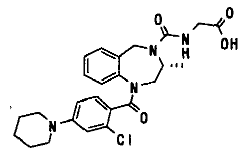 | MS(ESI, m/z). 485M+H)+ |
| Ex 3-19 | 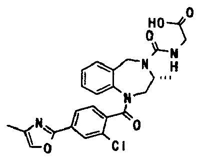 | MS(ESI, m/z) 483(M+H)+ |
| Ex 3-20 | 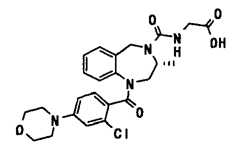 | MS(ESI, m/z) 487(M+H)+ |
| Ex 3-21 | 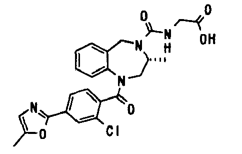 | MS(ESI, m/z) 483(M+H)+ |
| Ex 3-22 | 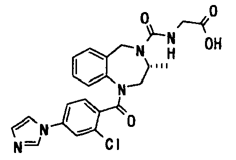 | MS(ESI, m/z) 468(M+H)+ |

**[Table 32]**

| No | Strc | Physical data |
|---|---|---|
| Ex 3-23 | 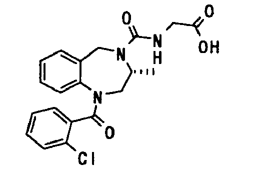 | 1H-NMR (CDCl3) δ ppm 0 80-1 60 (3H, m), 2 80-6.40 (7H, m), 6.40-8.00 (8H, m) MS(ESI, m/z) 402(M+H)+ |
| Ex 3-24 | 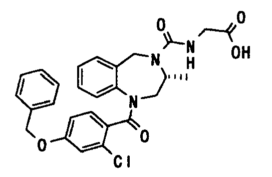 | MS(ESI, m/z): 536(M+H)+ |
| Ex 3-25 | 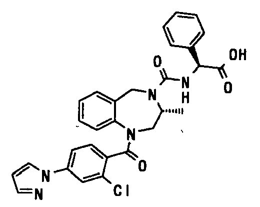 | MS(ESI, m/z) 544(M+H)+ |
| Ex 3-26 | 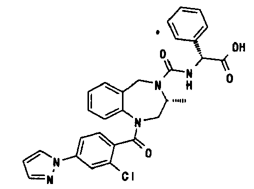 | MS(ESI, m/z) 544(M+H)+ |
| Ex 3-27 | 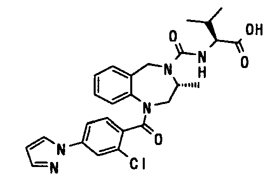 | MS(ESI, m/z) .510(M+H)+ |
| Ex 3-28 | 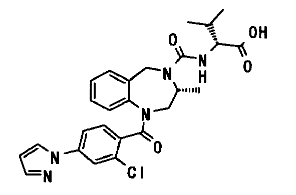 | MS(ESI, m/z) 510(M+H)+ |

**[Table 33]**

| No | Strc | Physical data |
|---|---|---|
| Ex 3-29 | 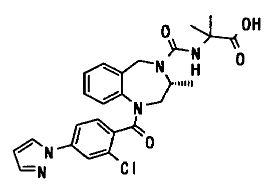 | MS(ESI, m/z) 496(M+H)+ |
| Ex 3-30 | 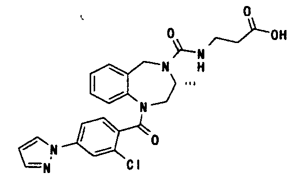 | MS(ESI, m/z) 482(M+H)+ |
| Ex 3-31 | 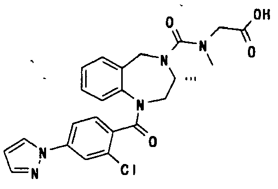 | MS(ESI, m/z) 482(M+H)+ |
| Ex 3-32 | 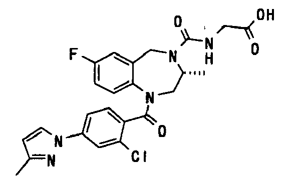 | 1H-NMR (DMSO-d6) δ ppm 1.10-1.45 (3H, m), 2 24 (3H, s), 2 80-5 25 (7H, m), 6 30-8 50 (8H, m), 12 20-12 50 (1H, m) |
| Ex 3-33 | 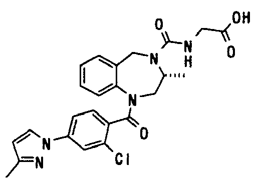 | 1H-NMR (DMSO-d6) δ ppm: 1.10-1 50 (3H, m), 2 23 (3H, s), 2 80-5.20 (7H, m), 6.30-8.45 (9H, m), 12 20-12.50 (1H, m) MS(ESI, m/z) 482(M+H)+ |
| Ex 3-34 | 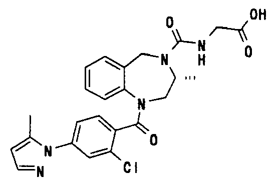 | MS(ESI, m/z) 482(M+H)+ |

**[Table 34]**

| No. | Strc | Physical data |
|---|---|---|
| Ex. 3-35 | 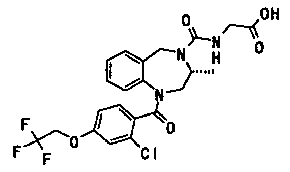 | 1H-NMR (CDCl3) δ ppm: 1.00-1.60 (3H, m), 2.50-6.20 (10H, m), 6.30-7.80 (7H, m) MS(ESI, m/z) 500(M+H)+ |
| Ex 3-36 | 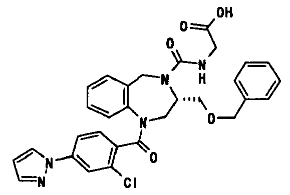 | MS(ESI, m/z) :574(M+H)+ |
| Ex 3-37 | 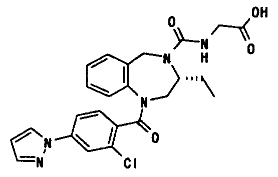 | 1H-NMR (CDCl3) δ ppm: 1.05-1 15 (3H, m), 1.60-2.15 (2H, m), 2.90-5 60 (7H, m), 6 40-8.00 (10H, m) MS(ESI, m/z) 482(M+H)+ |
| Ex 3-38 | 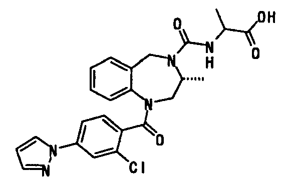 | 1H-NMR (CDCl3) δ ppm: 1.25-1 65 (6H, m), 2.95-5.50 (6H, m), 6.40-800 (10H, m) MS(ESI, m/z):482(M+H)+ |
| Ex 3-39 | 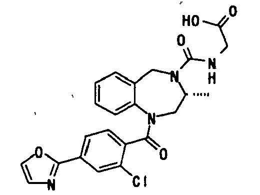 | 1H-NMR (CDCl3) δ ppm: 1.00-1.60 (3H, m), 2.95-8.20 (17H, m) |
| Ex 3-40 | 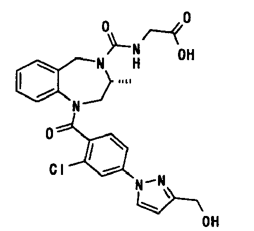 | 1H-NMR (DMSO-d6) δ ppm: 1 10-1.45 (3H, m), 3.20-5.20 (9H, m), 6 45-8.00 (8H, m), 8 40-8.50 (1H, m), 12 25-12.45 (1H, m) MS(ESI, m/z). 498(M+H)+ |

**[Table 35]**

| No | Strc | Physical data |
|---|---|---|
| Ex. 3-41 | 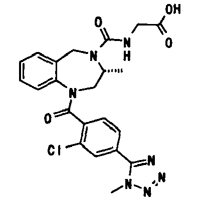 | MS(ESI, m/z): 482(M-H)- |
| Ex 3-42 | 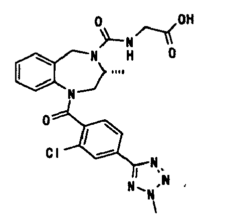 | 1H-NMR (CDCl3) δ ppm 1 00-1.65 (3H, m), 2 90-5 55 (10H, m), 6 50-8 30 (7H, m) MS(ESI, m/z) 484(M+H)+ |
| Ex 3-43 | 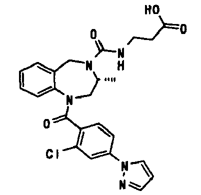 | 1H-NMR (CDCl3) δ ppm. 0.80-1 60 (3H, m), 2 35-2.65 (2H, m), 2 80-5 90 (8H, m), 6 40-8 05 (10H, m) MS(ESI, m/z) .482(M+H)+ |
| Ex 3-44 | 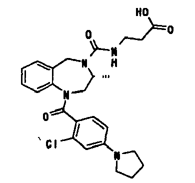 | 1H-NMR (CDCl3) δ ppm 0.90-1 60 (3H, m), 1 85-2 05 (4H, m), 2 40-2.55 (2H, m), 3 00-6 00 (12H, m), 6 00-7 70 (7H, m) |
| Ex 3-45 | 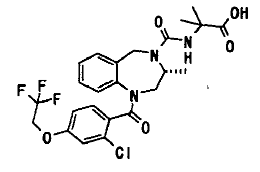 | 1H-NMR (CDCl3) δ ppm: 0.90-1 80 (9H, m), 2 80-5 80 (7H, m), 6 20-8 00 (7H, m) MS(ESI, m/z) 528(M+H)+ |
| Ex. 3-46 | 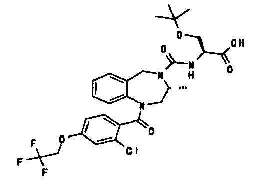 | 1H-NMR (DMSO-d6) δ ppm 0 80-1 45 (12H, m), 2 70-5 05 (10H, m), 6 0-7 60 (7H, m), 12 00-13 00 (1H, br) MS(ESI, m/z) 586(M+H)+ |

**[Table 36]**

| No | Strc | Physical data |
|---|---|---|
| Ex 3-47 | 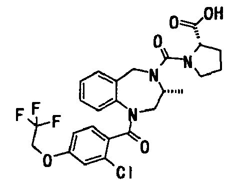 | 1H-NMR (DMSO-d6) δ ppm 0 80-2.40 (7H, m), 2 90-5 00 (10H, m), 6 40-7 60 (7H, m), 12 00-12.50 (1H, br) MS(ESI, m/z) 540 (M+H)+ |
| Ex 3-48 | 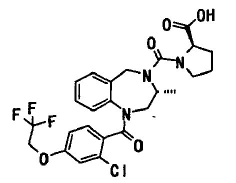 | 1H-NMR (DMSO-d6) δ ppm 0.80-2 40 (7H, m), 2.70-5 00 (10H, m), 6 40-7 60 (7H, m), 12 00-12 50 (1H, br) MS(ESI, m/z). 540(M+H)+ |
| Ex 3-49 | 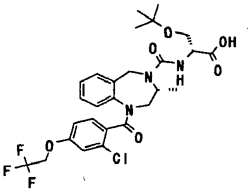 | 1H-NMR (DMSO-d6) δ ppm 0 80-1 50 (12H, m), 2 70-5 00 (10H, m), 6.00-7.80 (7H, m), 12 00-13 50 (1H, m) MS(ESI, m/z) : 586(M+H)+ |
| Ex 3-50 | 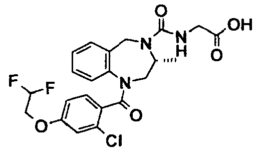 | 1H-NMR (CDCl3) δ ppm 1 00-1.60 (3H, m), 2 90-560 (9H, m), 5 65-6.20 (2H, m), 6 30-7 70 (7H, m) |
| Ex 3-51 | 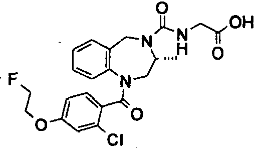 | 1H-NMR (DMSO-d6) δ ppm 0 90-1.45 (3H, m), 2 70-5 00 (11H, m), 6 35-7 80 (8H, m), 12 15-12 45 (1H, m) |
| Ex 3-52 | 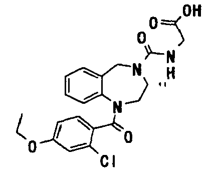 | 1H-NMR (DMSO-d6) δ ppm 0 80-1.50 (6H, m), 2 60-5 10 (9H, m), 640-770 (7H, m), 12 10-12 60 (1H, m) MS(ESI, m/z) 446(M+H)+ |
| Ex 3-53 | 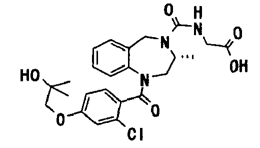 | MS(ESI, m/z) 490(M+H)+ |

### Example 4

### N-(2-Hydroxyethyl)-(R)-1-(2-chloro-4-pyrazol-1-ylbenzoyl)-3-methyl-1,2,3,5-tetrahydrobenzo[e]-1,4-diazepin-4-carboxamide.

To a solution of { [(R)-1-(2-chloro-4-pyrazol-1-ylbenzoyl)-3-methyl-1,2,3,5-tetrahydrobenzo[e]-1,4-diazepin-4-carbonyl]amino }acetic acid (0.0479 g) and N,N-diisopropylethylamine (0.0232 mL) in tetrahydrofuran (1.0 mL) was added isobutyl chloroformate (0.0148 mL) under ice-cooling, and the mixture was stirred at room temperature for an hour. To this reaction mixture was added lithium tetrahydroborate (0.0067 g) under ice-cooling, and the mixture was stirred at room temperature for an hour. To the reaction mixture was added water, and the organic layer extracted with ethyl acetate was washed with brine, dried over anhydrous magnesium sulfate. After filtration, the filtrate was concentrated under reduced pressure. The obtained crude product was purified by column chromatography on silica gel (eluent:ethyl acetate-hexane) to give N-(2-hydroxyethyl)-(R)-1-(2-chloro-4-pyrazol-1-ylbenzoyl)-3-methyl-1,2,3,5-tetrahydrobenzo[e]-1,4-diazepin-4-carboxamide (0.0324 g).
1H-NMR(CDCl3)δ ppm:
1.20-1.70 (3H, m)', 2.85-3.75 (6H, m), 4.20-5.30 (4H, m), 6.40-8.00 (10H, m).
MS(ESI, m/z) : 454(M+H)+

### Example 5-1

### N-Carbamoylmethyl-(R)-3-methyl-1-(4-pyrazol-1-ylbenzoyl)-1,2,3,5-tetrahydrobenzo[e]-1,4-diazepin-4-carboxamide

A suspension of {[(R)-3-methyl-1-(4-pyrazol-1-ylbenzoyl)-1,2,3,5-tetrahydrobenzo[e]-1, 4-diazepin-4-carbonyl]amino}acetic acid (0. 0427 g), ammonium chloride (0. 0158 g), hydroxybenzotriazole monohydrate (0.0256 g) and N,N-diisopropylethylamine (0.0600 mL) in N,N-dimethylformamide (2.0 mL) was stirred at room temperature for 15 minutes. To the reaction mixture was added 1-ethyl-3-(3-dimethylaminopropyl)carbodiimide hydrochloride (0.0321 g), and this mixture was stirred at room temperature for 3 days. To the reaction mixture were added water, ethyl acetate and the organic layer was separated. The aqueous layer was extracted with ethyl acetate. The combined organic layer was washed with water and brine, dried over anhydrous magnesium sulfate. After filtration, the filtrate was concentrated under reduced pressure. The obtained crude product was purified by column chromatography on aminopropylsilylated silica gel (eluent:ethyl acetate-methanol) to give N-carbamoylmethyl-(R)-3-methyl-1-(4-pyrazol-1-ylbenzoyl)-1,2,3,5-tetrahydrobenzo[e]-1,4-diazepin-4-carboxamide (0.018 g).
1H-NMR(CDCl3)δ ppm:
1.20-1.55 (3H, m), 2.90-3.30 (1H, m), 3.70-3.85 (2H, m), 4.30-5.70 (6H, m), 6.00-8.00 (11H, m).
MS (ESI, m/z) : 433(M+H)+

### Examples 5-2 to 5-80

The following compounds of Examples 5-2 to 5-80 were obtained with the use of the corresponding carboxylic acid derivatives and amine derivatives or the salts thereof in a similar manner to that described in Example 5-1. The structure formula and physical data of these compounds were shown in Tables 37 to 50.

**[Table 37]**

| No | Strc | Physical data |
|---|---|---|
| Ex 5-2 | 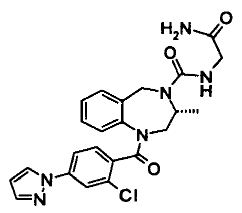 | MS(ESI, m/z). 489(M+Na)+ |
| Ex 5-3 | 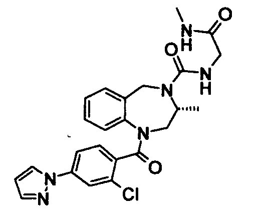 | MS(ESI, m/z): 479(M-H)- |
| Ex 5-4 | 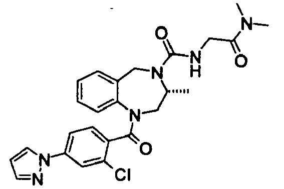 | MS(ESI, m/z): 495(M+H)+ |
| Ex. 5-5 | 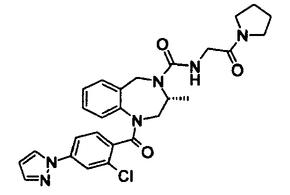 | MS(ESI, m/z). 521(M+H)+ |
| Ex. 5-6 | 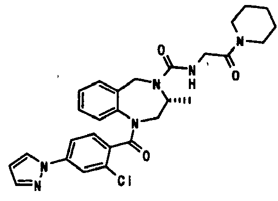 | MS(ESI, m/z) 535(M+H)+ |
| Ex 5-7 | 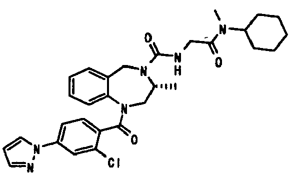 | MS(ESI, m/z) 563(M+H)+ |

**[Table 38]**

| No | Strc | Physical data |
|---|---|---|
| Ex. 5-8 | 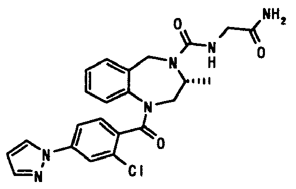 | MS(ESI, m/z): 453(M+H)+ |
| Ex 5-9 | 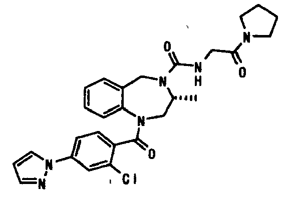 | MS(ESI, m/z) 507(M+H)+ |
| Ex. 5-10 | 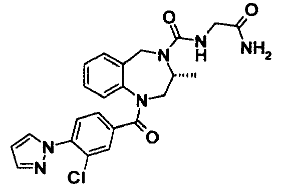 | 1H-NMR (CDCl3) δ ppm: 1.25-155 (3H, m), 2 90-3 30 (1H, m), 3.65-4 05 (2H, m), 4.30-5 70 (6H, m), 6.05-8.00 (11H, m) MS(ESI, m/z) 467(M+H)+ |
| Ex 5-11 | 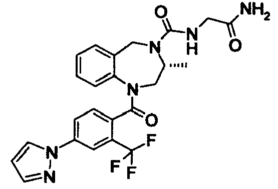 | 1H-NMR (CDCl3) δ ppm: 1 20-1.55 (3H, m), 2.95-3.35 (1H, m), 3 50-5.90 (8H, m), 6 20-8 25 (10H, m). MS(ESI, m/z) 501(M+H)+ |
| Ex. 5-12 | 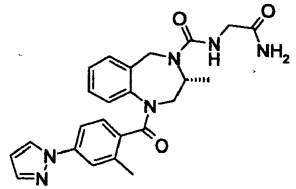 | 1H-NMR (CDCl3) δ ppm 1.20-1 45 (3H, m), 2.30-5.70 (12H, m), 6.20-8.00 (10H, m). MS (ESI, m/z) 447(M+H)+ |
| Ex 5-13 | 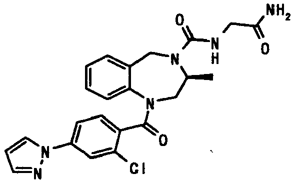 | MS(ESI, m/z): 467(M+H)+ |

**[Table 39]**

| No. | Strc | Physical data |
|---|---|---|
| Ex 5-14 | 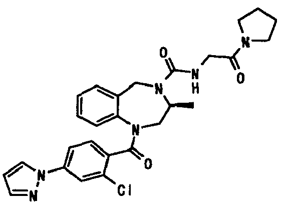 | MS(ESI, m/z) 521(M+H)+ |
| Ex 5-15 | 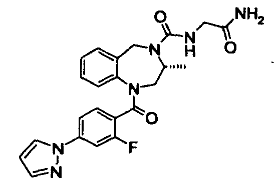 | 1H-NMR (CDCl3) 6 ppm 1.20-1.55 (3H, m), 2.95-3.30 (1H, m), 3.89 (1H, d, J=4.7Hz), 4 20-6 35 (6H, m), 6 40-8.10 (10H, m). MS(ESI, m/z): 451 (M+H)+ |
| Ex. 5-16 | 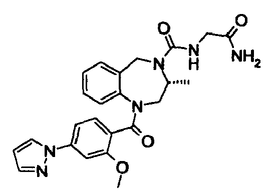 | 1H-NMR (CDCl3) δ ppm: 1 20-1.60 (3H, m), 2.80-5.60 (12H, m), 5.90-8 10 (10H, m) MS(ESI, m/z) : 463(M+H)+ |
| Ex. 5-17 | 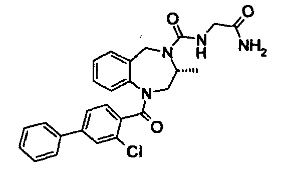 | 1H-NMR (CDCl3) δ ppm: 1 20-1.70 (3H, m), 2 90-3 40 (1H, m), 3 80-5 60 (8H, m), 6 00-8.00 (12H, m). MS(ESI, m/z) : 477(M+H)+ |
| Ex 5-18 | 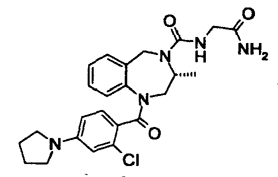 | 1H-NMR (CDCl3) δ ppm: 1 20-1 60 (3H, m), 1.90-2 00 (4H, m), 3.00-8 00 (20H, m). MS(ESI, m/z): 470(M+H)+ |
| Ex. 5-19 | 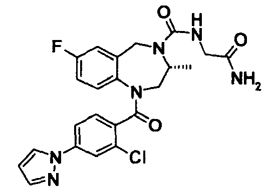 | 1H-NMR (CDCl3) δ ppm: 1.20-2 00 (3H, m), 2 80-5 95 (9H, m), 6 00-8.15 (10H, m) MS(ESI, m/z). 485(M+H)+ |

**[Table 40]**

| No | Strc | Physical data |
|---|---|---|
| Ex. 5-20 | 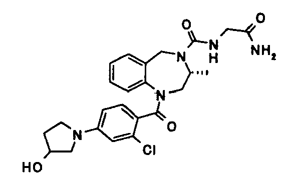 | MS(ESI, m/z) 486(M+H)⁺ |
| Ex. 5-21 | 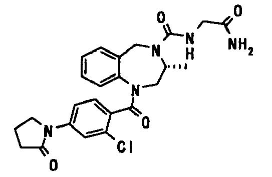 | SMS(ESI, m/z) 484(M+H)+ |
| Ex 5-22 | 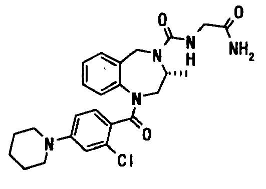 | 1H-NMR (CDCl3) δ ppm: 0 80-1 80 (7H, m), 2.80-5 80 (13H, m), 6 10-7 70 (7H, m) MS(ESI, m/z) : 484(M+H)+ |
| Ex. 5-23 | 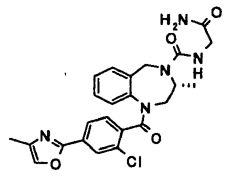 | 1H-NMR (CDCl3) δ ppm 0 80-1.70 (3H, m), 2.10-2 30 (3H, m), 2 80-5 80 (9H. m), 6 00-810 (8H, m) MS(ESI, m/z) 482(M+H)+ |
| Ex. 5-24 | 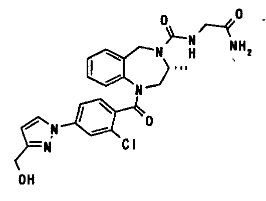 | 1H-NMR (DMSO-d6) δ ppm: 1 25-1.40 (4H, m), 3 55-4 20 (4H, m), 4 45-4 85 (7H, m), 6 40-6 50 (2H, m), 6 55-6 70 (2H, m), 6 90-7 15 (2H, m), 7 30-7.40 (1H, m), 7 50-7 65 (1H, m), 7 75-7 85 (1H, m), 8.30-8 35 (1H, m) MS(ESI, m/z). 497(M+H)+ |
| Ex. 5-25 | 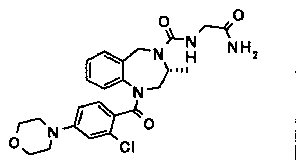 | 1H-NMR (CDCl₃) δ ppm. 1 00-1 60 (3H, m), 2.80-5 50 (15H, m), 6 10-7 70 (7H, m) MS(ESI, m/z) 486(M+H)+ |

**[Table 41]**

| No | Strc | Physical data |
|---|---|---|
| Ex 5-26 | 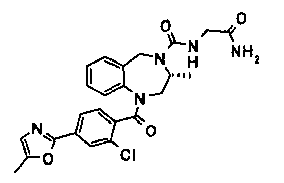 | 1H-NMR (CDCl3) δ ppm 1.00-1.60 (3H, m), 2.20-2.50 (3H, m), 2 80-6 00 (10H, m), 6.20-8.20 (8H, m) MS(ESI, m/z). 482(M+H)+ |
| Ex 5-27 | 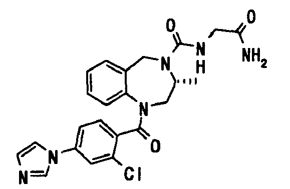 | MS(ESI, m/z), 467(M+H)+ |
| Ex. 5-28 | 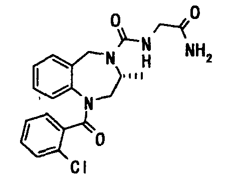 | 1H-NMR (CDCl3) δ ppm: 0.75-1.70 (3H, m), 2 80-5.90 (9H, m), 6.20-7 90 (8H, m) MS(ESI, m/z) 401(M+H)+ |
| Ex 5-29 | 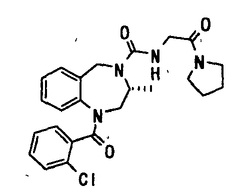 | 1H-NMR (CDCl3) δ ppm: 0 90-1.60 (3H, m), 1.75-2.05 (4H, m), 2.80-6.00 (12H, m), 6 40-7.85 (8H, m) MS(ESI, m/z) 455(M+H) + |
| Ex 5-30 | 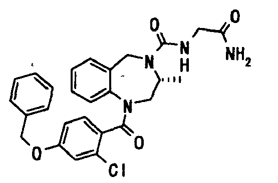 | 1H-NMR (CDCl3) δ ppm. 0.80-1.60 (3H, m), 2.80-6.00 (9H, m), 6 20-8 00 (12H, m) MS(ESI, m/z) 507(M+H)+ |
| Ex 5-31 | 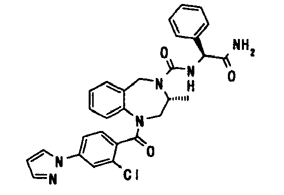 | 1H-NMR (DMSO-d6) δ ppm 0 90-1.50 (3H, m), 2.70-5.80 (6H, m), 6.30-8 90 (15H, m) MS(ESI, m/z) .543(M+H)+ |

**[Table 42]**

| No | Strc | Physical data |
|---|---|---|
| Ex 5-32 | 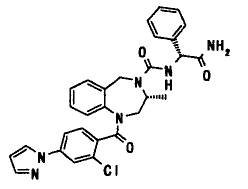 | MS(ESI, m/z) 543(M+H)+ |
| Ex. 5-33 | 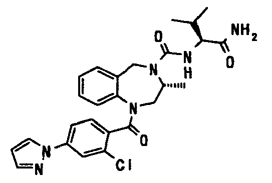 | 1H-NMR (CDCl3) δ ppm 0.60-1 60 (9H, m), 1 90-5.50 (7H, m), 6.30-8 00 (10H, m) MS(ESI, m/z) :509(M+H)+ |
| Ex 5-34 | 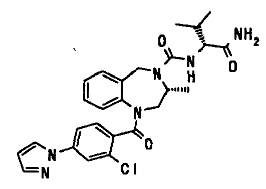 | 1H-NMR (CDCl3) δ ppm: 0 50-2.50 (10H, m), 2.70-5.80 (6H, m), 5.85-8.10 (11H, m) MS(ESI, m/z) 509(M+H)+ |
| Ex 5-35 | 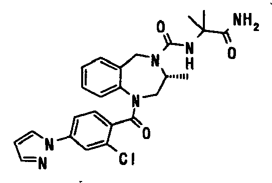 | 1H-NMR (CDCl3) δ ppm: 0 80-1.70 (9H ,m), 2.80-5.60 (5H, m), 6 00-8.30 (10H, m) MS(ESI, m/z) 495(M+H)+ |
| Ex. 5-36 | 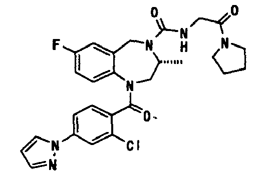 | 1H-NMR (CDCl3) δ ppm: 1.20-1.60 (3H, m), 1.80-2.05 (4H, m), 2.95-5.20 (11H, m), 5.60-5.85 (1H, m), 6.40-8.10 (9H, m) MS(ESI, m/z) .539(M+H)+ |
| Ex 5-37 | 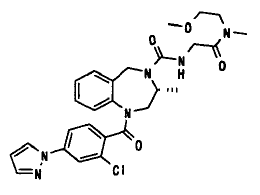 | 1H-NMR (CDCl3) δ ppm: 1.10-1 60 (3H, m), 2 90-5.20 (17H, m), 5 60-5 90 (1H, m), 6.40-8 00 (10H, m) MS(ESI, m/z) 539(M+H)+ |

**[Table 43]**

| No | Strc | Physical data |
|---|---|---|
| Ex. 5-38 | 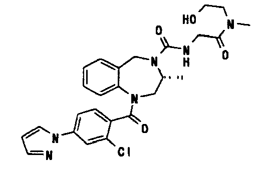 | 1H-NMR (CDCl3) δ ppm 1.10-1.60 (3H, m), 2.40-5.20 (14H, m), 5 55-5 90 (1H, m), 6.35-8 05 (10H, m) MS(ESI, m/z) 525(M+H)+ |
| Ex. 5-39 | 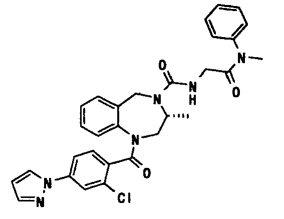 | 1H-NMR (CDCl3) δ ppm: 1.20-1.60 (3H, m), 290-5.10 (10H, m), 5.40-5 75 (1H, m), 6 40-8.00 (15H, m) |
| Ex 5-40 | 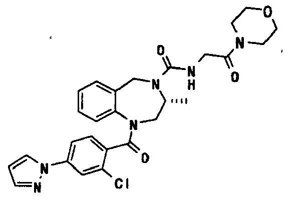 | 1H-NMR (CDCl3) δ ppm. 1 00-1.65 (3H, m), 2.85-5.30 (15H, m), 5 55-5.90 (1H, m), 6 40-8 00 (10H, m) MS(ESI, m/z) 537(M+H)+ |
| Ex 5-41 | 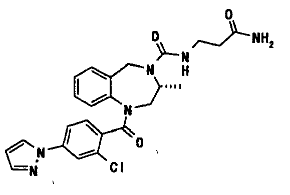 | 1H-NMR (CDCl3) δ ppm: 1.00-160 (3H, m), 2 00-6.20 (10H, m), 6 40-8 20 (10H, m) MS(ESI, m/z) : 481(M+H)+ |
| Ex 5-42 | 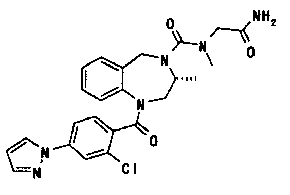 | 1H-NMR (CDCl3) δ ppm : 1 00-1.70 (3H, m), 2 80-5.50 (10H, m), 640-805 (10H, m) MS(ESI, m/z):481(M+H)+ |

**[Table 44]**

| No | Strc | Physical data |
|---|---|---|
| Ex 5-43 | 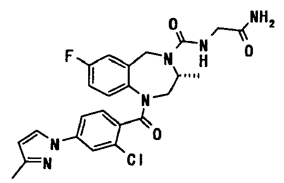 | 1H-NMR (CDCl3) δ ppm 1 00-1.75 (3H, m), 2.25-2 40 (3H, m), 2 90-5.90 (9H, m), 6.10-8 00 (8H, m) |
| Ex. 5-44 | 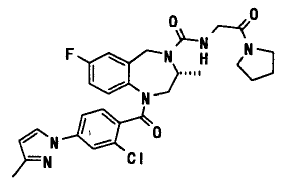 | 1H-NMR (CDCl3) δ ppm: 1.00-1.70 (3H, m), 1.80-2 05 (4H, m), 2.30-2.40 (3H, m), 2.90-5 20 (11H, m), 5.60-5.90 (1H, m), 6 20-8 00 (8H, m) |
| Ex 5-45 | 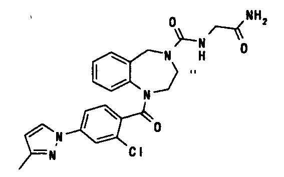 | 1H-NMR (CDCl3) δ ppm : 1.00-1.60 (3H, m), 2 30-2.40 (3H, m), 2 90-5.70 (8H, m), 6 00-8.00 (9H, m) MS(ESI, m/z) :481(M+H)+ |
| Ex. 5-46 | 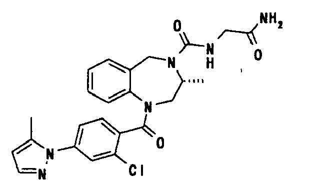 | 1H-NMR (CDCl3) δ ppm: 1 00-1.60 (3H, m), 2 25-2.45 (3H, m), 2.95-5.75 (8H, m), 6.05-8 05 (9H, m) MS(ESI, m/z) 481(M+H)+ |
| Ex 5-47 | 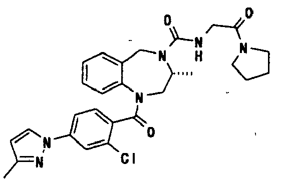 | 1H-NMR (CDCl3) δ ppm: 1.00-1 65 (3H, m), 1.80-2.05 (4H, m), 2.30-2.40 (3H, m), 2.90-5 20 (11H, m), 5 55-5.90 (1H, m), 5 90-7 90 (9H, m) MS(ESI, m/z) :535(M+H)+ |
| Ex 5-48 | 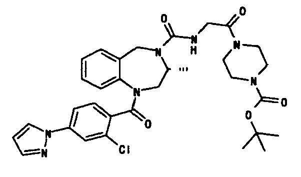 | 1H-NMR (CDCl3) δ ppm: 1 00-1.60 (12H, m), 2.90-5.20 (15H, m), 5 55-5.90 (1H, m), 6.40-8 00 (10H, m) MS(ESI, m/z) 636(M+H)+ |

**[Table 45]**

| No | Strc | Physical data |
|---|---|---|
| Ex 5-49 | 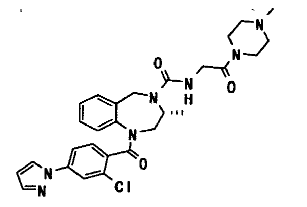 | 1H-NMR (CDCl3) δ ppm: 1.10-1.60 (3H, m), 2.30 (3H, s), 2 35-2.45 (4H, m), 2 80-5.20 (11H, m), 5 65-5 95 (1H, m), 6 40-8.20 (10H, m) MS(ESI, m/z) 550(M+H)+ |
| Ex 5-50 | 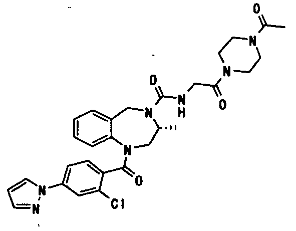 | 1H-NMR (CDCl3) δ ppm. 1.00-1 65 (3H, m), 2.10-2 15 (3H, m), 2 90-5.20 (15H, m), 5.50-5 85 (1H, m), 6 40-8.05 (10H, m) MS(ESI, m/z): 578(M+H)+ |
| Ex. 5-51 | 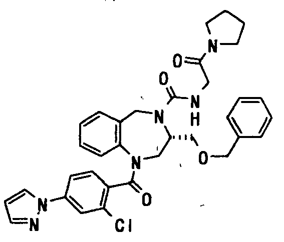 | 1H-NMR (CDCl3) δ ppm. 1 80-2 00 (4H, m), 3.00-5 20 (15H, m), 5.70-8.05 (16H, m) |
| Ex 5-52 | 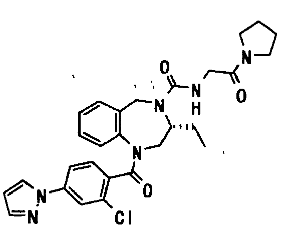 | 1H-NMR (CDCl3) δ ppm: 1.05-1.20 (3H, m), 1 80-2.10 (5H, m), 3.00-5.20 (11H, m), 5.65-5 90 (1H, m), 6 40-6.50 (1H, m), 6 60-8.00 (10H, m) MS(ESI, m/z) 535(M+H)+ |
| Ex. 5-53 | 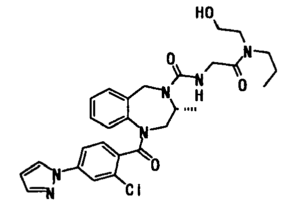 | 1H-NMR (CDCl3) δ ppm. 0 85-0 95 (3H, m), 1.20-1.65 (5H, m), 2 40-5 30 (13H, m), 5 60-5.90 (1H, m), 6.40-8 00 (10H, m) MS(ESI, m/z) 523(M+H)+ |

**[Table 46]**

| No | Strc | Physical data |
|---|---|---|
| Ex 5-54 | 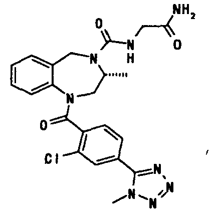 | MS(ESI, m/z) · 481 (M-H)- |
| Ex 5-55 | 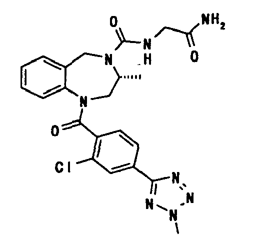 | 1H-NMR (CDCl3) δ ppm: 1 00-1.75 (3H, m), 2.70-6.00 (12H, m), 6.05-8 25 (7H, m) MS(ESI, m/z): 483(M+H)+ |
| Ex 5-56 | 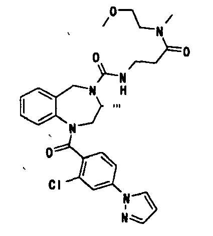 | 1H-NMR (CDCl3) δ ppm 0.95-1 65 (3H, m), 2.25-2.65 (2H, m), 2.80-5.20 (17H, m), 5.60-6 05 (1H, m), 6 40-8 10 (10H, m) MS(ESI, m/z) 553(M+H)+ |
| Ex 5-57 | 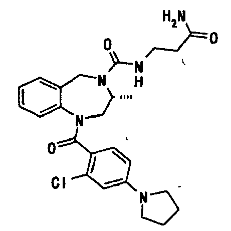 | MS(ESI, m/z) :484(M+H)+ |
| Ex 5-58 | 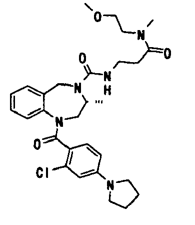 | 1H-NMR (CDCl3) δ ppm: 0.90-1.60 (3H, m), 1.90-2.15 (4H, m), 2.30-2.60 (2H, m), 2 80-5.20 (21H, m), 5 50-5.95 (1H, m), 6 00-7 80 (7H, m) MS(ESI, m/z) -556(M+H)+ |

**[Table 47]**

| No | Strc | Physical data |
|---|---|---|
| Ex 5-59 | 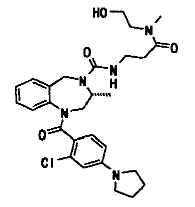 | MS(ESI, m/z) 542(M+H)+ |
| Ex 5-60 | 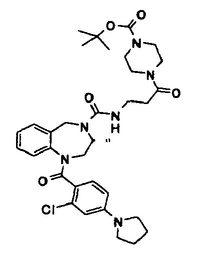 | MS(ESI, m/z) 653(M+H)+ |
| Ex. 5-61 | 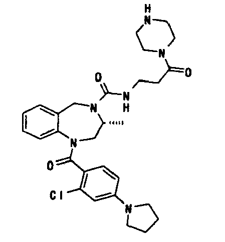 | MS(ESI, m/z) 553(M+H)+ |
| Ex 5-62 | 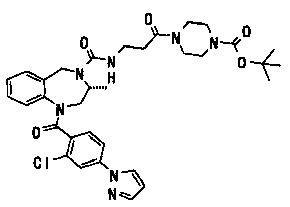 | MS(ESI, m/z) 650(M+H)+ |
| Ex. 5-63 | 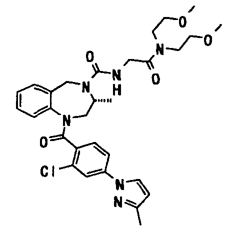 | 1H-NMR (CDCl3) δ ppm 1 00-1 65 (3H, m), 2 25-2 45 (3H, m), 2 80-5 20 (21H, m), 5 50-5 90 (1H, m), 6 20-8 05 (9H, m) MS(ESI, m/z) 597(M+H)+ |

**[Table 48]**

| No. | Strc | Physical data |
|---|---|---|
| Ex 5-64 | 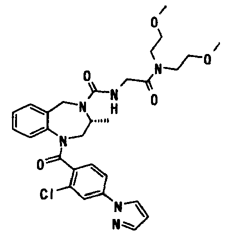 | 1H-NMR (CDCl3) δ ppm. 1 00-1 70 (3H, m), 2.80-5 20 (21H, m), 5 50-5 90 (1H, m), 6 35-8 00 (10H, m) MS(ESI, m/z) 583(M+H) + |
| Ex. 5-65 | 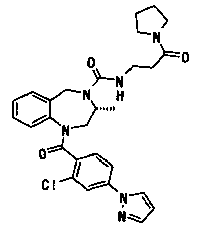 | 1H-NMR (CDCl3) δ ppm 1 00-1 65 (3H, m), 1 70-2.00 (4H, m), 2 20-2 50 (2H, m), 2 70-5 20 (11H, m), 5 70-6.10 (1H, m), 6 40-8.05 (10H, m) MS(ESI, m/z) 535(M+H)+ |
| Ex 5-66 | 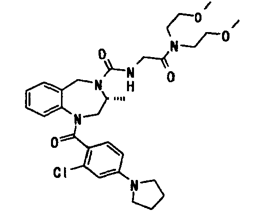 | 1H-NMR (CDCl3) δ ppm 1 00-1 60 (3H, m), 1 90-2.05 (4H, m), 2 80-5 20 (25H, m), 5 50-5 90 (1H, m), 6.00-7 70 (7H, m) MS(ESI, m/z) 586(M+H)+ |
| Ex 5-67 | 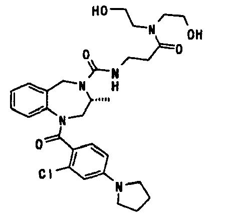 | MS(ESI, m/z) 572(M+H)+ |
| Ex 5-68 | 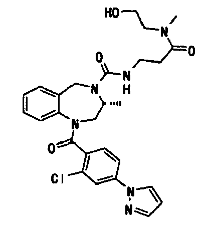 | MS(ESI, m/z) 539(M+H)+ |

**[Table 49]**

| No. | Strc | Physical data |
|---|---|---|
| Ex 5-69 | 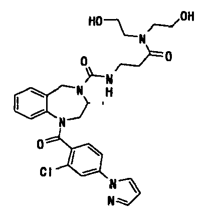 | 1H-NMR (CDCl3) δ ppm 1 00-1 70 (3H, m), 1 80-5 95 (19H, m), 6 40-8 00 (10H, m) |
| Ex 5-70 | 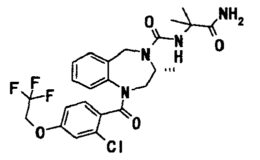 | 1H-NMR (DMSO-d6) δ ppm 0 80-1.45 (9H, m), 2 60-5 10 (7H, m), 6.10-7 90 (7H, m) MS(ESI, m/z) 527(M+H)+ |
| Ex 5-71 | 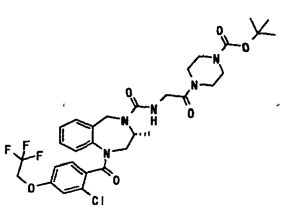 | 1H-NMR (CDCl3) δ ppm: 1.00-2 00 (12H, m), 2 80-5 20 (17H, m), 5 55-5 90 (1H, m), 6 40-8 00 (7H, m) MS(ESI, m/z) 668(M+H)+ |
| Ex 5-72 | 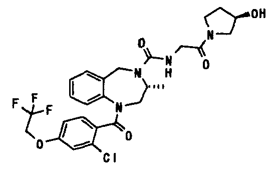 | 1H-NMR (CDCl3) δ ppm 1 00-1 70 (3H, m), 1 75-2.10 (2H, m), 2.90-5 35 (14H, m), 5 50-5 90 (1H, m), 6 40-7 90 (7H, m) MS(ESI, m/z) 569(M+H)+ |
| Ex 5-73 | 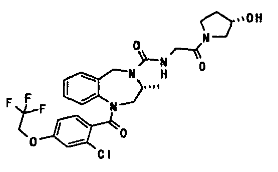 | 1H-NMR (CDCl3) δ ppm 1 00-1 65 (3H, m), 170-2 15 (2H, m), 2 80-5 20 (14H, m), 5.45-5 90 (1H, m), 6.30-7 90 (7H, m) MS(ESI, m/z) 569(M+H)+ |
| Ex. 5-74 | 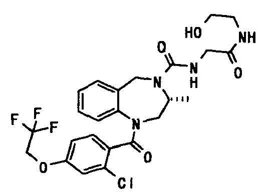 | 1H-NMR (CDCl3) δ ppm 1 00-1 65 (3H, m), 2 50-5.60 (14H, m), 6 30-7 90 (7H, m) MS(ESI, m/z) 543(M+H)+ |

**[Table 50]**

| No | Strc | Physical data |
|---|---|---|
| Ex 5-75 | 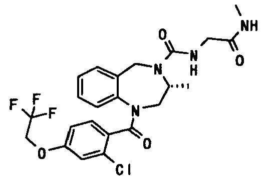 | 1H-NMR (CDCl3) δ ppm 1 00-1 60 (3H, m), 230-570 (13H, m), 6 10-7 90 (7H, m) MS(ESI, m/z) : 513(M+H)+ |
| Ex 5-76 | 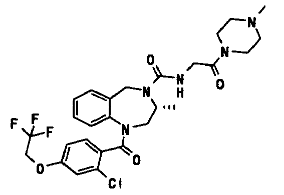 | 1H-NMR (CDCl3) δ ppm 1 00-1 70 (3H, m), 2 25-5.30 (20H, m), 5 50-5.95 (1H, m), 6 40-7.80 (7H, m) MS(ESI, m/z) · 582(M+H)+ |
| Ex 5-77 | 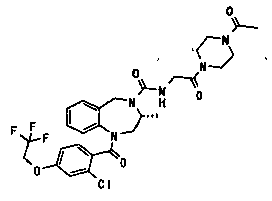 | 1H-NMR (CDCl3) δ ppm 1 00-1.70 (3H, m), 2 00-2.25 (3H, m), 2 70-5.30 (17H, m), 5 50-5 90 (1H, m). 6 30-7.80 (7H, m) MS(ESI, m/z) 610(M+H)+ |
| Ex 5-78 | 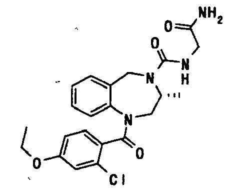 | 1H-NMR (CDCl3) δ ppm 0 80-1.80 (6H, m), 2 80-5 80 (9H, m), 6 10-7 80 (7H, m) MS(ESI, m/z). 445(M+H)+ |
| Ex 5-79 | 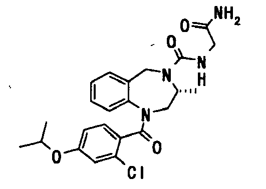 | 1H-NMR (CDCl3) δ ppm. MS(ESI, m/z) 457(M-H)- |
| Ex 5-80 | 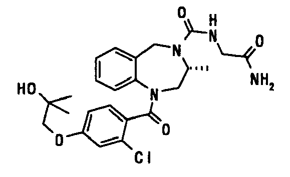 | 1H-NMR (CDCl3) δ ppm 1 00-1 65 (9H, m), 2 00-2 25 (1H, m), 2 90-5 50 (9H, m), 6 20-7 80 (7H, m) MS(ESI, m/z) 489(M+H)+ |

### Example 6-1

### (2-Chloro-4-pyrazol-1-ylphenyl)-[(R)-3-methyl-4-(pyrrolidin-1-carbonyl)-2,3,4,5-tetrahydrobenzo[e]-1,4-diazepin-1-yl]-methanone

To a solution of pyrrolidine (11.6 mg) and N,N-diisopropylethylamine (31.5 uL) in dichloromethane (0.60 mL) was added triphosgene (17.9 mg) under ice-cooling and the solution was stirred for 30 minutes under the same condition. To the stirred reaction solution were successively added (2-chloro-4-pyrazol-1-ylphenyl)-((R)-3-methyl-2,3,4,5-tetrahydrobenzo[e]-1,4-diazepin-1-yl)methanone (50.0 mg) and N,N-diisopropylethylamine (31.5 uL) under ice-cooling, and the mixture was stirred for an hour under ice-cooling. To the reaction solution was added water under ice-cooling and the mixture was extracted with ethyl acetate. The organic layer was washed with water and brine, dried over anhydrous magnesium sulfate, filtered. The filtrate was concentrated under reduced pressure. The residue was purified by column chromatography on,silica gel (eluent:ethyl acetate-hexane) to give (2-chloro-4-pyrazol-1-ylphenyl)-[(R)-3-methyl-4-(pyrrolidin-1-carbonyl)-2,3,4,5-tetrahydrobenzo[e]-1,4-diazepin-1-yl]methanone (45.8mg).

MS (ESI, m/z) : 463 (M+H) +

### Examples 6-2 to 6-63

The following compounds of Examples 6-2 to 6-33 were obtained with the use of the corresponding amine derivatives in a similar manner to that described in Example 6-1. The structure formula and physical data of these compounds were shown in Tables 51 to 55.

**[Table 51]**

| No | Strc | Physical data |
|---|---|---|
| Ex. 6-2 | 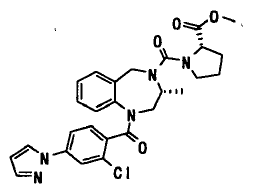 | 1H-NMR (CDCl3) δ ppm: 1 00-1.65 (3H, m), 1.75-2.30 (4H, m), 3.10-5.20 (11H, m), 6.40-8.00 (10H, m) MS(ESI, m/z). 522(M+H)+ |
| Ex 6-3 | 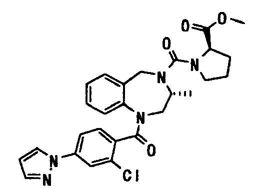 | 1H-NMR (CDCl3) δ ppm: 1 00-2.40 (7H, m), 3.10-5 10 (11H. m), 6.40-8.00 (10H, m) MS(ESI, m/z). 522(M+H)+ |
| Ex 6-4 | 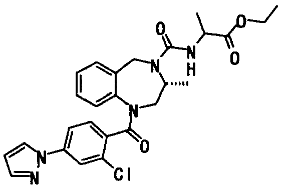 | MS(ESI, m/z) 510(M+H)+ |
| Ex 6-5 | 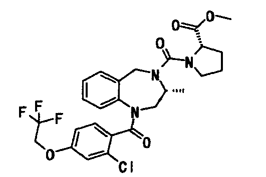 | 1H-NMR (CDCl3) δ ppm: 1.00-2.40 (7H, m), 3 00-5.20 (13H, m), 6.40-7.60 (7H, m) MS(ESI, m/z): 554 (M+H)+ |
| Ex 6-6 | 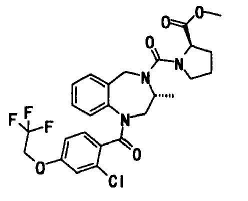 | 1H-NMR (CDCI3) δ ppm. 1.20-2.40 (7H, m), 3.00-5 20 (13H, m), 6 40-7 60 (7H, m) MS(ESI, m/z) 554(M+H)+ |
| Ex. 6-7 | 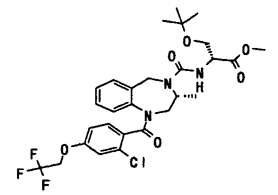 | 1H-NMR (CDCl3) δ ppm: 0.90-1.60 (12H, m), 2 80-5 60 (14H, m), 6.40-7.50 (7H, m) MS(ESI, m/z). 600(M+H)+ |

**[Table 52]**

| No | Strc | Physical data |
|---|---|---|
| Ex. 6-8 | 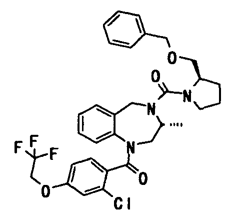 | 1H-NMR (CDCl3) δ ppm. 1.00-2.30 (7H, m), 2.80-5 10 (14H, m), 6 20-7 60 (12H, m) MS(ESI, m/z) : 616(M+H)+ |
| Ex 6-9 | 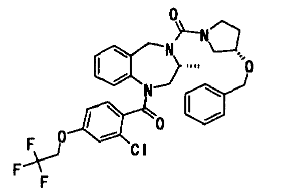 | 1H-NMR (CDCl3) δ ppm 1.00-2 20 (5H, m), 3 00-5 10 (14H, m), 6.20-7.50 (12H, m) MS(ESI, m/z) : 602(M+H)+ |
| Ex. 6-10 | 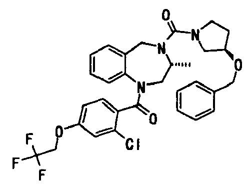 | 1H-NMR (CDCl3) δ ppm. 1.00-2 20 (5H, m), 3.00-5.10 (14H, m), 6.20-7.50 (12H, m) MS(ESI, m/z) : 602(M+H)+ |
| Ex. 6-11 | 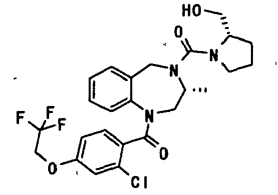 | 1H-NMR (CDCl3) δ ppm. 1 00-2 20 (7H, m), 2 80-510 (12H, m), 6.40-7 50 (7H, m) MS(ESI, m/z): 526(M+H)+ |
| Ex. 6-12 | 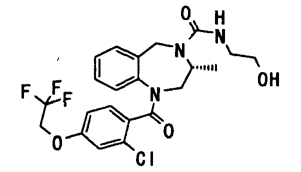 | 1H-NMR (CDCl3) δ ppm: 0.85-1.60 (3H, m), 1 85-2.10 (1H, m), 2.80-5.75 (12H, m), 6.30-7 90 (7H, m) MS(ESI, m/z) 486(M+H)+ |
| Ex. 6-13 | 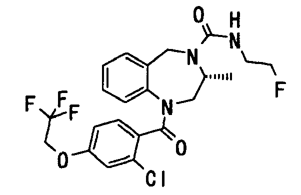 | 1H-NMR (CDCl3) δ ppm: 0.80-1.70 (3H, m), 2.90-5 40 (11H, m), 6.30-7.75 (7H, m) MS(ESI, m/z) 488(M+H)+ |

**[Table 53]**

| No | Strc | Physical data |
|---|---|---|
| Ex 6-14 | 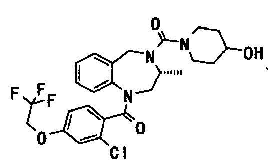 | 1H-NMR (CDCl3) δ ppm. 1 00-2 00 (7H, m), 2 75-5 05 (12H, m), 6 40-7 65 (7H, m) MS(ESI, m/z) 526(M+H)+ |
| Ex 6-15 | 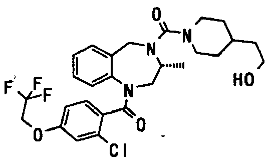 | 1H-NMR (CDCl3) δ ppm 1.00-1.85 (10H, m), 2 50-5.10 (12H, m), 6 50-7 50 (9H, m) MS(ESI, m/z) 554(M+H)+, |
| Ex 6-16 | 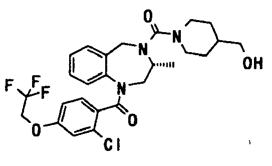 | 1H-NMR (CDCl3) δ ppm 1 00-1.85 (8H, m), 2 55-5.10 (13H, m), 6 35-7 70 (7H, m) MS(ESI, m/z) 540(M+H)+ |
| Ex 6-17 | 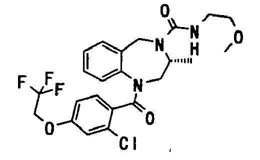 | 1H-NMR (CDCl3) δ ppm 1.00-1 65 (3H, m), 2.90-5 25 (14H, m), 6 40-7 85 (7H, m) MS(ESI, m/z) 500(M+H)+ |
| Ex 6-18 | 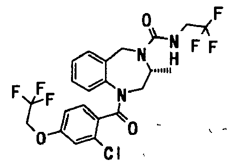 | MS(ESI, m/z) 524(M+H)+ |
| Ex 6-19 | 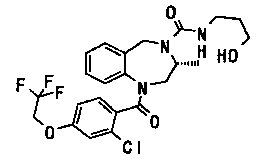 | 1H-NMR (CDCl3) δ ppm 1 00-1 75 (5H, m), 2 90-5 45 (11H, m), 6 40-7.85 (7H, m) MS(ESI, m/z) 500(M+H)+ |
| Ex 6-20 | 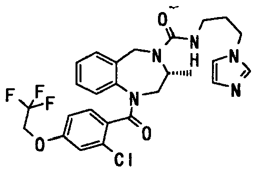 | 1H-NMR (CDCl3) δ ppm 1 00-2 10 (5H, m), 2.65-5 35 (11H, m), 6 30-7 90 (10H, m) MS(ESI, m/z) 550(M+H)+ |

**[Table 54]**

| No. | Strc | Physical data |
|---|---|---|
| Ex 6-21 | 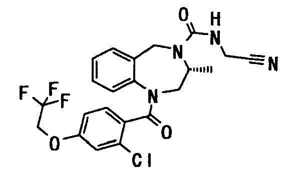 | 1H-NMR (CDCl3) δ ppm. 1.00-1 75 (3H, m), 2.90-5.45 (9H, m), 5.80-7 75 (8H, m) MS(ESI, m/z) 481 (M+H)+ |
| Ex 6-22 | 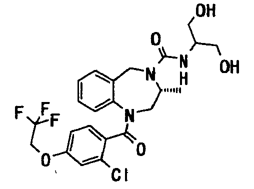 | MS(ESI, m/z) .516(M+H)+ |
| Ex. 6-23 | 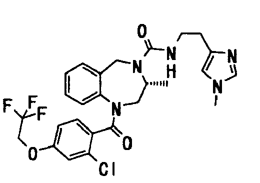 | MS(ESI, m/z) 550(M+H)+ |
| Ex 6-24 | 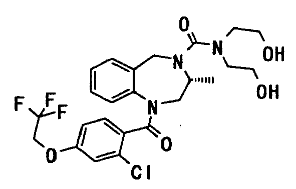 | 1H-NMR (CDCl3) δ ppm: 1 05-1.60 (3H, m), 2.30-5.35 (15H, m), 6.45-7 70 (7H, m) MS(ESI, m/z) .530(M+H)+ |
| Ex 6-25 | 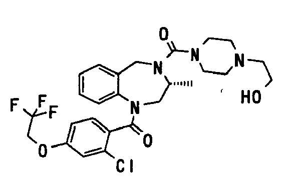 | 1H-NMR (CDCl3) δ ppm: 1 05-1 80 (3H, m), 2.15-5 35 (19H. m), 6 45-7 65 (7H, m) MS(ESI, m/z) 555(M+H)+ |
| Ex 6-26 | 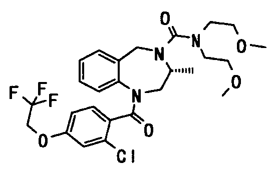 | 1H-NMR (CDCl3) δ ppm. 1.05-1 80 (3H, m), 2 15-5.35 (21H, m), 6 45-7.65 (7H, m) MS(ESI, m/z) 558(M+H)+ |

**[Table 55]**

| No | Strc | Physical data |
|---|---|---|
| Ex 6-27 | 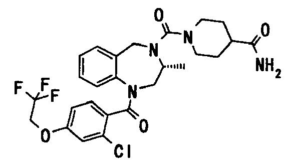 | 1H-NMR (CDCl3) δ ppm. 1 00-2 00 (7H, m), 2 15-5.70 (12H, m), 6.40-7 70 (7H, m) MS(ESI, m/z) :553(M+H)+ |
| Ex 6-28 | 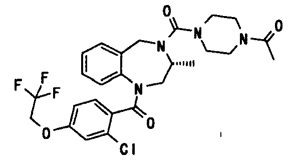 | 1H-NMR (CDCl3) δ ppm: 1.05-1.70 (3H, m), 2.00-2.20 (3H, m), 3 00-5 35 (15H, m), 6 45-7.65 (7H, m) MS(ESI, m/z) 553(M+H)+ |
| Ex 6-29 | 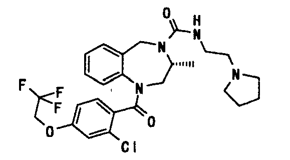 | 1H-NMR (CDCl3) δ ppm: 0.80-1.90 (7H, m), 2.20-5.60 (15H, m), 6.20-7.50 (7H, m) MS(ESI, m/z) :539 (M+H)+ |
| Ex 6-30 | 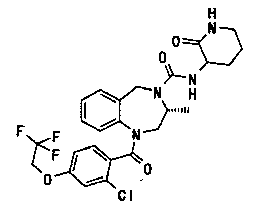 | 1H-NMR (CDCl3) δ ppm. 0.75-2 00 (7H, m), 2 30-2.70 (1H, m), 2.90-3.40 (3H, m), 3.95-5 30 (6H, m), 5 65-6.10 (1H, m), 6.35-7 85 (7H, m) MS(ESI, m/z) .539 (M+H)+ |
| Ex. 6-31 | 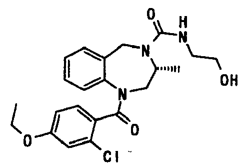 | 1H-NMR (CDCl3) δ ppm 0.80-1.60 (6H, m), 2 00-5.55 (11H, m), 6.30-7.80 (7H, m) MS(ESI, m/z):432(M+H)+ |
| Ex. 6-32 | 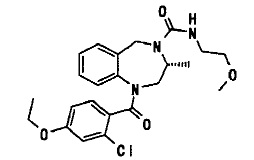 | 1H-NMR (CDCl3) δ ppm. 1.00-1 60 (6H. m), 2.90-5 35 (14H, m), 6 30-7.75 (7H, m) MS(ESI, m/z) :446 (M+H)+ |
| Ex. 6-33 | 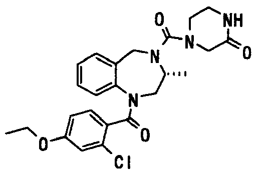 | 1H-NMR (CDCl3) δ ppm: 1.05-1 70 (6H, m), 3.00-5 15 (13H, m), 5 95-6 20 (1H, m), 6.40-7 70 (7H, m) MS(ESI, m/z) :471 (M+H)+ |

### Example 7-1

### Methyl (S)-{[(R)-1-(2-chloro-4-pyrazol-1-ylbenzoyl)-3-methyl-1,2,3,5-tetrahydrobenzo[e]-1,4-diazepin-4-carbonyl]amino}-phenylacetate

To a suspension of methyl (S)-aminophenylacetate hydrochloride (66.0 mg) and pyridine (53.0 uL) in dichloromethane (1.0 mL) was added 4-nitrophenyl chloroformate (66.0 mg) and the suspension was stirred at room temperature for 10 minutes (suspension H). To a solution of (2-chloro-4-pyrazol-1-ylphenyl)-((R)-3-methyl-2,3,4,5-tetrahydrobenzo[e]-1,4-diazepin-1-yl)methanone (60.0 mg) in dichloromethane (1.0'mL) were successively added the suspension H and N,N-diisopropylethylamine (114 uL) under the same condition and the mixture was stirred for 2 days under the same condition. To the mixture was added an aqueous solution of sodium hydrogen carbonate under the same condition (pH=8), and the mixture was stirred for 5 minutes. The organic layer was separated and the solvent was removed under reduced pressure. The obtained residue was purified by column chromatography on silica gel (eluent:ethyl acetate-hexane) to give methyl (S)-{[(R)-1-(2-chloro-4-pyrazol-1-ylbenzoyl)-3-methyl-1,2,3,5-tetrahydrobenzo[e]-1,4-diazepin-4-carbonyl]amino}phenylacetate (85.3 mg).
1H-NMR(CDCl3)δ ppm: 1.00-1.60 (3H, m), 2.70-6.00 (10H, m), 6.10-8.10 (15H, m).
MS(ESI, m/z) : 558 (M+H) +

### Examples 7-2 to 7-8

The following compounds of Examples 7-2 to 7-8 were obtained with the use of the corresponding amine derivatives in a similar manner to that described in Example 7-1. The structure formula and physical data of these compounds were shown in Table 56.

**[Table 56]**

| No | 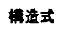 | Physical data |
|---|---|---|
| Ex 7-2 | 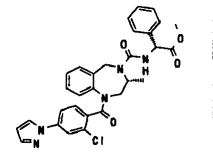 | 1H-NMR (CDCl3) δ ppm 1 00-1 70 (3H, m), 2 80-6 00 (10H, m), 640-820 (15H, m) MS(ESI, m/z) 558(M+H)+ |
| Ex 7-3 | 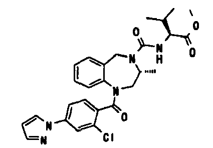 | 1H-NMR (CDCl3) δ ppm 0 60-1 80 (10H, m), 2 00-2.25 (1H, m), 280-530 (9H, m), 6 20-8 10 (10H, m) MS(ESI, m/z) 524(M+H)+ |
| Ex 7-4 | 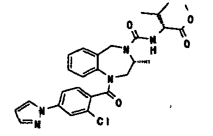 | 1H-NMR (CDCl3) δ ppm 0 50-1 75 (9H, m), 1 90-2 20 (1H, m), 2 50-5 30 (9H, m), 6 00-8 05 (10H, m) MS(ESI, m/z) 524(M+H)+ |
| Ex 7-5 | 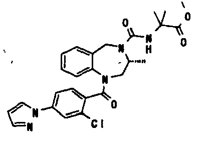 | 1H-NMR (CDCl3) δ ppm 1 20-1 65 (9H, m), 2 80-5 50 (8H, m), 6 40-8 00 (10H, m) MS(ESI, m/z) 510(M+H)+ |
| Ex 7-6 | 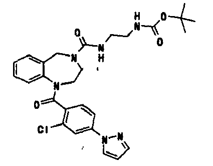 | 1H-NMR (CDCl3) δ ppm 1 00-1 65 (12H, m), 2 90-5 90 (10H, m), 6 40-8 05 (10H, m) MS(ESI, m/z) 553(M+H)+ |
| Ex 7-7 | 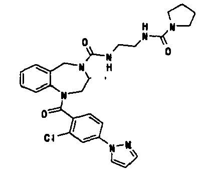 | 1H-NMR (CDCl3) δ ppm 1 00-2 20 (7H, m), 2 80-5 50 (13H, m), 5 70-6 00 (1H, m), 6 40-8 20 (10H, m) MS(ESI, m/z) 550(M+H)+ |
| Ex 7-8 | 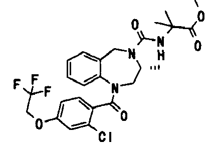 | 1H-NMR (CDCl3) δ ppm 0 80-2 20 (9H, m), 2 90-5 80 (10H, m), 6 30-8 20 (7H, m) MS(ESI, m/z) 542(M+H)+ |

### Example 8-1

### N-[2-Oxo-2-(piperazin-1-yl)]ethyl-(R)-1-[2-chloro-4-(3-methylpyrazol-1-yl)benzoyl]-3-methyl-1,2,3,5-tetrahydrobenzo[e]-1,4-diazepin-4-carboxamide

To a mixture of ({(R)-1-[2-chloro-4-(3-methylpyrazol-1-yl)benzoyl]-3-methyl-1,2,3,5-tetrahydrobenzo[e]-1,4-diazepin-4-carbonyl}amino)acetic acid(20.0 mg) and N-cyclohexylcarbodiimide, N'-methyl polystyrene (load:1.95 mmol/g, 85.0 mg) in dichloromethane (0.80 mL) was added piperazine (71.0 mg) at room temperature. The mixture was stirredat roomtemperature for 2 days, and without work-up, the mixture was purified by column chromatography on silica gel (eluent:ethyl acetate-ethanol) to give N-[2-oxo-2-(piperazin-1-yl)]ethyl-(R)-1-[2-chloro-4-(3-methylpyrazol-1-yl)benzoyl]-3-methyl-1,2,3,5-tetrahydrobenzo[e]-1,4-diazepin-4-carboxamide (12.9 mg).
1H-NMR(CDCl3) δ ppm:
1. 00-1. 60 (3H, m), 1.70-5.20 (18H, m), 5.65-5.95 (1H, m), 6.20-7.90 (9H, m).
MS(ESI, m/z) : 550(M+H)+

### Examples 8-2 to 8-68

The following compounds of Examples 8-2 to 8-68 were obtained with the use of the corresponding carboxylic acid derivatives and the corresponding amine derivatives in a similar manner to that described in Example 8-1. The structure formula and physical data of these compounds were shown in Table 57 to 67.

**[Table 57]**

| No. | Strc | Physical data |
|---|---|---|
| Ex 8-2 | 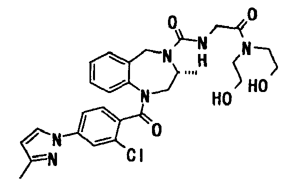 | 1H-NMR (CDCl3) δ ppm. 1.00-1.50 (3H, m), 2 20-2.40 (3H, m), 2.60-5.20 (15H, m), 5.60-5.90 (1H, m), 620-810 (9H, m) MS(ESI, m/z): 569(M+H)+ |
| Ex 8-3 | 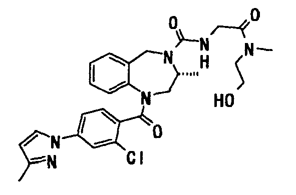 | 1H-NMR (CDCl3) δ ppm 1 00-1 70 (3H, m), 2.30-2 40 (3H, m), 2.70-5 20 (14H, m), 5 55-5.75 (1H, m), 6 20-8 00 (9H, m) MS(ESI, m/z) : 539(M+H)+ |
| Ex. 8-4 | 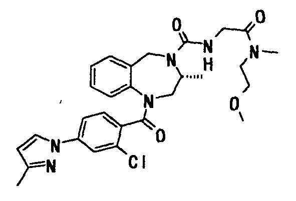 | 1H-NMR (CDCl3) δ ppm 1.00-1.70 (3H, m), 2.25-2.45 (3H, m), 2.90-5.20 (17H, m), 5 60-5.90 (1H, m), 6.90-7.90 (9H, m) MS(ESI, m/z): 553(M+H)+ |
| Ex. 8-5 | 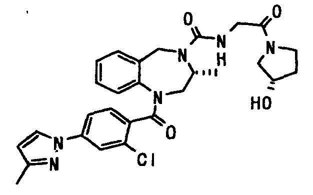 | 1H-NMR (CDCl3) δ ppm 1 00-2.15 (5H, m), 2.25-2 40 (3H, m), 2.90-5 20 (12H, m), 5.50-5.90 (1H, m), 6.20-8.00 (9H, m) MS(ESI, m/z) 551 (M+H)+ |
| Ex. 8-6 | 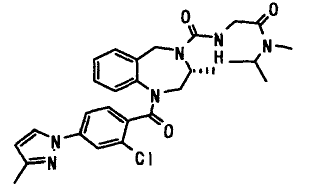 | 1H-NMR (CDCl3) δ ppm. 1.00-1.65 (9H, m), 2.25-2 45 (3H, m), 2.70-5.20 (11H, m), 5.70-6.05 (1H, m), 6.50-8 00 (9H, m) MS(ESI, m/z). 537(M+H)+ |
| Ex 8-7 | 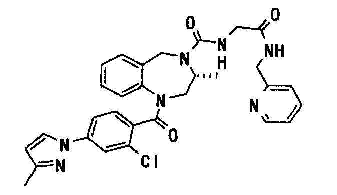 | 1H-NMR (CDCl3) δ ppm. 1.00-1.65 (3H, m), 2.30-2.45 (3H, m), 2.70-5 30 (9H, m), 5.45-5 75 (1H, m), 6.15-7.95 (12H, m), 8 40-8 60 (1H, m) MS(ESI, m/z) : 572(M+H)+ |

**[Table 58]**

| No | Strc | Physical data |
|---|---|---|
| Ex 8-8 | 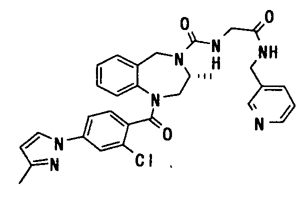 | 1H-NMR (CDCl₃) δ ppm: 1.00-1 65 (3H, m), 2.30-2.45 (3H, m), 2 90-5 30 (9H, m), 5 55-5.70 (1H, m), 6.20-8 80 (13H, m) MS(ESI, m/z) 572(M+H)+ |
| Ex 8-9 | 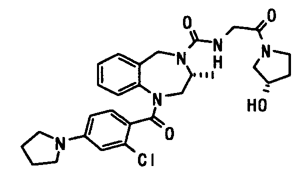 | 1H-NMR (CDCl₃) δ ppm 1 00-2.15 (9H, m), 2 80-5 25 (16H, m), 5 50-5 90 (1H, m), 6 00-7 70 (7H, m) MS(ESI, m/z). 540(M+H)+ |
| Ex 8-10 | 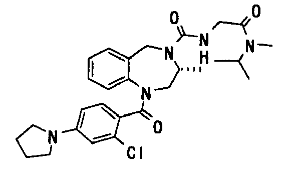 | 1H-NMR (CDCl₃) δ ppm: 1.00-1.60 (9H, m), 1.90-2.05 (4H, m), 2.70-2.85 (3H, m), 2.85-5.30 (12H, m), 5.70-6.00 (1H, m), 6 10-7.70 (7H, m) MS(ESI, m/z) 526(M+H)+ |
| Ex 8-11 | 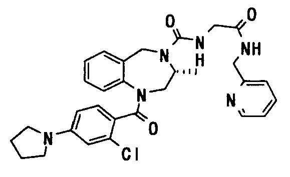 | 1H-NMR (CDCl3) δ ppm. 1.00-1 65 (3H, m), 1 90-2.05 (4H, m), 2 70-5 30 (13H, m), 5 35-5 55 (1H, m), 5.90-7 75 (10H, m), 8 40-8 60 (1H, m) MS(ESI, m/z) 561(M+H)+ |
| Ex 8-12 | 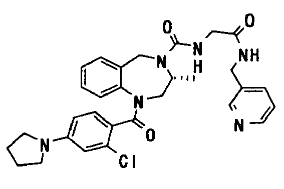 | 1H-NMR (CDCl3) δ ppm: 0.75-1 60 (3H, m), 1 90-210 (4H, m), 2.90-5.30 (13H, m), 5.40-5 70 (1H, m), 5 80-8.70 (12H, m) MS(ESI, m/z) 561(M+H)+ |
| Ex 8-13 | 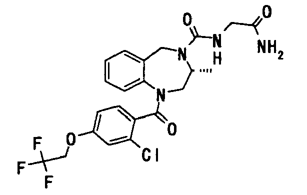 | 1H-NMR (CDCl3) δ ppm 1.00-1 70 (3H, m), 2 90-5 50 (9H, m), 580-790 (8H, m) |

**[Table 59]**

| No | Strc | Physical data |
|---|---|---|
| Ex 8-14 | 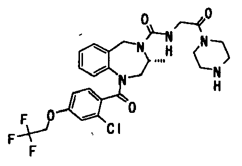 | MS(ESI, m/z) 568(M+H)+ |
| Ex 8-15 | 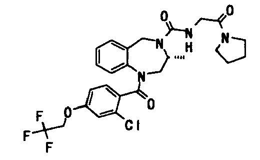 | 1H-NMR (CDCl3) δ ppm 1 00-1 60 (3H, m), 1 80-2 05 (4H, m), 2 80-5 20 (13H, m), 5 50-5 90 (1H, m), 6 30-7 70 (7H, m) MS(ESI, m/z) 553(M+H)+ |
| Ex 8-16 | 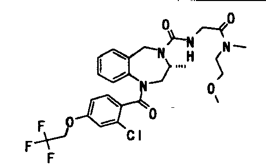 | 1H-NMR (CDCl3) δ ppm 1 00-1 65 (3H, m), 2 95-3 05 (3H, m), 3.25-5 20 (16H, m), 5 55-5 95 (1H, m), 6.30-7 70 (7H, m) MS(ESI, m/z) 571(M+H)+ |
| Ex 8-17 | 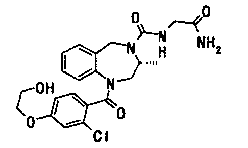 | 1H-NMR (DMSO-d6) δ ppm 1 10-1 45 (3H, m), 2 80-5 00 (11H, m), 6 40-7 60 (7H, m) |
| Ex 8-18 | 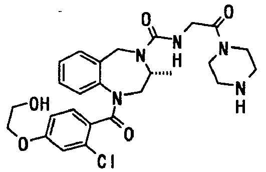 | 1H-NMR (CDCl3) δ ppm 1 00-1 65 (3H, m), 2 40-2 70 (1H, m), 2 75-5 30 (19H, m), 5 60-5 95 (1H, m), 6 30-5 80 (7H, m) MS(ESI, m/z) 530(M+H)+ |
| Ex 8-19 | 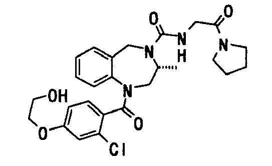 | 1H-NMR (CDCl3) δ ppm 1 00-1 65 (3H, m), 1 80-2 15 (5H, m), 2 80-5 30 (15H, m), 5 50-5 95 (1H, m), 6 20-7 55 (7H, m), MS(ESI, m/z) 515(M+H)+ |
| Ex 8-20 | 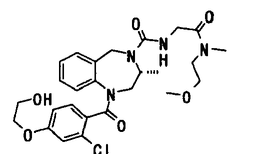 | 1H-NMR (CDCl3) δ ppm 1 00-1 60 (3H, m), 1 94 (1H, t, J=5 8Hz), 3 00 (3H, s), 3 25-5 20 (18H, m), 5 55-5 95 (1H, m), 6 30-7 70 (7H, m) MS(ESI, m/z). 533(M+H)+ |

**[Table 60]**

| No | Strc | Physical data |
|---|---|---|
| Ex 8-21 | 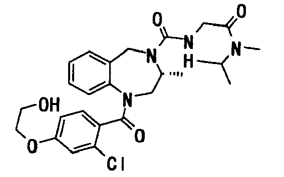 | 1H-NMR (CDCl3) δ ppm. 1.00-1 60 (9H, m), 1 98 (1H, t, J=6 1Hz). 2 70-2 85 (3H, m), 2 90-5 25 (12H, m), 5 65-6.05 (1H, m), 6 25-7 80 (7H, m) |
| Ex. 8-22 | 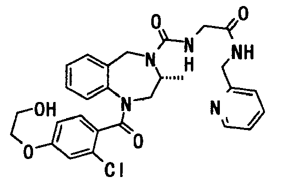 | 1H-NMR (CDCl3) δ ppm: 1.00-1.60 (3H, m), 190-2 80 (1H, m), 2 90-5.35 (13H, m), 5.40-5 75 (1H, m), 6 20-7 80 (10H, m), 8.40-8 60 (12H, m) MS(ESI, m/z) 552(M+H)+ |
| Ex. 8-23 | 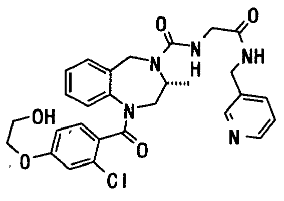 | 1H-NMR (CDCl3) δ ppm. 1 00-1 65 (3H, m), 2.80-5 25 (13H, m), 5 35-5.80 (1H, m), 6 20-7 80 (10H, m), 8.40-8 70 (1H, m) MS(ESI, m/z) 552(M+H)+ |
| Ex 8-24 | 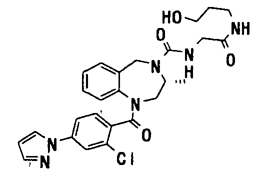 | 1H-NMR (CDCl3) δ ppm 1.00-1 60 (3H, m), 2.70-5 80 (14H, m), 6 40-8.10 (10H, m) MS(ESI, m/z) 525(M+H)+ |
| Ex 8-25 | 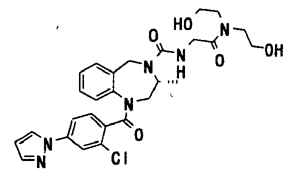 | 1H-NMR (CDCl3) δ ppm. 0 90-2.00 (5H, m), 2 80-5 10 (15H, m), 5 20-5 90 (1H, m), 6 35-8 10 (10H, m) MS(ESI, m/z) 555(M+H)+ |
| Ex 8-26 | 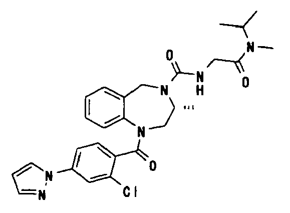 | 1H-NMR (CDCl3) δ ppm 1 00-1.70 (9H, m), 2.70-2.85 (3H, m), 2 90-5.20 (8H, m), 5 70-6 05 (1H, m), 6.40-8.05 (10H, m) MS(ESI, m/z) 523(M+H)+ |

**[Table 61]**

| No | Strc | Physical data |
|---|---|---|
| Ex 8-27 | 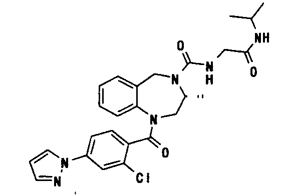 | 1H-NMR (CDCl3) δ ppm 0 70-180 (9H, m), 2 70-5 75 (8H, m), 5.70-6 05 (1H, m), 6 00-8 20 (11H, m) |
| Ex. 8-28 | 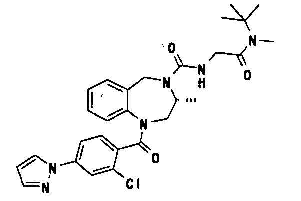 | 1H-NMR (CDCl3) δ ppm: 1 00-1.70 (12H, m), 2 84 (3H, s), 2.90-5 20 (7H, m), 5 75-6 05 (1H, m), 6 40-8.05 (10H, m) MS(ESI, m/z) 537(M+H)+ |
| Ex 8-29 | 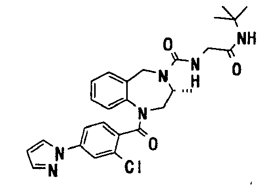 | 1H-NMR (CDCl3) δ ppm 1 00-1.70 (12H, m), 2 90-5 65 (7H, m), 5 75-6 20 (1H, m), 645-820 (10H, m) MS(ESI, m/z) -523(M+H)+ |
| Ex 8-30 | 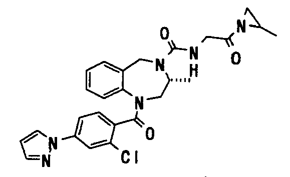 | MS(ESI, m/z) 507(M+H)+ |
| Ex 8-31 | 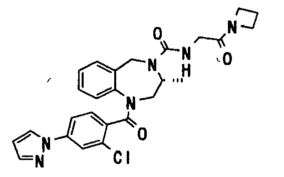 | 1H-NMR (CDCl3) δ ppm: 1 00-1 70 (3H, m), 2 25-2 45 (2H, m), 2 85-5 20 (11H, m), 5.30-5.65 (1H, m), 6 40-8 05 (10H, m) MS(ESI, m/z) 507(M+H)+ |
| Ex 8-32 | 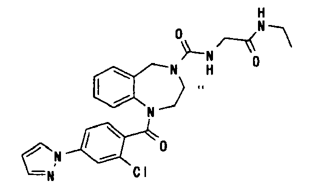 | 1H-NMR (CDCl3) δ ppm 0 80-1 70 (6H, m), 2 80-5 25 (9H, m), 5 45-5 70 (1H, m), 6 15-8 05 (11H, m) |

**[Table 62]**

| No | Strc | Physical data |
|---|---|---|
| Ex 8-33 | 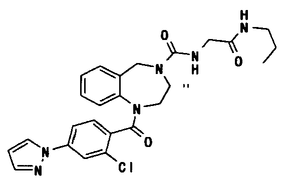 | 1H-NMR (CDCl3) δ ppm: 0 80-1.85 (8H, m), 2 60-5 40 (9H, m), 5 45-5.80 (1H. m), 6 20-8 20 (11H, m) |
| Ex 8-34 | 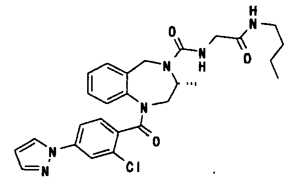 | 1H-NMR (CDCl3) δ ppm 0 70-1.60 (10H, m), 2.80-5 20 (9H, m), 5 35-5.65 (1H, m), 6.10-8 20 (11H, m) |
| Ex 8-35 | 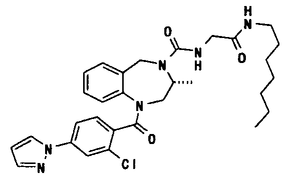 | 1H-NMR (CDCl3) δ ppm: 0.70-2 00 (16H, m), 2.70-5.25 (9H, m), 5 45-5.80 (1H. m), 6.20-8 30 (11H. m) MS(ESI, m/z) 565(M+H)+ |
| Ex 8-36 | 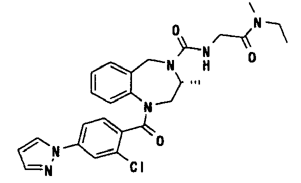 | 1H-NMR (CDCl3) δ ppm 0 70-1.65 (6H, m), 2 85-5.25 (12H, m), 5.65-5.95 (1H, m), 6.40-8.00 (10H, m) MS(ESI, m/z) :509(M+H)+ |
| Ex 8-37 | 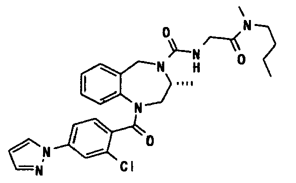 | 1H-NMR (CDCl3) δ ppm 0.70-1.70 (10H, m), 2 85-5.25 (12H, m), 5 65-6 00 (1H, m), 6.40-8 05 (10H, m) MS(ESI, m/z) 537(M+H)+ |
| Ex 8-38 | 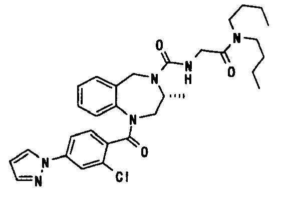 | 1H-NMR (CDCl3) δ ppm: 0 75-1.70 (17H, m), 2 95-5 20 (11H, m), 5 65-6 00 (1H, m), 6 40-8.00 (10H, m) MS(ESI, m/z) 579(M+H)+ |

**[Table 63]**

| No | Strc | Physical data |
|---|---|---|
| Ex 8-39 | 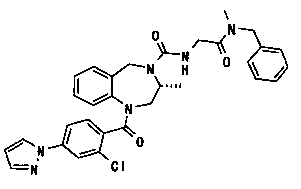 | 1H-NMR (CDCl3) δ ppm 1.00-1 50 (3H, m), 2.70-5 20 (12H, m), 5 60-6.00 (1H, m), 6 40-8 10 (15H, m) MS(ESI, m/z) .571 (M+H)+ |
| Ex 8-40 | 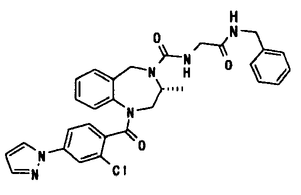 | 1H-NMR (CDCl3) δ ppm: 1.00-160 (3H, m), 2 80-5 35 (9H, m), 5.45-5 75 (1H, m), 6 40-8.20 (15H, m) |
| Ex 8-41 | 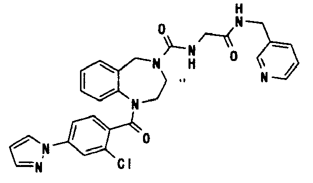 | 1H-NMR (CDCl3) δ ppm. 1.00-1 60 (3H, m), 2 85-5 30 (9H, m), 5 45-5 65 (1H, m), 6.40-8 65 (14H, m) |
| Ex 8-42 | 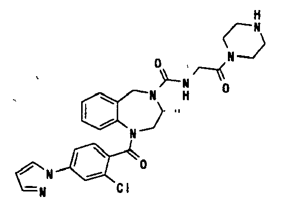 | 1H-NMR (CDCl3) δ ppm: 1 00-1 60 (3H, m), 2 40-2.55 (1H, br), 2.75-5.20 (15H, m), 5.25-5 95 (1H, m), 6 40-8 10 (10H, m) |
| Ex 8-43 | 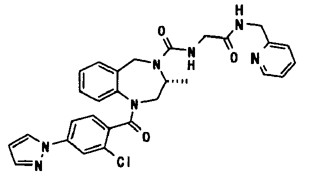 | 1H-NMR (CDCl3) δ ppm 1 00-1 60 (3H, m), 2 95-5 65 (10H, m), 6 40-8 65 (14H, m) MS(ESI, m/z) 588(M+H)+ |
| Ex 8-44 | 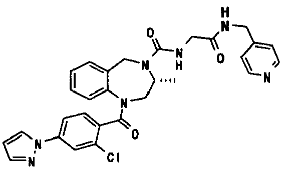 | 1H-NMR (CDCl3) δ ppm 1.00-1.60 (3H, m), 2 85-5 30 (9H, m), 5.55-5.85 (1H, m), 6 40-8 70 (14H, m) MS(ESI, m/z) 558(M+H)+ |

**[Table 64]**

| No | Strc | Physical data |
|---|---|---|
| Ex 8-45 | 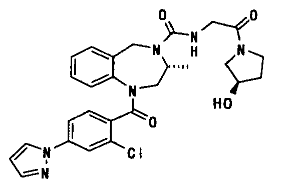 | 1H-NMR (CDCl3) δ ppm. 1 00-2 65 (6H, m), 2.75-5 30 (12H, m), 5 55-5 90 (1H, m), 6.40-8 05 (10H, m) MS(ESI, m/z) 537(M+H)+ |
| Ex 8-46 | 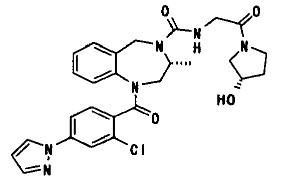 | 1H-NMR (CDCl3) δ ppm: 1 00-2.60 (6H, m), 2 65-5.40 (12H, m), 5 55-5 90 (1H, m), 6.40-8.05 (10H, m) MS(ESI, m/z) 537(M+H)+ |
| Ex 8-47 | 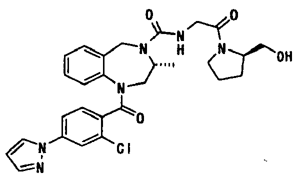 | 1H-NMR (CDCl3) δ ppm 1.00-2 15 (7H, m), 2 80-5.20 (12H, m), 5 45-5 85 (1H, m), 6 35-8.00 (10H, m) MS(ESI, m/z) 551 (M+H)+ |
| Ex 8-48 | 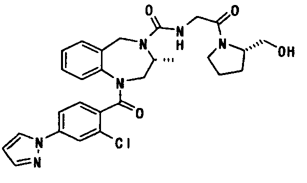 | 1H-NMR (CDCl3) δ ppm. 1.00-2 10 (7H, m), 2 80-5.20 (12H, m), 5 40-5 85 (1H, m), 6 35-8.00 (10H, m) MS(ESI, m/z) 551(M+H)+ |
| Ex 8-49 | 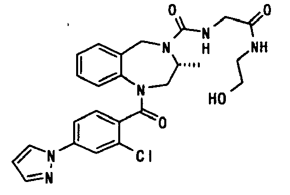 | 1H-NMR (CDCl3) δ ppm: 1.00-1.70 (3H, m), 2.70-5 80 (13H, m), 6 40-8 05 (10H, m) MS(ESI, m/z) 511 (M+H)+ |
| Ex 8-50 | 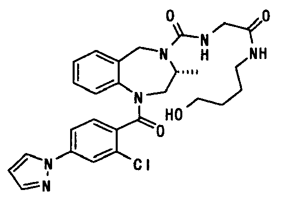 | 1H-NMR (CDCl3) δ ppm 1 00-1 70 (7H, m), 2 30-5.60 (12H, m), 6 35-8.05 (10H, m) MS(ESI, m/z) 539(M+H)+ |

**[Table 65]**

| No | Strc | Physical data |
|---|---|---|
| Ex 8-51 | 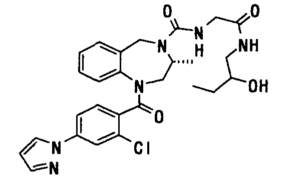 | 1H-NMR (CDCl3) δ ppm. 0 70-1 75 (8H, m), 2 60-5.70 (12H, m), 6 40-8 00 (10H, m) MS(ESI, m/z) 539(M+H)+ |
| Ex. 8-52 | 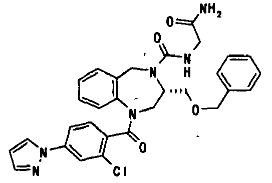 | 1H-NMR (CDCl3) δ ppm 3 00-5 00 (11H, m), 5 55-8 05 (18H, m) MS(ESI, m/z) 573(M+H)+ |
| Ex 8-53 | 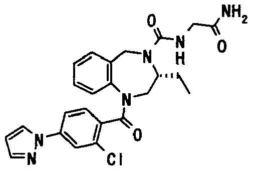 | 1H-NMR (CDCl3) δ ppm 0 75-1 15 (3H, m), 1 55-1.80 (2H, m), 2 95-5 70 (8H, m), 6 00-8 00 (10H, m) MS(ESI, m/z) 481(M+H)+ |
| Ex. 8-54 | 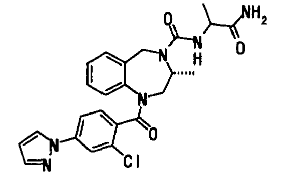 | 1H-NMR (CDCl3) δ ppm 1 15-1 60 (6H, m), 2 95-5 60 (6H, m), 6 40-8 35 (10H, m) MS(ESI, m/z) 481(M+H)+ |
| Ex. 8-55 | 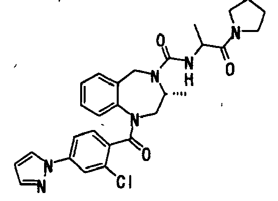 | 1H-NMR (CDCl3) δ ppm 1 00-2 00 (10H, m), 2 90-5 30 (10H, m), 5 40-5 90 (1H, m), 6 40-8 00 (10H, m) |
| Ex 8-56 | 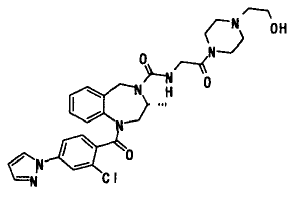 | 1H-NMR (CDCl3) δ ppm 1 00-1 75 (3H, m), 2 20-5 15 (20H, m), 565-590 (1H, m), 6 45-8 00 (10H, m) MS(ESI, m/z) 580(M+H)+ |

**[Table 66]**

| No | Strc | Physical data |
|---|---|---|
| Ex 8-57 | 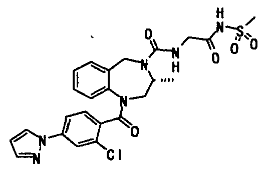 | MS(ESI, m/z) 545(M+H)+ |
| Ex 8-58 | 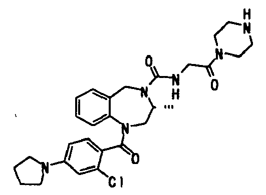 | 1H-NMR (CDCl3) δ ppm 1 00-1 60 (3H, m), 1 90-2 05 (8H, m), 2 40-2 55 (1H, m), 2 75-5 30 (19H, m), 5 70-5 90 (1H, m), 6 10-7 70 (7H, m) MS(ESI, m/z) 539(M+H)+ |
| Ex. 8-59 | 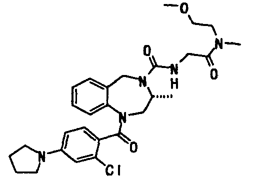 | 1H-NMR (CDCl3) δ ppm 1 30-1 60 (3H, m), 1 90-2 10 (4H, m), 2 80-5 30 (21H, m), 5 60-5 90 (1H, m), 6 05-7 60 (7H, m) |
| Ex. 8-60 | 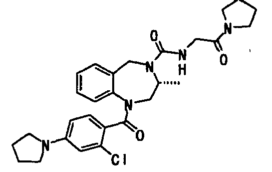 | 1H-NMR (CDCl3) δ ppm 1 00-1 65 (3H, m), 1 85-2 05 (8H, m), 2 80-5 30 (15H, m), 5 65-5 90 (1H, m), 6 10-7 80 (7H, m) |
| Ex 8-61 | 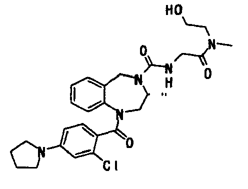 | 1H-NMR (CDCl3) δ ppm 1 00-1 55 (3H, m), 1 90-2.05 (4H, m), 2 20-5 20 (19H, m), 5 65-5 85 (1H, m), 6 10-6 45 (2H, m), 6 65-7 65 (5H, m) MS(ESI, m/z) 528(M+H)+ |
| Ex 8-62 | 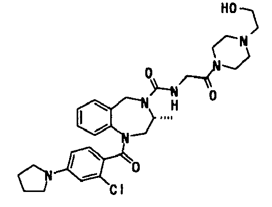 | 1H-NMR (CDCl3) δ ppm 1 00-1 60 (3H, m), 1 90-2 10 (4H, m), 2 25-5 25 (23H, m), 5 65-5 90 (1H, m), 6 10-7 65 (7H, m) MS(ESI, m/z) 583(M+H)+ |

**[Table 67]**

| No | Strc | Physical data |
|---|---|---|
| Ex 8-63 | 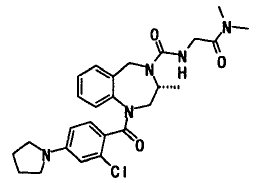 | 1H-NMR (CDCl₃) δ ppm 1 00-1 60 (3H, m), 1 80-2 05 (4H, m), 3 10-5 25 (15H, m), 5 65-5 90 (1H, m), 6 10-6 50 (2H, m), 6 65-7 60 (5H, m) |
| Ex 8-64 | 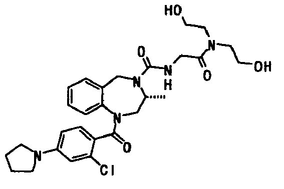 | 1H-NMR (CDCl₃) δ ppm 1 10-1 60 (3H, m), 1 85-2 10 (4H, m), 2 80-5 25 (19H, m), 5 70-5 90 (1H, m), 6 05-7 75 (7H, m) |
| Ex 8-65 | 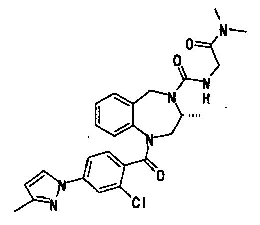 | 1H-NMR (CDCl₃) δ ppm 1 00-1 60 (3H, m), 2 30-2 40 (3H, m), 2 70-5 30 (13H, m), 5 60-6 00 (1H, m), 6 20-8 00 (9H, m) |
| Ex 8-66 | 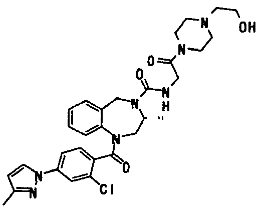 | 1H-NMR (CDCl3) δ ppm 0 90-1 95 (3H, m), 2 25-5 30 (22H, m), 5 60-5 90 (1H, m), 6 15-8 00 (9H, m) |
| Ex 8-67 | 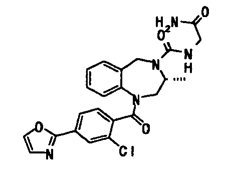 | MS(ESI, m/z) 468(M+H)+ |
| Ex 8-68 | 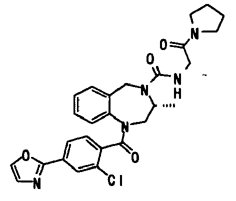 | MS(ESI, m/z) 522(M+H)+ |

### Example 9-1

### N-[N-Ethyl-N-(2-hydroxyethyl)carbamoyl]methyl-(R)-1-[2-chloro-4-(3-methylpyrazol-1-yl)benzoyl]-3-methyl-1,2,3,5-tetrahydrobenzo[e]-1,4-diazepin-4-carboxamide

To a mixture of ({(R)-1-[2-chloro-4-(3-methylpyrazol-1-yl)-benzoyl]-3-methyl-1,2,3,5-tetrahydrobenzo[e]-1,4-diazepin-4-carbonyl)amino}acetic acid (20.0 mg) and 1-ethyl-3-(3-dimethylaminopropyl) carbodiimide hydrochloride (15. 9 mg) in dichloromethane (800 uL) was added 2-ethylaminoethanol (8.0 uL) at room temperature and the mixture was stirred for a day. The solvent was removed under reduced pressure. The residue was purified by column chromatography on silica gel (eluent:ethyl acetate-ethanol) to give N-[N-ethyl-N-(2-hydroxyethyl)carbamoyl]methyl-(R)-1-[2-chloro-4-(3-methylpyrazol-1-yl)benzoyl]-3-methyl-1,2,3,5-tetrahydrobenzo[e]-1,4-diazepin-4-carboxamide (13.4 mg).
1H-NMR(CDCl3)δ ppm:
1.00-1.70 (6H, m), 2.25-2.40 (3H, m), 2.70-5.25 (13H, m), 5.55-5.90 (1H, m), 6.20-8.00 (9H, m).

### Examples 9-2 to 9-66

The following compounds of Examples 9-2 to 9-66 were obtained with the use of the corresponding carboxylic acid derivatives and the corresponding amine derivatives in a similar manner to that described in Example 9-1. The experiments were executed in a similar manner to that described in Example 9-1 using amine tosylate (Examples 9-51 and 9-52) or amine hydrochloride (Examples 9-57 and 9-66) that were treated with diisopropylethylamine. The structure formula and physical data of these compounds were shown in Tables 68 to 79.

**[Table 68]**

| No | Strc | Physical data |
|---|---|---|
| Ex. 9-2 | 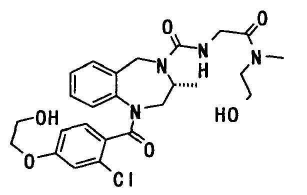 | 1H-NMR (CDCl3) δ ppm: 1.00-1 65 (3H, m), 1 90-5.30 (20H, m), 5.50-5 90 (1H, m), 6 30-7 70 (7H, m) MS(ESI, m/z) 519(M+H)+ |
| Ex. 9-3 | 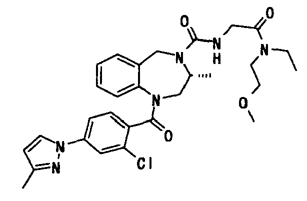 | 1H-NMR (CDCl3) δ ppm 1 00-1.65 (6H, m), 2.30-2 40 (3H, m), 2.90-5 30 (16H, m), 5.60-6 00 (1H, m), 6 20-8 00 (9H, m) MS(ESI, m/z) : 567(M+H)+ |
| Ex. 9-4 | 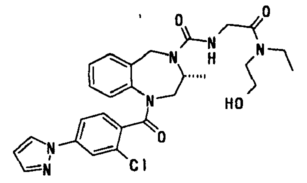 | 1H-NMR (CDCl3) δ ppm 1 00-1 70 (6H, m), 2 50-5.20 (13H, m), 5 55-5 90 (1H, m), 6 40-8 00 (10H, m) MS(ESI, m/z). 539(M+H)+ |
| Ex. 9-5 | 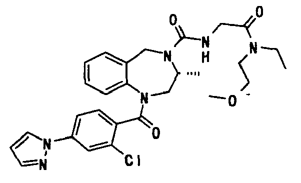 | 1H-NMR (CDCl3) δ ppm: 1 00-1.70 (6H, m), 2 70-5 30 (16H, m), 5.60-5 95 (1H, m), 6 40-8 00 (10H, m) MS(ESI, m/z) : 553(M+H)+ |
| Ex 9-6 | 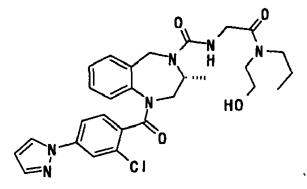 | 1H-NMR (CDCl3) δ ppm. 1.00-1 70 (8H, m), 2 70-5.30 (13H, m), 555-5.90 (1H, m), 6 40-8.10 (10H, m) |

**[Table 69]**

| No | Strc | Physical data |
|---|---|---|
| Ex. 9-7 | 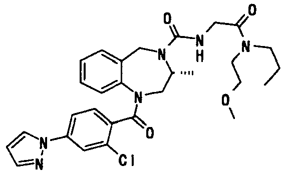 | 1H-NMR (CDCl3) δ ppm 1 00-1 65 (8H, m), 2 80-5.30 (16H, m), 5.60-6.00 (1H, m), 6.40-8.10 (10H, m) MS(ESI, m/z) 567(M+H)+ |
| Ex 9-8 | 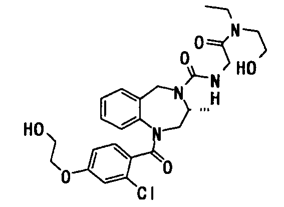 | 1H-NMR (CDCl3) δ ppm. 0 90-2.30 (8H, m), 2 70-5.20 (17H, m), 5 55-5 90 (1H, m), 6 20-7.80 (7H, m) MS(ESI, m/z). 533(M+H)+ |
| Ex 9-9 | 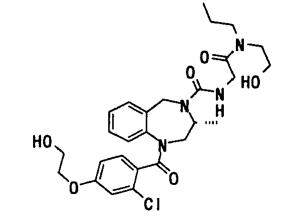 | 1H-NMR (CDCl3) δ ppm. 1.00-1 70 (8H, m), 1 90-2.50 (1H. m), 2.70-5.20 (17H, m), 5 50-5.90 (1H, m), 6 20-7.70 (7H, m) MS(ESI, m/z) 547(M+H)+ |
| Ex. 9-10 | 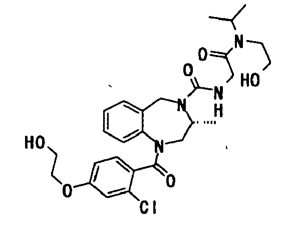 | 1H-NMR (CDCl3) δ ppm. 0 90-1 70 (9H, m), 1.85-2.30 (1H, m), 2.60-5 20 (16H, m), 5 50-5 90 (1H, m), 6.20-7.70 (7H, m) MS(ESI, m/z). 547(M+H)+ |
| Ex 9-11 | 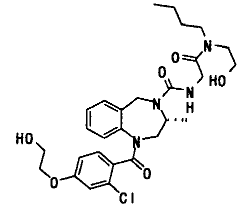 | 1H-NMR (CDCl3) δ ppm. 0.85-1.65 (13H, m), 1.90-2 50 (1H, m), 2 70-5.15 (17H, m), 5 50-5 85 (1H, m), 6 20-7.70 (7H, m) MS(ESI, m/z). 561(M+H)+ |

**[Table 70]**

| No | Strc | Physical data |
|---|---|---|
| Ex 9-12 | 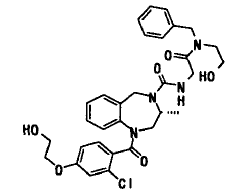 | 1H-NMR (CDCl₃) δ ppm 0.90-1.70 (3H, m), 1.85-5.30 (18H, m), 5.55-5 90 (1H, m), 6 20-7 80 (12H, m) MS(ESI, m/z): 595(M+H)+ |
| Ex 9-13 | 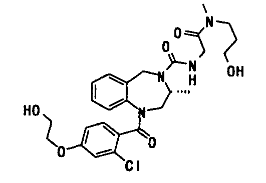 | 1H-NMR (CDCl₃) δ ppm. 0.90-2.25 (6H, m), 2.60-5 20 (18H, m), 5 50-5.90 (1H, m), 6 30-7 80 (7H, m) MS(ESI, m/z): 533(M+H)+ |
| Ex 9-14 | 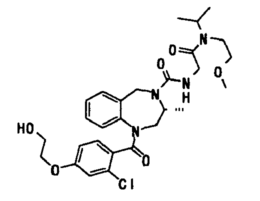 | 1H-NMR (CDCl3) δ ppm: 0.85-1 70 (9H, m), 1 95-2 10 (1H, m), 2.80-5 30 (19H, m), 5.60-6.00 (1H, m), 6 30-7.80 (7H, m) MS(ESI, m/z) : 561 (M+H)+ |
| Ex 9-15 | 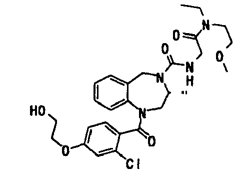 | 1H-NMR (CDCl3) δ ppm 0 90-1.70 (6H, m), 1.90-2.15 (1H, m), 2.70-5 30 (20H, m), 5 60-6 00 (1H, m), 6.30-7 70 (7H, m) MS(ESI, m/z) 547(M+H)+ |
| Ex 9-16 | 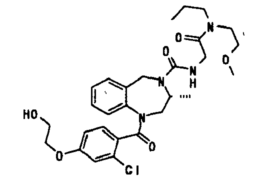 | 1H-NMR (CDCl3) δ ppm: 0 80-1 70 (8H, m), 1 95-2 15 (1H, m), 2.70-5 30 (20H, m), 5 60-5 95 (1H, m), 6 30-7.70 (7H, m) MS(ESI, m/z): 561(M+H)+ |
| Ex 9-17 | 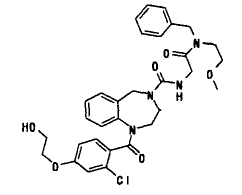 | 1H-NMR (CDCl3) δ ppm: 1.00-1 60 (3H, m), 1.90-2.05 (1H, m), 2 90-5 20 (20H, m), 5.60-6 00 (1H,m). 6.30-7.80 (12H, m) MS(ESI, m/z). 609(M+H)+ |

**[Table 71]**

| No | Strc | Physical data |
|---|---|---|
| Ex. 9-18 | 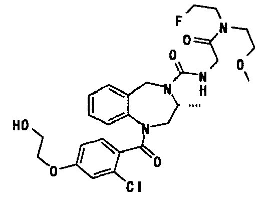 | MS(ESI, m/z) : 565(M+H)+ |
| Ex 9-19 | 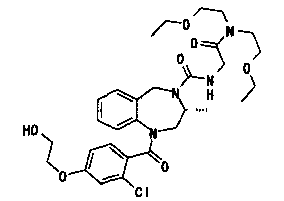 | 1H-NMR (CDCl3) δ ppm 0 90-1.70 (9H, m), 1 95-2.20 (1H, m), 2 50-5 30 (23H, m), 5.50-5 90 (1H, m), 6 30-7.70 (7H, m) MS(ESI, m/z): 605(M+H)+ |
| Ex 9-20 | 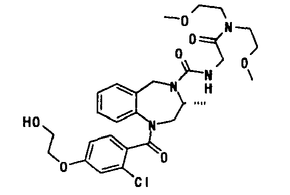 | 1H-NMR (CDCl3) δ ppm: 0 90-1.70 (3H, m), 1 90-2.15 (1H, m), 2 50-5 30 (25H, m), 5,60-5.90 (1H, m), 6 30-7 70 (7H, m) MS(ESI, m/z) 577(M+H)+ |
| Ex. 9-21 | 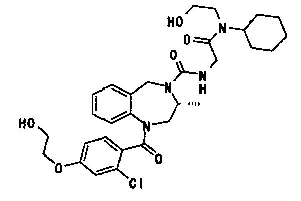 | 1H-NMR (CDCl3) δ ppm 0 90-2 20 (14H, m), 2 70-5 20 (16H, m), 5 55-5.95 (1H, m), 6 30-7 70 (7H, m) MS(ESI, m/z) : 587(M+H)+ |
| Ex. 9-22 | 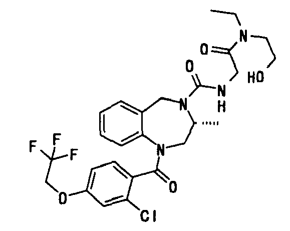 | 1H-NMR (CDCl3) δ ppm. 0 90-1.60 (6H, m), 2 40-5 40 (15H, m), 5.55-6 00 (1H. m), 6 40-8 20 (7H, m) MS(ESI, m/z) : 571(M+H)+ |

**[Table 72]**

| No. | Strc | Physical data |
|---|---|---|
| Ex. 9-23 | 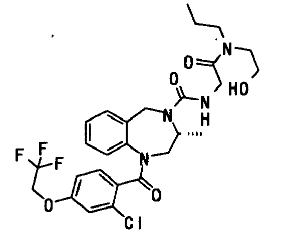 | 1H-NMR (CDCl3) δ ppm. 0 85-1 70 (8H, m), 2.35-5 20 (15H, m), 5.50-5 90 (1H, m), 6 00-7 80 (7H, m) MS(ESI, m/z) 585(M+H)+ |
| Ex. 9-24 | 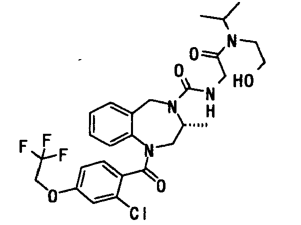 | 1H-NMR (CDCl3) δ ppm: 0 80-1 60 (9H, m), 2 30-5 20 (14H, m), 5 50-5.90 (1H, m), 6 20-7 80 (7H, m) MS(ESI, m/z) 585(M+H)+ |
| Ex 9-25 | 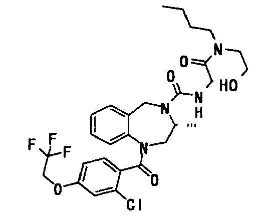 | 1H-NMR (CDCl3) δ ppm: 0 80-1.60 (11H, m), 2.40-5 20 (15H, m), 550-590 (1H, m), 6 00-7 80 (7H, m) MS(ESI, m/z) 599(M+H)+ |
| Ex. 9-26 | 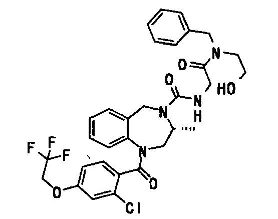 | MS(ESI, m/z). 633(M+H)+ |
| Ex. 9-27 | 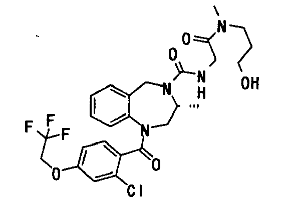 | 1H-NMR (CDCl3) δ ppm. 1.00-1 60 (5H, m), 2.20-5.20 (16H, m), 5 40-5 85 (1H, m), 6 00-7 80 (7H, m) MS(ESI, m/z), 571(M+H)+ |

**[Table 73]**

| No | Strc | Physical data |
|---|---|---|
| Ex 9-28 | 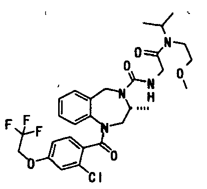 | 1H-NMR (CDCl3) δ ppm 1 00-1.60 (9H, m), 2 70-5 20 (17H, m), 5 60-5 95 (1H, m), 6 30-7 80 (7H, m) MS(ESI, m/z). 599(M+H)+ |
| Ex 9-29 | 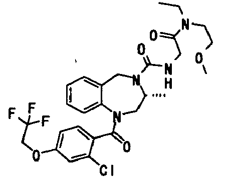 | 1H-NMR (CDCl3) δ ppm. 1 00-1 60 (6H, m), 2.30-5.20 (18H, m), 5 50-5 90 (1H, m), 6 20-7.80 (7H, m) MS(ESI, m/z) 585(M+H)+ |
| Ex. 9 - 30 | 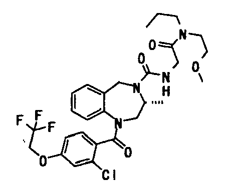 | 1H-NMR (CDCl3) δ ppm: 0 80-1.70 (8H, m), 2.50-5 20 (18H, m), 5 55-5.95 (1H, m), 6 00-7 80 (7H, m) MS(ESI, m/z) . 599(M+H)+ |
| Ex 9-31 | 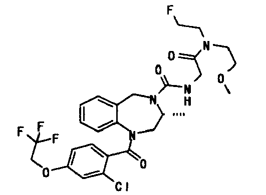 | MS(ESI, m/z) . 603(M+H)+ |
| Ex. 9-32 | 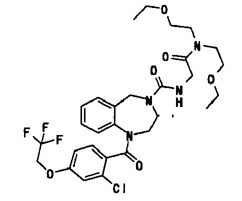 | 1H-NMR (CDCl3) δ ppm 0 70-1.70 (9H, m), 2 50-5.20 (21H, m), 5.50-5.90 (1H, m), 6 30-7 80 (7H, m) MS(ESI, m/z) 643(M+H)+ |
| Ex 9-33 | 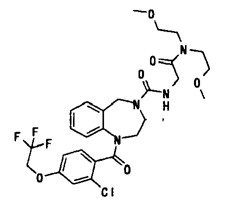 | 1H-NMR (CDCl3) δ ppm 1.00-1.65 (3H, m), 2.70-5 20 (23H, m), 5 50-5 90 (1H, m), 6 10-7 80 (7H, m) MS(ESI, m/z) 615(M+H)+ |

**[Table 74]**

| No | Strc | Physical data |
|---|---|---|
| Ex. 9-34 | 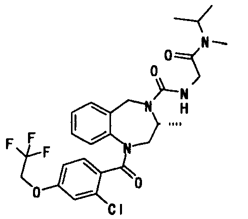 | 1H-NMR (CDCl3) δ ppm 1 00-1.60 (9H, m), 2 60-5.20 (13H, m), 5 70-6.00 (1H, m), 6.30-7 80 (7H, m) MS(ESI, m/z) : 555(M+H)+ |
| Ex. 9-35 | 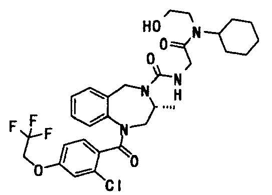 | 1H-NMR (CDCl3) δ ppm: 1.00-1 9- (13H, m), 2 80-5.20 (14H, m), 5.50-5 90 (1H, m), 6 30-7.80 (7H, m) MS(ESI, m/z) 625(M+H)+ |
| Ex. 9-36 | 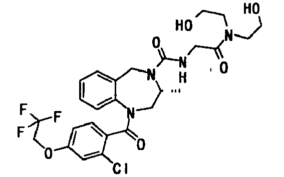 | 1H-NMR (CDCl3) δ ppm: 1 00-1 65 (3H, m), 2 70-5.30 (17H, m), 5 60-6 00 (1H, m), 6 30-7 80 (7H, m) |
| Ex. 9-37 | 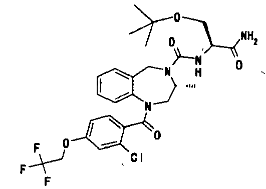 | 1H-NMR (CDCl3) δ ppm: 1.00-1.65 (12H, m), 2.80-5 90 (13H, m), 610-8.80 (7H, m) MS(ESI, m/z). 585(M+H)+ |
| Ex 9-38 | 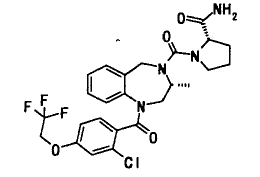 | 1H-NMR (CDCl3) δ ppm: 0.70-2 45 (7H, m), 3.00-6 00 (12H, m), 6.30-8.00 (7H, m) MS(ESI, m/z): 539(M+H)+ |
| Ex 9-39 | 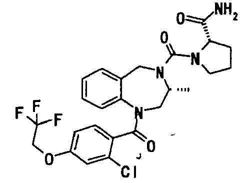 | 1H-NMR (CDCl3) δ ppm 0 70-2 45 (7H, m), 3.00-6.00 (12H, m), 6 30-8 00 (7H, m) MS(ESI, m/z) 539(M+H)+ |

**[Table 75]**

| No. | Strc | Physical data |
|---|---|---|
| Ex 9-40 | 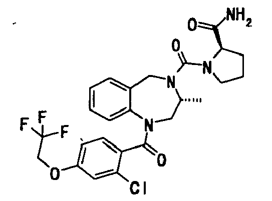 | 1H-NMR (CDCl3) δ ppm 1 30-2.30 (7H, m), 2.95-5.30 (10H, m), 6 30-7 30 (7H, m) MS(ESI, m/z) 539(M+H)+ |
| Ex. 9-41 | 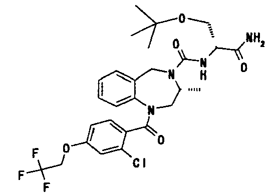 | 1H-NMR (CDCl3) δ ppm. 0.70-1.70 (12H, m), 2 50-5.90 (11H, m), 6 05-790 (7H, m) MS(ESI, m/z): 585(M+H)+ |
| Ex. 9-42 | 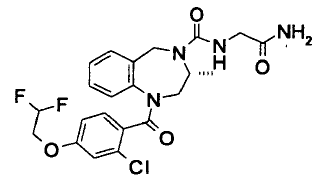 | 1H-NMR (CDCl3) δ ppm: 1.00-1 65 (3H, m), 2.85-5 65 (10H, m), 6 03 (1H, tt, J=54.9, 4.1Hz), 6.20-7.90 (7H, m) MS(ESI, m/z) 481(M+H)+ |
| Ex. 9-43 | 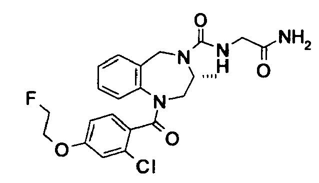 | 1H-NMR (CDCl3) δ ppm 0 90-1 60 (3H, m), 2 85-5 25 (11H, m), 5 50-7.85 (10H, m) MS(ESI, m/z) 463(M+H)+ |
| Ex. 9-44 | 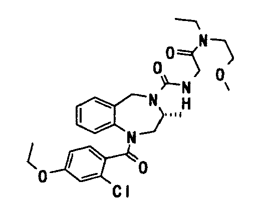 | 1H-NMR (CDCl3) δ ppm. 0 80-1.60 (9H, m), 2 80-5 30 (18H, m), 5.55-5 95 (1H, m), 6.25-7 85 (7H, m) MS(ESI, m/z). 531(M+H)+ |
| Ex 9-45 | 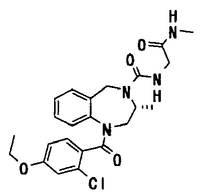 | 1H-NMR (CDCl3) δ ppm 0.80-1 80 (6H, m), 2 30-5.70 (12H, m), 5.80-7.90 (7H, m) MS(ESI, m/z) 459(M+H)+ |

**[Table 76]**

| No. | Strc | Physical data |
|---|---|---|
| Ex. 9-46 | 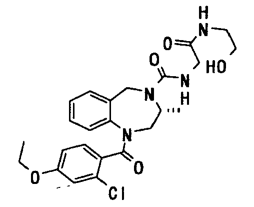 | 1H-NMR (CDCl3) δ ppm 0 80-1 80 (6H, m), 2 70-5.80'(13H, m), 6 20-7 80 (7H, m) MS(ESI, m/z) : 489(M+H)+ |
| Ex. 9-47 | 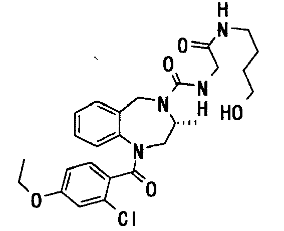 | 1H-NMR (CDCl3) δ ppm: 0 80-1.80 (10H, m), 2 10-5 80 (13H, m), 6.20-7 80 (7H, m) MS(ESI, m/z) : 517(M+H)+ |
| Ex. 9-48 | 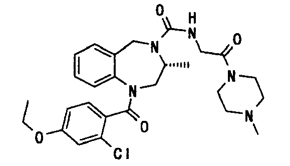 | 1H-NMR (CDCl3) δ ppm: 0.80-1.70 (6H, m), 1.90-6 00 (21H, m), 6.40-7 80 (7H, m) |
| Ex. 9-49 | 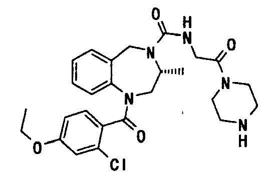 | 1H-NMR (CDCl3) δ ppm: 0 80-1.80 (6H, m), 2.80-6 00 (18H, m), 6.40-7 80 (7H, m) MS(ESI, m/z) 514 (M+H)+ |
| Ex. 9-50 | 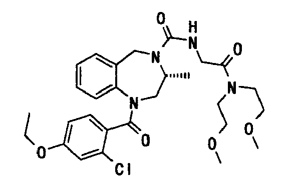 | 1H-NMR (CDCl3) δ ppm. 0.80-1.80 (6H, m), 2.80-6 00 (24H, m), 6.40-7.80 (7H, m) MS(ESI, m/z) .561 (M+H)+ |
| Ex. 9-51 | 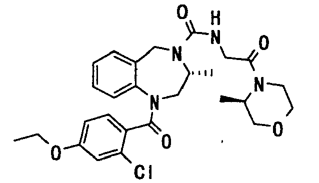 | 1H-NMR (CDCl3) δ ppm. 0.80-1.70 (9H, m), 2 80-5 25 (16H, m), 5.55-5 95 (1H, m), 6 35-7.80 (7H, m) MS(ESI, m/z) .529 (M+H)+ |

**[Table 77]**

| No | Strc | Physical data |
|---|---|---|
| Ex 9-52 | 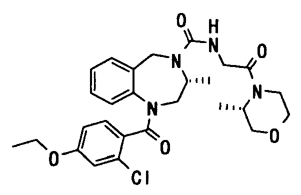 | 1H-NMR (CDCl3) δ ppm: 0 80-1 60 (9H, m), 2.80-5.25 (16H, m), 5 55-5 95 (1H, m), 6.30-7 80 (7H, m) MS(ESI, m/z) 529 (M+H)+ |
| Ex 9-53 | 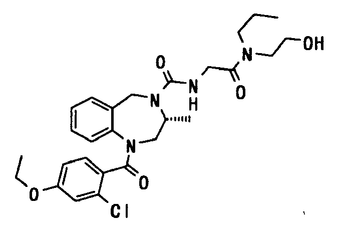 | 1H-NMR (CDCl3) δ ppm 0 70-1.80 (11H, m), 2 00-2 45 (1H, m), 2.80-5.20 (15H, m), 5 50-6.00 (1H, m), 6 40-7 80 (7H, m) MS(ESI, m/z) :531 (M+H)+ |
| Ex 9-54 | 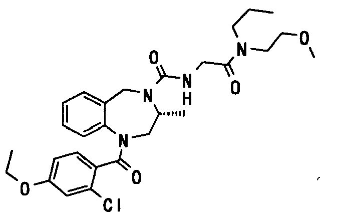 | 1H-NMR (CDCl3) δ ppm. 0.70-1 80 (11H, m), 2.80-5 20 (18H, m), 5 50-6 00 (1H, m), 6 40-7 80 (7H, m) MS(ESI, m/z) 545 (M+H)+ |
| Ex. 9-55 | 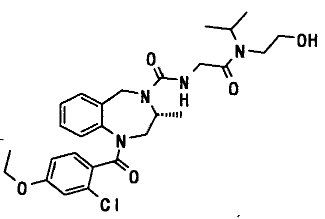 | 1H-NMR (CDCl3) δ ppm: 0.80-1.80 (12H, m), 2.80-5.20 (14H, m), 5 60-6 00 (1H, m), 6 40-7 80 (7H, m) MS(ESI, m/z) .531 (M+H)+ |
| Ex. 9-56 | 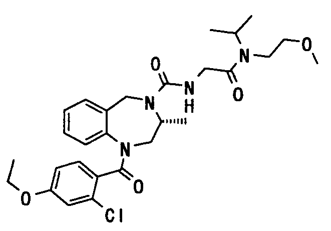 | 1H-NMR (CDCl3) δ ppm 0 70-1.80 (12H, m), 2.80-5.20 (17H, m), 5 60-6 00 (1H, m), 6 40-7 80 (7H, m) MS(ESI, m/z) 545 (M+H)+ |

**[Table 78]**

| No. | Strc | Physical data |
|---|---|---|
| Ex. 9-57 | 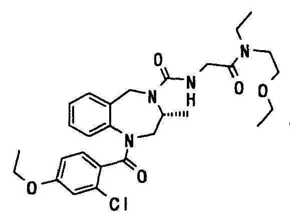 | 1H-NMR (CDCl3) δ ppm. 0 75-1 75 (12H, m), 2 80-5.25 (17H, m), 5 55-5.95 (1H, m), 6 25-7 75 (7H, m) MS(ESI, m/z) 545 (M+H)+ |
| Ex. 9-58 | 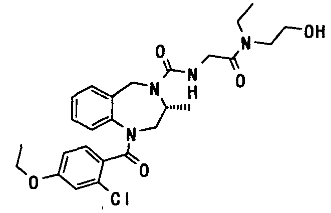 | 1H-NMR (CDCl3) δ ppm 0.85-1.70 (9H, m), 2.80-5 35 (15H, m), 5.50-5 95 (1H, m), 6 35-7 75 (7H, m) MS(ESI, m/z).517 (M+H)+ |
| Ex 9-59 | 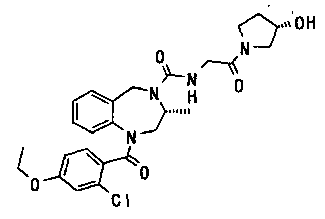 | 1H-NMR (CDCl3) δ ppm: 0 80-2.15 (8H, m), 2 25-5.20 (14H, m), 5 50-5 85 (1H, m), 6 30-7 70 (7H, m) MS(ESI, m/z) 515 (M+H)+ |
| Ex 9-60 | 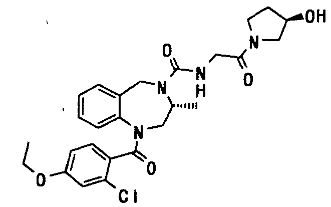 | 1H-NMR (CDCI3) δ ppm 0 90-2 10 (8H, m), 2 20-5 20 (14H, m), 5 50-5 90 (1H, m), 6 40-7 75 (7H, m9 MS(ESI, m/z) 515 (M+H)+ |
| Ex 9-61 | 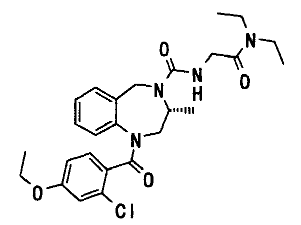 | 1H-NMR (CDCl3) δ ppm 0 80-1 75 (12H, m), 2 85-5.35 (13H, m), 5 60-6 05 (1H, m), 6.40-7 80 (7H, m) MS(ESI, m/z) 501 (M+H)+ |

**[Table 79]**

| No. | Strc | Physical data |
|---|---|---|
| Ex. 9-62 | 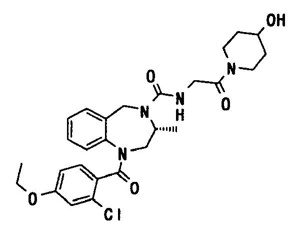 | 1H-NMR (CDCl3) δ ppm: 0.80-1.70 (8H, m), 1 80-1.95 (2H, m), 2.80-5 35 (14H, m), 5.60-5 95 (1H, m), 6 40-7.70 (7H, m) MS(ESI, m/z) .529 (M+H)+ |
| Ex. 9-63 | 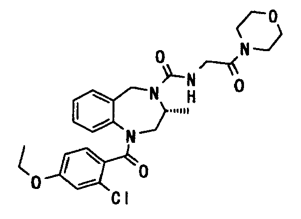 | MS(ESI, m/z) 515 (M+H)+ |
| Ex. 9-64 | 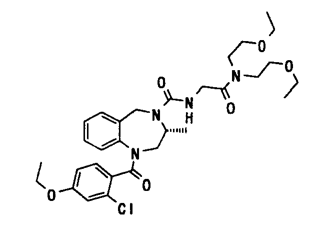 | 1H-NMR(CDCl3) δ ppm. 0 80-1 70 (12H, m), 2 70-5 40 (21H, m), 5 55-5 90 (1H, m), 6 30-7 75 (7H, m) MS(ESI, m/z) 589 (M+H)+ |
| Ex. 9-65 | 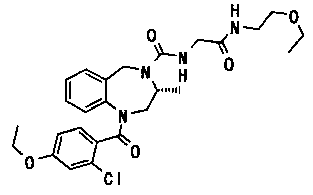 | 1H-NMR (CDCl3) δ ppm: 0.75-1 80 (9H, m), 280-550 (16H, m), 6 10-780 (7H, m) MS(ESI, m/z) :517 (M+H)+ |
| Ex 9-66 | 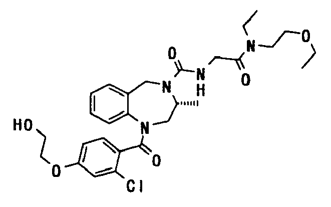 | 1H-NMR (CDCl3) δ ppm: 0 80-2 05 (10H, m), 2.90-6 00 (19H, m), 6 35-7 80 (7H, m) MS(ESI, m/z) 561 (M+H)+ |

### Example 10-1

### N-Carbamoylmethyl-(R)-1-(2-chloro-4-hydroxybenzoyl)-3-methyl-1,2,3,5-tetrahydrobenzo[e]-1,4-diazepin-4-carboxamide

To a solution of N-carbamoyl-(R)-1-(4-benzyloxy-2-chlorobenzoyl)-3-methyl-1,2,3,5-tetrahydrobenzo[e]-1,4-diazepin-4-carboxamide (46.0 mg) in tetrahydrofuran (1.5 mL) was added 10% palladium-carbon (10.0 mg) at room temperature under an inert gas atmosphere. The suspension was stirred at room temperature for 4.5 hours under a hydrogen gas atmosphere. To the suspension was added 10% palladium-carbon (10.0 mg) and the suspension was stirred at room temperature for 6 hours under a hydrogen gas atmosphere. The mixture was passed through a Celite pad and the filtrate was concentrated under reduced pressure. The obtained crude product was purified by column chromatography on silica gel (elueht:ethyl acetate-methanol-hexane) to give N-carbamoylmethyl-(R)-1-(2-chloro-4-hydroxybenzoyl)-3-methyl-1,2,3,5-tetrahydrobenzo[e]-1,4-diazepin-4-carboxamide (26.4 mg).
1H-NMR(DMSO-d6)δ ppm:
1.00-1.45 (3H, m), 2.70-5.20 (7H, m), 6.20-7.60 (9H, m), 9.55-10.55 (1H, br).
MS(ESI, m/z) : 417(M+H)+

### Examples 10-2 to 10-7

The following compounds of Examples 10-2 to 10-7 were obtained with the use of the corresponding benzyl ether derivatives in a similar manner to that described in Example 10-1. The structure formula and physical data of these compounds were shown in Table 80.

**[Table 80]**

| Ex. No. | Strc | Physical data |
|---|---|---|
| 10-2 | 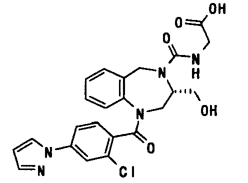 | 1H-NMR (CDCl3) δ ppm 200-530 (11H, m), 6 00-6 15 (1H, m), 6 35-8 25 (10H, m) MS(ESI, m/z) 484(M+H)+ |
| 10-3 | 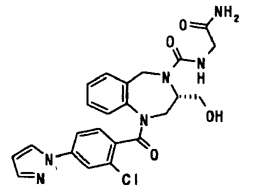 | MS(ESI, m/z) 483(M+H)+ |
| 10-4 | 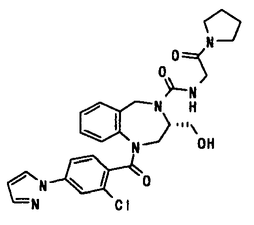 | 1H-NMR (CDCl3) δ ppm 1 80-2 05 (4H, m), 3 00-5 30 (13H, m), 585-610 (1H, m), 6 40-7 90 (10H, m) MS(ESI,-m/z) 537(M+H)+ |
| 10-5 | 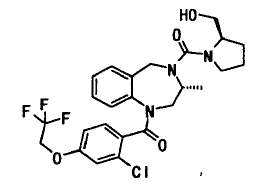 | 1H-NMR (CDCl3) δ ppm 1 20-2 30 (7H, m), 2 90-5 10 (12H, m), 6 40-7 60 (7H, m) MS(ESI, m/z) 526(M+H)+ |
| 10-6 | 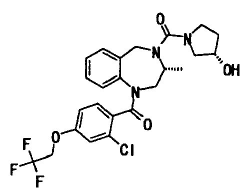 | 1H-NMR (CDCl3) δ ppm 1 20-2 20 (5H, m), 2 90-5 20 (12H, m), 6 40-7 70 (7H, m) MS(ESI, m/z) 512(M+H)+ |
| 10-7 | 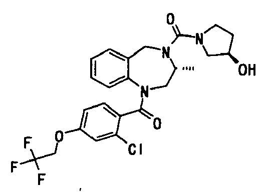 | 1H-NMR (CDCl3) δ ppm 1 20-2 20 (5H, m), 2 90-5 20 (12H, m), 6 40-7 70 (7H, m) MS(ESI, m/z) 512(M+H)+ |

### Example 11-1

### N-[3-Oxo-(3-piperazin-1-yl)propyl]-(R)-1-(2-chloro-4-pyrazol-1-ylbenzoyl)-3-methyl-1,2,3,5-tetrahydrobenzo[e]-1,4-diazepin-4-carboxamide

To a solution of tert-butyl 4-(3-{[(R)-1-(2-chloro-4-pyrazol-1-ylbenzoyl)-3-methyl-1,2,3,5-tetrahydrobenzo[e]-1,4-diazepin-4-carbonyl]amino}propionyl)piperazin-1-carboxylate (20.0 mg) in dichloromethane (1.0 mL) was added trifluoroacetic acid (0.30 g) at room temperature. The reaction mixture was stirred at room temperature for 3 hours. The reaction mixture was purified by column chromatography on aminopropylsilylated silica gel (eluent:ethyl acetate-ethanol) without work-up to give N-[3-oxo-(3-piperazin-1-yl)propyl]-(R)-1-(2-chloro-4-pyrazol-1-ylbenzoyl)-3-methyl-1,2,3,5-tetrahydrobenzo[e]-1,4-diazepin-4-carboxamide (16.4 mg).
MS(ESI, m/z) : 550(M+H)+

### Example 11-2

### N-(2-Aminoethyl)-(R)-1-(2-chloro-4-pyrazol-1-ylbenzoyl)-3-methyl-1,2,3,5-tetrahydrobenzo[e]-1,4-diazepin-4-carboxamide

N-(2-Aminoethyl)-(R)-1-(2-chloro-4-pyrazol-1-ylbenzoyl)-3-methyl-1,2,3,5-tetrahydrobenzo[e]-1,4-diazepin-4-carboxamide was obtained with the use of the corresponding amine derivative in a similar manner to that described in Example 11-1.
1H-NMR(CDCl3)δ ppm: 1.00-1.60 (3H, m), 2.50-5.30 (9H, m), 6.40-8.00 (10H, m).
MS(ESI, m/z) :453(M+H)+

### Example 11-3

### N-[2-Oxo-(2-piperazin-1-yl)ethyl]-(R)-1-[2-chloro-4-(2,2,2-trifluoroethoxy)benzoyl]-3-methyl-1,2,3,5-tetrahydrobenzo[e]-1,4-diazepin-4-carboxamide

N-[2-Oxo-(2-piperazin-1-yl)ethyl]-(R)-1-[2-chloro-4-(2,2,2-trifluoroethoxy)benzoyl]-3-methyl-1,2,3,5-tetrahydrobenzo[e]-1,4-diazepin-4-carboxamide was obtained with the use of the corresponding amine derivative in a similar manner to that described in Example 11-1.
1H-NMR(CDCl3)δ ppm:
1.00-1.70 (3H, m), 2.80-5.20 (17H, m), 5.60-5.95 (1H, m), 6.40-7.80 (7H, m).
MS (ESI, m/z) : 568(M+H)+

### Example 12

### Methyl ({(R)-1-[2-chloro-4-(2-hydroxyethoxy)benzoyl]-3-methyl-1,2,3,5-tetrahydrobenzo[e]-1,4-diazepin-4-carbonyl}amino)-acetate

To a stirred mixture of ethyl ({(R)-1-[2-chloro-4-(2-acetoxyethoxy)benzoyl]-3-methyl-1,2,3,5-tetrahydrobenzo[e]-1, 4-diazepin-4-carbonyl}amino)acetate (42.7 mg) in methanol (0.80 mL) was added 1 mol/L aqueous solution of sodium hydrogen carbonate (80.0 uL) at room temperature. The mixture was stirred at room temperature for 3 hours and the solvent was removed under reduced pressure. The residue was purified by column chromatography on silica gel (eluent: ethyl acetate-hexane) to give methyl ({(R)-1-[2-chloro-4-(2-hydroxyethoxy)benzoyl]-3-methyl-1,2,3,5-tetrahydrobenzo[e]-1,4-diazepin-4-carbonyl}amino)acetate (24.6 mg).
1H-NMR(CDCl3)δ ppm:
0.85-1.60 (3H, m), 1.70-2.45 (1H, m), 2.85-5.60 (14H, m), 6.35-7-90 (7H, m) .
MS (ESI, m/z) : 475(M+H)+

### Example 13

### Ethyl ({(R)-1-[2-Chloro-4-(2-hydroxyethoxy)benzoyl]-3-methyl-1,2,3,5-tetrahydrobenzo[e]-1,4-diazepin-4-carbonyl}amino)-acetate

A solution of ({(R)-1-[2-chloro-4-(2-hydroxyethoxy)-benzoyl]-3-methyl-1,2,3,5-tetrahydrobenzo[e]-1,4-diazepin-4-carbonyl}amino)acetic acid (23.9 mg) in 20wt% ethanolic solution of hydrogen chloride (2.0 mL) was stirred at an external temperature of 40 °C for 3 hours and the solvent was removed under reduced pressure. The residue was purified by column chromatography on silica gel (eluent:ethyl acetate-hexane) to give ethyl ({(R)-1-[2-chloro-4-(2-hydroxyethoxy)benzoyl]-3-methyl-1,2,3,5-tetrahydrobenzo[e]-1,4-diazepin-4-carbonyl}amino)acetate (21.5 mg).
1H-NMR(CDCl3)δ ppm:
0.80-1.60 (6H, m), 1.85-1.95 (1H, m), 3.65-5.70 (13H, m), 6.40-7.90 (7H, m).
MS(ESI, m/z) : 490(M+H)+

### Example 14-1

### N-[N-Cyclohexyl-N-(2-methoxyethyl)carbamoyl]methyl-(R)-1-[2-chloro-4-(2,2,2-trifluoroethoxy)benzoyl]-3-methyl-1,2,3,5-tetrahydrobenzo[e]-1,4-diazepin-4-carboxamide

To a mixture of N-{N-cyclohexyl-N-(2-hydroxyethyl)-methyl}-(R)-1-[2-chloro-4-(2,2,2-trifluoroethoxy)benzoyl]-3-methyl-1,2,3,5-tetrahydrobenzo[e]-1,4-diazepin-4-carboxamide (25.0 mg) and methyl iodide (0.15 mL) in dichloromethane (1.0 mL) was added silver oxide (11.2 mg) at room temperature and the mixture was stirred at room temperature for 16 hours. The mixture was filtered. The filtrate was concentrated under reduced pressure. The residue was purified by column chromatography on octadecylsilylated silica gel (eluent:acetonitrile-0.1% aqueous solution of formic acid) to give N-[N-cyclohexyl-N-(2-methoxyethyl)carbamoyl]methyl-(R)-1-[2-chloro-4-(2,2,2-trifluoroethoxy)benzoyl]-3-methyl-1,2,3, 5-tetrahydrobenzo[e]-1,4-diazepin-4-carboxamide (3.40 mg).
MS(ESI, m/z) : 639(M+H)+

### Examples 14-2 to 14-6

The following compounds of Examples 14-2 to 14-6 were obtained with the use of the corresponding alcohol derivatives in a similar manner to that described in Example 14-1. The structure formula and physical data of these compounds were shown in Table 81.

**[Table 81]**

| Ex. No. | Strc | Physical data |
|---|---|---|
| 14-2 | 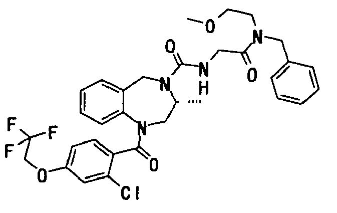 | MS(ESI, m/z) 647(M+H)+ |
| 14-3 | 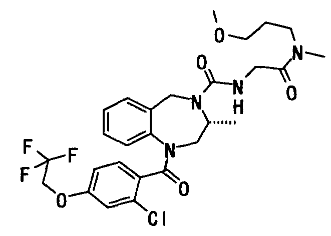 | 1H-NMR (CDCl3) δ ppm 1 00-1 60 (3H, m), 1 75-1.85 (2H, m), 2 50-5.20 (19H, m), 5.50-5 90 (1H, m), 6 30-7.70 (7H, m) MS(ESI, m/z) 585(M+H)+ |
| 14-4 | 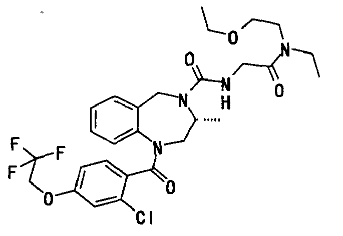 | MS(ESI, m/z) 599(M+H)+ |
| 14-5 | 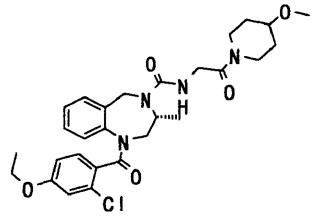 | 1H-NMR (CDCl3) δ ppm. 0 85-1.70 (8H, m), 1 75-1.90 (2H, m), 2 70-5 35 (15H, m), 5 60-6 00 (1H, m), 6 25-7.70 (7H, m) MS(ESI, m/z) 543 (M+H)+ |
| 14-6 | 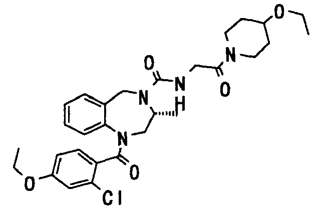 | 1H-NMR (CDCl3) δ ppm: 0.80-1 65 (11H, m), 1 70-1 95 (2H, m), 2.75-5 35 (16H, m), 5.60-5.90 (1H, m), 6 35-7 70 (7H, m) MS(ESI, m/z) 557 (M+H)+ |

### Example 15

### Ethyl {[(R)-1-(2-chloro-4-pyrazol-1-ylbenzoyl)-3-methyl-1,2,3,5-tetrahydrobenzo[e]-1,4-diazepin-4-carbonyl]methylamino}acetate

To a solution of ethyl {[(R)-1-(2-chloro-4-pyrazol-1-yl-benzoyl)-3-methyl-1,2,3,5-tetrahydrobenzo[e]-1,4-diazepin-4-carbonyl]amino} acetate (135mg) in tetrahydrofuran (1.0mL) was added sodium hydride (purity 60%, 16.3mg) at room temperature. The mixture was stirred at room temperature for 30 minutes. To the reaction mixture was added methyl iodide (46.4 mg) under ice-cooling, and the mixture was stirred at room temperature for 13.5 hours. To the reaction mixture were added water and ethyl acetate, and the organic layer was separated. The aqueous layer was extracted with ethyl acetate. The combined organic layer was washed with brine, and then dried over anhydrous magnesium sulfate. The layer was concentrated under reduced pressure and the residue was purified by column chromatography on silica gel (eluent:hexane-ethyl acetate) to give ethyl {[(R)-1-(2-chloro-4-pyrazol-1-ylbenzoyl)-3-methyl-1,2,3,5-tetrahydrobenzo[e]-1,4-diazepin-4-carbonyl]methylamino}acetate (49.5 mg).
1H-NMR(CDCl3)δ ppm: 1.05-1.65 (6H, m), 2.75-5.10 (12H, m), 6.40-8.10 (10H, m).
MS(ESI, m/z) : 510(M+H)+

### Example 16-1

### N-((S)-1-Carbamoyl-2-hydroxy)ethyl-(R)-1-[2-chloro-4-(2,2,2-trifluoroethoxy)benzoyl]-3-methyl-1,2,3,5-tetrahydrobenzo[e]-1,4-diazepin-4-carboxamide

To a mixture of N-((S)-2-tert-butoxy-1-carbamoylethyl)-(R)-1-[2-chloro-4-(2,2,2-trifluoroethoxy)benzoyl]-3-methyl-1,2,3,5-tetrahydrobenzo[e]-1,4-diazepin-4-carboxamide (114 mg) in dichloromethane (1.0 mL) was added trifluoroacetic acid (1.00 mL) at room temperature. The mixture was stirred at room temperature overnight. The mixture was concentrated under reduced pressure. To the residue was added dichloromethane, then the mixture was neutralized by addition of 2 mol/L aqueous solution of sodiumhydroxide under ice-cooling. To the mixture was added dichloromethane and the organic layer was separated. The layer was successively washed with a solution of sodium hydrogen carbonate and brine and the layer was concentrated under reduced pressure. The obtained crude product was purified by column chromatography on silica gel (eluent:methanol-ethyl acetate) to give N-((S)-1-carbamoyl-2-hydroxy)ethyl-(R)-1-[2-chloro-4-(2,2,2-trifluoroethoxy)benzoyl]-3-methyl-1,2,3,5-tetrahydrobenzo[e]-1,4-diazepin-4-carboxamide (51.6 mg).
1H-NMR(DMSO-d6)δ ppm:
0.90-1.50 (3H, m), 2.80-5.00 (10H, m), 6.00-7.90 (7H, m).

### Example 16-2

### N-((R)-1-Carbamoyl-2-hydroxyethyl)-(R)-1-[2-chloro-4-(2,2,2-trifluoroethoxy)benzoyl]-3-methyl-1,2,3,5-tetrahydrobenzo[e]-1,4-diazepin-4-carboxamide

N-((R)-1-Carbamoyl-2-hydroxyethyl)-(R)-1-[2-chloro-4-(2,2,2-trifluoroethoxy)benzoyl]-3-methyl-1,2,3,5-tetrahydrobenzo[e]-1,4-diazepin-4-carboxamide was obtained with the use of the corresponding alcohol derivative in a similar manner to that described in example 16-1.
1H-NMR(DMSO-d6) δ ppm: 0.50-1.70 (3H, m), 2.00-5.20 (10H, m), 6.00-7.80 (7H, m).
MS(ESI, m/z) : 529 (M+H)+

### Example 17-1

### {(R)-1-[2-Chloro-4-(2,2,2-trifluoroethoxy)benzoyl]-3-methyl-1,2,3,5-tetrahydrobenzo[e]-1,4-diazepin-4-yl}((R)-2-methoxymethylpyrrolidin-1-yl)methanone

A solution of {(R)-1-[2-chloro-4-(2,2,2-trifluoroethoxy)-benzoyl]-3-methyl-1,2,3,5-tetrahydrobenzo[e]-1,4-diazepin-4-yl}-((R)-2-hydroxymethylpyrrolidin-1-yl)methanone (40.0 mg) in tetrahydrofuran (300 uL) was added dropwise to a suspension of sodium hydride (purity 60%, 4.00 mg) in tetrahydrofuran (700 uL) under ice-cooling. The mixture was stirred for 15 minutes under an argon gas atmosphere, while ice-cooling. To the mixture was added methyl iodide (5.00 uL) under ice-cooling and the mixture was stirred at room temperature for 16 hours under ice-cooling. To the mixture were added sodium hydride (purity 60%, 2.00 ug), methyl iodide (15.0 uL) under ice-cooling and the mixture was stirred at room temperature for 4 hours. To the mixture were added piperazine (28.4 mg) and water under ice-cooling and the mixture was stirred. To the mixture was added dichloromethane (1.5 mL) and the organic layer was separated. The layer was dried over anhydrous sodium sulfate and then the mixture was concentrated under reduced pressure. The obtained crude product was purified by column chromatography on silica gel (eluent:hexane-ethyl acetate) to give {(R)-1-[2-chloro-4-(2,2,2-trifluoroethoxy)benzoyl]-3-methyl-1,2,3,5-tetrahydrobenzo[e]-1,4-diazepin-4-yl}((R)-2-methoxymethylpyrrolidin-1-yl)methanone (32.8 mg).
1H-NMR (CDC13) δ ppm: 0.70-2.20 (7H, m), 2.90-5.10 (15H, m), 6.40-7.70 (7H, m).
MS(ESI, m/z) : 540 (M+H) +

### Examples 17-2 to 17-4

The following compounds of Examples 17-2 to 17-4 were obtained with the use of the corresponding alcohol derivatives in a similar manner to that described in Example 17-1. The structure formula and physical data of these compounds were shown in Table 82.

**[Table 82]**

| Ex. No. | Strc | Physical data |
|---|---|---|
| 17-2 | 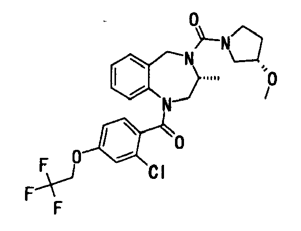 | 1H-NMR (CDCl3) δ ppm. 1.00-2.20 (5H, m), 300-510 (15H, m), 6 40-7.50 (7H, m) MS(ESI, m/z). 526(M+H)+ |
| 17-3 | 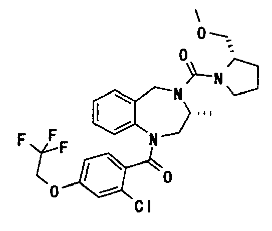 | 1H-NMR (CDCl3) δ ppm 1.00-2.20 (7H, m), 2.90-5 10 (15H, m), 6 40-7.80 (7H, m) MS(ESI, m/z). 540(M+H)+ |
| 17-4 | 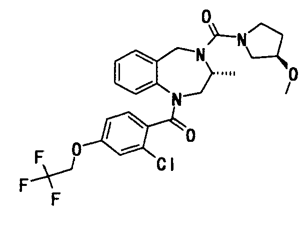 | 1H-NMR (CDCl3) δ ppm. 1 00-2 20 (5H, m), 3 00-5.10 (15H, m), 6.40-7 80 (7H, m) MS(ESI, m/z) 526(M+H)+ |

### Example 18

### Ethyl {[(R)-1-,(2-chloro-4-isopropoxybenzoyl)-3-methyl-1,2,3,5-tetrahydrobenzo[e]-1,4-diazepin-4-carbonyl]amino}acetate

To a solution of (2-chloro-4-isopropoxyphenyl)-((R)-3-methyl-2,3,4,5-tetrahydrobenzo[e]-1,4-diazepin-1-yl)methanone (33.7 mg) in tetrahydrofuran (0.80 mL) was added ethyl isocyanatoacetate (14.6 mg) under ice-cooling and the mixture was stirred at room temperature for an hour. Without work-up, the reaction solution was purified by column chromatography on silica gel (eluent:hexane-ethyl acetate) to give ethyl { [(R)-1-(2-chloro-4-isopropoxybenzoyl)-3-methyl-1,2,3,5-tetrahydrobenzo[e]-1,4-diazepin-4-carbonyl]amino}acetate (42.9 mg).

### Example 19

### {[(R)-1-(2-Chloro-4-isopropoxybenzoy)-3-methyl-1,2,3,5-tetrahydrobenzo[e]-1,4-diazepin-4-carbonyl]amino}acetic acid

To a solution of ethyl {[(R)-1-(2-chloro-4-isopropoxybenzoyl)-3-methyl-1,2,3,5-tetrahydrobenzo[e]-1,4-diazepin-4-carbonyl]amino}acetate (42.0 mg) in ethanol (0.10 mL) was added 5 mol/L aqueous solution of sodiumhydroxide (38.0 uL) under ice-cooling and the mixture was stirred at room temperature for 3 hours. The reaction solution was acidified by addition of 1 mol/L hydrochloric acid under ice-cooling. This solution was extracted with ethyl acetate. The organic layer was washed with water and brine to give {[(R)-1-(2-chloro-4-isopropoxybenzoy)-3-methyl-1,2,3,5-tetrahydrobenzo[e]-1,4-diazepin-4-carbonyl]amino}acetic acid (39.3 mg).

### Example 20

### Ethyl {[(R)-1-(2-Chloro-4-piperidin-1-ylbenzoyl)-3-methyl-1,2,3,5-tetrahydrobenzo[e]-1,4-diazepin-4-carbonyl]amino}-acetate

To a suspension of ethyl { [(R) -1-(4-bromo-2-chlorobenzoyl)-3-methyl-1,2,3,5-tetrahydrobenzo[e]-1,4-diazepin-4-carbonyl]-amino}acetate (50.0 mg), palladium acetate (II) (1.10 mg), 2,2'-bis(diphenylphosphino)-1,1'-binaphtyl (4.60 mg), cesium carbonate (64.1 mg) in toluene (1. 5 mL) was added piperidine (12.0 uL) at room temperature and the suspension was stirred at an external temperature of 100 °C under an argon atmosphere for 42 hours. After the suspension was allowed to cool, the suspension was passed through a Celite pad and the filtrate was concentrated under reduced pressure. The obtained crude product was purified by column chromatography on silica gel (eluent:hexane-ethyl acetate) to give ethyl {[(R)-1-(2-Chloro-4-piperidin-1-ylbenzoyl)-3-methyl-1,2,3,5-tetrahydrobenzo[e]-1,4-diazepin-4-carbonyl]amino}acetate (43.6 mg).
1H-NMR(CDCl3)δ ppm:
1.00-1.80 (9H, m), 2.70-5.30 (13H, m), 6.30-7.80 (7H, m).
MS(ESI, m/z) : 513 (M+H)+

### Test example 1

### Binding experiment for human V2 receptor

Test compounds were dissolved in dimethylsulfoxide at 10 mM. The affinities for human V2 receptor were determined by an inhibition against [³H]-Arginie vasopressin (AVP) (Perkin elmer Japan) binding to the human V2 receptor using CHO cell membranes expressed human V2 receptor (Packard BioScience). Cell membranes suspension was prepared by suspending the cell membranes as mentioned above with Assay buffer (50 mM Tris-HCl, 10 mM MgCl2, 0.1% bovine serum albumin (BSA), pH7.4) at an adequate protein concentration. The test compound solutions were prepared by diluting the dimethylsulfoxide solution of the test compound as mentioned above with Assay buffer at final concentration of 10 nM, 100 nM, 1 µM and 10 µM (in this diluting procedure, concentration of dimethylsulfoxide was adjusted to be 0.5% at each concentration of the test compound). Cell membranes suspension (50 µL), 3 nM of [³H]-AVP (50 µL), Assay buffer (50 µL) and the solution of the test compounds at each concentrations (50 µL) were added to MultiScreen 96-well plate (Millipore) and the mixture was incubated for an hour at 25 °C with shaking slightly. After filtration by aspiration, the plate was washed with ice-cold washing buffer (50 mM Tris-HCl, 10 mM MgCl2, pH 7.4) for three times. After dry the plate, Microscinti-20 (Packard) was added to each well and the plate was shaken slightly, and then radioactivities of each well were counted with a micro plate scintillation counter, TopCount (Packard). Non-specific binding of [³H] -AVP for cell membranes was determined by adding 1 µM of cold AVP substituted for the test compounds. The concentration of the test compounds inhibiting the specific binding of [³H]-AVP by 50 % was considered as IC50 value. The Ki value of the test compounds was calculated from Kd value determined by the method described below and was considered as indication of affinity to the human V2 receptor. The results were shown in Table 83 as below.

### The calculation of Kd value of [³H]-AVP for the cell membranes expressed human V2 receptor

The suspension of the cell membranes expressed human V2 receptor was prepared with diluting adequately with the Assay buffer as mentioned above. In consideration for the radioactivity of [³H]-AVP, six different concentrations (final concentration; ranging from approximately 100 pM to 6000 pM) of [³H]-AVP were prepared by serial dilution by from 2 to 3 fold with the Assay buffer. The cell membranes suspension (50 µL), each concentrations of [³H]-AVP (50 µL), the Assay buffer (50 µL) and 1 µM of cold AVP or the Assay buffer (50 µL) were added to MultiScreen 96-well plate (Millipore) and the mixture was incubated for an hour at 25 °C with shaking slightly. The specific bindings (B value) of each concentrations of the [³H]-AVP were determined by the method as mentioned above and free-bound contents (F value) at each concentrations of the [³H] -AVP were determined. The Kd value was calculated by Scatchard analysis using the B value and the F value.

**[Table 83]**

| Test compound EX. No. | Affinity to human V2 receptor Ki (nM) |
|---|---|
| 3-1 | 19.7 |
| 4 | 61.9 |
| 5-2 | 35.5 |
| 5-3 | 14.0 |
| 5-18 | 27.8 |

### Test example 2

### The study to confirm the agonism of human V2 receptor

The experiment to confirm the response of the test compounds to human V2 receptor was carried out using the cells prepared as described below in order to use this confirmation study to see the agonism of human V2 receptor of the test compounds.
The test compounds were dissolved in dimethylsulfoxide at 10 mM and solutions of the test compounds were prepared by 10-fold serial dilution with the Assay buffer (0.1% BSA, 20 mM HEPES/Hank's balanced salt solution, pH 7.4) at the concentration of 0.1 nM to 10 µM, which were used in this study.
Human V2 receptor couples with Gs protein, one of the G-coupling protein, and consequently produces cyclic adenosine 3', 5'-monophosphate (cAMP) via adenylate cyclase. This cAMP produce can be substituted to intracellular increase of Ca²⁺ with coexpressing Gqs, a chimeric protein, and human V2 receptor (Mol. Pharmacol., Vol. 50, pp. 885-890, 1996). The responses of the test compounds for human V2 receptor were quantified by measuring this intracellular Ca²⁺. The changes of intracellular Ca²⁺ after adding the test compounds at each concentration as mentioned above (0.1 nM to 10 µM) were measured with a FlexStation (Molecular Devices) using a FLIPR CALCIUM ASSAY KIT (Molecular Devices).
The intrinsic activities (IA) of the test compounds were calculated from the maximum response of the test compounds as that of AVP was considered to be 1.00. In case of a full agonist, the EC50 values of the test compounds were calculated as the maximum response of AVP was considered to be 100% and in the case of a partial agonist, EC50 values of test compounds were calculated as the maximum response of own test compounds were considered to be 100%. The concentration that achieved to 50% response of the maximum response in a concentration-response curve was considered as EC50 value. The values of EC50 obtained in this study were shown in Table 84 described below as indications of the agonism of human V2 receptor.

### The preparation of the cells using confirmation study of an agonism of human V2 receptor (HEK293 cells coexpressed human V2 receptor and Gqs chimeric protein)

HEK293 cells (American Type Culture Collection) were incubated in the Eagle's Minimum Essential Medium (EMEM, Invitrogen) containing 1 mM sodium pyruvate, nonessential amino acids (0.1 mM), streptomycin (100 µg/mL), penicillin (100 U/mL), 10% fetal calf serum (Sanko chemicals) in an incubator with 5% CO2 at 37°C. The trans fection was carried out by addingpCI-neo hV2 expression vector, expression vector inserted with the sequence coding Gqs chimeric protein (pLEC-Gqs5, LiveWare, Molecular Devices) and Lipofectamine2000 (Invitrogen), all diluted with OPTI-MEM I Reduced Serum Medium I (Invitrogen), to the cell suspension which was prepared from the confluent cells suspended with EMEM as mentioned above without antibiotics at 1X10⁶ cells/mL. After the transfection, the cells (HEK293 cells coexpressed human V2 receptor and Gqs chimeric protein) were incubated in an incubator with 5% CO2 for two days and were used as cells for confirming agonism of human V2 receptor, and used for assessment of the test compounds. The expression plasmid vector of human V2 receptor represented as pCI-neo/hV2 above was constructed by the method as described below.

### The method of construction of human V2 receptor expression plasmid vector

cDNA library was obtained from reverse transcription of human kidney total RNA using SuperScript II RNase H-reverse transcriptase (Invitrogen) and oligo dT. The DNA fragment encoding human V2 receptor was amplified by the PCR method using the cDNA library as a template, primers used in combination of each forward primer (sequence no. 1 to 3 shown below) and each reverse primer (sequence no. 4-6shownbelow) respectively and pfu DNA polymerase (Stratagene). This amplified DNA fragment andpCR-blunt kit (Invitrogen), a cloning plasmid vector, was ligated by the general method of the kit. The ligate-productions were introduced into E. coli TOP10 cells (Invitrogen) by the general method, and the transformant cells were selected by LB agar medium containing 50 µg/mL of kanamycin. One of the transformant was grown in LB liquid medium and the vectors were extracted from the transformant and purified. The vectors were clevaged with restriction enzyme Eco RI to obtain DNA fragments.
As the same time, pCI-neo (Promega), a mammalian expression plasmid vector, was digested by restriction enzyme Eco RI and treated with calf intestinal alkaline phosphatase to protect from a self ligation. Then this pCI-neo and the DNA fragments obtained by Eco RI digestion as mentioned above were ligated by Quick ligation Kit (New England BioLabs). After the ligated-productions were introduced into E. coli TOP10 cells by the general method, the transformants were selected with LB agar medium containing 100 µg/mL of ampicillin. One of the transformant was grown in LB liquid medium and the vectors were extracted from the transformant and purified. The sequence of the DNA fragment inserted at multi-cloning site of this vector was determined and corresponded to the sequence of human V2 receptor administered as accession no.AF030626 in GenBank/EMBL data base. This expression plasmid vector encoding human V2 receptor was termed as pCI-neo/hV2.

Sequence no.1 AGTCCGCACATCACCTCCAG
Sequence no.2 ATGCTCATGGCGTCCACCAC
Sequence no.3 GCCCTCAGAACACCTGC
Sequence no.4 GCTCCTCACGATGAAGTGTC
Sequence no.5 GCAAGACACCCAACAGCTCC
Sequence no.6 GCTGAGCTTCTCAAAGCCTCT

**[Table 84]**

| Test compound Ex. No. | Agonism of human V2 receptor EC₅₀ (nM) | I A |
|---|---|---|
| 3-1 | 9.4 | 0.59 |
| 3-6 | 15.4 | 0.93 |
| 4 | 16.0 | 0.75 |
| 5-2 | 7.3 | 0.71 |
| 5-3 | 9.1 | 0.48 |
| 5-4 | 27.6 | 0.49 |
| 5-5 | 24.9 | 0.83 |
| 5-6 | 17.3 | 0.92 |
| 5-11 | 8.6 | 0.75 |
| 5-12 | 6.5 | 0.79 |
| 5-15 | 9.1 | 0.71 |
| 5-18 | 2.2 | 0.89 |
| 5-19 | 15.6 | 0.57 |

### Test example 3

The study of antidiuretic effect - the confirmation study of antidiuretic effect on the diuretic activity induced by loading hypotonic solution in the anesthetized rats infused with hypotonic solution
It has been reported that plasma AVP level was decreased with intravenous infusion of hypotonic solution (J. Endocrinol., Vol. 141, pp. 59-67, 1994). The antidiuretic effect of the test compounds in the rats induced a diuretic condition was determined by the method as reported by Angchanpen et al (Br. J. Pharmacol., Vol. 93, pp. 151-155, 1988). The test compounds were dissolved in dimethylsulfoxide at 10 mM, and used in the study. Male SD rats (200-400 g weight) were anesthetized with 100 mg/kg of Inactin (SIGMA) intraperitonealy and each cannula was inserted into trachea, jugular vein, bladder and femoral vein, respectively. The hypotonic solution (0.3% NaCl, 0.83% glucose) was infused via femoral vein at 9 mL/hour. The urine volume obtained via cannula inserted into bladder was measured every 10 minutes. After steady-state of urine volume for three 10 minutes periods, test compounds prepared as mentioned above were administered intravenously at 10 µg/kg via cannula inserted into jugular vein. Dimethylsulfoxide/saline (0.3%) was used as a vehicle. The average of urine volume measured for three 10 minutes periods before the administration was defined as the pre value (0%). After the administration of the test compounds, the urine volume was measured every 10 minutes. The antidiuretic effect, namely the decrease of the urine volume, induced by administration'of test compounds was calculated from the decreasing rate of urine volume (minus %) against the pre value. Because maximum decreasing rate of the urine volume after adminiatration of vehicle was -20% in this study, the period that decreasing rate of urine volume restored to -20% after administration of the test compounds was considered as indication of duration time of the test compounds. Each of the result was shown in Table 85.

**[Table 85]**

| Test compound Ex. No. | Antidiuretic effect (decreasing rate of urine volume) | Duration time |
|---|---|---|
| (Untreated) | 0.0 | - |
| 3-1 | -88.7% | 60 min |
| 5-2 | -83.9% | 50 min |
| 5-4 | -90.4% | 80 min |
| 5-5 | -89.4% | 90 min |

The abbreviations used above were shown below.
AVP: arginine vasopressin
[³H]-AVP: tritium labeled-vasopressin
HEPES: 4-(2-Hydroxyethyl)-1-piperazineethanesulfonic Acid
HEK: human embryonic kidney
Tris: 2-Amino-2-hydroxymethl-1,3-propanediol
CHO: chinese hamster ovary cell

### Test example 4

### Acute toxicity test

Male SD rats (250-300 g weight) were divided into some groups (N=2) and each cannula was inserted into trachea and jugular vein under anesthesia with urethane (1.5 g/kg, subcutaneously). The test compound solutions were prepared with an adequate solvent to be a dosage of 10 mg/kg, and then the soluton was administered intravenously via cannula. The survival rate was determined for an hour experimental period. As the result was shown in Table 86, it was not observed that animal was dead and then it was suggested that the compounds of the present invention had low toxicity.

**[Table 86]**

| Test compound Ex. No. | Dead |
|---|---|
| 5-2 | 0/2 |
| 5-4 | 0/2 |
| 5-5 | 0/2 |

### Industrial Applicability

The compounds represented by the above general formula (A) of the present invention, for example, in a binding experiment for human V2 receptor and a study to confirm the agonism of human V2 receptor, exerted a strong agonism of human V2 receptor. Thence the compounds represented by the above general formula (A) of the present invention can decrease urine volume significantly. Therefore the compounds represented by the above general formula (A) of the present invention have a profile based on the effect such as antidiuretic activity and a releasing activity of coagulation factor VIII and von-Wiliebrand factor, and are useful for a various problems of urination, and a large volume of urine and a bleeding tendency, are preferably an agent for the treatment or prevention of a disease associated with micturition, urinary incontinence, enuresis, central diabetes insipidus, nocturia, spontaneous bleeding, hemophilia, von-Wiliebrand disease, congenital/acquired dysfunction of blood platelets or the like.

## Claims

1. A urea derivative represented by the general formula (A) : 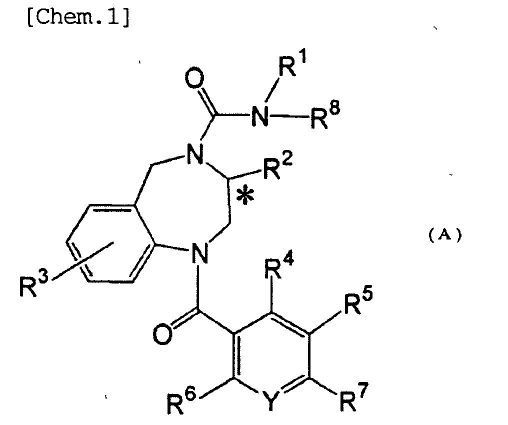 wherein R¹ and R⁸ bind together with the nitrogen atom bound to them to form an alicyclic amine, or are indipendently the following a) to o) :
a) a hydrogen atom,
b) a C₃₋₇ cycloalkyl group,
c) a C₁₋₇ alkyl group,
d) a halo(C₁₋₇ alkyl) group,
e) a C₆₋₁₀ aryl group,
f) a heteroaryl group,
g) a hydroxy(C₁₋₇ alkyl) group,
h) a C₃₋₇ cycloalkyl(C₁₋₇ alkyl) group,
i) a C₁₋₆ alkoxy(C₁₋₇ alkyl) group,
j) a C₂₋₇ acyloxy(C₁₋₇ alkyl) group,
k) a C₆₋₁₀ aryl(C₁₋₇ alkyl) group,
l) a heteroaryl(C₁₋₇ alkyl) group,
m) -M¹-COOR¹¹,
n) -M¹-CONR¹²R¹³, or
o) -M¹-NR¹²-SO₂R¹³;
M¹ is a C₁₋₇ alkylene group;
R¹¹ is a hydrogen atom or a C₁₋₇ alkyl group;
R¹² and R¹³ bind together with the nitrogen atom bound to them to form an alicyclic amino group, or are independently the following a) to i):
a) a hydrogen atom,
b) a C₆₋₁₀ aryl group,
c) a C₁₋₇ alkyl group
d) a hydroxy(C₁₋₇ alkyl) group,
e) a C₁₋₆ alkoxy(C₁₋₇ alkyl) group,
f) a heteroaryl(C₁₋₇ alkyl) group,
g) a C₆₋₁₀ aryl(C₁₋₇ alkyl) group,
h) -M²-CONR¹⁴NR¹⁵, or
i) -M²-NR¹⁶SO₂R¹⁷;
M² is a C₁₋₇ alkylene group;
R¹⁴ and R¹⁵ bind together with the nitrogen atom bound to them to form an alicyclic amino group, or are independently the following a) to f):
a) a hydrogen atom,
b) a C₁₋₇ alkyl group,
c) a hydroxy(C₁₋₇ alkyl) group,
d) a C₁₋₆ alkoxy(C₁₋₇ alkyl) group,
e) a heteroaryl(C₁₋₇ alkyl) group, or
f) a C₆₋₁₀ aryl(C₁₋₇ alkyl) group;
R¹⁶ is a hydrogen atom or a C₁₋₇ alkyl group;
R¹⁷ is a C₁₋₇ alkyl group;
R² is the following a) to g):
a) a hydrogen atom,
b) a C₁₋₇ alkyl group,
c) a hydroxy(C₁₋₇ alkyl) group,
d) a C₁₋₆ alkoxy(C₁₋₇ alkyl) group,
e) a C₆₋₁₀ aryl(C₁₋₇ alkyl) group,
f) -M¹-CONR¹²R¹³ (in the formula, M¹, R¹² and R¹³ have the same meanings as defined above), or
g) -M¹-COOR¹¹ (in the formula, M¹ and R¹¹ have the same meanings as defined above);
R³ is the following a) to d):
a) a hydrogen atom,
b) a halogen atom,
c) a hydroxy group, or
d) a C₁₋₆ alkoxy group;
R⁴, R⁵ and R⁶ are independently the following a) to f):
a) a hydrogen atom,
b) a halogen atom,
c) a C₁₋₇ alkyl group,
e) a C₁₋₆ alkoxy group, or
f) a halo (C₁₋₇ alkyl) group;
R⁷ is the following a) to d):
a) a group represented by the general formula 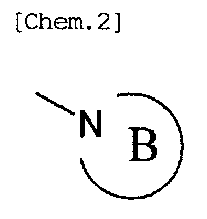 wherein B ring is a heteroaryl group or an alicyclic amino group,
b) a group represented by the general formula 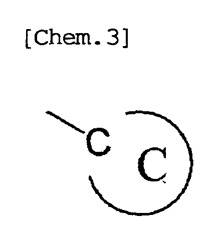 wherein C ring is a C₆₋₁₀ aryl group, a heterocycloalkyl group or a heteroaryl group, or
c) - M³-R⁷¹;
M³ is a single bond, -O-, -C(CH₃)₂- or -CF₂-;
R⁷¹ is the following a) to e):
a) a hydrogen atom,
b) a halogen atom,
c) a C₁₋₇ alkyl group,
d) a halo (C₁₋₇ alkyl) group, or
e) a hydroxy(C₁₋₇ alkyl) group;
Y is N or CH; and
a carbon atom marked with * represents a carbon atom having R-configuration or S-configuration, or a mixture thereof;
or a pharmaceutically acceptable salt thereof, or a prodrug thereof.

2. A urea derivative as claimed in claim 1 wherein the carbon atom marked with * has the configuration represented by the general formula (A-1): 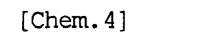 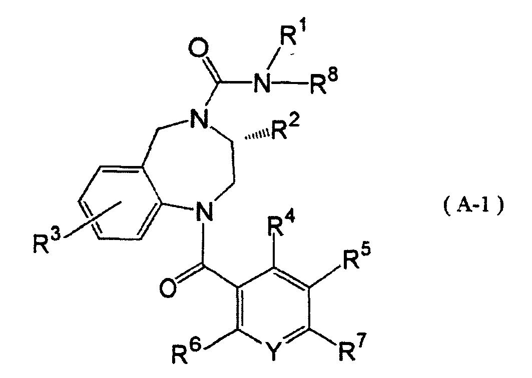 or a pharmaceutically acceptable salt thereof, or a prodrug thereof.

3. A urea derivative as claimed in claim 1 or 2 wherein R⁸ is a hydrogen atom, or a pharmaceutically acceptable salt thereof, or a prodrug thereof.

4. A urea derivative as claimed in any one of claims 1 to 3 wherein R⁵ and R⁶ are a hydrogen atom, or a pharmaceutically acceptable salt thereof, or a prodrug thereof.

5. A urea derivative as claimed in any one of claims 1 to 4 wherein R³ is a hydrogen atom or a halogen atom, or a pharmaceutically acceptable salt thereof, or a prodrug thereof.

6. A urea derivative as claimed in any one of claims 1 to 5 wherein R² is a C₁₋₇ alkyl group, or a pharmaceutically acceptable salt thereof, or a prodrug thereof.

7. A urea derivative as claimed in any one of claims 1 to 6 wherein R⁴ is the following a) to c):
a) a hydrogen atom,
b) a halogen atom, or
c) a halo(C₁₋₇alkyl) group, or a pharmaceutically acceptable salt thereof, or a prodrug thereof.

8. A urea derivative as claimed in any one of claims 1 to 7 wherein R⁷ is any group selected from a group consisting of the following groups: 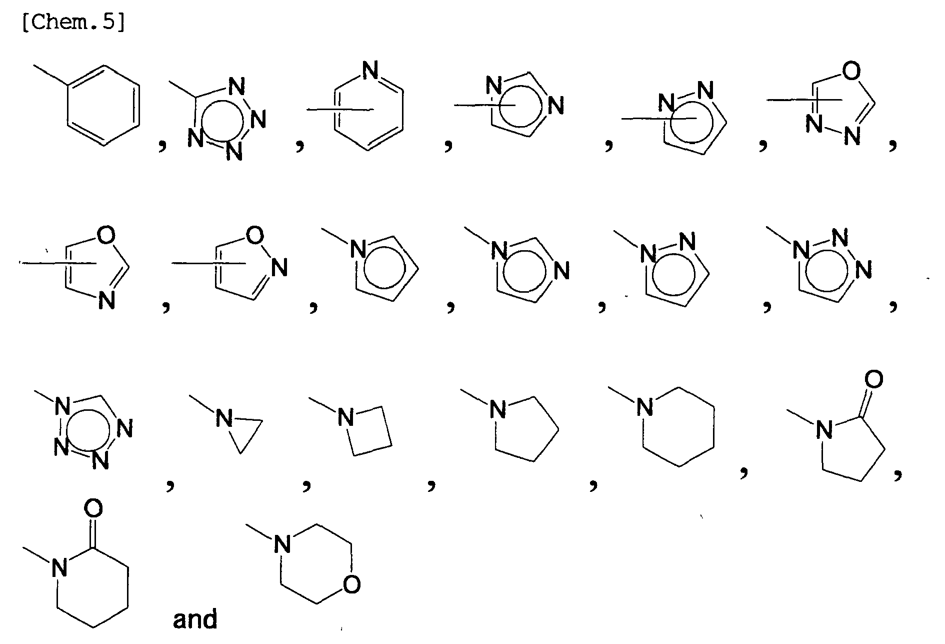 wherein the ring may be substituted by 1 to 3 groups independently selected from a group consisting of a halogen atom, a C₁₋₇ alkyl group, a halo(C₁₋₇ alkyl) group, a C₁₋₆ alkoxy group, a hydroxyC₁₋₇ alkyl group and a C₁₋₆ alkoxy(C₁₋₇ alkyl) group;
a C₁₋₆ alkoxy group, a hydroxy(C₁₋₆ alkoxy) group or a halo(C₁₋₆ alkoxy) group, or a pharmaceutically acceptable salt thereof, or a prodrug thereof.

9. A pharmaceutical composition comprising as an active ingredient a urea derivative as claimed in any one of claims 1 to 7, or a pharmaceutically acceptable salt thereof, or a prodrug thereof.

10. A human type-2 arginine vasopressin receptor agonist comprising as an active ingredient a urea derivative as claimed in anyone of claims 1 to 7, or a pharmaceutically acceptable salt thereof, or a prodrug thereof.

11. An agent for the treatment or prevention of central diabetes insipidus, comprising as an active ingredient a urea derivative as claimed in any one of claims 1 to 7, or a pharmaceutically acceptable salt thereof, or a prodrug thereof.

12. An agent for the treatment or prevention of noctuira, comprising as an active ingredient a urea derivative as claimed in any one of claims 1 to 7, or a pharmaceutically acceptable salt thereof, or a prodrug thereof.

13. An agent for the treatment or prevention of nocturnal enuresis, comprising as an active ingredient a urea derivative as claimed in any one of claims 1 to 7, or a pharmaceutically acceptable salt thereof, or a prodrug thereof.

14. A method for the treatment or prevention of central diabetes insipidus, comprising administering an effective amount of a urea derivative as claimed in any one of claims 1 to 7 or a pharmaceutically acceptable salt thereof, or a prodrug thereof.

15. A method for the treatment or prevention of noctuira, comprising administering an effective amount of a urea derivative as claimed in any one of claims 1 to 7 or a pharmaceutically acceptable salt thereof, or a prodrug thereof.

16. A method for the treatment or prevention of nocturnal enuresis, comprising administering an effective amount of a urea derivative as claimed in any one of claims 1 to 7 or a pharmaceutically acceptable salt thereof, or a prodrug thereof.

17. A use of a urea derivative as claimed in any one of claims 1 to 7 or a pharmaceutically acceptable salt thereof, or a prodrug thereof, for manufacturing a pharmaceutical composition for the treatment or prevention of central diabetes insipidus.

18. A use of a urea derivative as claimed in any one of claims 1 to 7 or a pharmaceutically acceptable salt thereof, or a prodrug thereof, for manufacturing a pharmaceutical composition for the treatment or prevention of noctuira.

19. A use of a urea derivative as claimed in any one of claims 1 to 7 or a pharmaceutically acceptable salt thereof, or a prodrug thereof, for manufacturing a pharmaceutical composition for the treatment or prevention of nocturnal enuresis.

20. A pharmaceutical composition comprising in combination Drug Group (A): a urea derivative as claimed in any one of claims 1 to 7 or a pharmaceutically acceptable salt thereof, or a prodrug thereof, and Drug Group (B) : at least one agent selected from a group consisting of an agent for the treatment of central diabetes insipidus, an agent for the treatment of noctuira and an agent for the treatment of nocturnal enuresis, other than a type-2 arginine vasopressin receptor agonist.

21. A pharmaceutical composition comprising in combination Drug Group (A) : a urea derivative as claimed in any one of claims 1 to 7 or a pharmaceutically acceptable salt thereof, or a prodrug thereof, and Drug Group (B) : at least one agent selected from a group consisting of an α₁-adrenoceptor blocker, a cholinergic blocking agent, a cholinergic agent, an antispasmodic agent, an anti-androgen agent, an antidepressant, a calcium antagonist, a potassium-channel opener, a sensory nerve blocking agent, a β-adrenergic agonist, an acetylcholinesterase inhibitor and anti-inflammatory agent.

22. A method for the treatment or prevention of central diabetes insipidus, comprising administering in combination each effective amount of Drug Group (A) : a urea derivative as claimed in any one of claims 1 to 7 or a pharmaceutically acceptable salt thereof, or a prodrug thereof, and Drug Group (B) : at least one agent selected from a group consisting of an agent for the treatment of central diabetes insipidus, an agent for the treatment of noctuira and an agent for the treatment of nocturnal enuresis, other than a type-2 arginine vasopressin receptor agonist.

23. A method for the treatment or prevention of noctuira, comprising administering in combination each effective amount of Drug Group (A): a urea derivative as claimed in any one of claims 1 to 7 or a pharmaceutically acceptable salt thereof, or a prodrug thereof, and Drug Group (B) : at least one agent selected from a group consisting of an agent for the treatment of central diabetes insipidus, an agent for the treatment of noctuira and an agent for the treatment of nocturnal enuresis, other than a type-2 arginine vasopressin receptor agonist.

24. A method for the treatment or prevention of nocturnal enuresis, comprising administering in combination each effective amount of Drug Group (A): a urea derivative as claimed in any one of claims 1 to 7 or a pharmaceutically acceptable salt thereof, or a prodrug thereof, and Drug Group (B) : at least one agent selected from a group consisting of an agent for the treatment of central diabetes insipidus, an agent for the treatment of noctuira and an agent for the treatment of nocturnal enuresis, other than a type-2 arginine vasopressin receptor agonist.

25. A method for the treatment or prevention of central diabetes insipidus, comprising administering in combination each effective amount of Drug Group (A) : a urea derivative as claimed in any one of claims 1 to 7 or a pharmaceutically acceptable salt thereof, or a prodrug thereof, and Drug Group (B) : at least one agent selected from a group consisting of an α₁-adrenoceptor blocker, a cholinergic blocking agent, a cholinergic agent, an antispasmodic agent, an anti-androgen agent, an antidepressant, a calcium antagonist, a potassium-channel opener, a sensory nerve blocking agent, a β-adrenergic agonist, an acetylcholinesterase inhibitor and anti-inflammatory agent.

26. A method for the treatment or prevention of noctuira, comprising administering in combination each effective amount of Drug Group (A): a urea derivative as claimed in any one of claims 1 to 7 or a pharmaceutically acceptable salt thereof, or a prodrug thereof, and Drug Group (B) : at least one agent selected from a group consisting of an α₁-adrenoceptor blocker, a cholinergic blocking agent, a cholinergic agent, an antispasmodic agent, an anti-androgen agent, an antidepressant, a calcium antagonist, a potassium-channel opener, a sensory nerve blocking agent, a β-adrenergic agonist, an acetylcholinesterase inhibitor and anti-inflammatory agent.

27. A method for the treatment or prevention of nocturnal enuresis, comprising administering in combination each effective amount of Drug Group (A): a urea derivative as claimed in any one of claims 1 to 7 or a pharmaceutically acceptable salt thereof, or a prodrug thereof, and Drug Group (B) : at least one agent selected from a group consisting of an α₁-adrenoceptor blocker, a cholinergic blocking agent, a cholinergic agent, an antispasmodic agent, an anti-androgen agent, an antidepressant, a calcium antagonist, a potassium-channel opener, a sensory nerve blocking agent, a β-adrenergic agonist, an acetylcholinesterase inhibitor and anti-inflammatory agent.

28. A use of Drug Group (A) : a urea derivative as claimed in any one of claims 1 to 7 or a pharmaceutically acceptable salt thereof, or a prodrug thereof, and Drug Group (B) : at least one agent selected from a group consisting of an agent for the treatment of central diabetes insipidus, an agent for the treatment of noctuira and an agent for the treatment of nocturnal enuresis, other than a type-2 arginine vasopressin receptor agonist, for manufacturing a pharmaceutical composition for the treatment or prevention of central diabetes insipidus, noctuira or nocturnal enuresis.

29. A use of Drug Group (A) : a urea derivative as claimed in any one of claims 1 to 7 or a pharmaceutically acceptable salt thereof, or a prodrug thereof, and and Drug Group (B): at least one agent selected from a group consisting of an α₁-adrenoceptor blocker, a cholinergic blocking agent, a cholinergic agent, an antispasmodic agent, an anti-androgen agent, an antidepressant, a calcium antagonist, a potassium-channel opener, a sensory nerve blocking agent, a β-adrenergic agonist, an acetylcholinesterase inhibitor and anti-inflammatory agent, for manufacturing a pharmaceutical composition for the treatment or prevention of central diabetes insipidus, noctuira or nocturnal enuresis..

30. A method for the treatment or prevention of a disease which can be improved by stimulating V2 receptor, which includes administering an effective amount of a urea derivative as claimed in any one of claims 1 to 7 or a pharmaceutically acceptable salt thereof, or a prodrug thereof.

31. A method as claimed in claim 30 wherein the disease which can be improved by stimulating V2 receptor is a disease selected from a group consisting of central diabetes insipidus, noctuira and nocturnal enuresis.
